(19) Europäisches Patentamt
European Patent Office
Office européen des brevets

(11) EP 4 434 978 A1

(12) **EUROPEAN PATENT APPLICATION**
published in accordance with Art. 153(4) EPC

(43) Date of publication:
**25.09.2024 Bulletin 2024/39**

(21) Application number: **22894916.0**

(22) Date of filing: **17.11.2022**

(51) International Patent Classification (IPC):
***C07D 401/12*** (2006.01) ***C07D 417/12*** (2006.01)
***C07D 401/02*** (2006.01) ***C07D 401/14*** (2006.01)
***A61K 31/427*** (2006.01) ***A61K 31/496*** (2006.01)
***A61K 31/506*** (2006.01)

(52) Cooperative Patent Classification (CPC):
**A61K 31/427; A61K 31/496; A61K 31/498; A61K 31/506; A61K 31/675; A61P 3/00; A61P 9/00; A61P 29/00; A61P 35/00; A61P 35/02; A61P 37/06; C07D 401/02; C07D 401/12; C07D 401/14; C07D 403/14;** (Cont.)

(86) International application number:
**PCT/CN2022/132632**

(87) International publication number:
**WO 2023/088385 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2021 CN 202111362590**
**18.04.2022 CN 202210405555**

(71) Applicant: **TYK Medicines Inc.**
**Huzhou, Zhejiang 313100 (CN)**

(72) Inventors:
- **LIANG, APeng**
  **Huzhou, Zhejiang 313100 (CN)**
- **WU, Yusheng**
  **Huzhou, Zhejiang 313100 (CN)**
- **CHEN, Shaoqing**
  **Huzhou, Zhejiang 313100 (CN)**
- **LI, Jun**
  **Huzhou, Zhejiang 313100 (CN)**
- **HAN, Shunxun**
  **Huzhou, Zhejiang 313100 (CN)**
- **DONG, Shengli**
  **Huzhou, Zhejiang 313100 (CN)**
- **NIU, Chengshan**
  **Huzhou, Zhejiang 313100 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

# (54) COMPOUND FOR DEGRADING EGFR PROTEIN AND USE THEREOF

(57) The present invention relates to a compound for degrading an EGFR protein and the use thereof. Specifically, the present invention relates to a compound as represented by formula (I), and the definitions of each group and each substituent are as described in the description. The present invention further relates to the use of the compound in the preparation of a drug for regulating the activity of an EGFR tyrosine kinase or treating EGFR-related diseases, particularly non-small cell lung cancer.

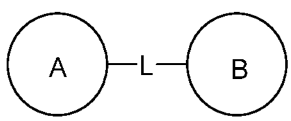

I

EP 4 434 978 A1

(52) Cooperative Patent Classification (CPC): (Cont.)
**C07D 405/14; C07D 417/12; C07D 417/14; C07D 471/04; C07D 487/08; C07D 487/10; C07D 519/00; C07F 9/6558**

## Description

### Technical field

The present invention relates to the field of pharmaceutical technology, specifically relates to a compound for degrading EGFR and its application in modulating EGFR kinase activity or in the treatment of EGFR-related diseases, in particular cancer.

### Background

The receptor tyrosine kinases of HER family are mediators of cell growth, differentiation and survival. This receptor family includes four distinct members, namely the epidermal growth factor receptor (EGFR, ErbB1 or HER1), HER2 (ErbB2), HER3 (ErbB3) and HER4 (ErbB4). Upon ligand binding, the receptor forms homodimer or heterodimer, and subsequent activation of endogenous tyrosine kinase activity leads to receptor autophosphorylation and activation of downstream signaling molecules. Regulation of EGFR activation due to overexpression or mutation has been shown to be implicated in multiple types of human cancers including colorectal, pancreatic, glioma, head and neck, and lung cancers, particularly non-small cell lung cancer (NSCLC), and multiple EGFR-targeting agents have been developed over the years, with three generations of drugs already in clinical use.

In the actual clinical application, after using the first generation or second generation EGFR inhibitors, patients usually produce the drug-resistant mutation of EGFRT790M in 8-12 months, which leads to the loss of therapeutic effect of the drug. Although, the later marketed third-generation EGFR inhibitors, such as oshitiniband almonertinib, etc., can effectively overcome the EGFRT790M mutation resistance. However, after taking for a period of time, drug-resistant mutations such as EGFRC797S will still appear, leading to the progression of the disease.

The frequent problem of mutation resistance and non-classical EGFR mutations during the treatment with EGFR small-molecule tyrosine kinase inhibitors has become an urgent clinical challenge to be addressed. Although some EGFR variant inhibitor compounds, such as EAI045, have been reported to overcome C797S resistance, the clinical results are limited. In recent years, a series of PROTAC-type compounds capable of overcoming C797S resistance by degrading EGFR proteins have been reported in some patents (WO2019149922, WO2021127561), which has become a new research direction. Although some progress has been made on EGFR allosteric inhibitors and EGFR degraders, there is still a need to find more and more clinically valuable EGFR protein modulating drugs for the treatment of diseases caused by current EGFR dysregulation, especially in the field of EGFR-positive non-small cell lung cancer.

### Summary

The purpose of the present invention is to provide a compound of formula I having EGFR kinase inhibitory activity and degradative activity and its use for modulating EGFR activity or preventing and/or treating EGFR-related diseases.

In the first aspect of the present invention, provided is a compound, and the compound is a compound shown in Formula I, or a pharmaceutically acceptable salt thereof, a stereoisomer, a tautomer, a hydrate, a solvate, an isotopic compound, or a prodrug thereof,

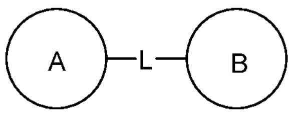

I

wherein,

L is selected from the group consisting of:

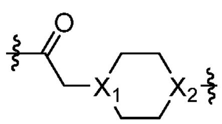

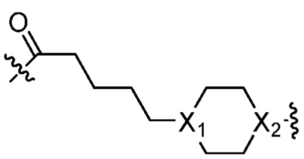

,

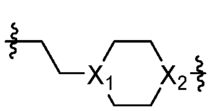 , 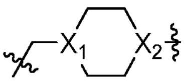 , 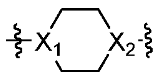 , 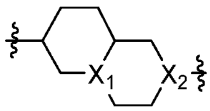 , 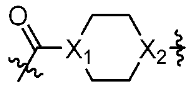 ,

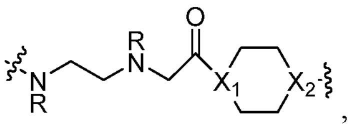 , 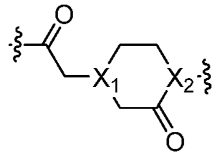 , 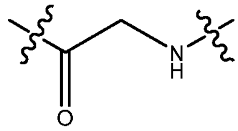 , 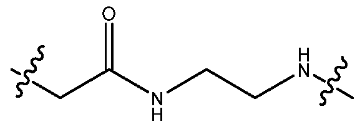 ,

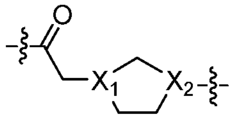 , 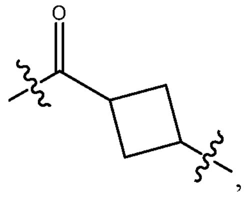 , 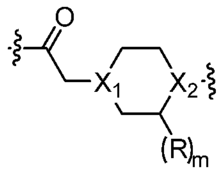 and 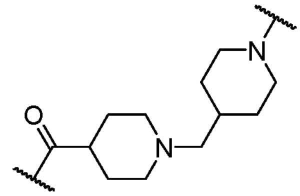 ;

A is selected from the group consisting of

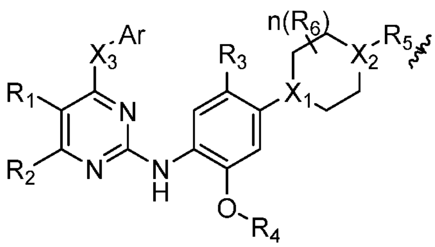 ,

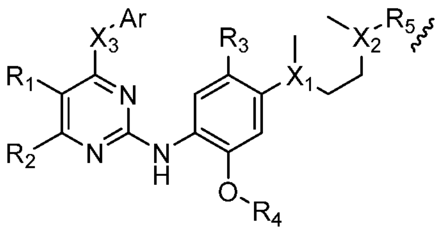 , 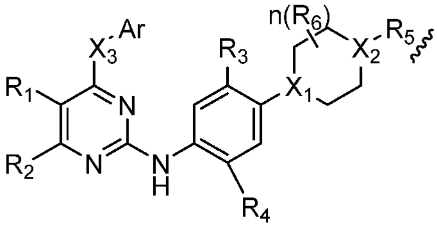 , 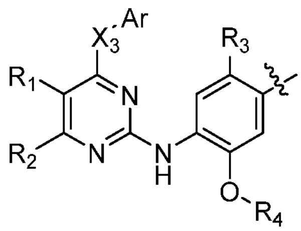 ,

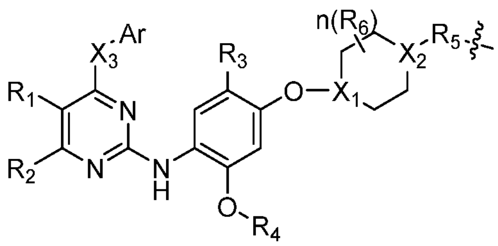 , 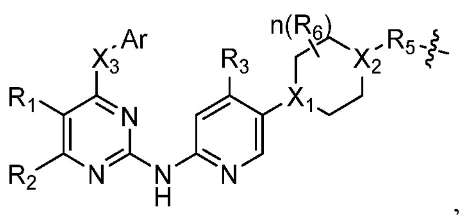 ,

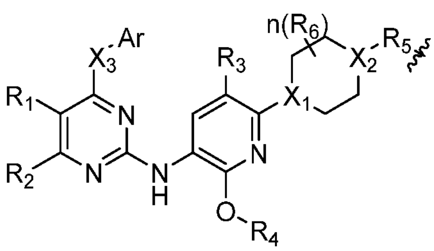 , 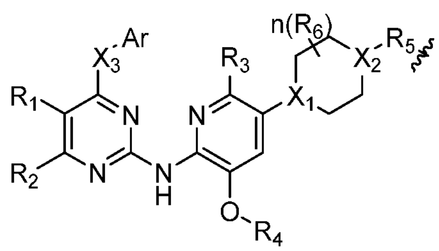 ,

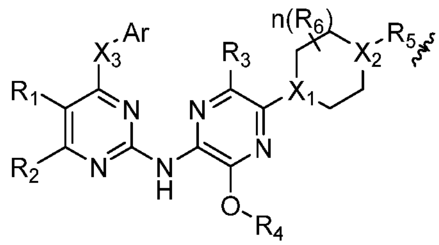
,
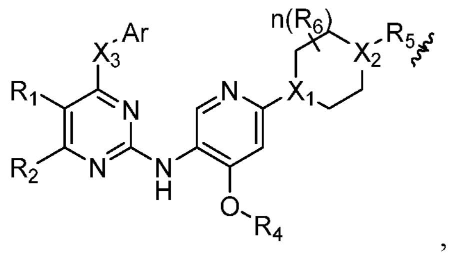
,

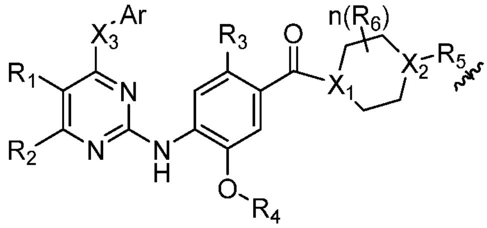
,
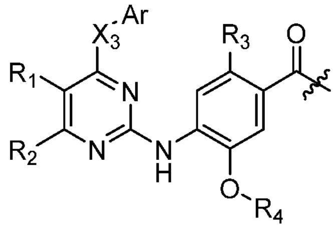
,

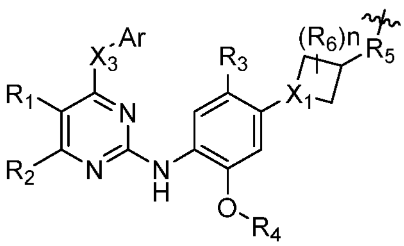
,
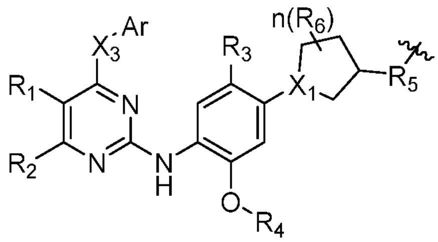
,

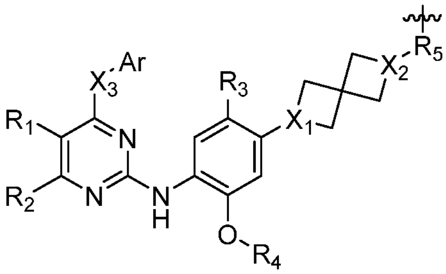
.
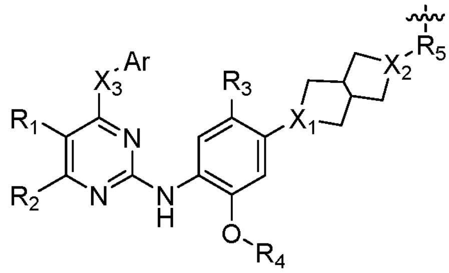
.

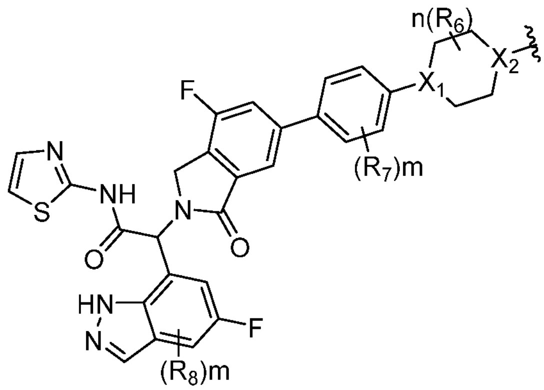
,
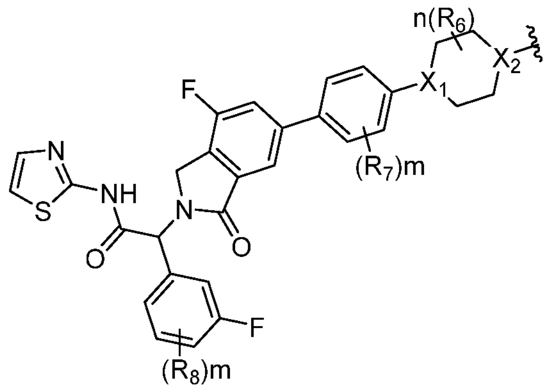
,

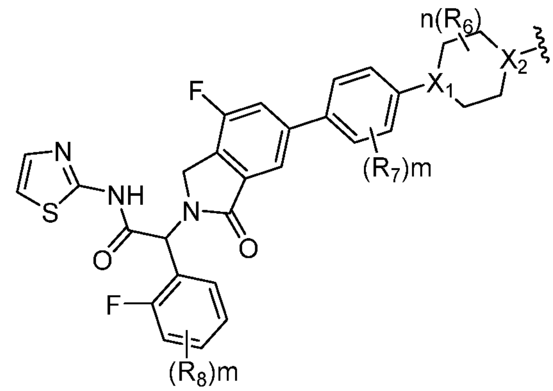
,
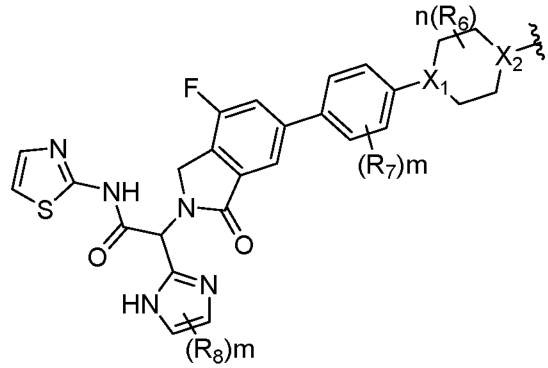
,

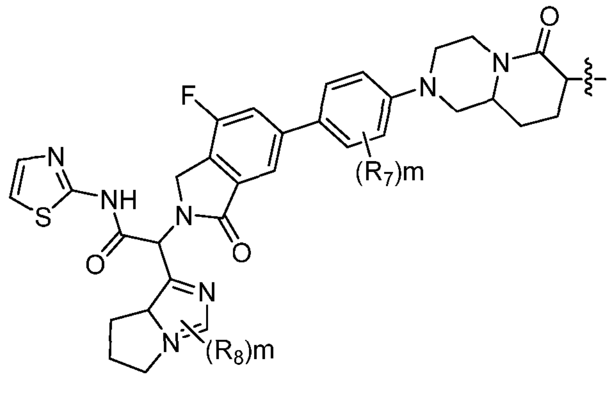
,
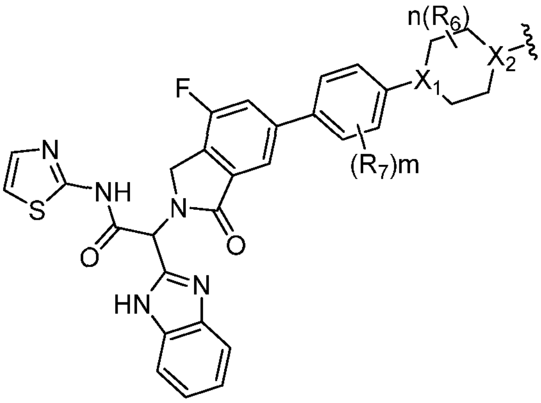
,

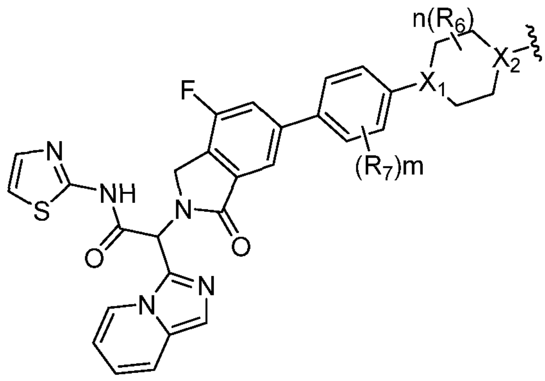
,
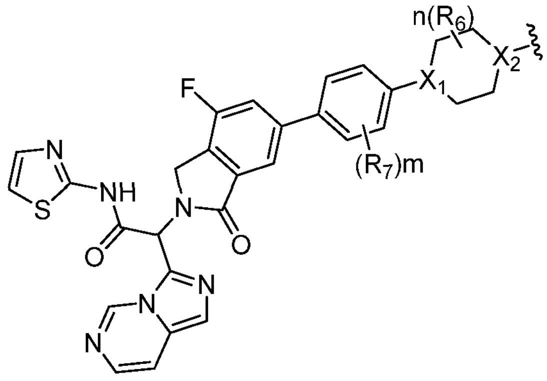

and

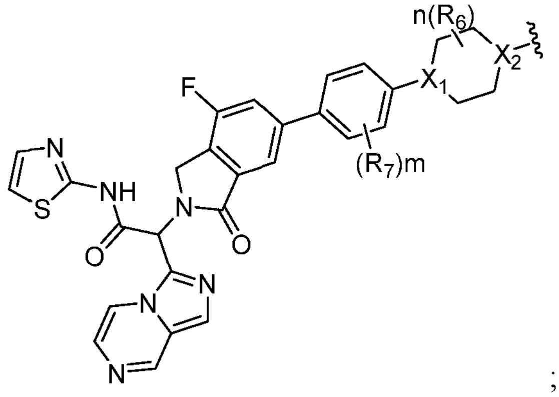
;

B is selected from the group consisting of:

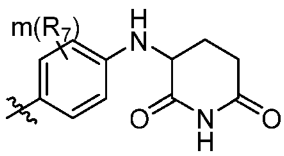, 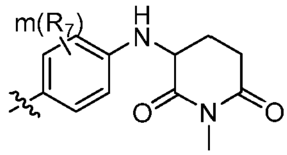,

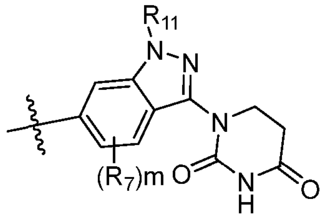, 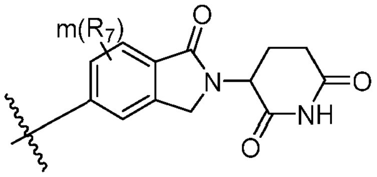, 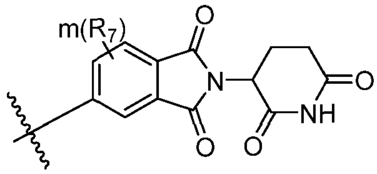,

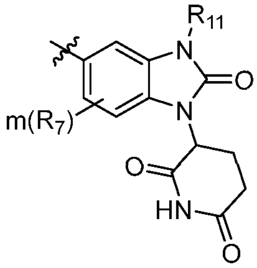, 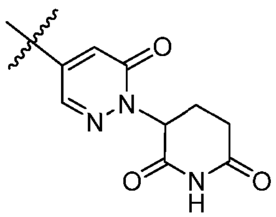, 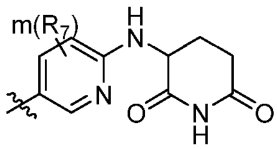, 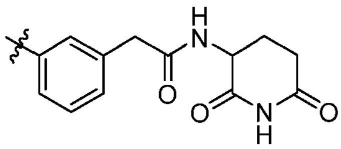,

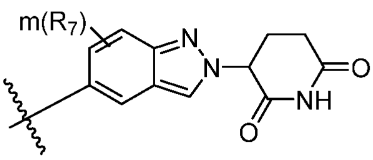, 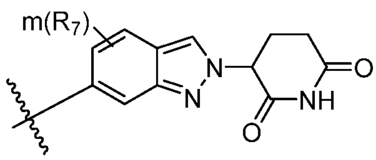, 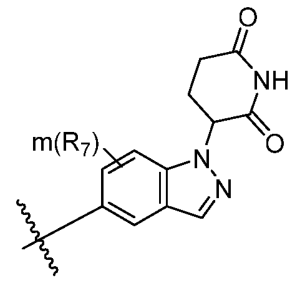,

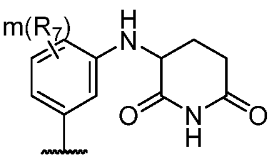, 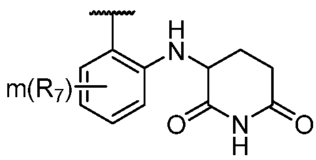, 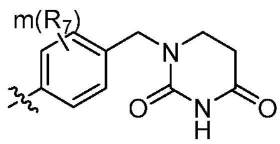,

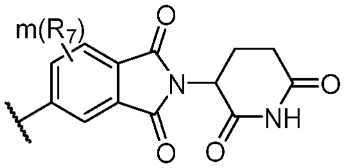 and 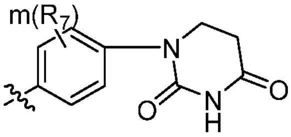;

in each formula:

each $X_1$ and $X_2$ are independently selected from the group consisting of: CR and N;
each $X_3$ is independently selected from the group consisting of: NH, O and none;
each Ar is independently selected from the group consisting of: phenyl or substituted phenyl, heteroaryl or

substituted heteroaryl, and

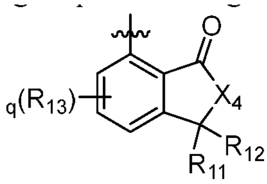

;

$X_4$ is selected from the group consisting of: $CR_{11}R_{12}$, O and NR; the substituted phenyl, and substituted heteroaryl has one or more (e.g., 2, 3, or 4) substituents selected from the group consisting of: halogen, $C_{1-6}$alkyl, $C_{1-6}$haloalkyl, hydroxy-substituted $C_{1-6}$alkyl, $C_{3-6}$cycloalkyl, $C_{1-6}$alkylamino, $C_{1-6}$alkoxy, $C_{1-6}$haloalkoxy, $C_{3-6}$halocycloalkyl, cyano, oxo, $-NR_9C(O)R_{10}$, $-OC(O)NR_9R_{10}$, $-NR_9C(O)OR_{10}$, $-C(O)NR_9R_{10}$, methanesulfonyl, $-NR_9$-methanesulfonyl,

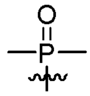

,

-CO-$C_{1-6}$alkyl, -C(=O)O-$C_{1-6}$alkyl, -CO-$C_{3-6}$cycloalkyl, -CO-$C_{1-6}$haloalkyl, and -CO-$C_{3-6}$halocycloalkyl;

each $R_1$ is independently selected from the group consisting of: hydrogen, halogen, $C_{1-6}$haloalkyl and cyano;

each $R_2$, and $R_3$ are independently selected from the group consisting of: H, halogen, $C_{1-6}$alkyl, $C_{3-6}$cycloalkyl, $C_{1-6}$alkylamino, $C_{1-6}$alkoxy, $C_{1-6}$haloalkoxy, $C_{1-6}$haloalkyl, cyano, $C_{3-6}$halocycloalkyl, $-NR_9C(O)R_{10}$, $-C(O)NR_9R_{10}$, methanesulfonyl,

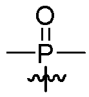

,

and unsubstituted or $C_{1-6}$alkyl-substituted 5-10-membered heteroaryl containing 1, 2 or 3 heteroatoms selected from N, O and S;

each $R_4$ is independently selected from the group consisting of $C_{1-6}$alkyl, $C_{1-6}$haloalkyl, $C_{3-6}$cycloalkyl, and $C_{3-6}$halocycloalkyl;

each $R_5$ is independently absent, $C_{1-6}$alkyl, NR,

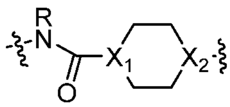

,

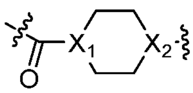

and

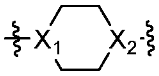

;

each $R_6$, $R_7$, and $R_8$ are independently selected from the group consisting of: H, halogen, $C_{1-6}$alkyl, $C_{3-6}$cycloalkyl, $C_{1-6}$alkylamino, $C_{1-6}$alkoxy, $C_{1-6}$haloalkoxy, $C_{1-6}$haloalkyl, cyano, $C_{3-6}$halocycloalkyl-$NR_9C(O)R_{10}$, $-C(O)NR_9R_{10}$, methanesulfonyl,

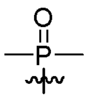

,

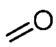

and hydroxy, or $R_6$ together with the attached ring form a 3- to 6-membered ring; each $R_9$ is independently selected from the group consisting of: H, $C_{1-6}$alkyl, $C_{6-10}$ aryl, $C_{1-6}$haloalkyl, $C_{3-6}$cycloalkyl, and $C_{3-6}$halocycloalkyl;

each $R_{10}$ is independently selected from the group consisting of: H, $C_{1-6}$alkyl, $C_{2-6}$alkenyl, $C_{2-6}$alkynyl, $C_{3-6}$cycloalkyl, $C_{1-6}$alkoxy, $C_{1-6}$haloalkoxy, $C_{1-6}$haloalkyl, $C_{3-6}$halocycloalkyl, methyl sulfonyl, and

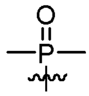 ;

each m, n, and q are independently selected from the group consisting of: 0, 1, 2, 3, 4 and 5;

each $R_{11}$, and $R_{12}$ are independently selected from the group consisting of: H, $C_{1-6}$alkyl, $C_{1-6}$haloalkyl, $C_{3-6}$cycloalkyl, and $C_{3-6}$halocycloalkyl; or $R_{11}$ and $R_{12}$ together with the attached moiety form a 5-7 membered ring;

each $R_{13}$ is independently selected from the group consisting of: H, halogen, $C_{1-6}$alkyl, $C_{1-6}$haloalkyl, $C_{3-6}$cycloalkyl, and $C_{3-6}$halocycloalkyl; and

each R is independently selected from the group consisting of: H, $C_{1-6}$alkyl, hydroxyl, halogen and $C_{1-6}$haloalkyl.

In another preferred embodiment,

L is selected from the group consisting of:

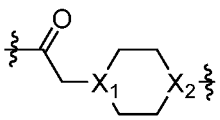 , 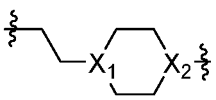 ,

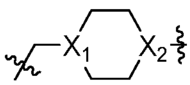 , 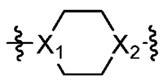 , 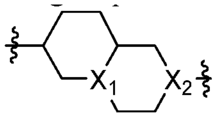 and 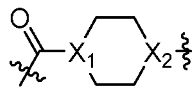 ;

A is selected from the group consisting of:

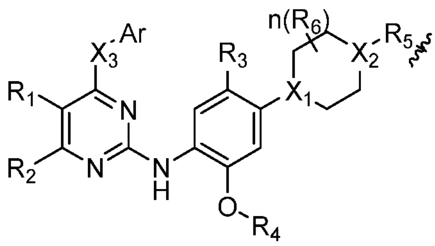 ,

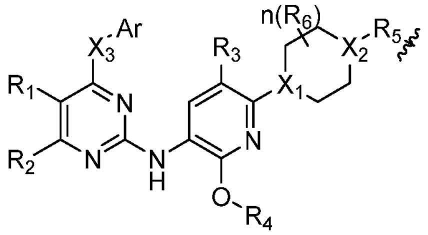 , 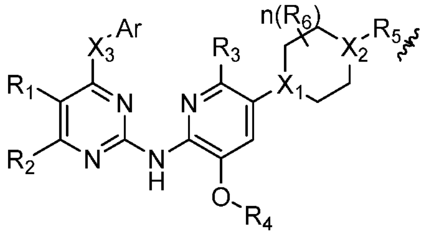 ,

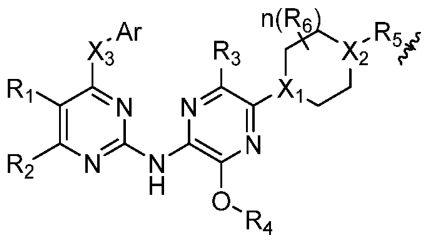
,
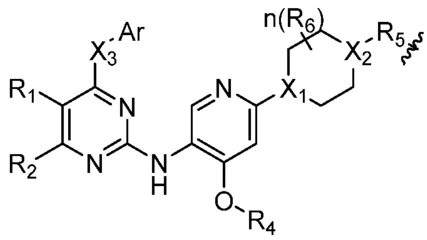
,

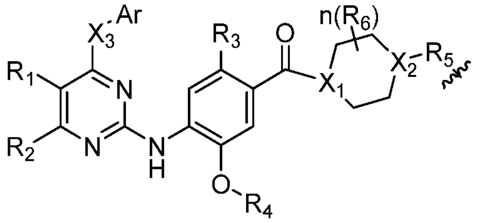
,
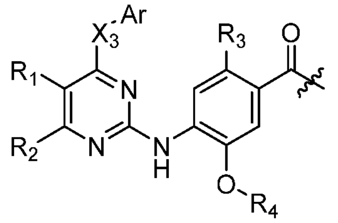
,

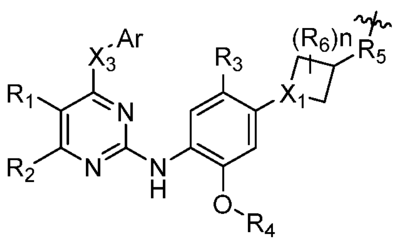
,
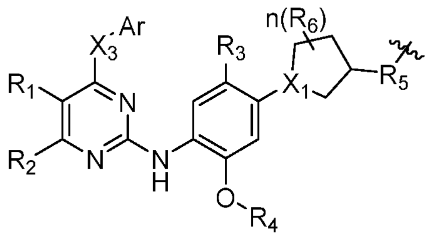
,

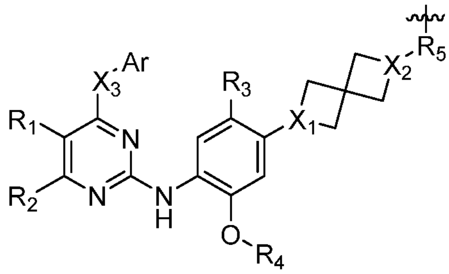
,

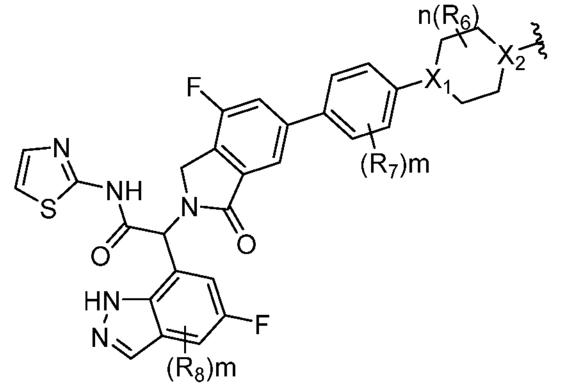
,
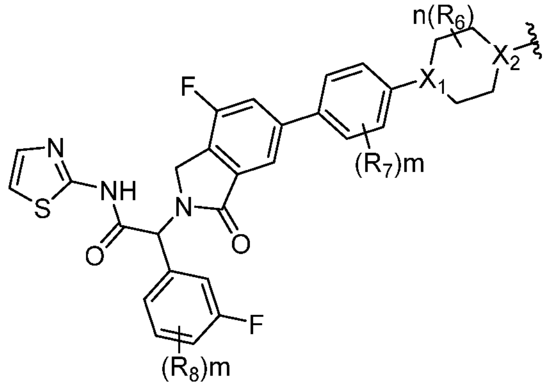
,

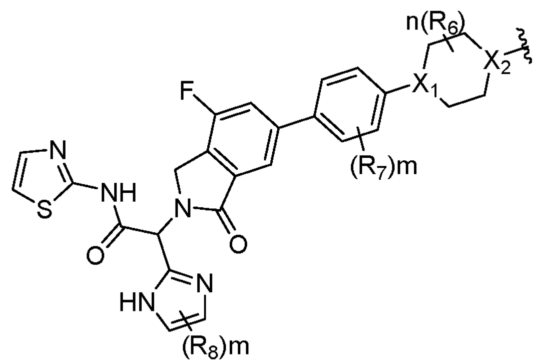 ,

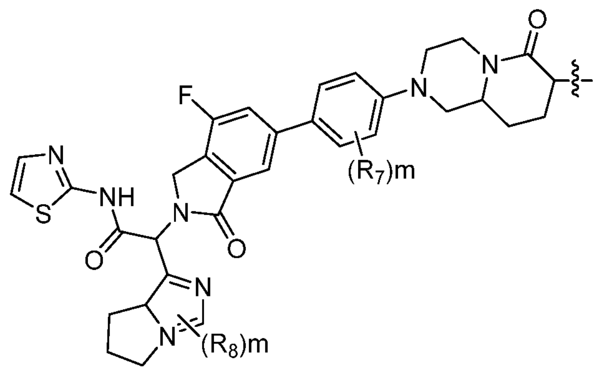 ,

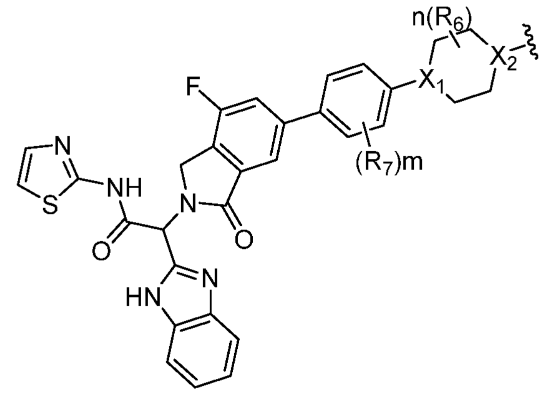 ,

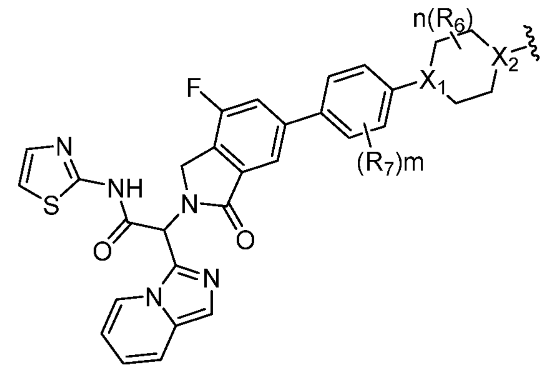 ,

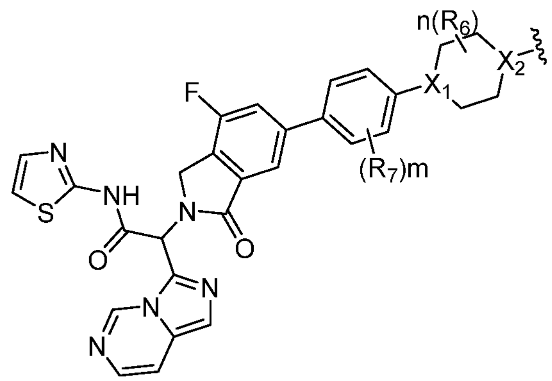 and 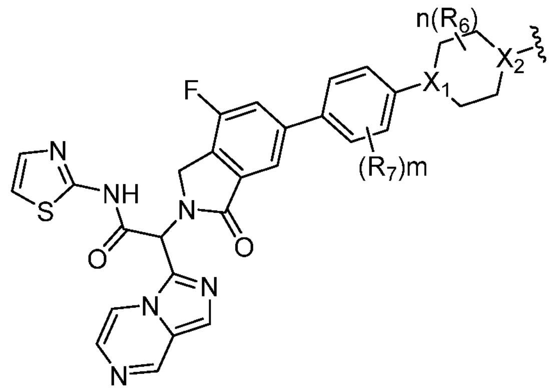 ;

B is selected from the group consisting of:

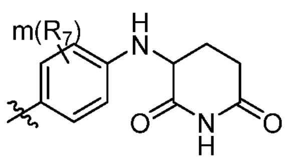 ,

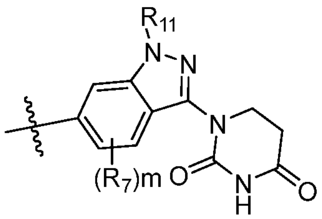 , 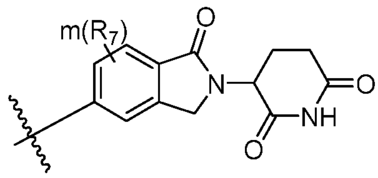 , 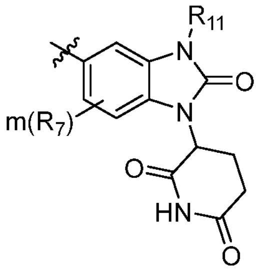 ,

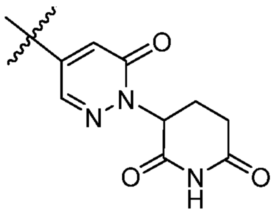 , 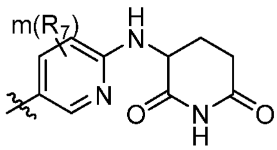 , 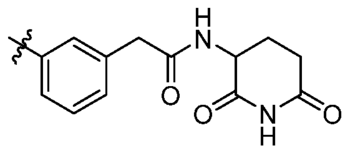 ,

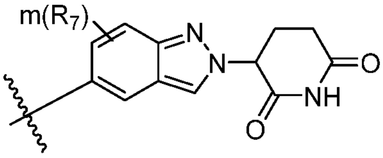 , 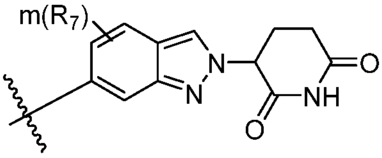 and 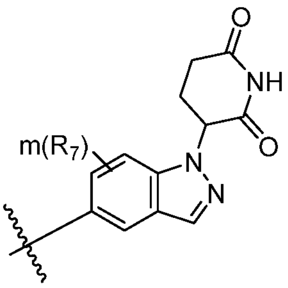 ;

in each formula:

each $X_1$ and $X_2$ are independently selected from the group consisting of: CR and N;
each $X_3$ is independently selected from the group consisting of: NH, O and none;
each Ar is independently selected from the group consisting of: phenyl or substituted phenyl, heteroaryl or substituted heteroaryl, and

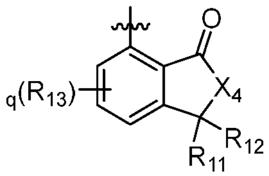 ;

$X_4$ is selected from the group consisting of: $CR_{11}R_{12}$, O, and NR; and the substituted phenyl, and substituted heteroaryl have one or more (e.g. 2, 3 or 4) substituents selected from the group consisting of: halogen, $C_{1-6}$alkyl, $C_{1-6}$haloalkyl, $C_{3-6}$cycloalkyl, $C_{1-6}$alkylamino, $C_{1-6}$alkoxy, $C_{1-6}$haloalkoxy, $C_{3-6}$halocycloalkyl, cyano, $-NR_9C(O)R_{10}$, $-OC(O)NR_9R_{10}$, $-NR_9C(O)OR_{10}$, $-C(O)NR_9R_{10}$, methylsulfonyl,

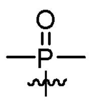 ,

$-CO-C_{1-6}$alkyl, $-CO-C_{3-6}$cycloalkyl, $-CO-C_{1-6}$haloalkyl, and $-CO-C_{3-6}$halocycloalkyl;
each $R_1$ is independently selected from the group consisting of: hydrogen, halogen, $C_{1-6}$haloalkyl, and cyano;
each $R_2$, and $R_3$ are independently selected from the group consisting of: H, halogen, $C_{1-6}$alkyl, $C_{3-6}$cycloalkyl,

$C_{1-6}$alkylamino, $C_{1-6}$alkoxy, $C_{1-6}$haloalkoxy, $C_{1-6}$haloalkyl, cyano, $C_{3-6}$halocycloalkyl, $-NR_9C(O)R_{10}$, $-C(O)NR_9R_{10}$, methylsulfonyl, and

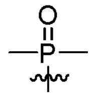 ;

each $R_4$ is independently selected from the group consisting of $C_{1-6}$alkyl, $C_{1-6}$haloalkyl, $C_{3-6}$cycloalkyl, and $C_{3-6}$halocycloalkyl;
each $R_5$ is independently absent, $C_{1-6}$alkyl, NR or

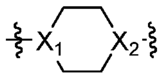 ;

each $R_6$, $R_7$, and $R_8$ are independently selected from the group consisting of: H, halogen, $C_{1-6}$alkyl, $C_{3-6}$cycloalkyl, $C_{1-6}$alkylamino, $C_{1-6}$alkoxy, $C_{1-6}$haloalkoxy, $C_{1-6}$haloalkyl, cyano, $C_{3-6}$halocycloalkyl-$NR_9C(O)R_{10}$, $-C(O)NR_9R_{10}$, methanesulfonyl,

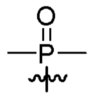 , 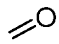 ,

and hydroxy, or $R_6$ together with the attached ring form a 3- to 6-membered ring (i.e., $R_6$ together with

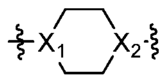

form a bridged ring);
each $R_9$ is independently selected from the group consisting of: H, $C_{1-6}$alkyl, $C_{1-6}$haloalkyl, $C_{3-6}$cycloalkyl, and $C_{3-6}$halocycloalkyl;
each $R_{10}$ is independently selected from the group consisting of: H, $C_{1-6}$alkyl, $C_{2-6}$alkenyl, $C_{2-6}$alkynyl, $C_{3-6}$cycloalkyl, $C_{1-6}$alkoxy, $C_{1-6}$haloalkoxy, $C_{1-6}$haloalkyl, $C_{3-6}$halocycloalkyl, methylsulfonyl, and

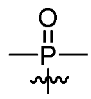 ;

each m, n, and q are independently selected from the group consisting of: 0, 1, 2, 3, 4 and 5;
each $R_{11}$, and $R_{12}$ are independently selected from the group consisting of: H, $C_{1-6}$alkyl, $C_{1-6}$haloalkyl, $C_{3-6}$cycloalkyl, and $C_{3-6}$halocycloalkyl; or $R_{11}$ and $R_{12}$ together with the attached moiety form a 5- to-7 membered ring;
each $R_{13}$ is independently selected from the group consisting of: H, halogen, $C_{1-6}$alkyl, $C_{1-6}$haloalkyl, $C_{3-6}$cycloalkyl, and $C_{3-6}$halocycloalkyl; and
each R is independently selected from the group consisting of: H, $C_{1-6}$alkyl and $C_{1-6}$haloalkyl.

In another preferred embodiment, the heteroaryl is each independently a 5-to 10-membered heteroaryl containing 1, 2 or 3 heteroatoms selected from N, O and S.
In another preferred embodiment, L is selected from the group consisting of:

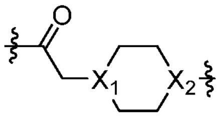, 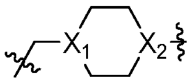 and 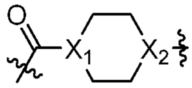.

In another preferred embodiment, both $X_1$ and $X_2$ are N; or one of $X_1$ is N and the other is both CR.

In another preferred embodiment, $R_1$ is selected from the group consisting of: hydrogen, halogen, trifluoromethyl and cyano.

In another preferred embodiment, $R_6$ together with the attached ring form a 3- to 6-membered ring, thereby forming a spiro, fused or bridged ring, preferably, $R_6$ is $C_{1-4}$alkylene and together with the attached ring form

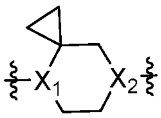, 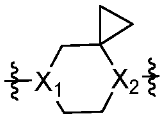,

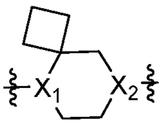, 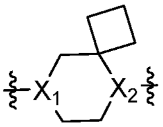, 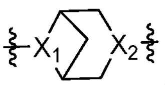, 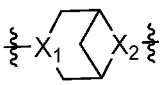, 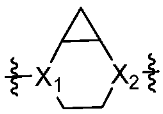 and 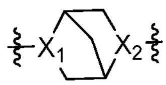,

more preferably, $R_6$ is methylene.

In another preferred embodiment, each Ar is independently selected from the group consisting of: phenyl or substituted phenyl, heteroaryl or substituted heteroaryl; wherein the heteroaryl is selected from the group consisting of: pyridinyl

( 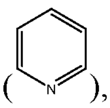 ),

thiazolyl

( 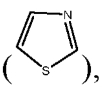 ),

indolyl

( 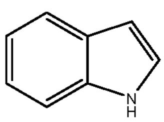 ),

quinolinyl

( 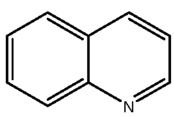 ), 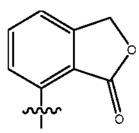, 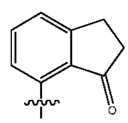, 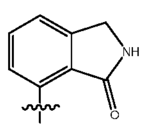

and

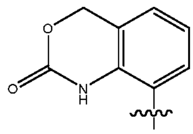.

In another preferred embodiment, Ar is selected from the group consisting of:

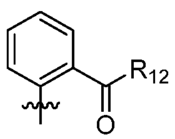, 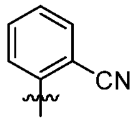, 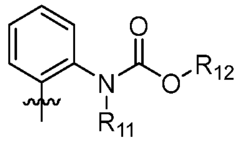, 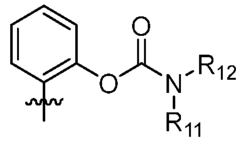, 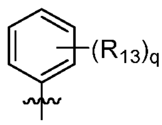,

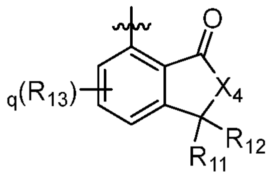 and 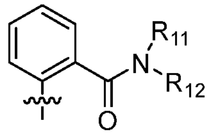;

wherein $X_4$ is selected from the group consisting of: $CR_{11}R_{12}$, O and NR;

each $R_{11}$ is independently selected from the group consisting of: H, $C_{1-6}$alkyl, $C_{1-6}$haloalkyl, $C_{3-6}$cycloalkyl and $C_{3-6}$halocycloalkyl;

each $R_{12}$ is independently selected from the group consisting of: H, $C_{1-6}$alkyl, $C_{1-6}$haloalkyl, $C_{3-6}$cycloalkyl, and $C_{3-6}$halocycloalkyl; or $R_{11}$ and $R_{12}$ together with the attached moiety form a 5-to 7 membered ring;

each $R_{13}$ is independently halogen; and

q is selected from the group consisting of: 0, 1, 2, 3, 4 and 5.

In another preferred embodiment, A is selected from the group consisting of:

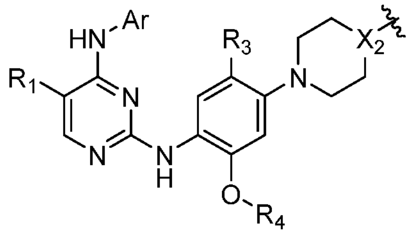, 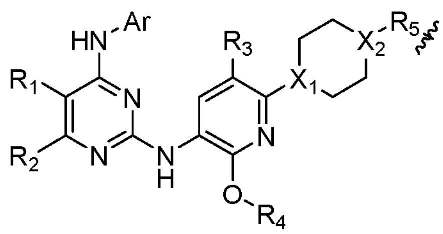,

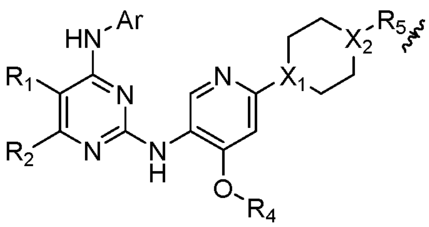, 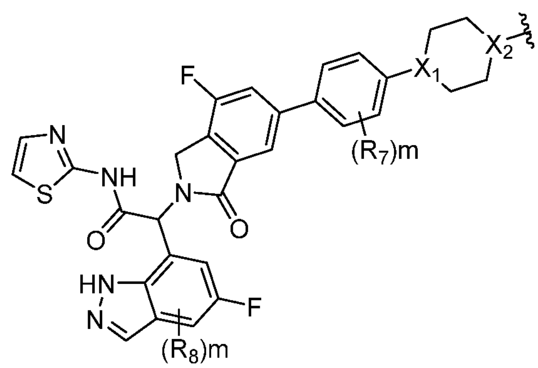.

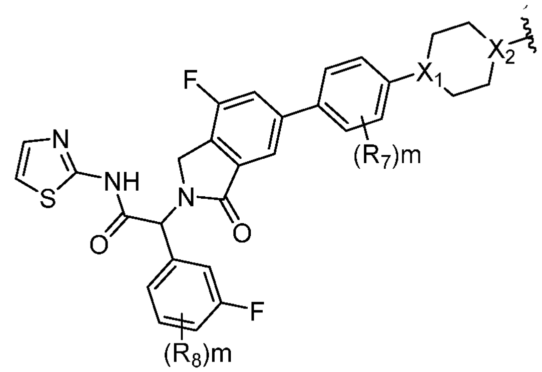

and

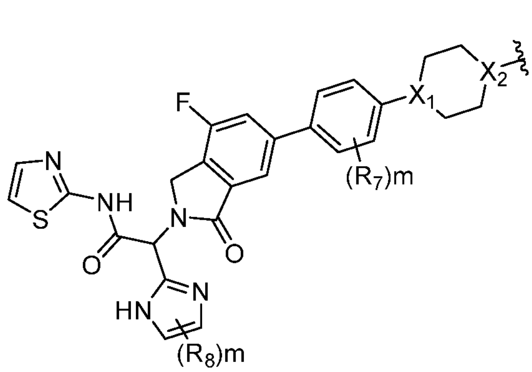

,

wherein $R_1$ is selected from the group consisting of: chlorine, trifluoromethyl and bromine;
Ar is selected from the group consisting of:

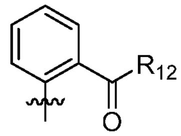

,

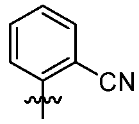

,

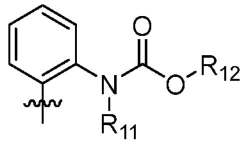

,

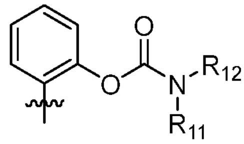

,

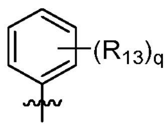

,

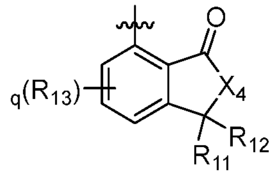

and

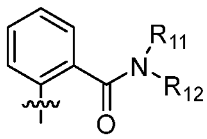

;

each $R_3$ is independently selected from the group consisting of: H, halogen, $C_{1-6}$alkyl, $C_{1-6}$haloalkyl, cyano, and $-NR_9C(O)R_{10}$ ;
each $R_{12}$ is independently selected from the group consisting of: H, $C_{1-6}$alkyl, $C_{1-6}$haloalkyl, $C_{3-6}$cycloalkyl, and $C_{3-6}$halocycloalkyl.
each $R_{13}$ is independently halogen;
q is selected from the group consisting of: 0, 1, 2, 3, 4 and 5;
$X_4$, $R_2$, $R_4$, $R_7$, $R_8$, $R_9$, $R_{10}$, $R_{11}$, $R_5$, $X_1$, $X_2$, and m are defined as described above.

In another preferred embodiment, B is selected from the group consisting of:

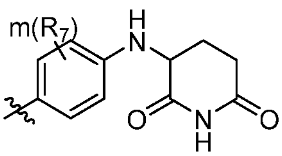

,

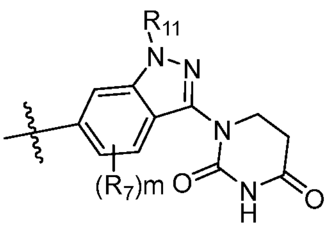

,

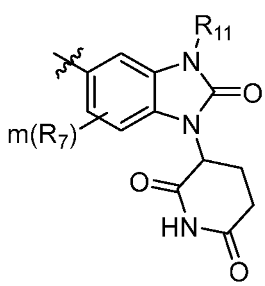

and

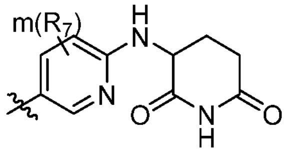

,

wherein $R_7$, $R_{11}$ and m are as defined above.

In another preferred embodiment, L is selected from

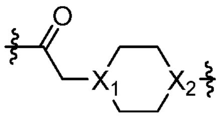

,

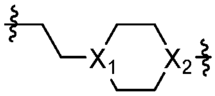

,

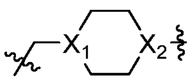

,

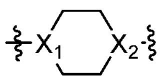

and

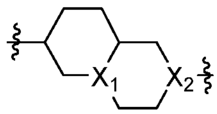

;

each $X_1$, and $X_2$ are independently selected from CR and N; each R is independently selected from the group consisting of: H and $C_{1-6}$alkyl;

A is selected from the group consisting of:

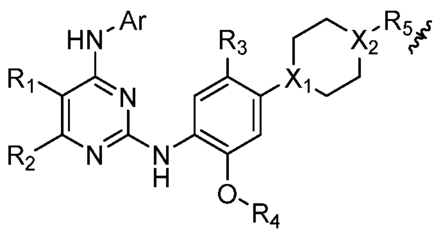

,

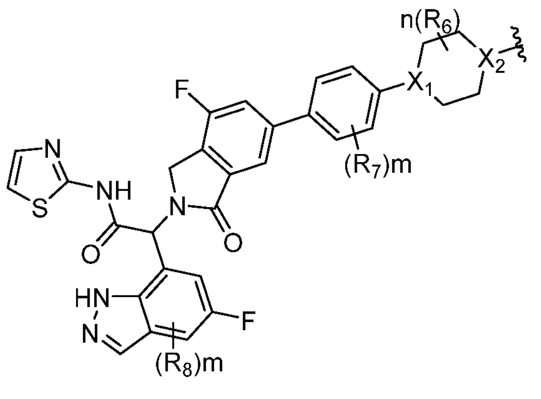

,

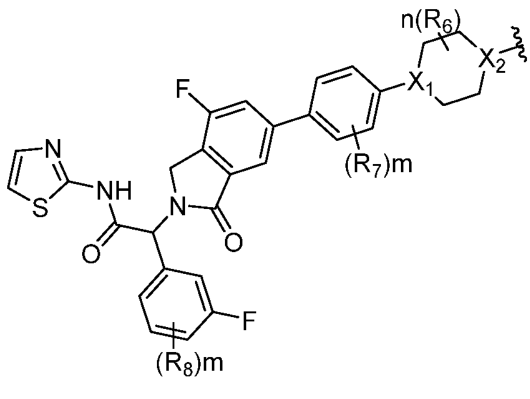

,

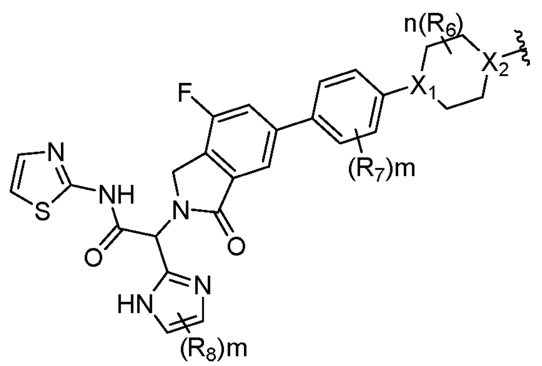,
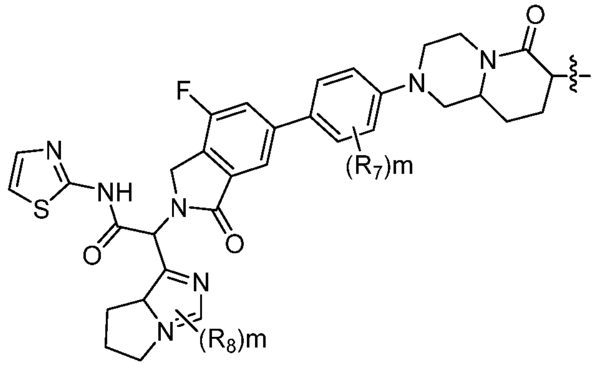,

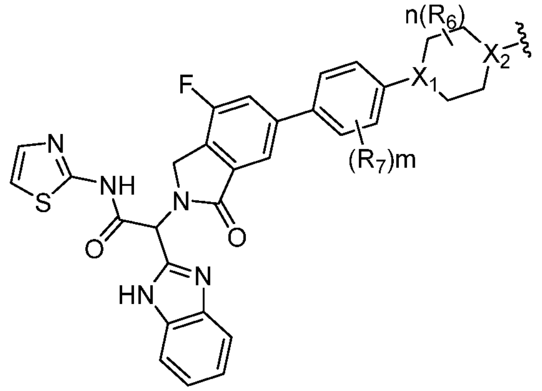,
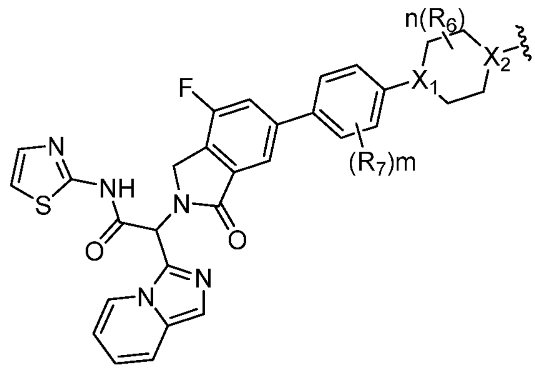,

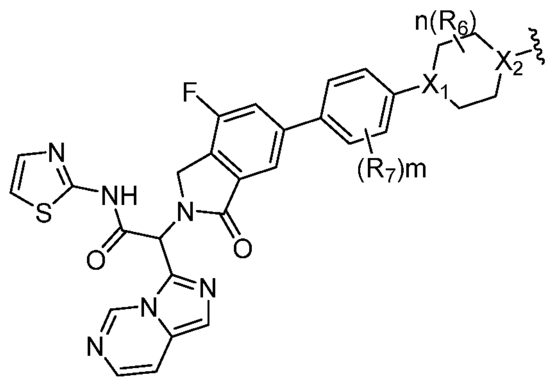
and
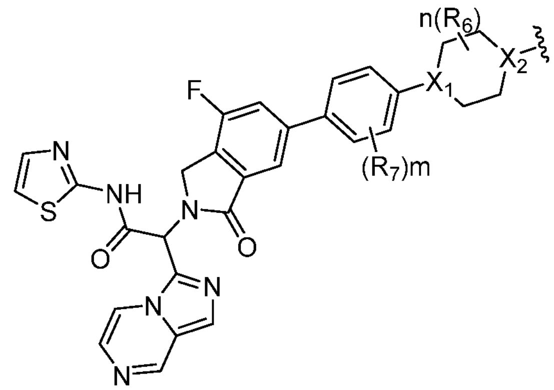;

wherein,

Ar is selected from phenyl or substituted phenyl, heteroaryl or substituted heteroaryl; and the substituents of substituted phenyl, and substituted heteroaryl are halogen, $C_{1-6}$alkyl, $C_{3-6}$cycloalkyl, $C_{1-6}$alkylamino, $C_{1-6}$alkoxy, $C_{1-6}$haloalkoxy, $C_{3-6}$halocycloalkyl, cyano, $-NR_9C(O)R_{10}$, methylsulfonyl,

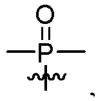,

and $-CO-C_{1-6}$alkyl;

$R_1$ is selected from hydrogen, halogen, trifluoromethyl and cyano;

$R_2$, and $R_3$ are each independently selected from H, halogen, $C_{1-6}$alkyl, $C_{3-6}$cycloalkyl, $C_{1-6}$alkylamino, $C_{1-6}$alkoxy, $C_{1-6}$haloalkoxy, $C_{3-6}$halocycloalkyl, cyano, $-NR_9C(O)R_{10}$, methylsulfonyl and

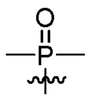;

$R_4$ is selected from $C_{1-6}$alkyl, $C_{1-6}$haloalkyl, $C_{3-6}$cycloalkyl, and $C_{3-6}$halocycloalkyl;
$R_5$ is absent, $C_{1-6}$alkyl, or

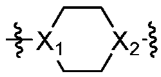;

each $R_6$, $R_7$, and $R_8$ are independently selected from H, halogen, $C_{1-6}$alkyl, $C_{3-6}$cycloalkyl, $C_{1-6}$alkylamino, $C_{1-6}$alkoxy, $C_{1-6}$haloalkoxy, $C_{3-6}$halocycloalkyl, cyano, -$NR_9C(O)R_{10}$, methanesulfonyl,

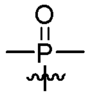, 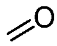,

and hydroxy, or $R_6$ together with the attached ring form a 3- to 6-membered ring;
$R_9$ is selected from H, $C_{1-6}$alkyl, $C_{1-6}$haloalkyl, $C_{3-6}$cycloalkyl, and $C_{3-6}$halocycloalkyl;
$R_{10}$ is selected from $C_{1-6}$alkyl, $C_{3-6}$cycloalkyl, $C_{1-6}$alkoxy, $C_{1-6}$haloalkoxy, $C_{3-6}$halocycloalkyl, methanesulfonyl, and

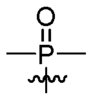;

each m, and n are independently selected from 0, 1, 2, 3, 4 and 5;
B is selected from the group consisting of:

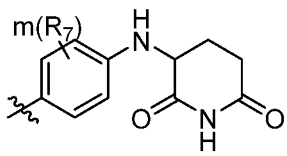,

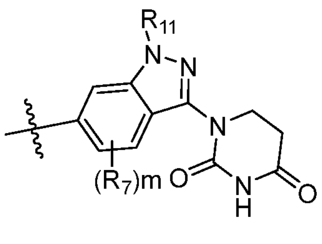, 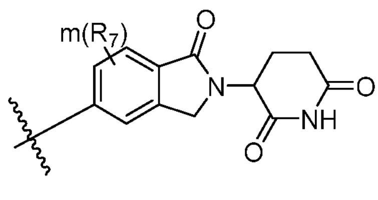, 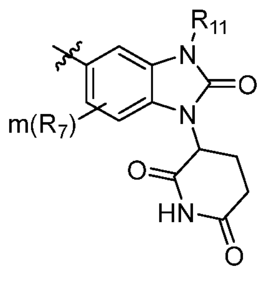,

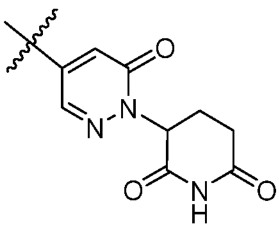, 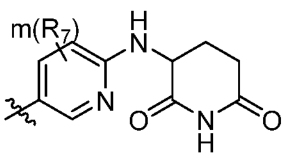 and 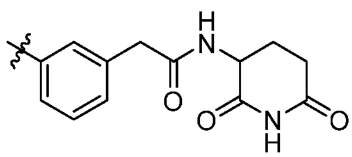;

wherein $R_{11}$ is selected from H, $C_{1-6}$alkyl, $C_{1-6}$haloalkyl, $C_{3-6}$cycloalkyl, and $C_{3-6}$halocycloalkyl.

In another preferred embodiment,

L is selected from

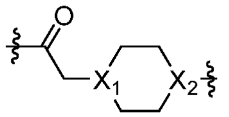

and

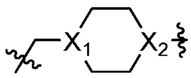

;

each $X_1$ and $X_2$ are as defined above.

In another preferred embodiment,
A is selected from the group consisting of:

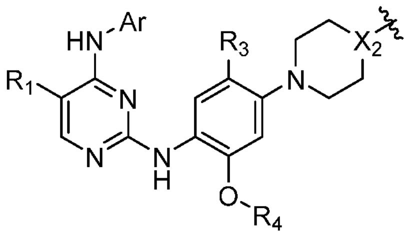

,

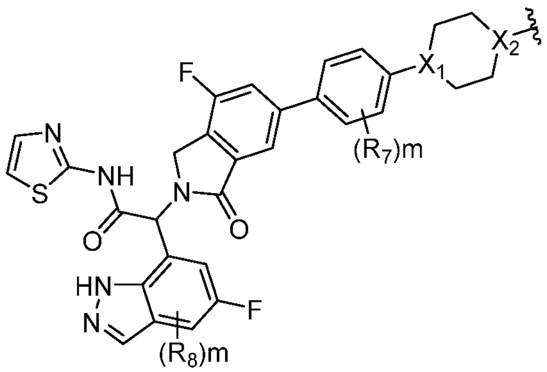

,

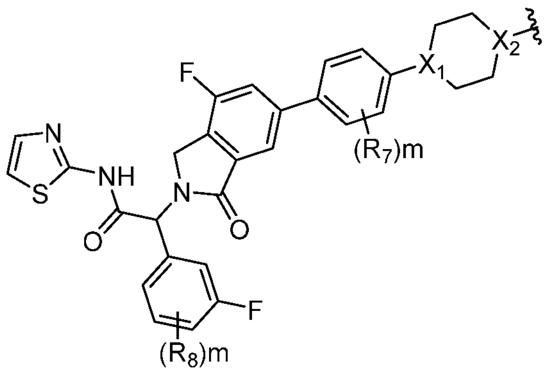

and

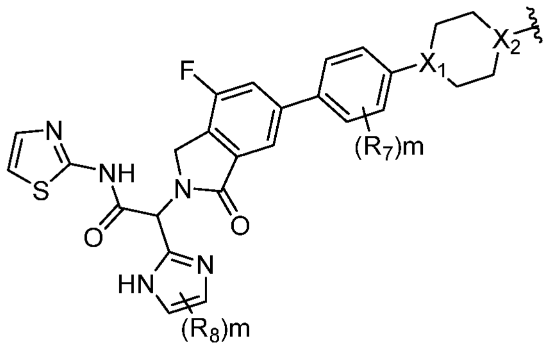

;

wherein $R_1$ is selected from: chlorine, and trifluoromethyl;
Ar is selected from phenyl or substituted phenyl, and heteroaryl or substituted heteroaryl;
$R_3$, $R_4$, $R_7$, $R_8$, $X_1$, $X_2$, and m are as described above.

In another preferred embodiment,
B is selected from

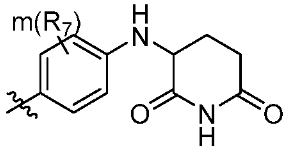,
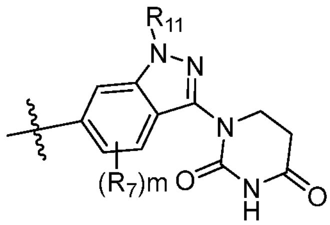,
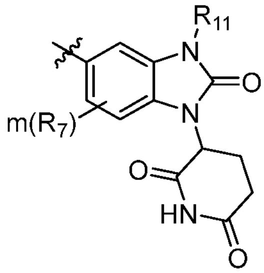

and

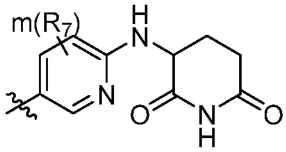,

wherein $R_7$, $R_{11}$, and m are as described above.

In another preferred embodiment, the compound is selected from the group consisting of:

| | |
|---|---|
| 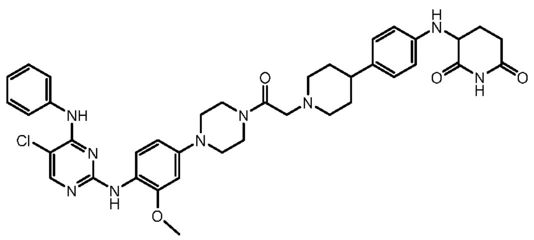 T-01 | 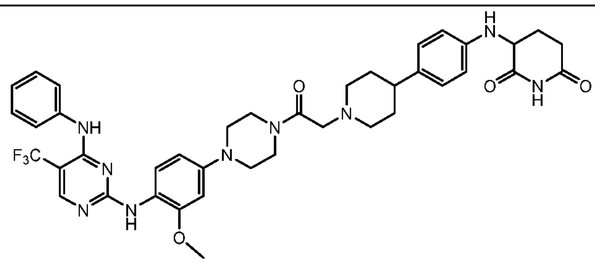 T-02 |
| 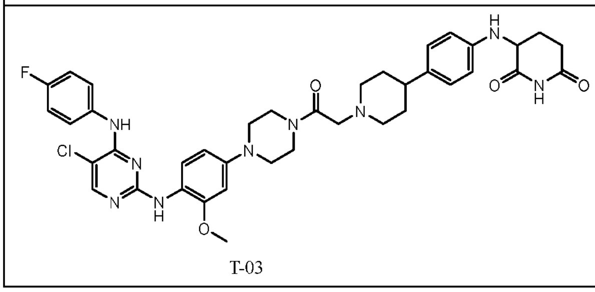 T-03 | 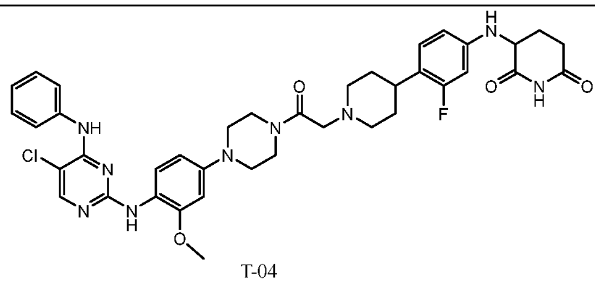 T-04 |

| | |
|---|---|
| 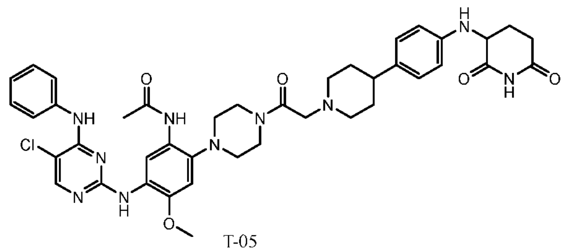 T-05 | 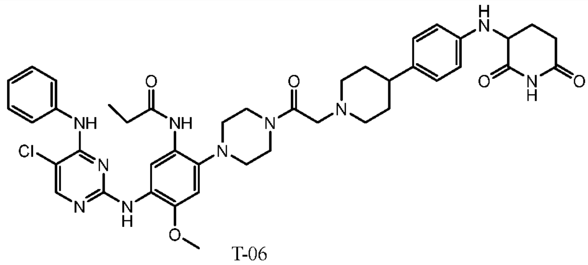 T-06 |
| 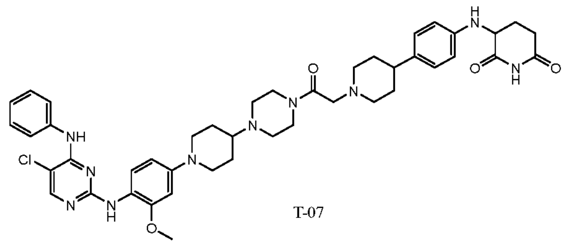 T-07 | 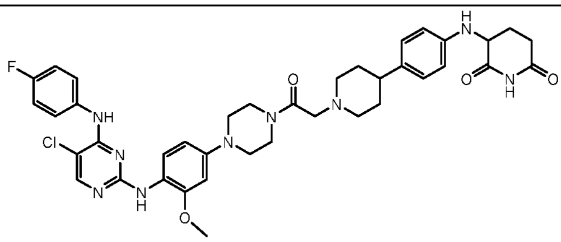 T-08 |
| 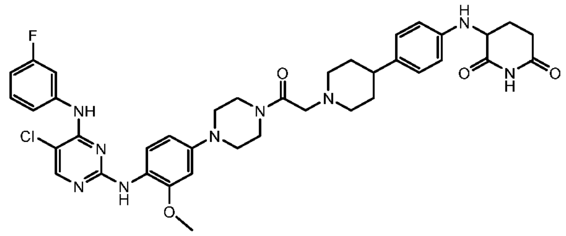 T-09 | 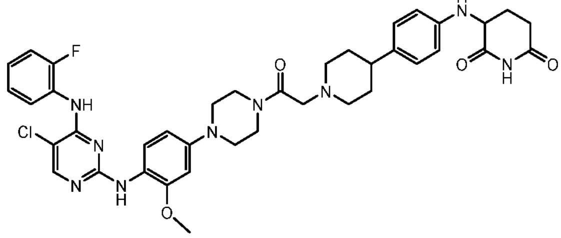 T-10 |
| 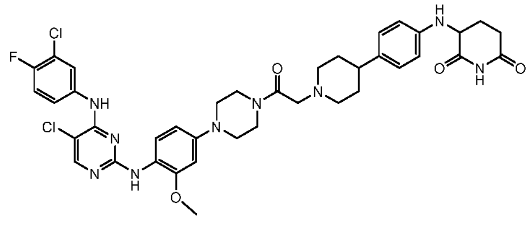 T-11 | 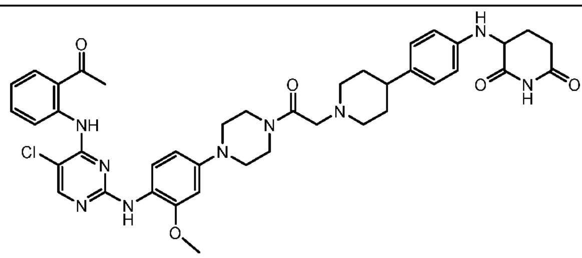 T-12 |
| 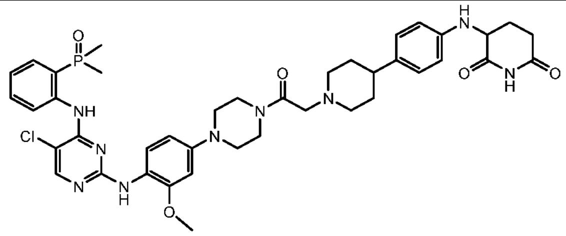 T-13 | 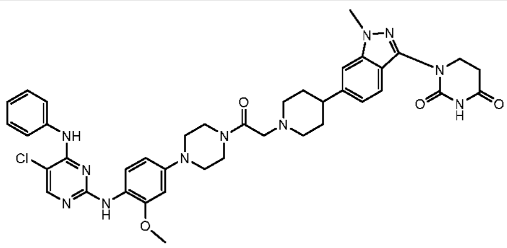 T-14 |
| 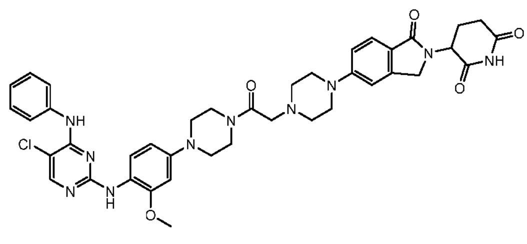 T-15 | 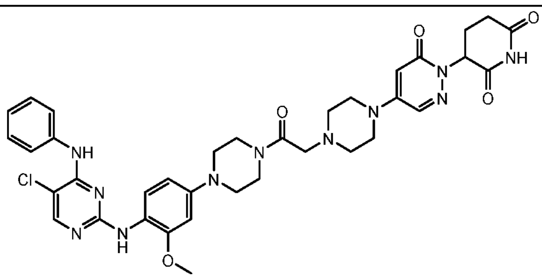 T-16 |

| | |
|---|---|
| 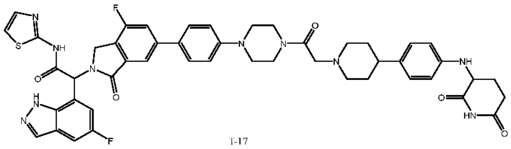 T-17 | 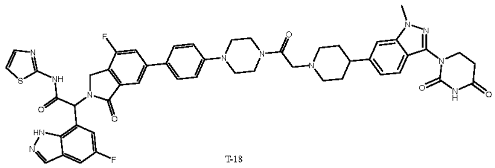 T-18 |
| 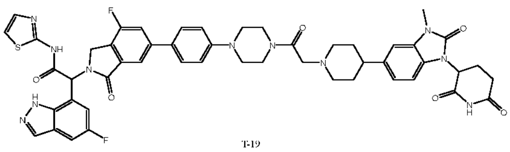 T-19 | 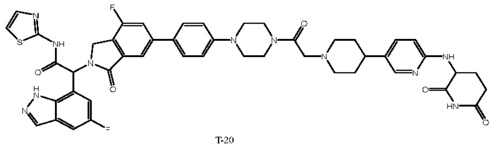 T-20 |
| 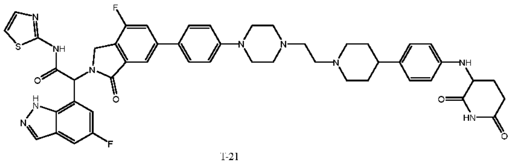 T-21 | 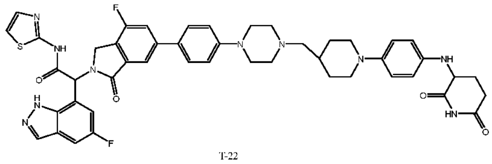 T-22 |
| 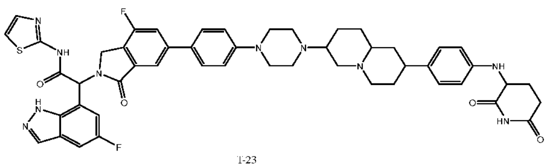 T-23 | 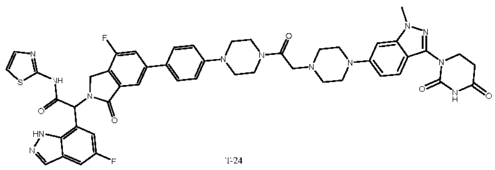 T-24 |
| 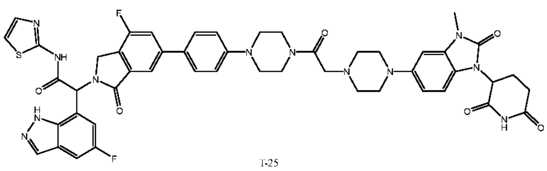 T-25 | 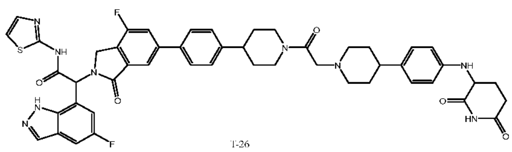 T-26 |
| 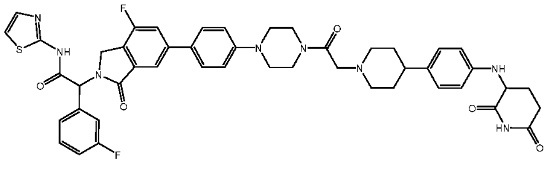 T-27 | 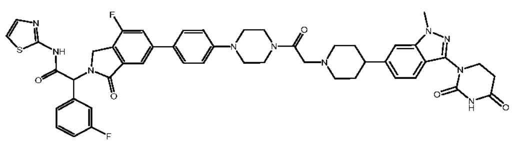 T-28 |
| 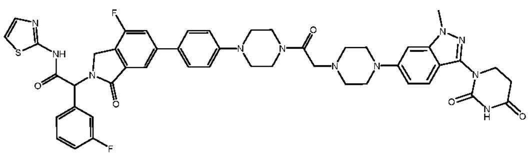 T-29 | 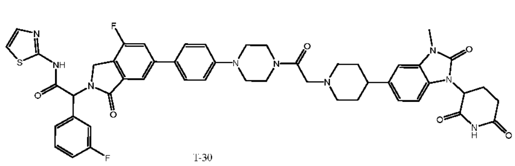 T-30 |

| | |
|---|---|
| 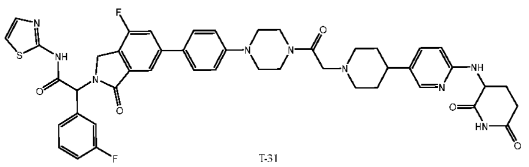 T-31 | 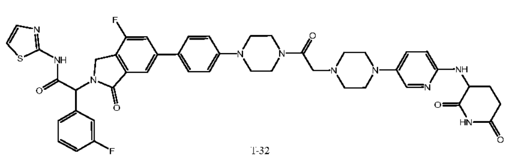 T-32 |
| 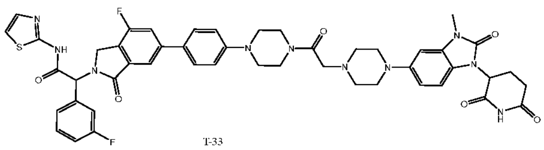 T-33 | 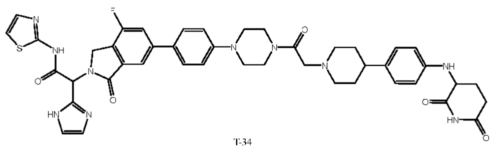 T-34 |
| 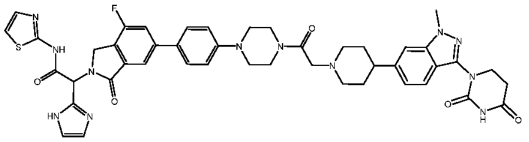 T-35 | 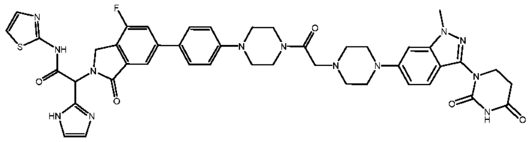 T-36 |
| 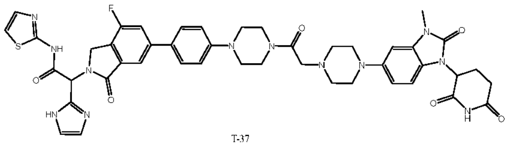 T-37 | 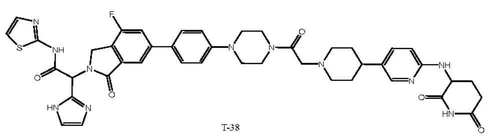 T-38 |
| 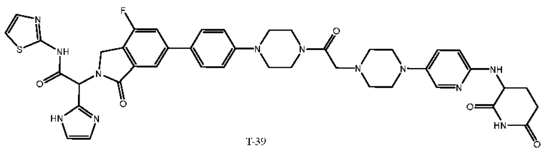 T-39 | 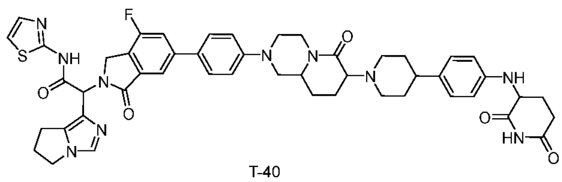 T-40 |
| 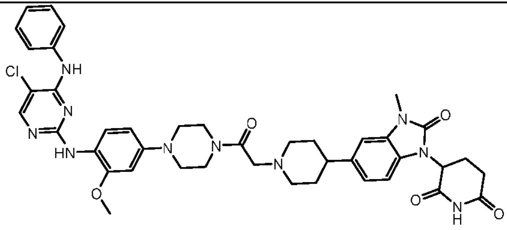 T-41 | 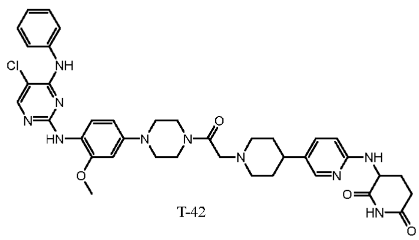 T-42 |
| 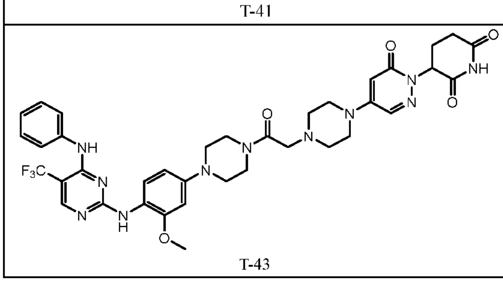 T-43 | 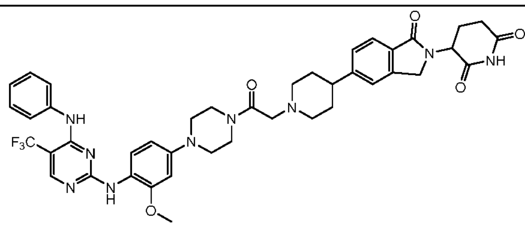 T-44 |

| | |
|---|---|
| 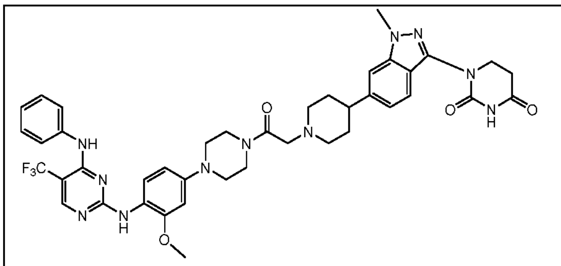<br>T-45 | 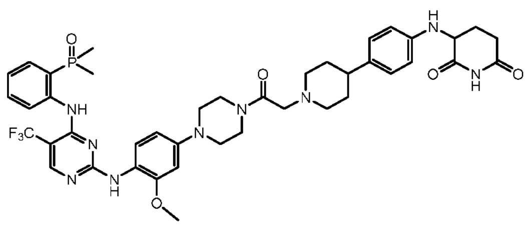<br>T-46 |
| 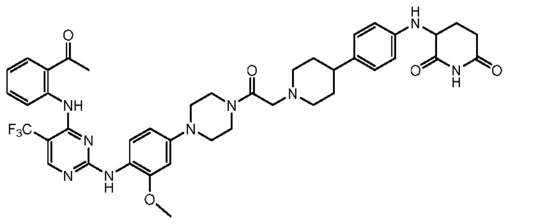<br>T-47 | 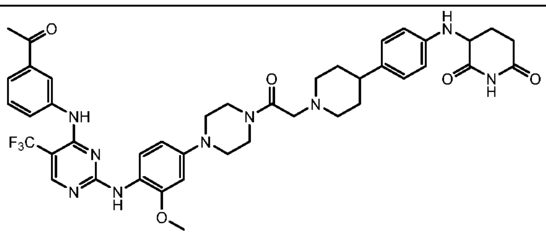<br>T-48 |
| 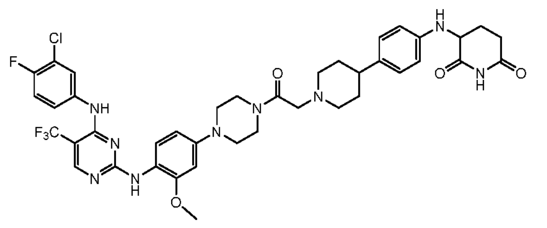<br>T-49 | 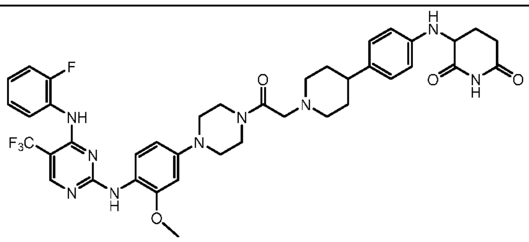<br>T-50 |
| 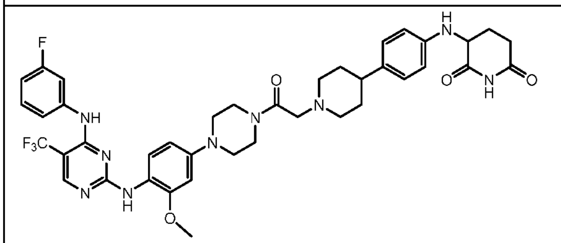<br>T-51 | 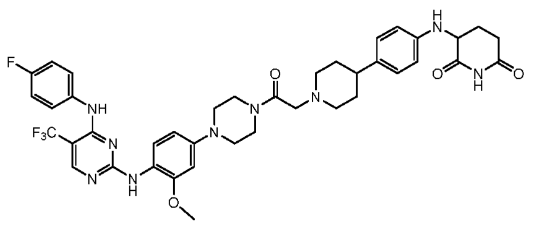<br>T-52 |
| 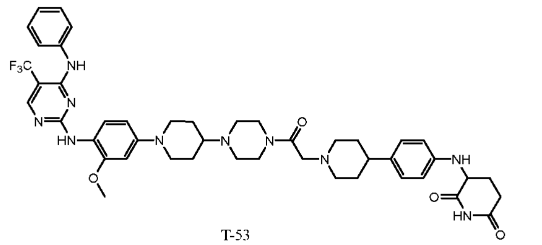<br>T-53 | 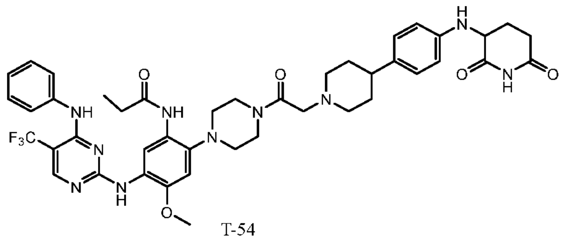<br>T-54 |
| 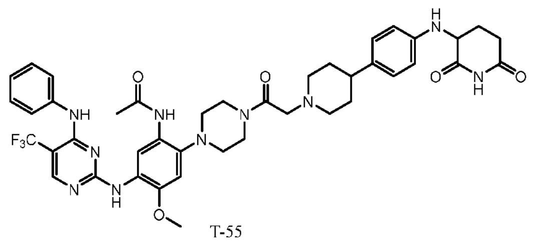<br>T-55 | 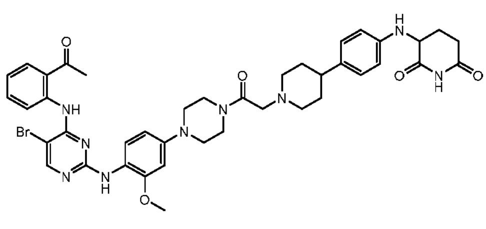<br>T-56 |

| | |
|---|---|
| 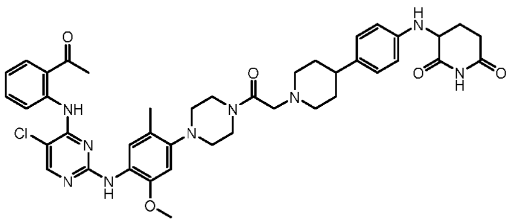 T-57 | 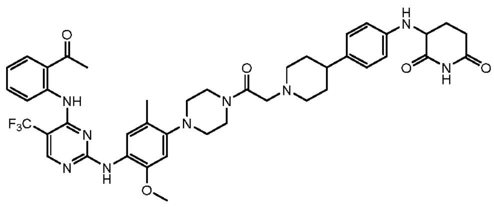 T-58 |
| 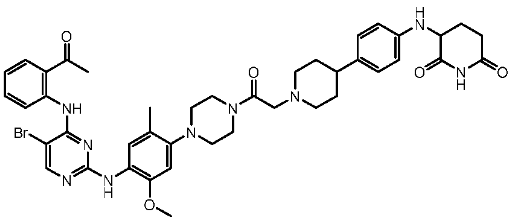 T-59 | 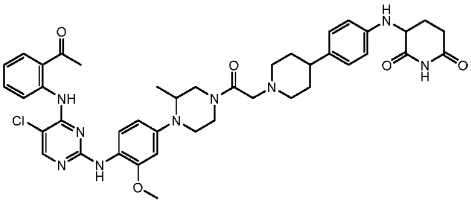 T-60 |
| 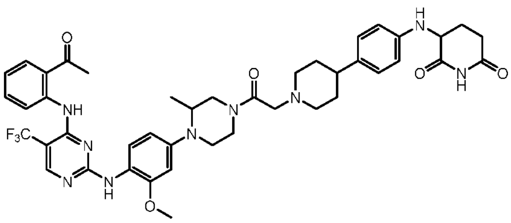 T-61 | 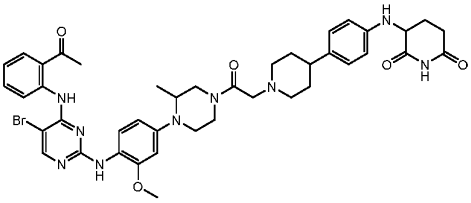 T-62 |
| 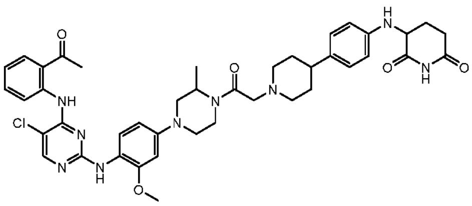 T-63 | 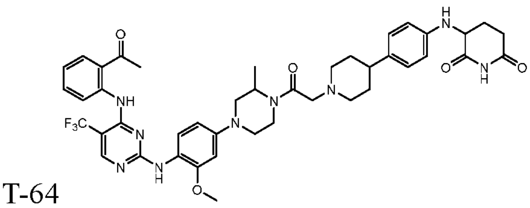 T-64 |
| 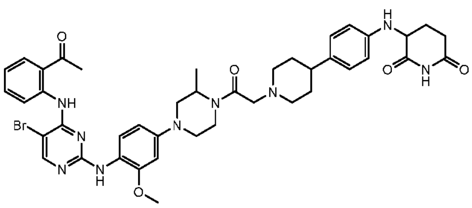 T-65 | 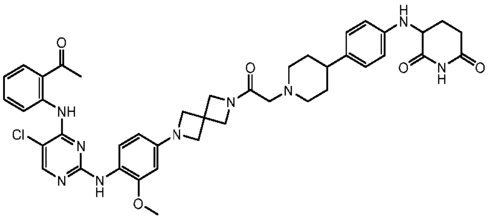 T-66 |
| 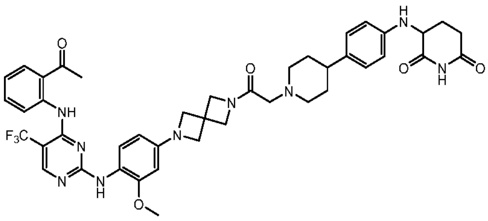 T-67 | 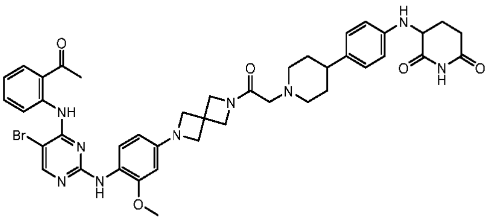 T-68 |
| 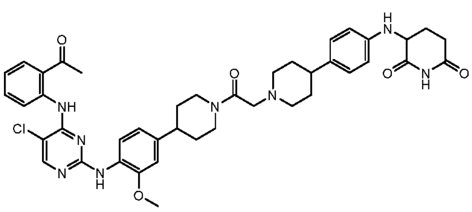 T-69 | 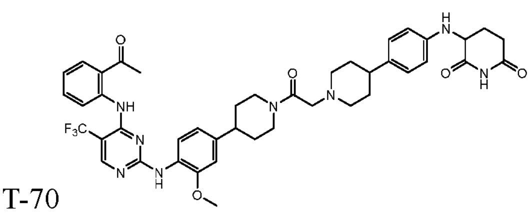 T-70 |

| | |
|---|---|
| 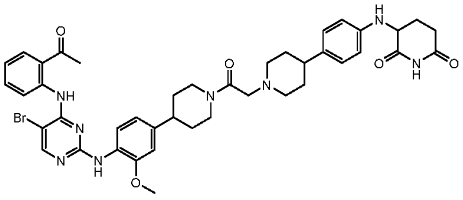 T-71 | 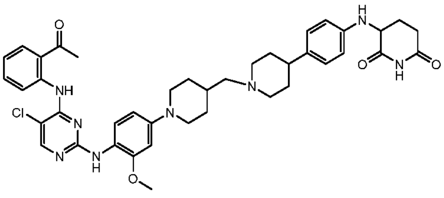 T-72 |
| 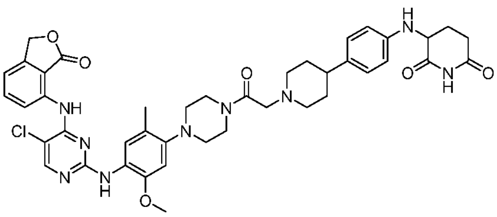 T-73 | 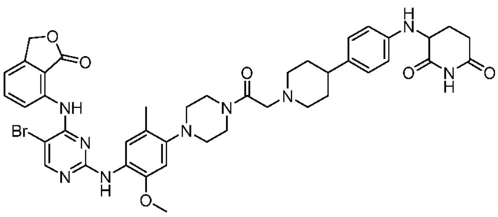 T-74 |
| 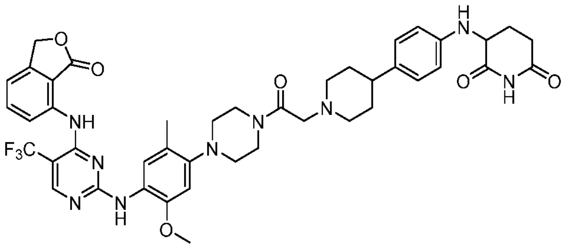 T-75 | 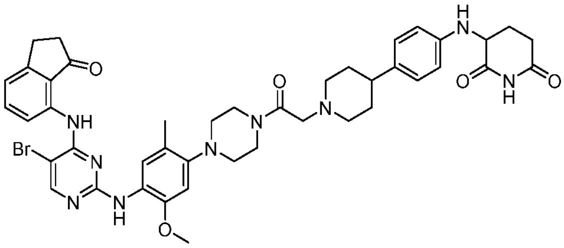 T-76 |
| 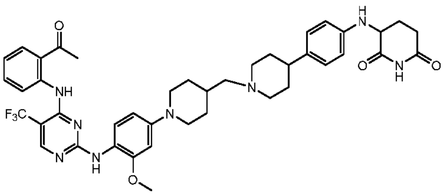 T-77 | 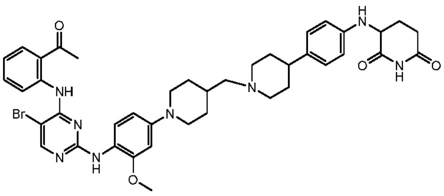 T-78 |
| 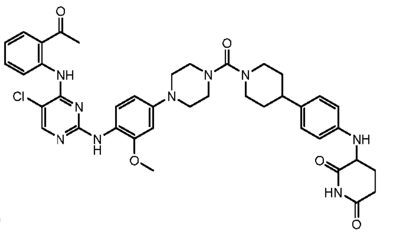 T-79 | 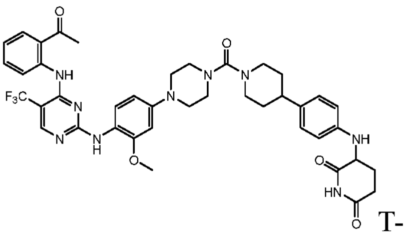 T-80 |
| 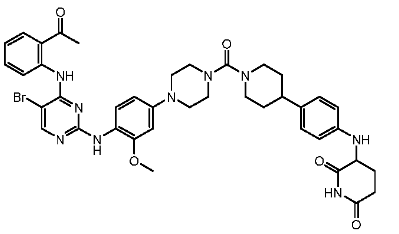 T-81 | 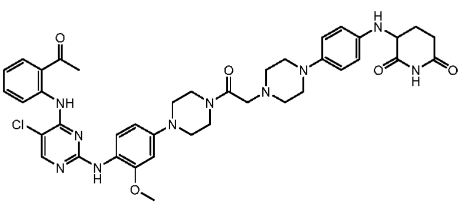 T-82 |
| 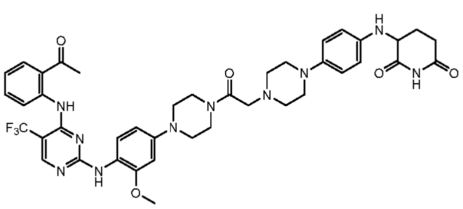 T-83 | 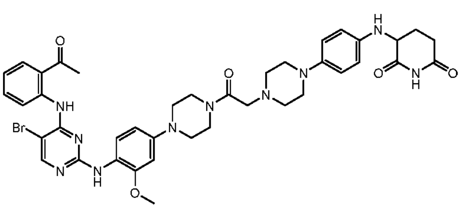 T-84 |

| | |
|---|---|
| 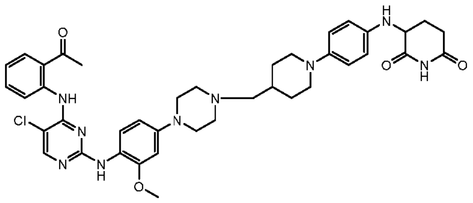 T-85 | 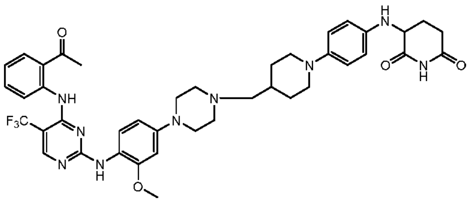 T-86 |
| 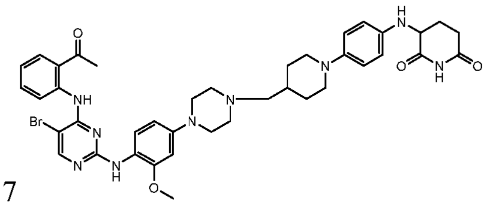 T-87 | 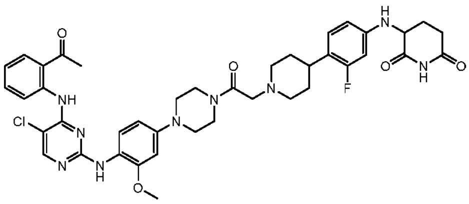 T-88 |
| 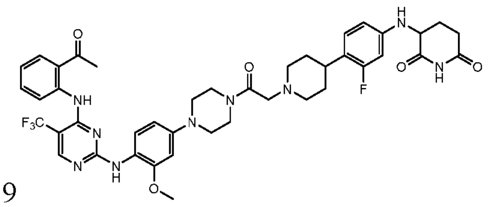 T-89 | 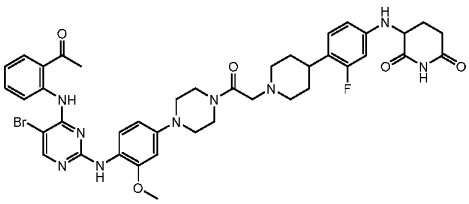 T-90 |
| 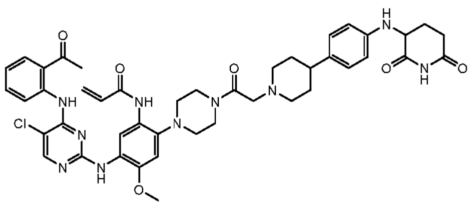 T-91 | 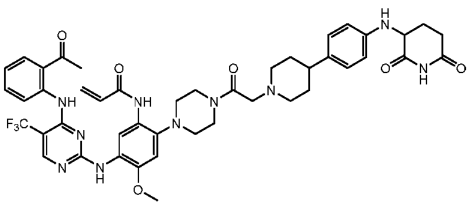 T-92 |
| 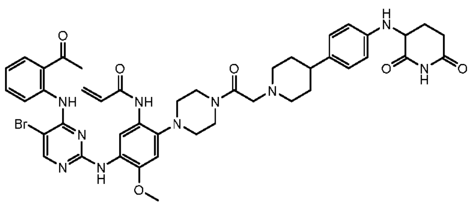 T-93 | 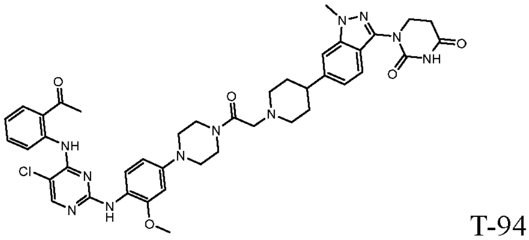 T-94 |
| 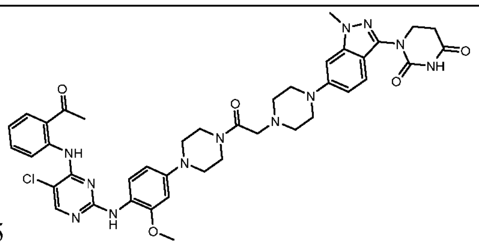 T-95 | 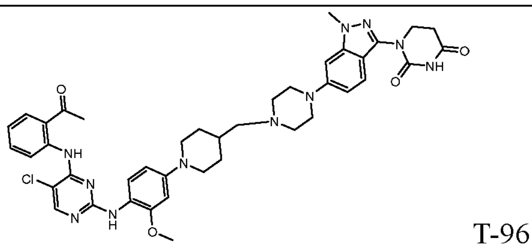 T-96 |
| 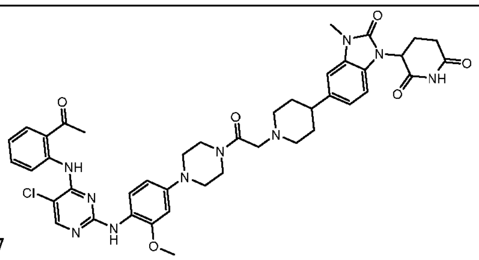 T-97 | 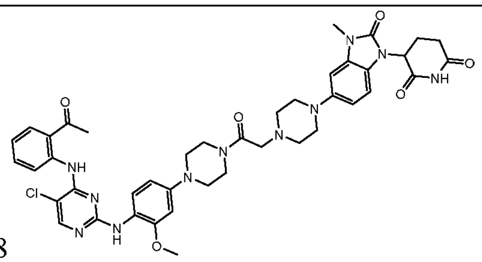 T-98 |

| | |
|---|---|
| 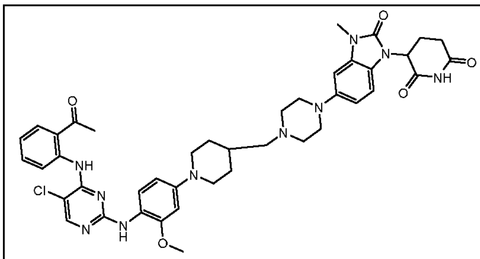 T-99 | 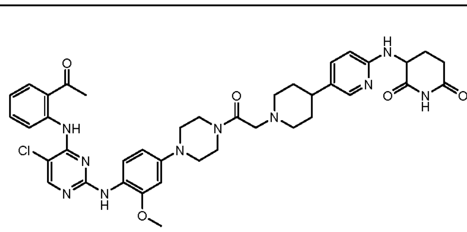 T-100 |
| 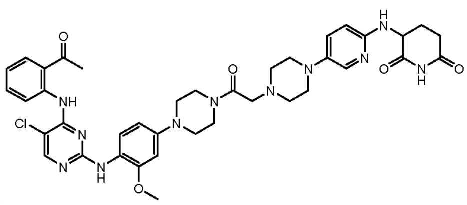 T-101 | 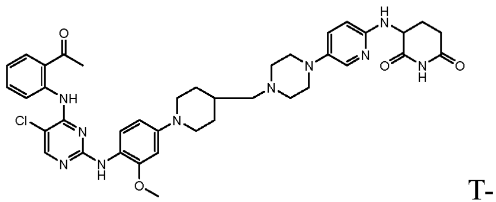 T-102 |
| 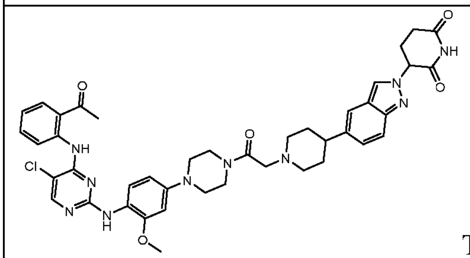 T-103 | 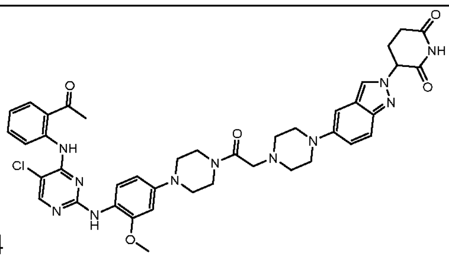 T-104 |
| 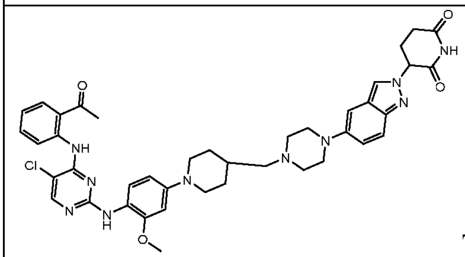 T-105 | 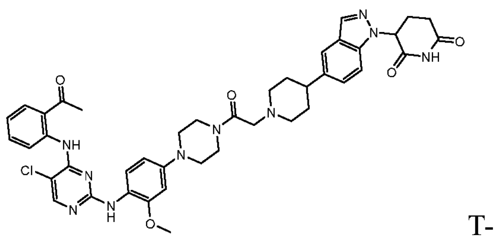 T-106 |
| 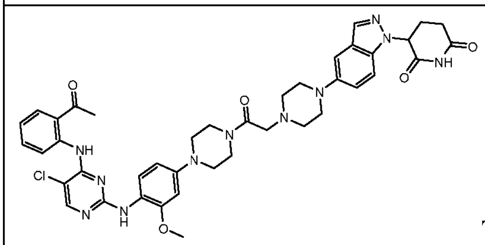 T-107 | 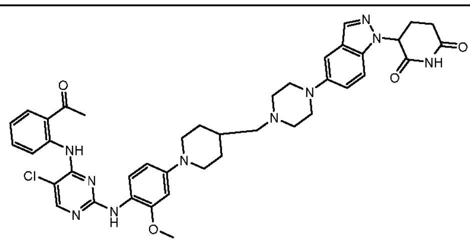 T-108 |
| 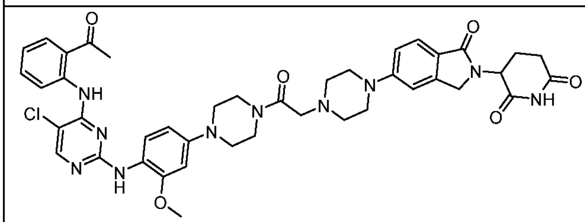 T-109 | 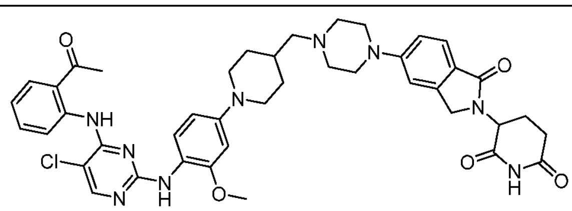 T-110 |
| 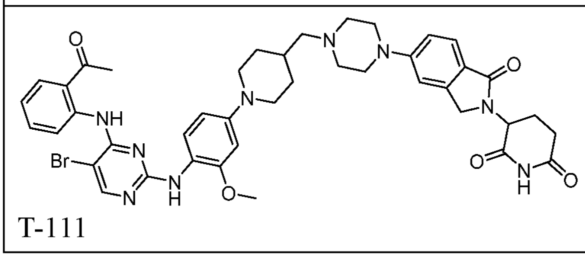 T-111 | T-112 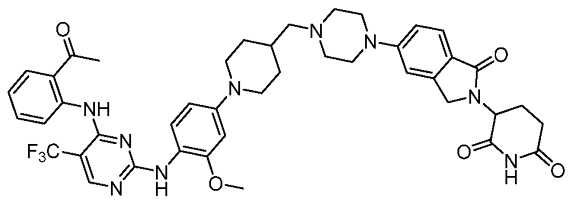 |

| | |
|---|---|
| 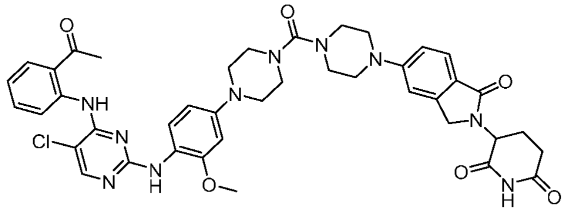 T-113 | 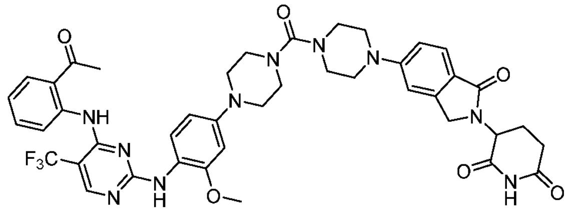 T-114 |
| 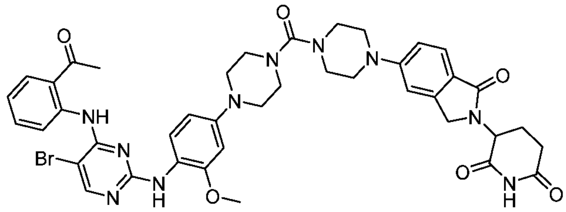 T-115 | 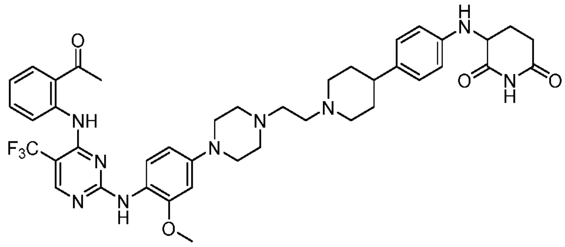 T-116 |
| 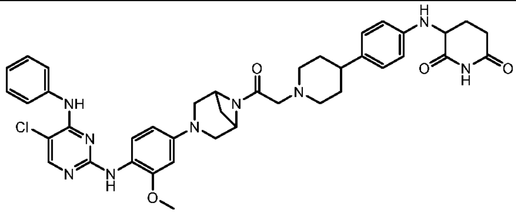 T-117 | 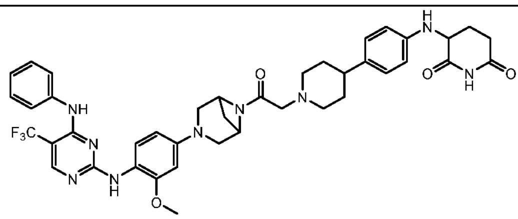 T-118 |
| 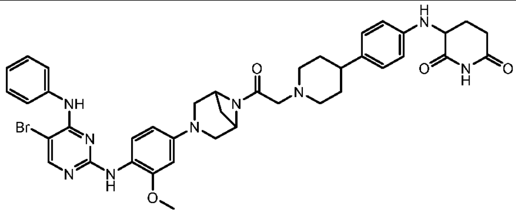 T-119 | 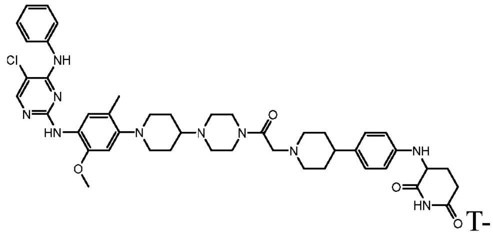 T-120 |
| 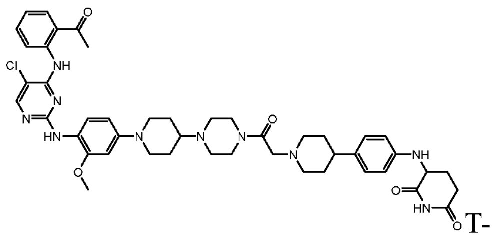 T-121 | 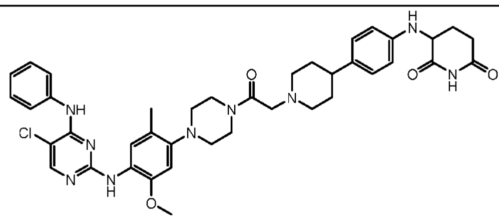 T-122 |
| 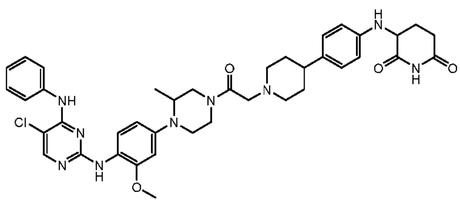 T-123 | 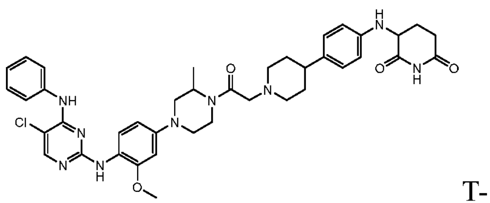 T-124 |
| 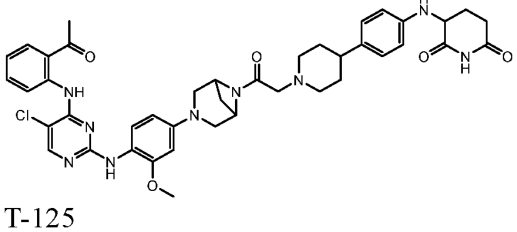 T-125 | 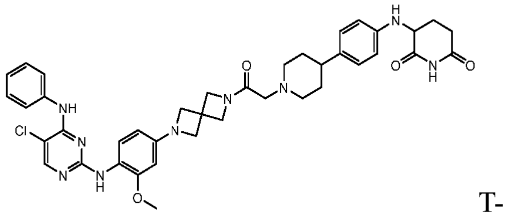 T-126 |

| | |
|---|---|
| 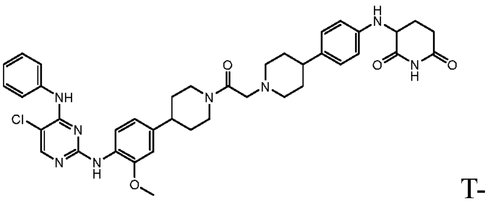 T-127 | 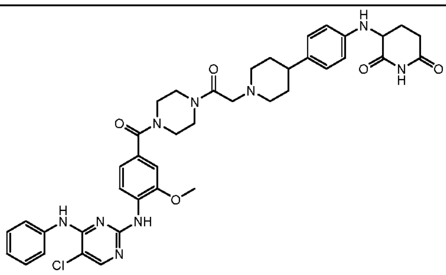 T-128 |
| 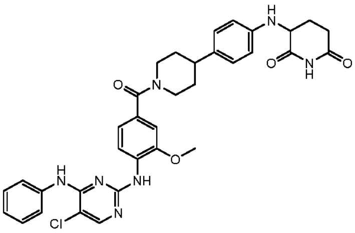 T-129 | 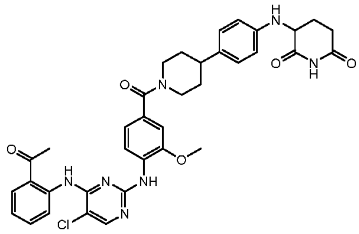 T-130 |
| 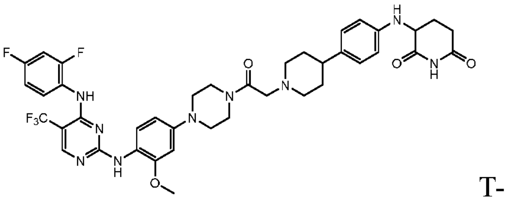 T-131 | 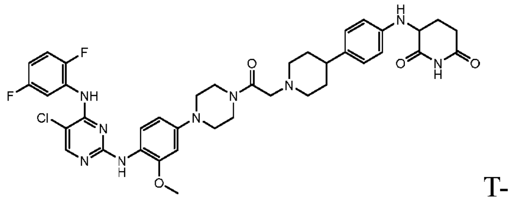 T-132 |
| 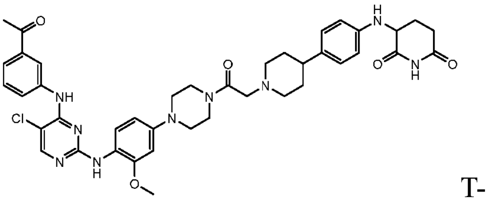 T-133 | 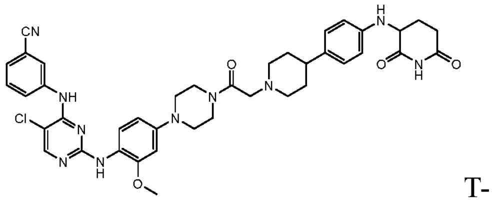 T-134 |
| 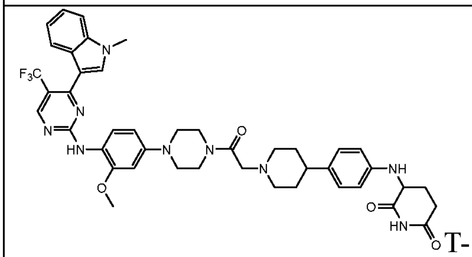 T-135 | 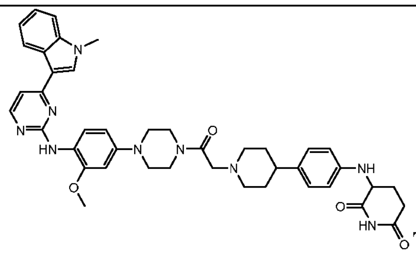 T-136 |
| 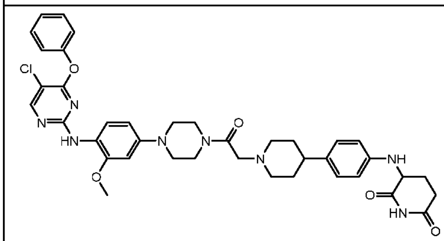 T-137 | 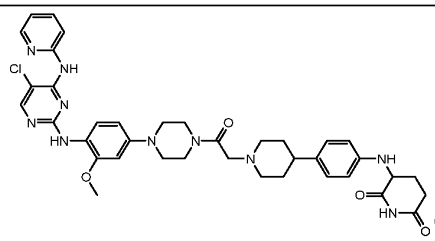 T-138 |
| 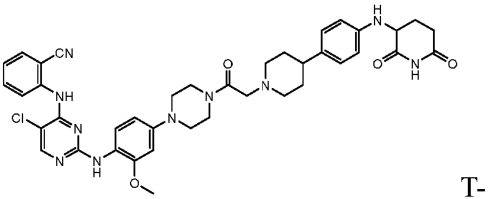 T-139 | 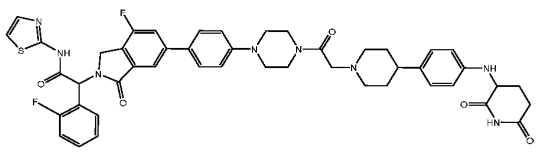 T-140 |

| | |
|---|---|
| 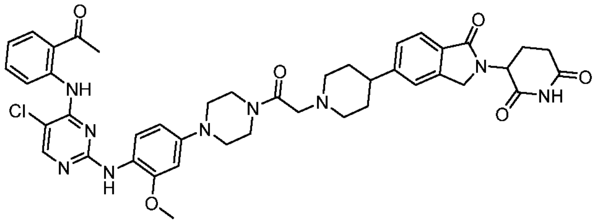 T-141 | 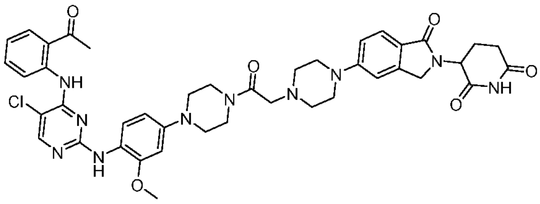 T-142 |
| 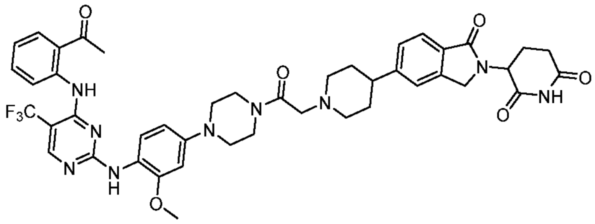 T-143 | 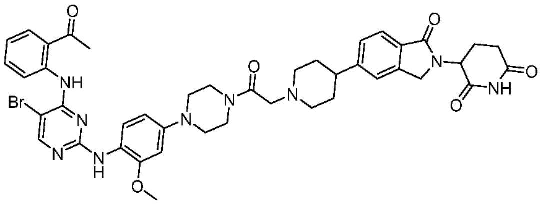 T-144 |
| 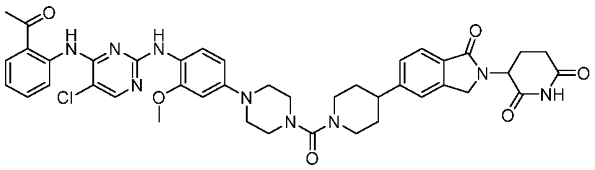 T-145 | 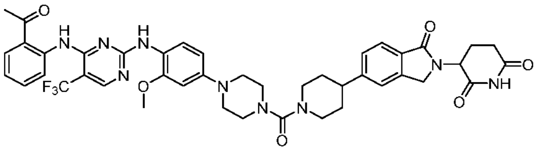 T-146 |
| 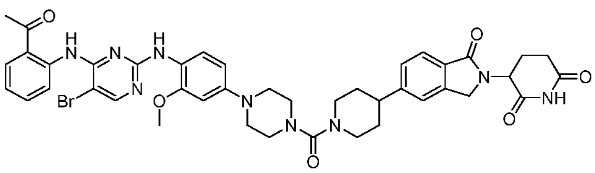 T-147 | 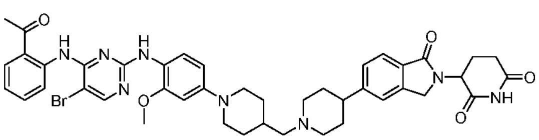 T-148 |
| 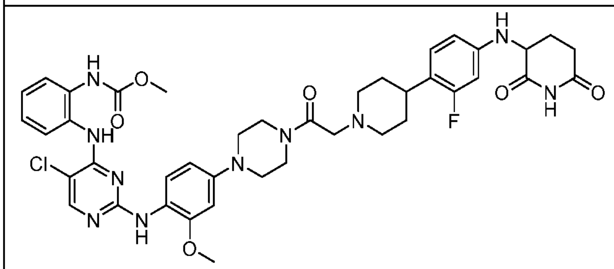 T-149 | 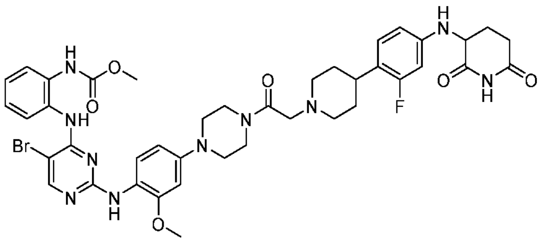 T-150 |
| 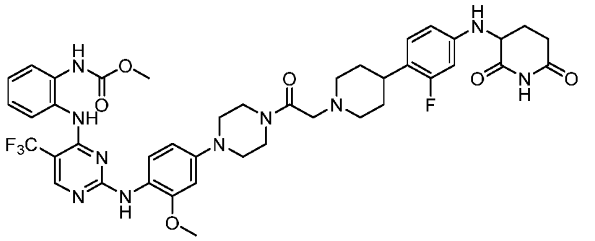 T-151 | 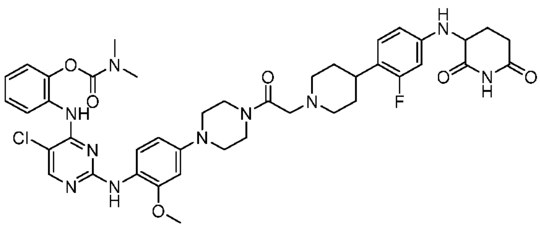 T-152 |

| | |
|---|---|
| 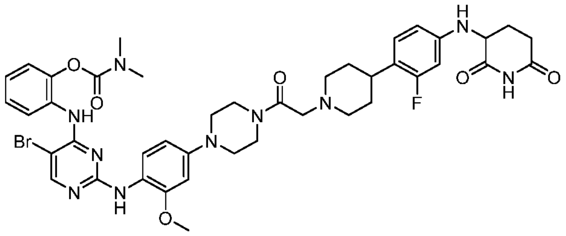 T-153 | 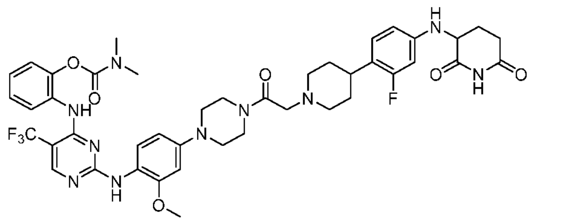 T-154 |
| 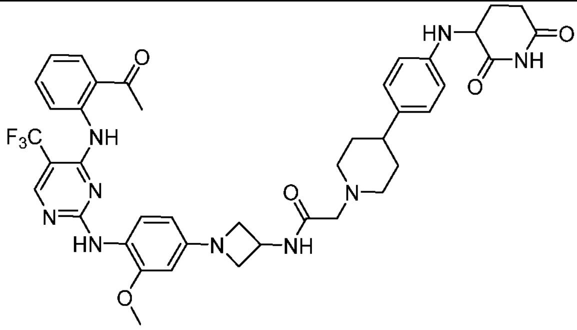 T-155 | 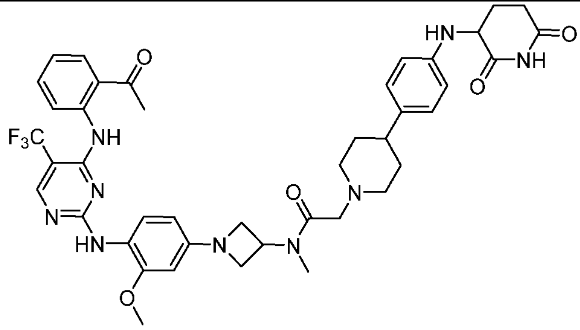 T-156 |
| 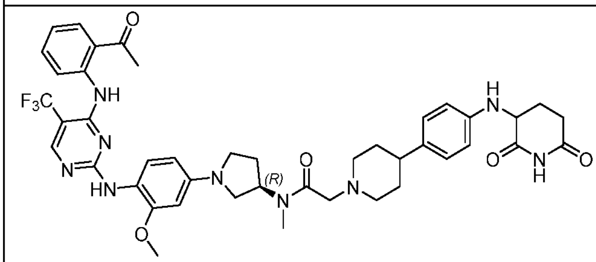 T-157 | 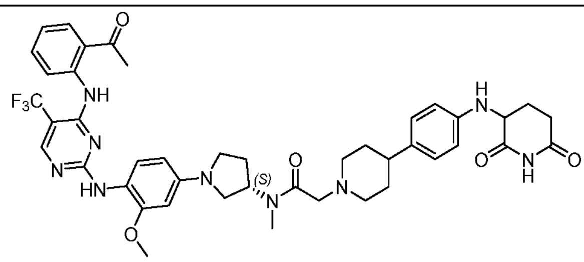 T-158 |
| 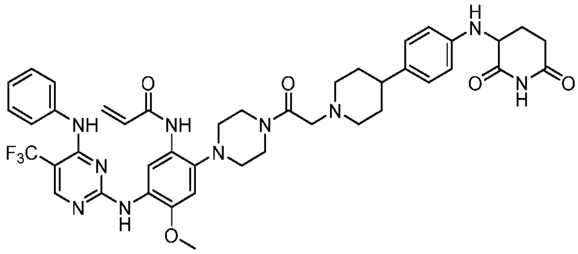 T-159 | 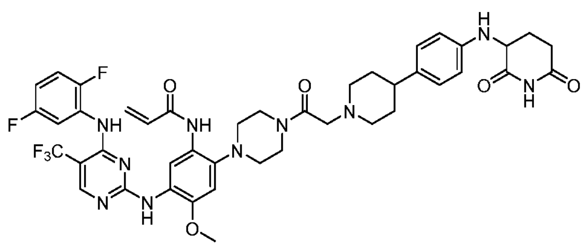 T-160 |
| 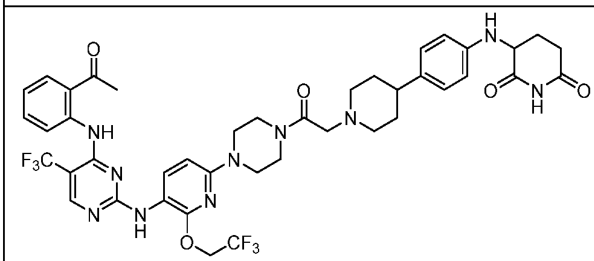 T-161 | 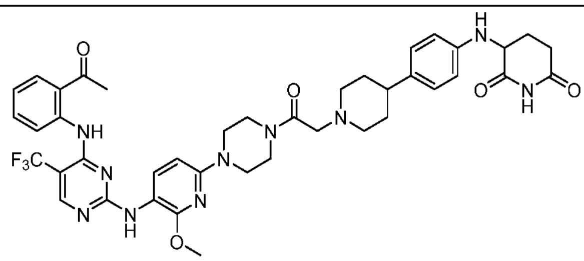 T-162 |
| 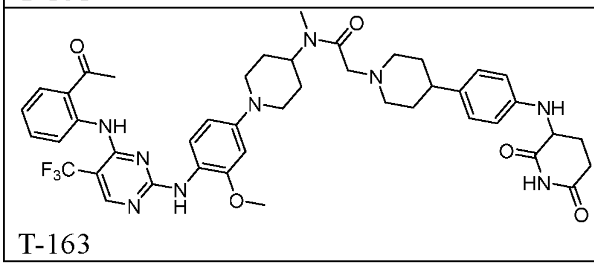 T-163 | 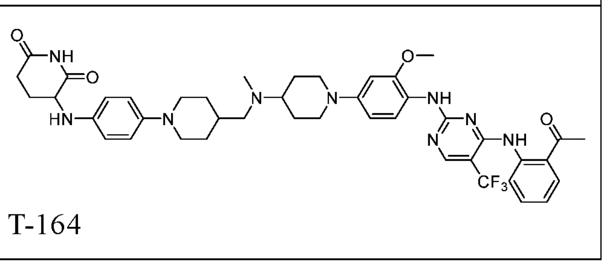 T-164 |

| | |
|---|---|
| 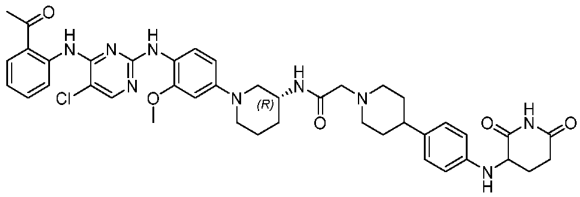 T-165 | 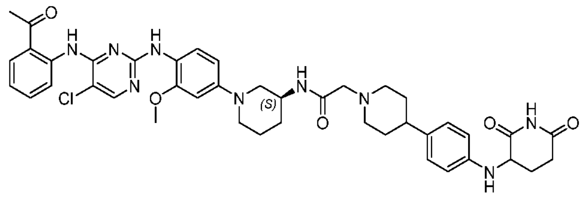 T-166 |
| 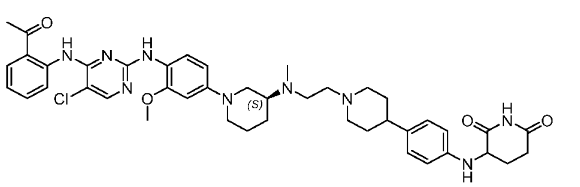 T-167 | 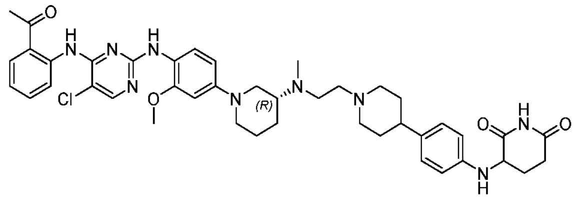 T-168 |
| 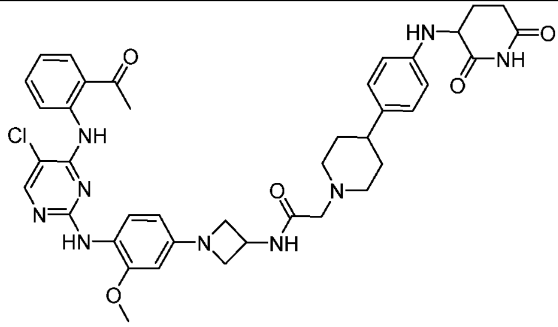 T-169 | 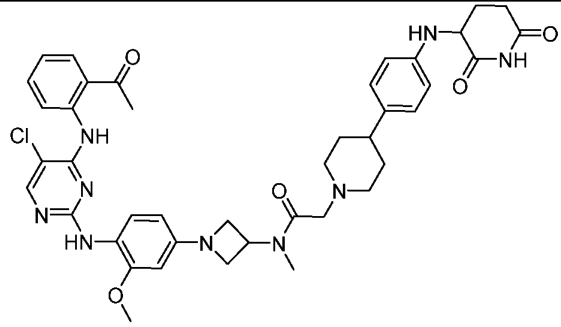 T-170 |
| 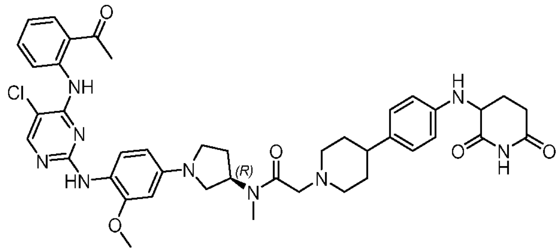 T-171 | 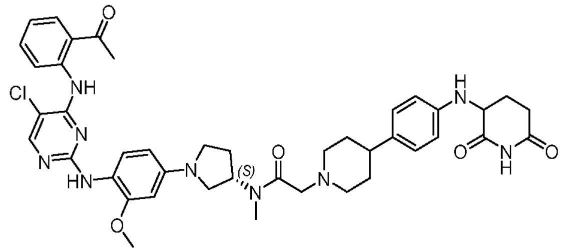 T-172 |
| 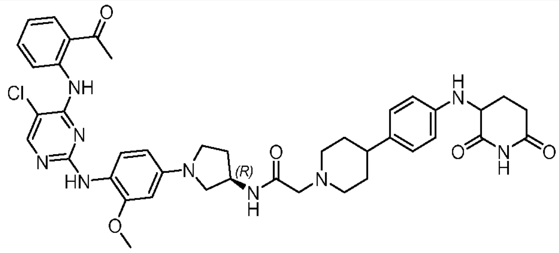 T-173 | 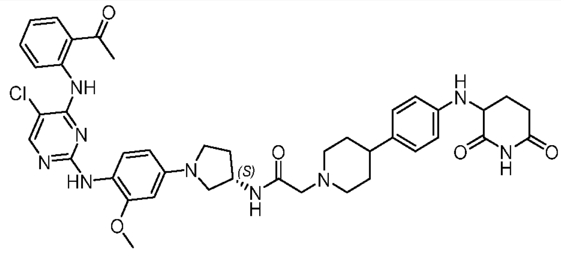 T-174 |
| 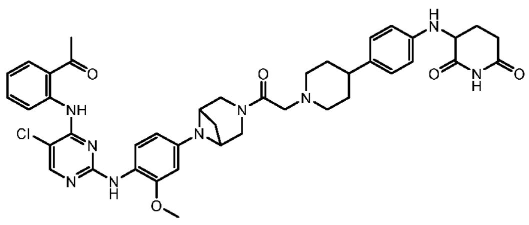 T-175 | 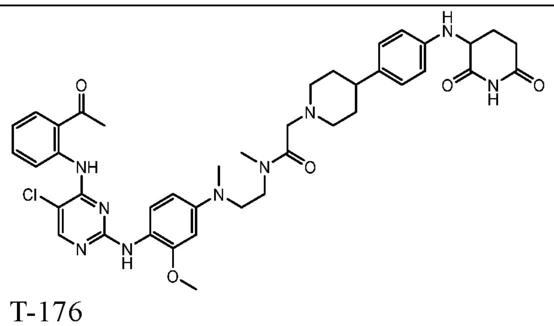 T-176 |

| | |
|---|---|
| 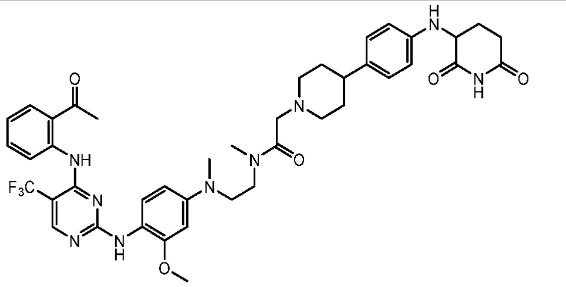 T-177 | 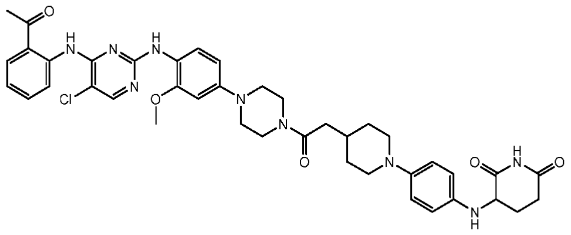 T-178 |
| 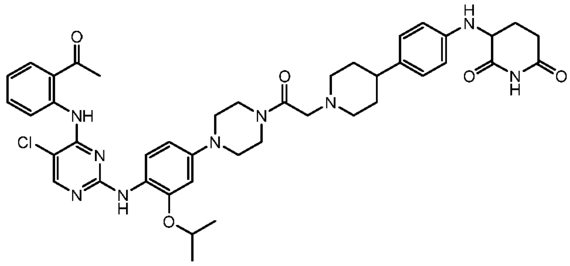 T-179 | 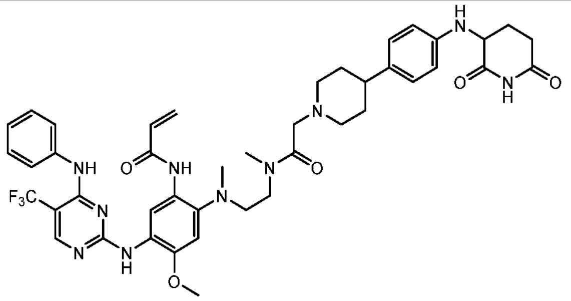 T-180 |
| 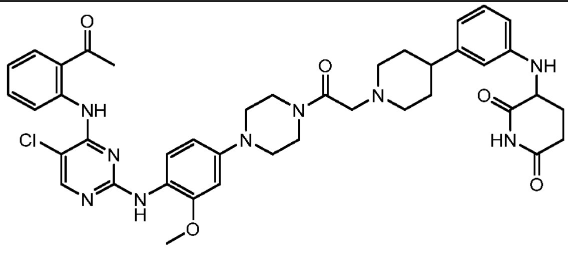 T-181 | 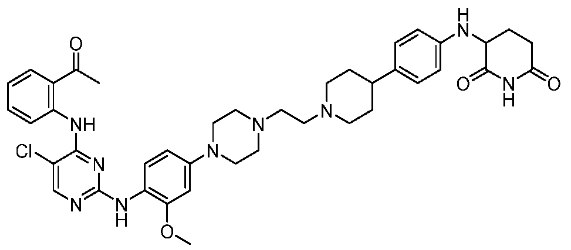 T-182 |
| 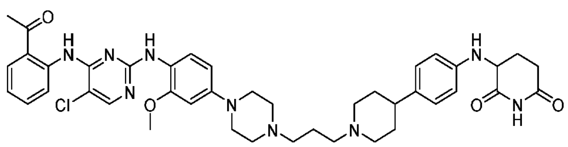 T-183 | 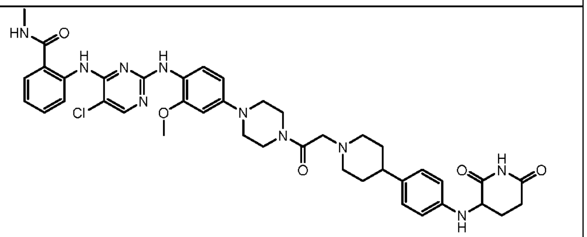 T-184 |
| 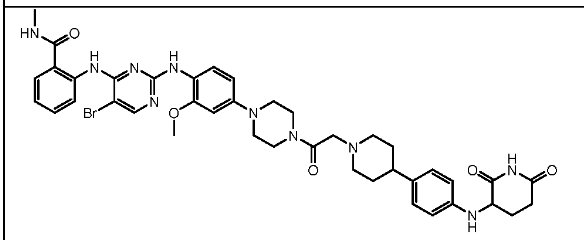 T-185 | 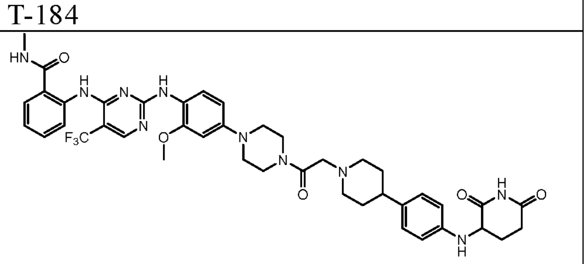 T-186 |
| 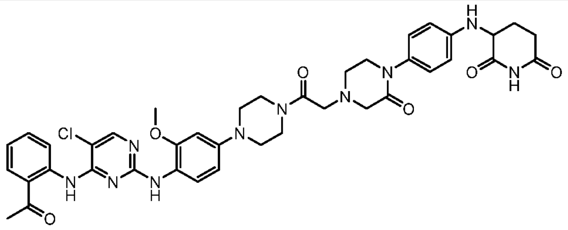 T-187 | 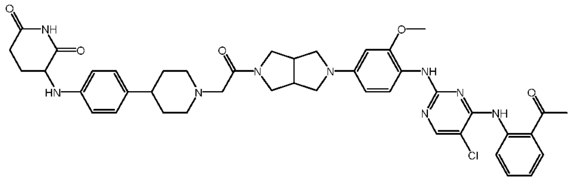 T-188 |

| | |
|---|---|
| 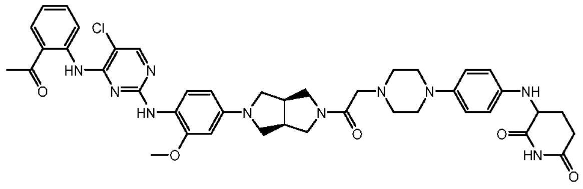 T-189 | 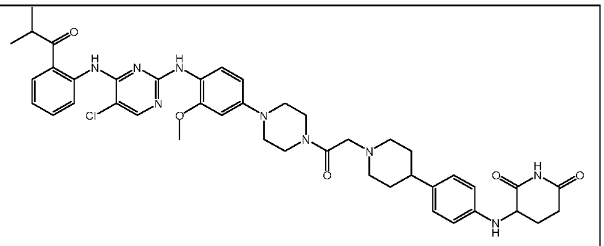 T-190 |
| 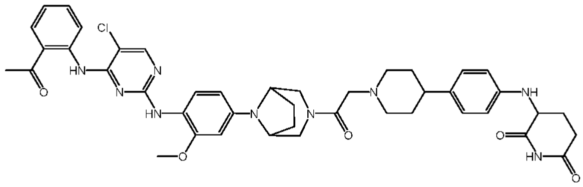 T-191 | 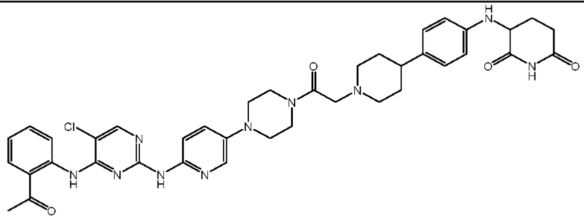 T-192 |
| 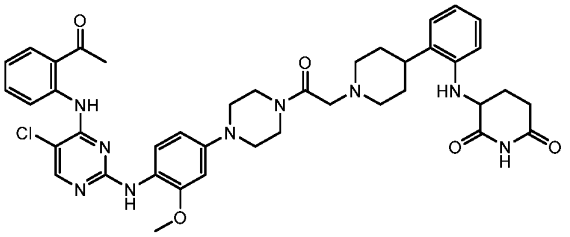 T-193 | 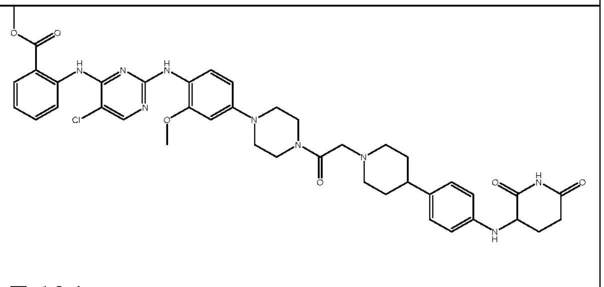 T-194 |
| 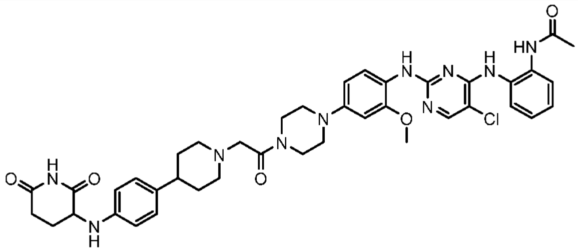 T-195 | 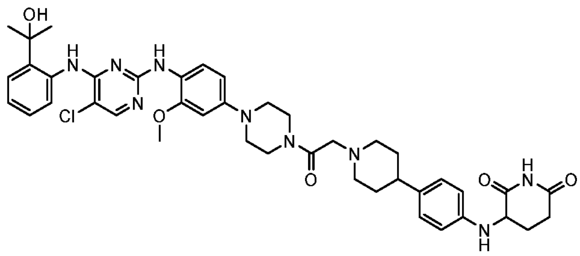 T-196 |
| 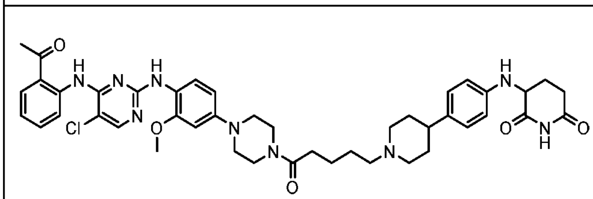 T-197 | 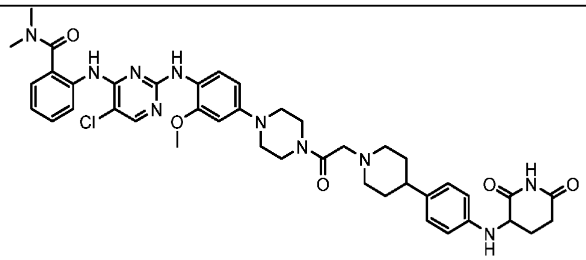 T-198 |
| 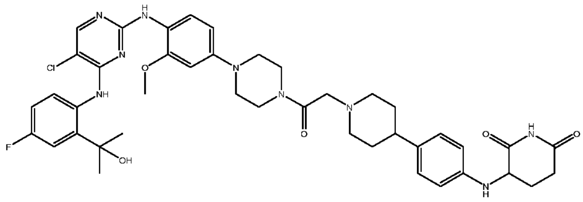 T-199 | 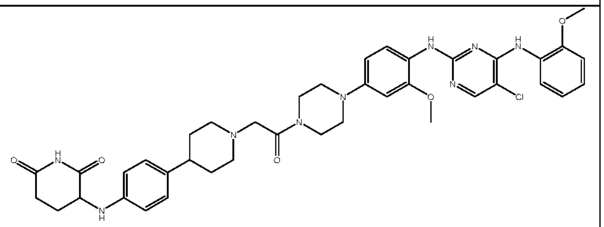 T-200 |

| | |
|---|---|
| 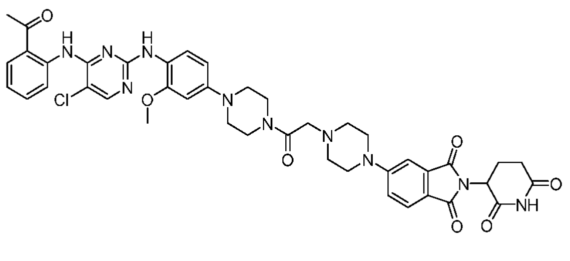<br>T-201 | 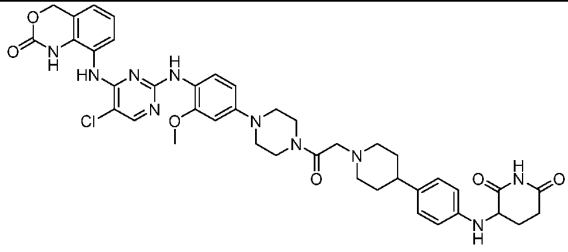<br>T-202 |
| 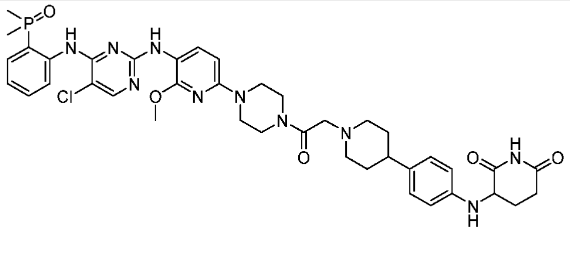<br>T-203 | 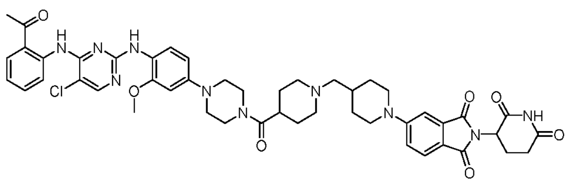<br>T-204 |
| 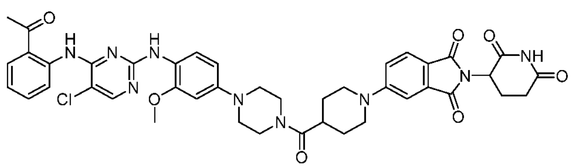<br>T-205 | 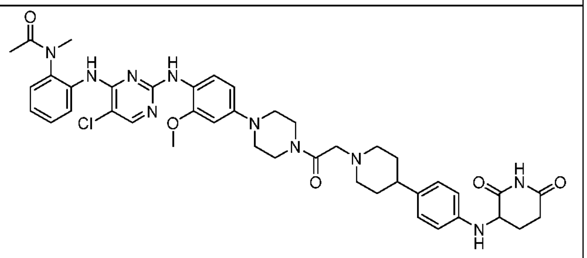<br>T-206 |
| 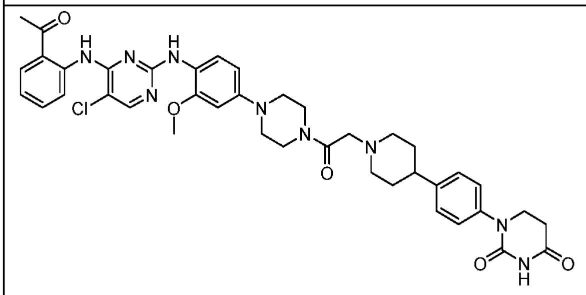<br>T-207 | 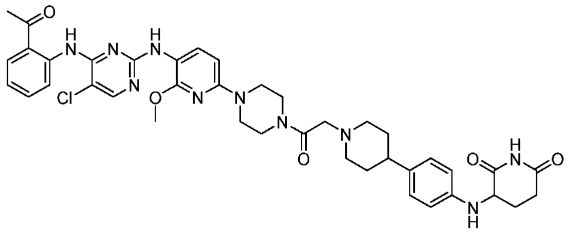<br>T-208 |
| 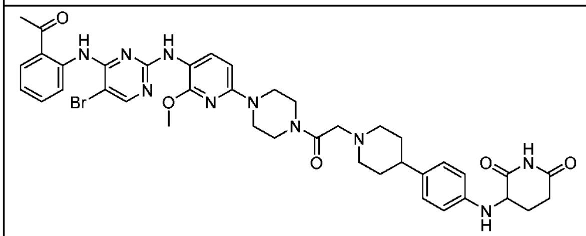<br>T-209 | 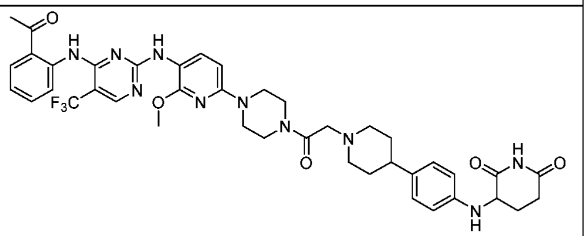<br>T-210 |
| 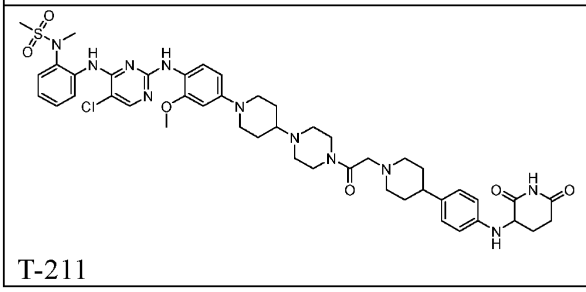<br>T-211 | 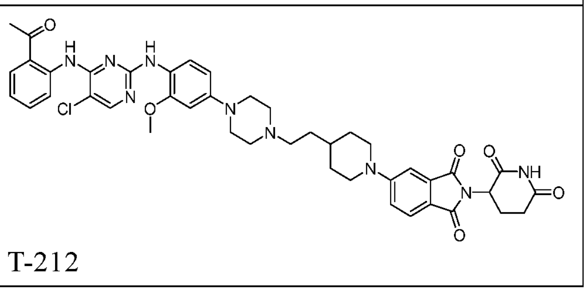<br>T-212 |

| | |
|---|---|
| 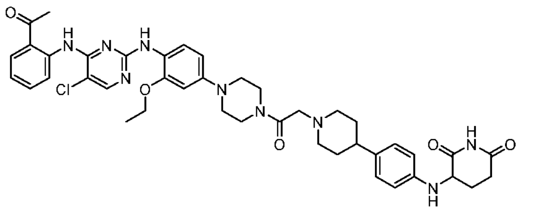 T-213 | 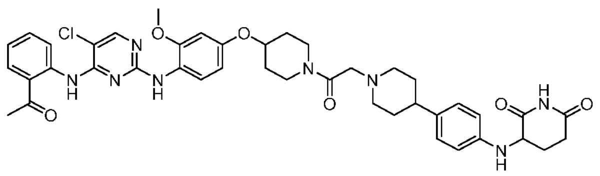 T-214 |
| 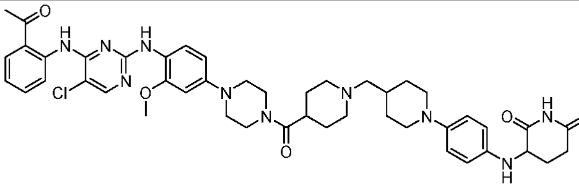 T-215 | 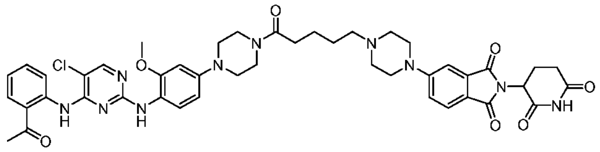 T-216 |
| 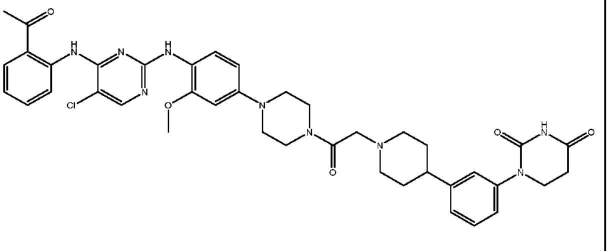 T-217 | 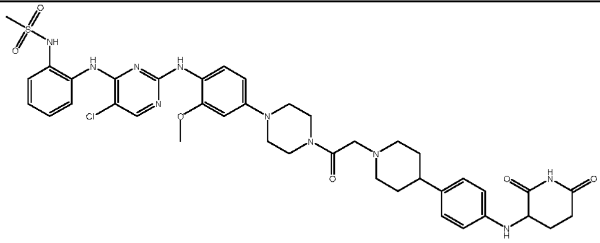 T-218 |
| 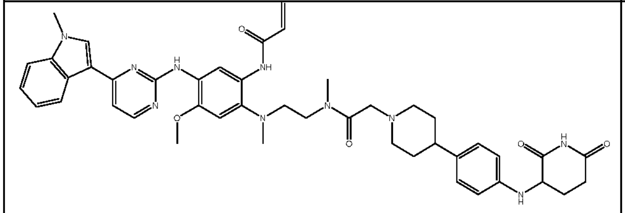 T-219 | 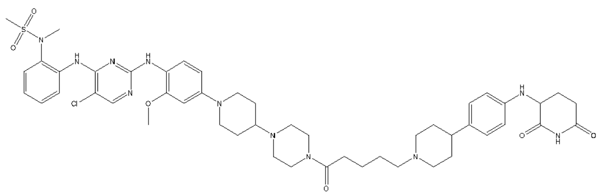 T-220 |
| 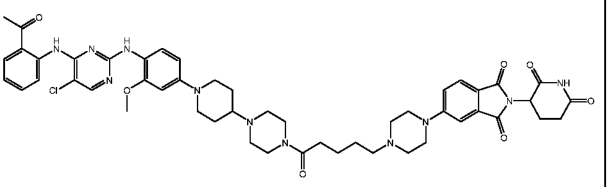 T-221 | 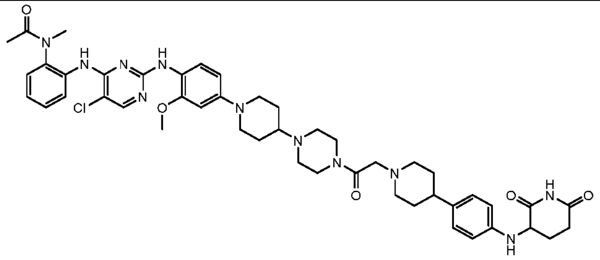 T-222 |
| 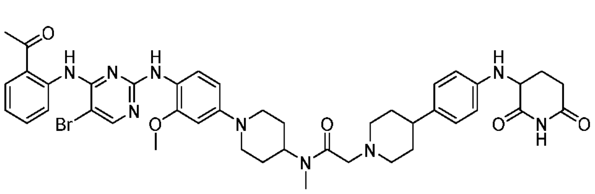 T-223 | 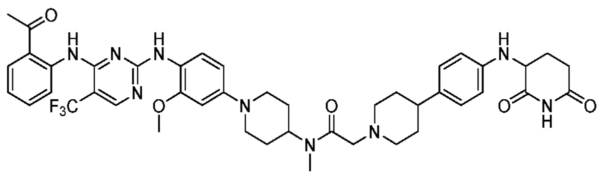 T-224 |

| | |
|---|---|
| 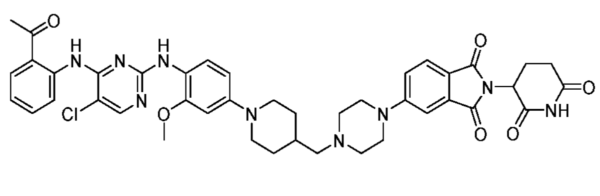 T-225 | 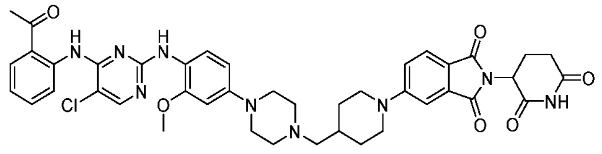 T-226 |
| 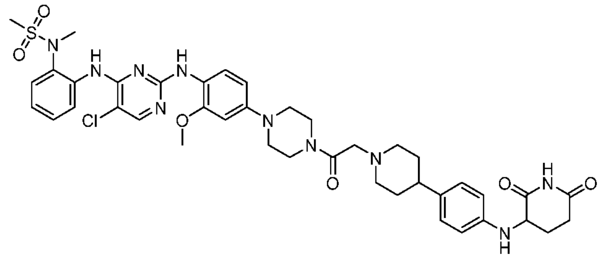 T-227 | 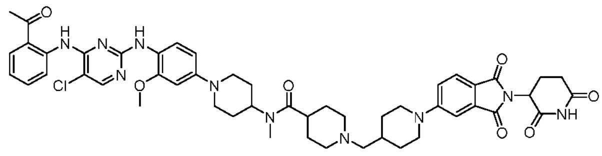 T-228 |
| 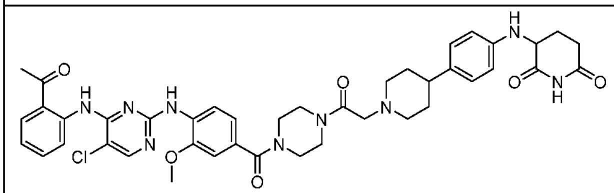 T-229 | 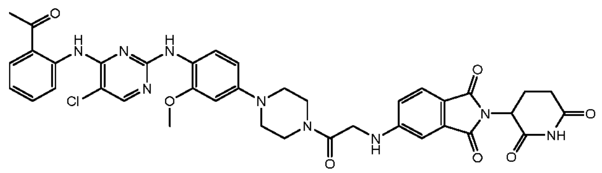 T-230 |
| 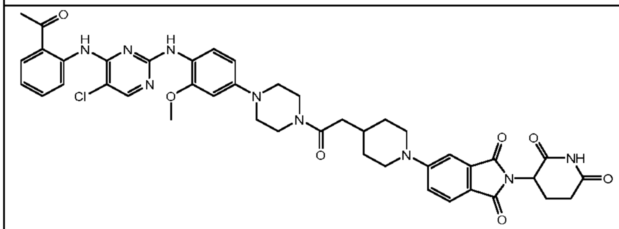 T-231 | 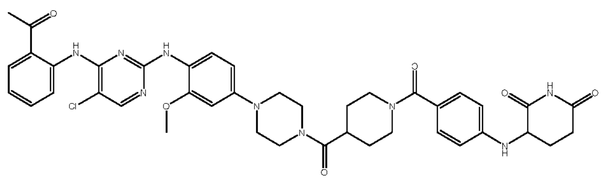 T-232 |
| 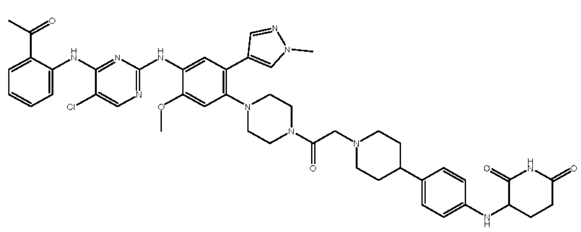 T-233 | 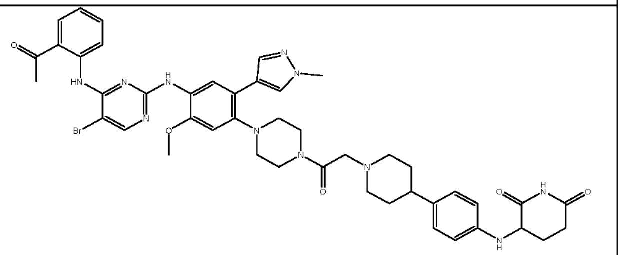 T-234 |
| 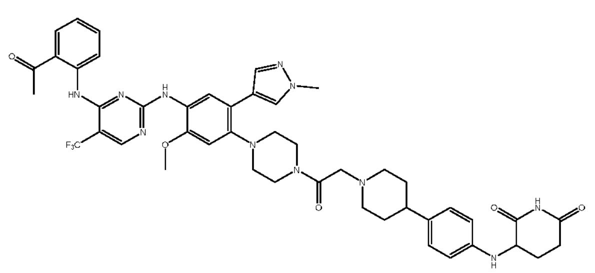 T-235 | 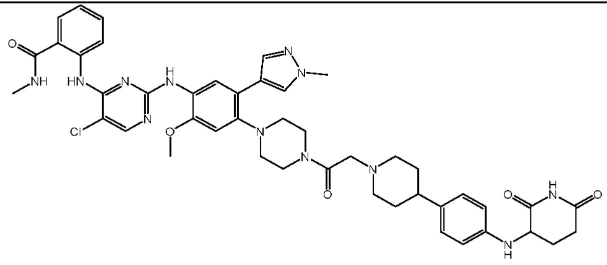 T-236 |

| | |
|---|---|
| 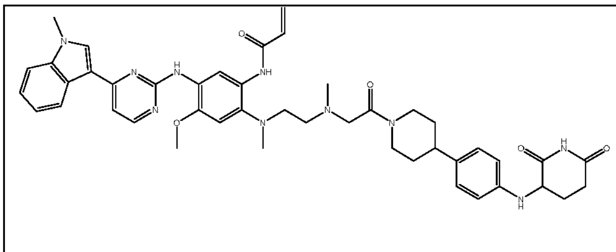<br>T-237 | 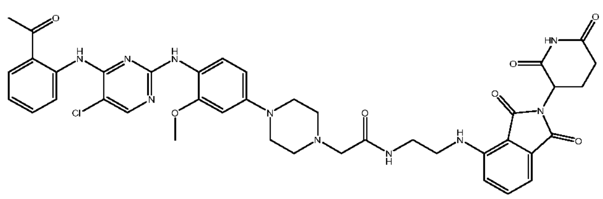<br>T-238 |
| 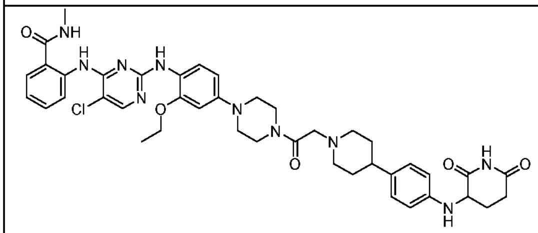<br>T-239 | 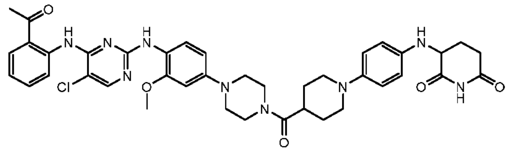<br>T-240 |
| 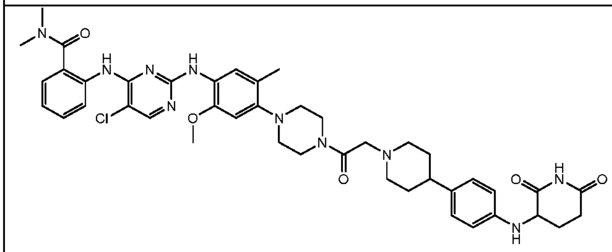<br>T-241 | 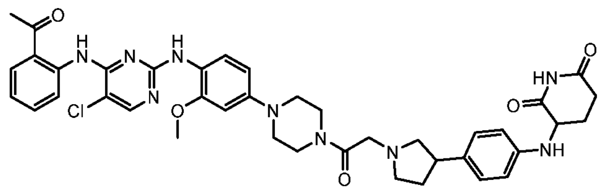<br>T-242 |
| 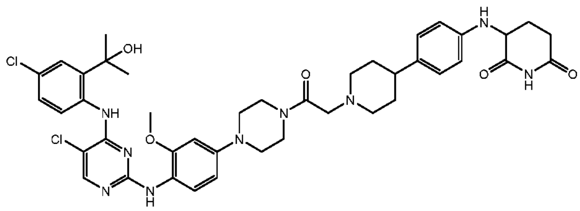<br>T-243 | 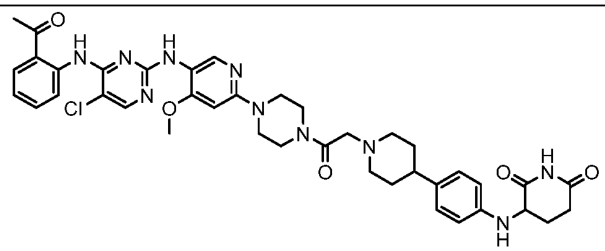<br>T-244 |
| 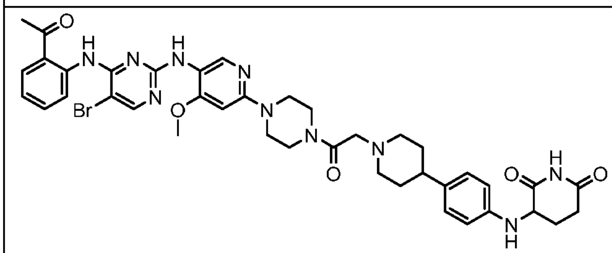<br>T-245 | 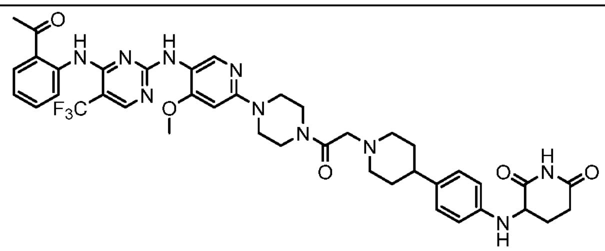<br>T-246 |
| 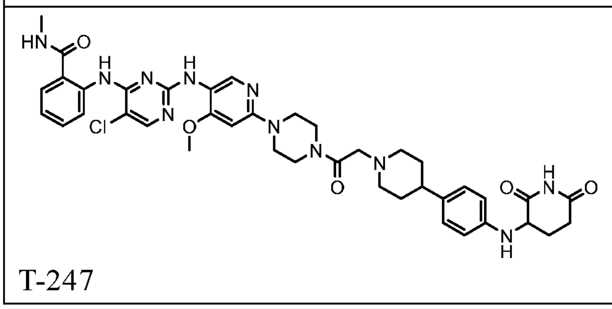<br>T-247 | 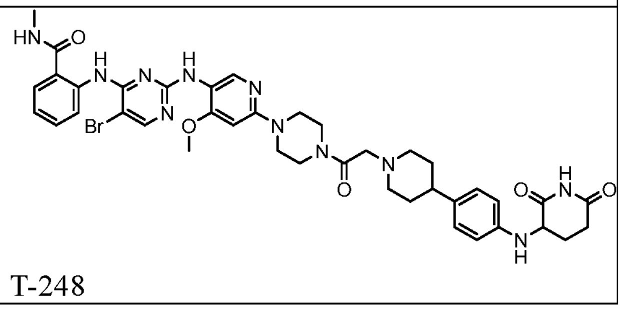<br>T-248 |

| | |
|---|---|
| 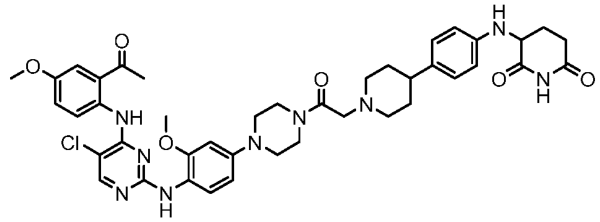 T-249 | 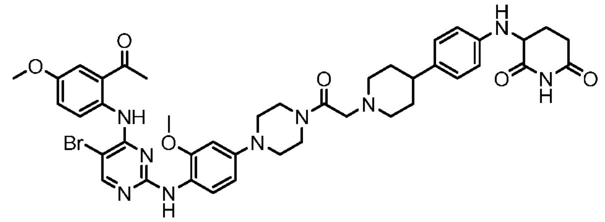 T-250 |
| 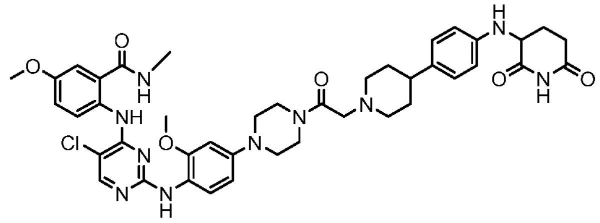 T-251 | 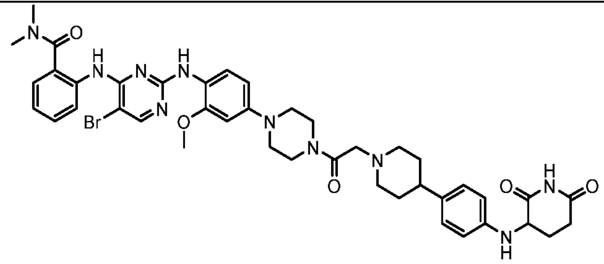 T-252 |
| 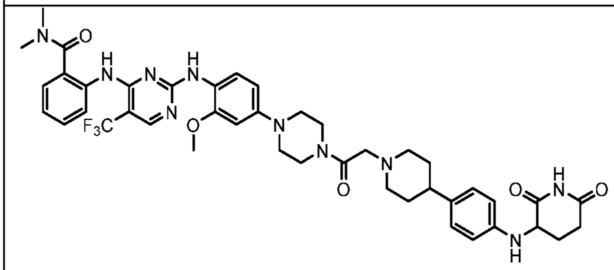 T-253 | 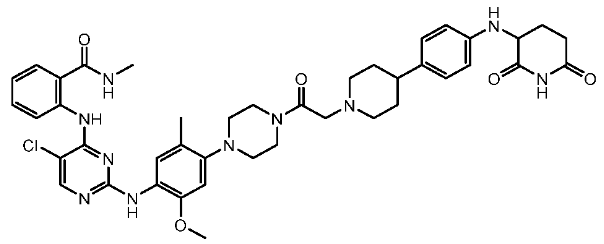 T-254 |
| 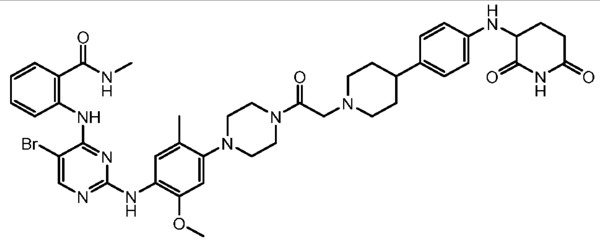 T-255 | 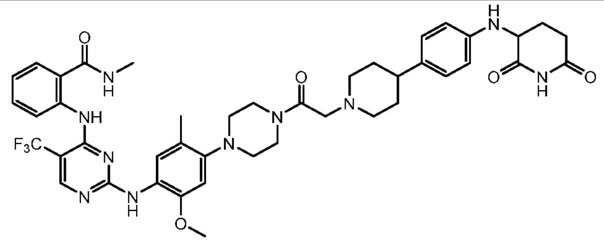 T-256 |
| 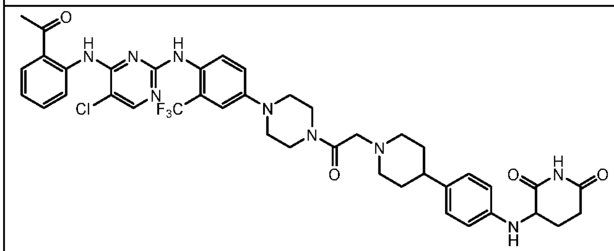 T-257 | 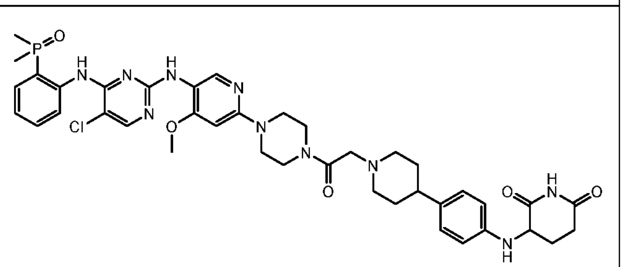 T-258 |
| 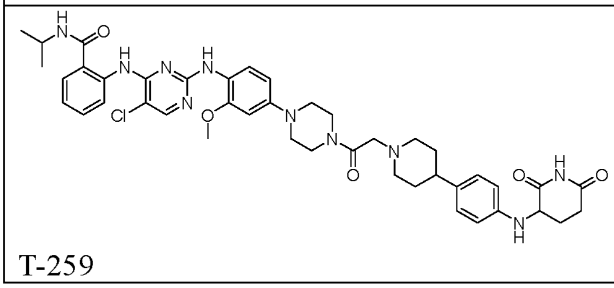 T-259 | 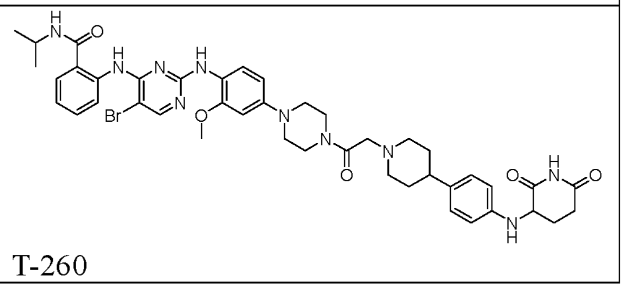 T-260 |

| | |
|---|---|
| 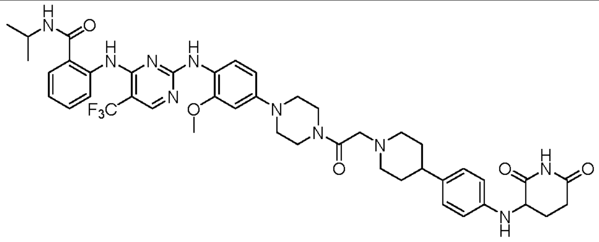 T-261 | 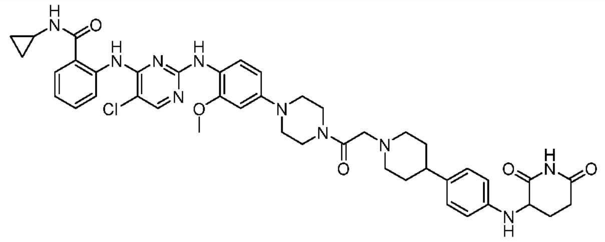 T-262 |
| 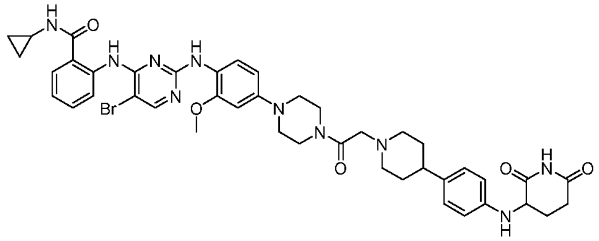 T-263 | 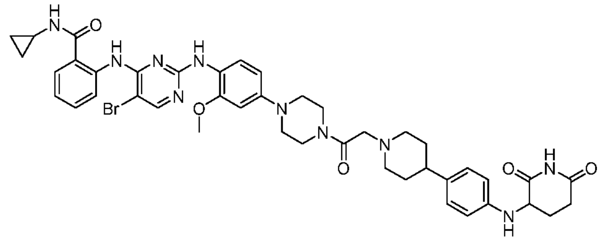 T-264 |
| 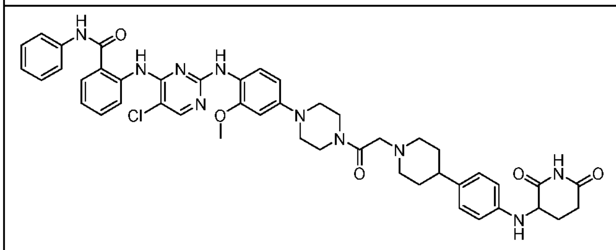 T-265 | 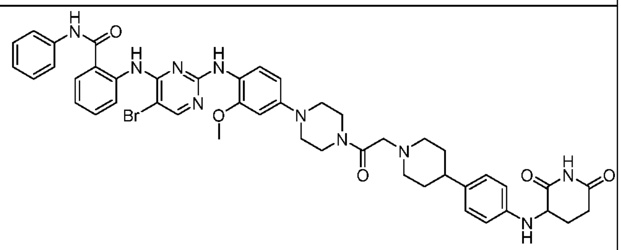 T-266 |
| 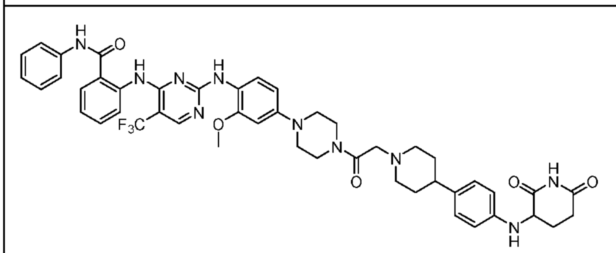 T-267 | 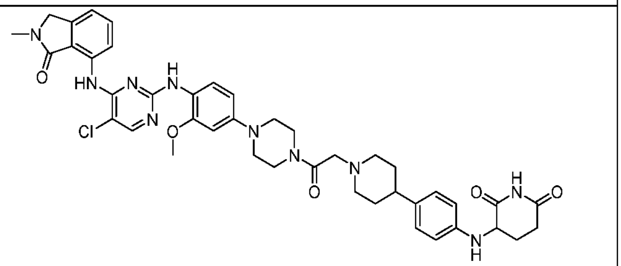 T-268 |
| 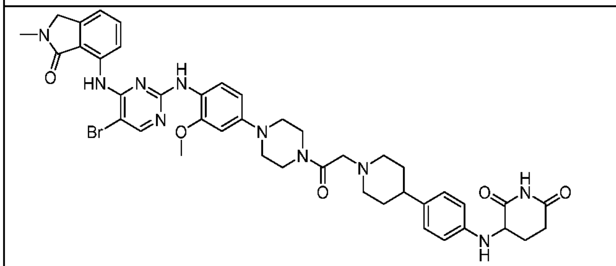 T-269 | 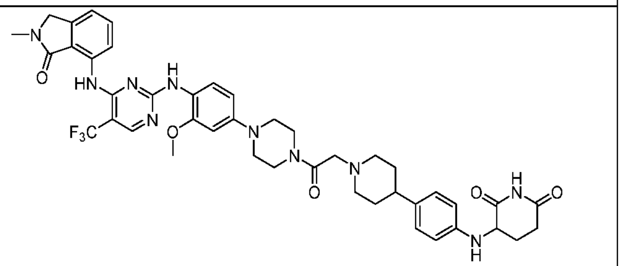 T-270 |
| 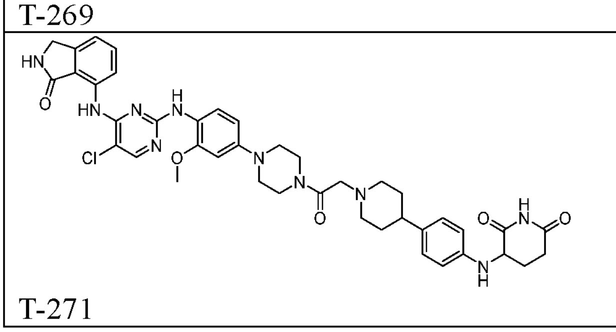 T-271 | 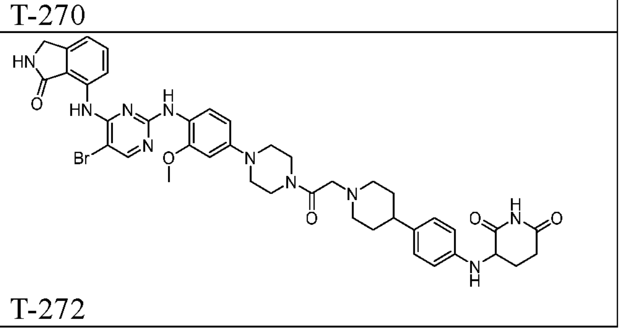 T-272 |

| | |
|---|---|
| 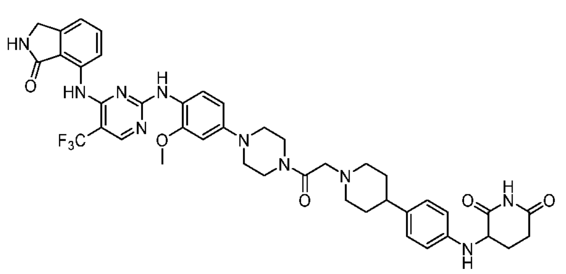 T-273 | 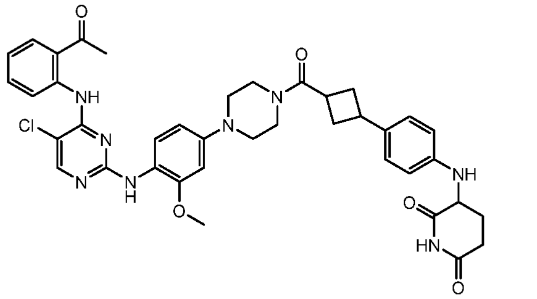 T-274 |
| 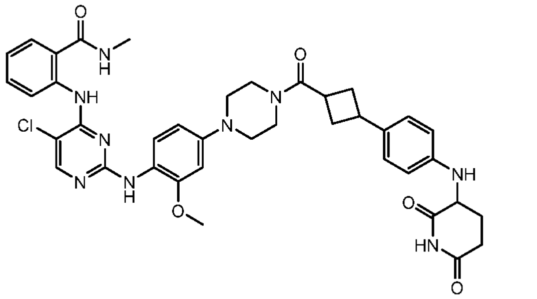 T-275 | 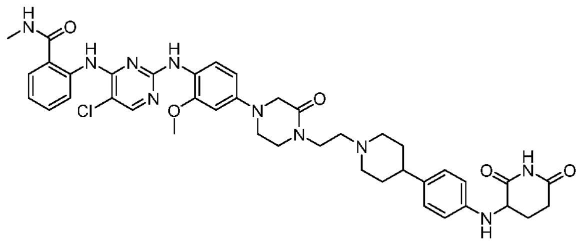 T-276 |
| 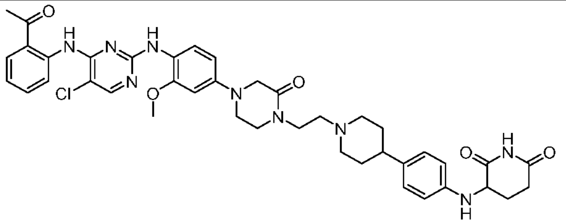 T-277 | 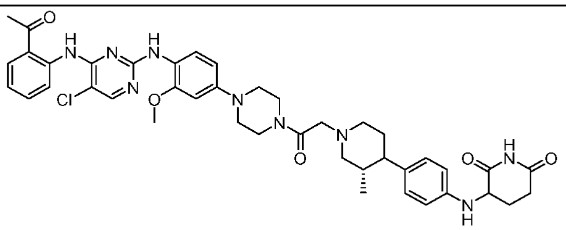 T-278 |
| 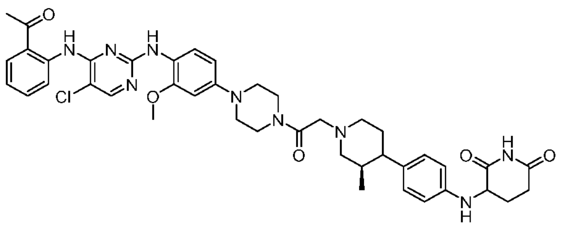 T-279 | 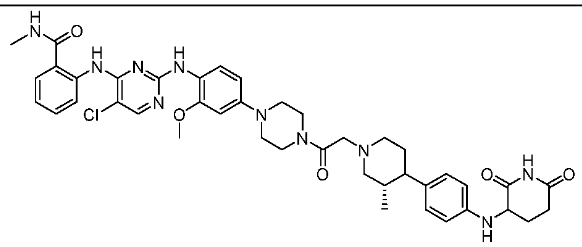 T-280 |
| 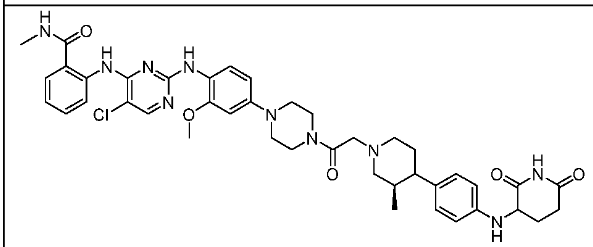 T-281 | 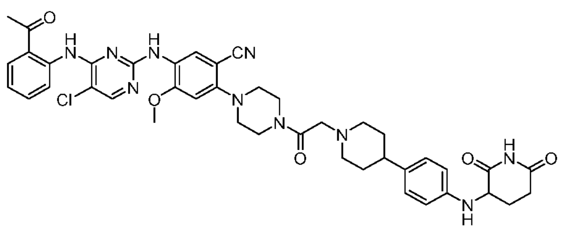 T-282 |
| 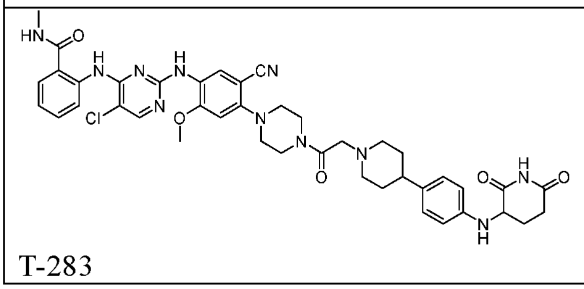 T-283 | 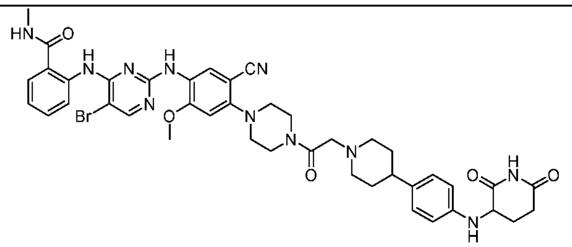 T-284 |

| | |
|---|---|
| 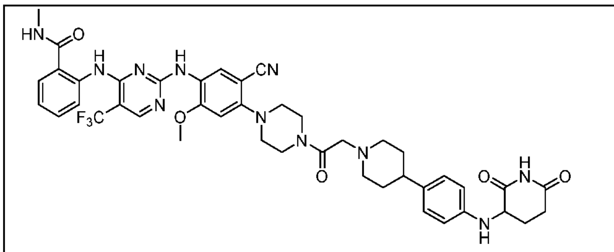 T-285 | 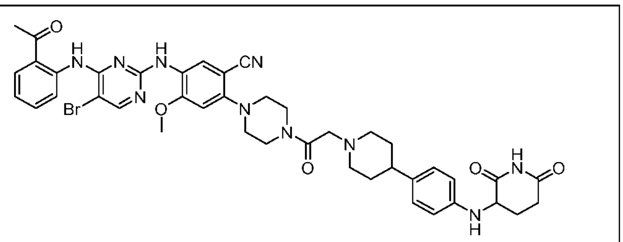 T-286 |
| 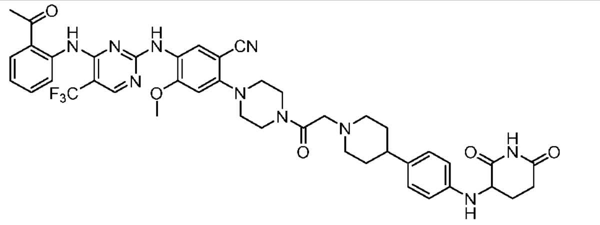 T-287 | 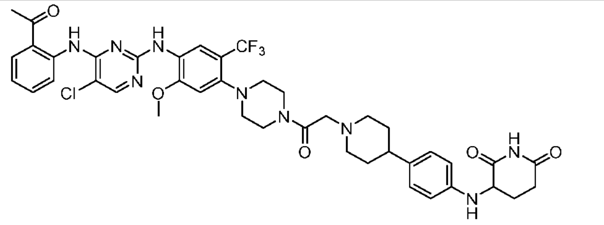 T-288 |
| 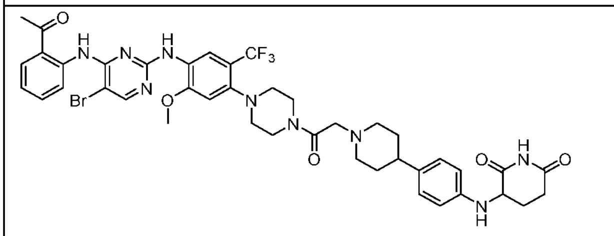 T-289 | 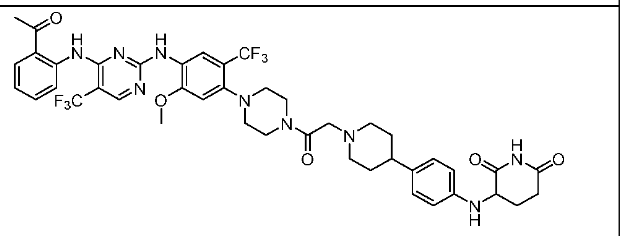 T-290 |
| 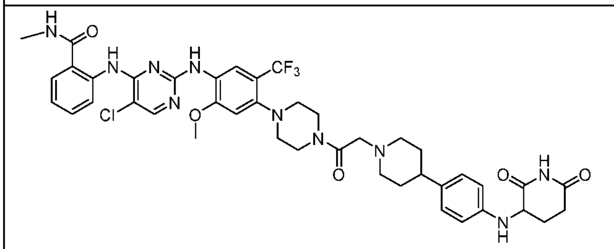 T-291 | 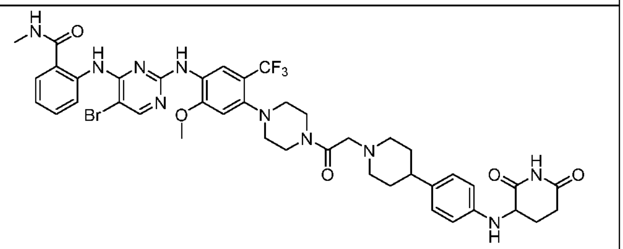 T-292 |
| 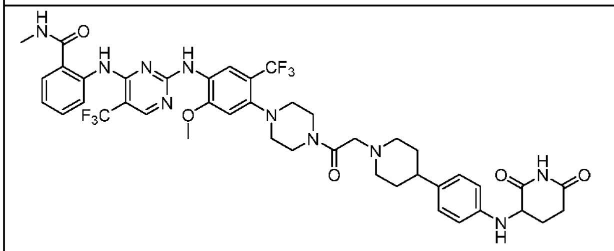 T-293 | 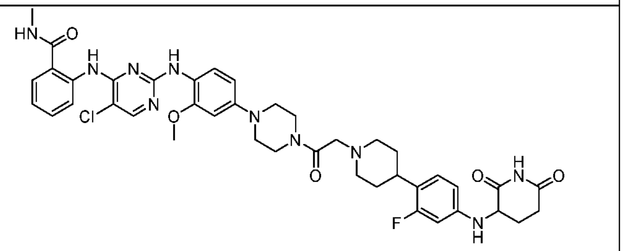 T-294 |
| 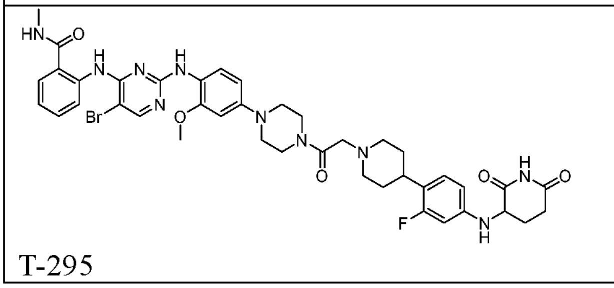 T-295 | 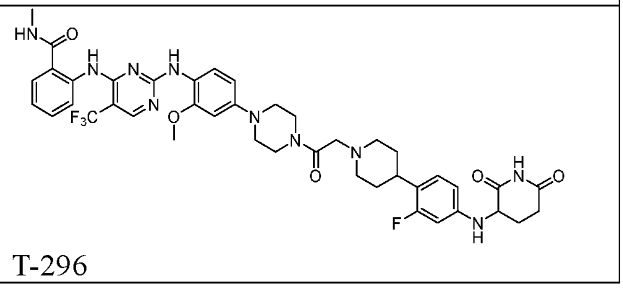 T-296 |

| | |
|---|---|
| 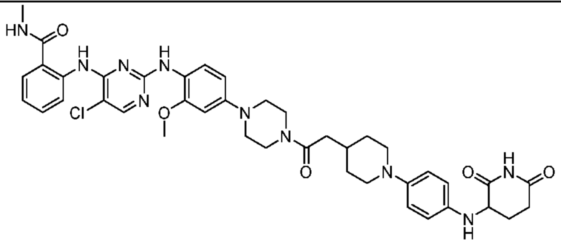 T-297 | 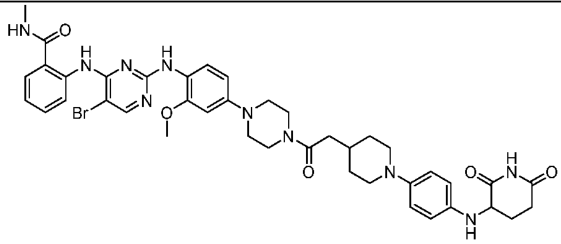 T-298 |
| 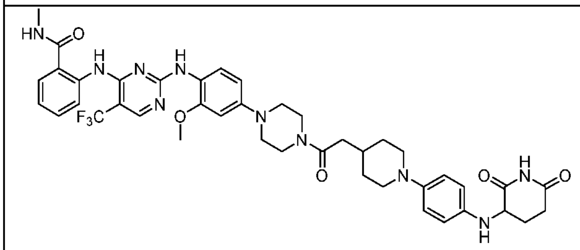 T-299 | 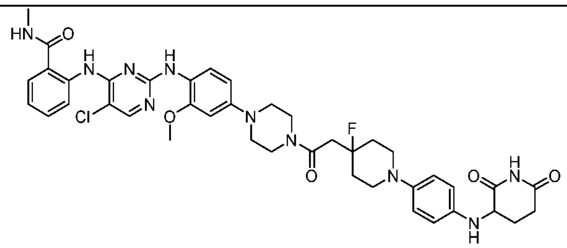 T-300 |
| 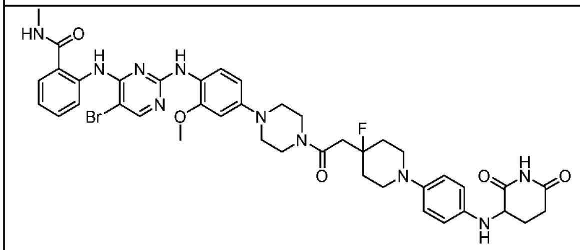 T-301 | 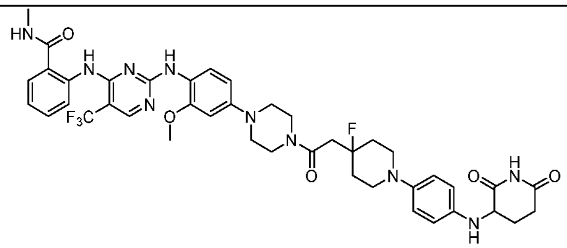 T-302 |
| 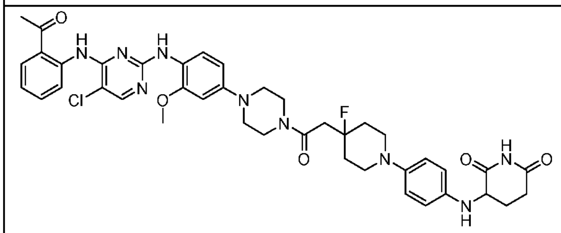 T-303 | 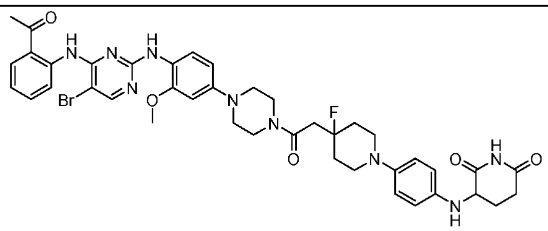 T-304 |
| 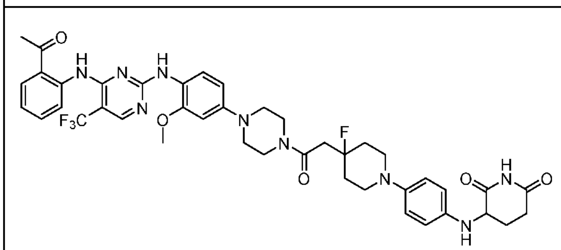 T-305 | 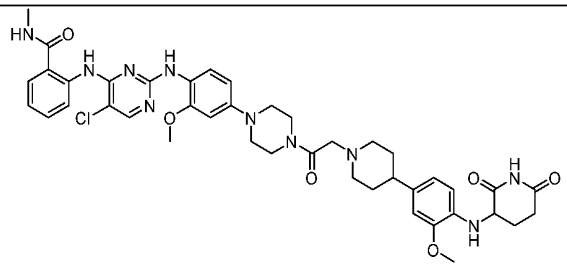 T-306 |
| 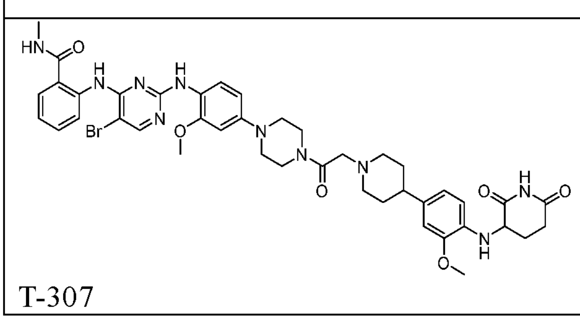 T-307 | 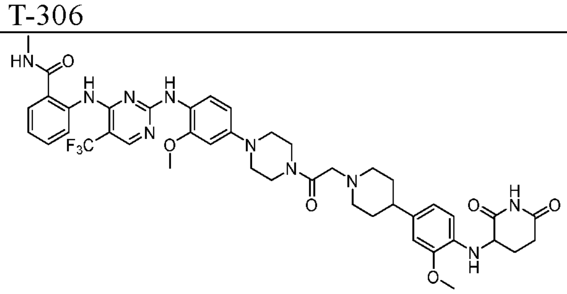 T-308 |

| | |
|---|---|
| 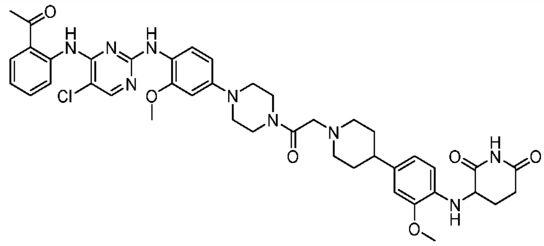 T-309 | 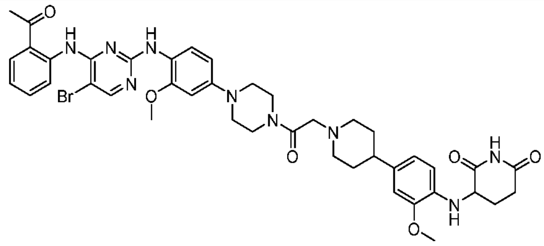 T-310 |
| 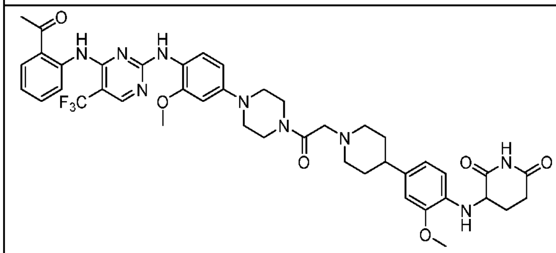 T-311 | 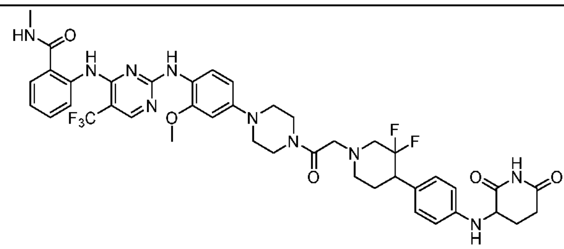 T-312 |
| 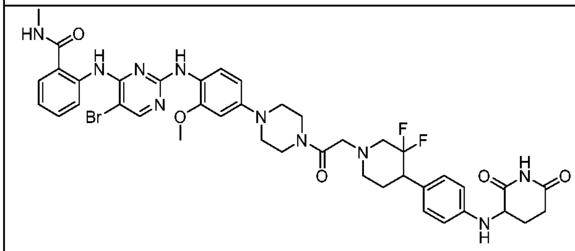 T-313 | 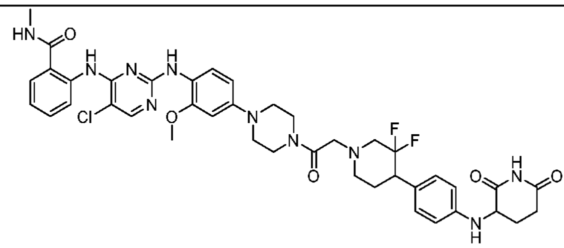 T-314 |
| 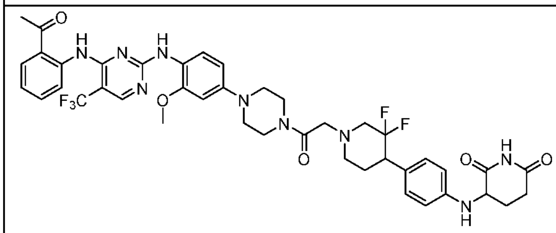 T-315 | 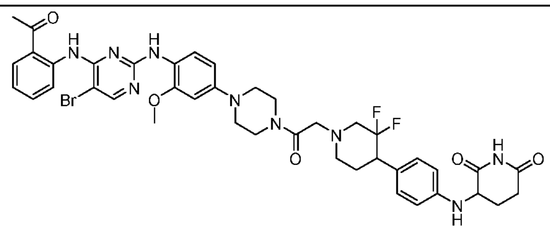 T-316 |
| 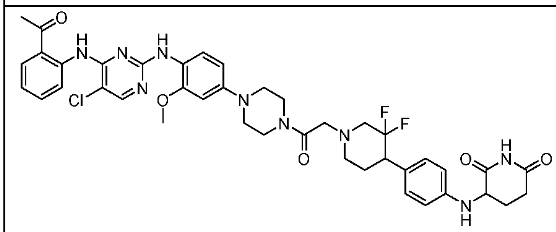 T-317 | 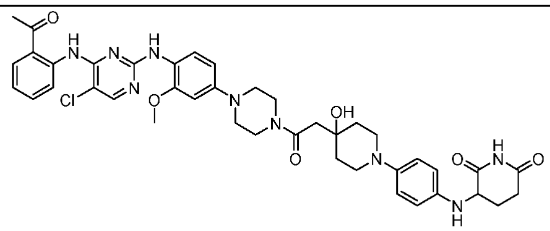 T-318 |
| 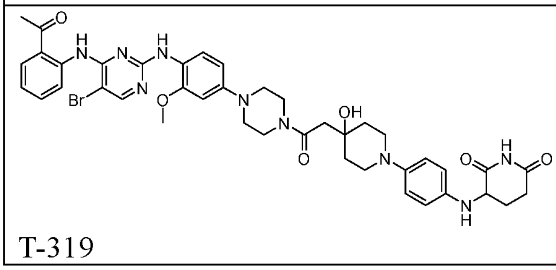 T-319 | 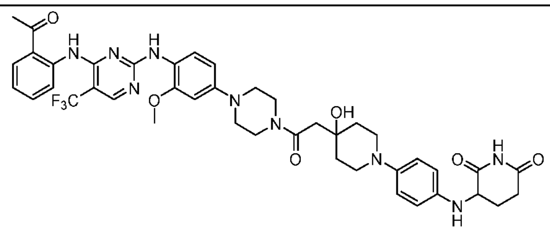 T-320 |

| | |
|---|---|
| 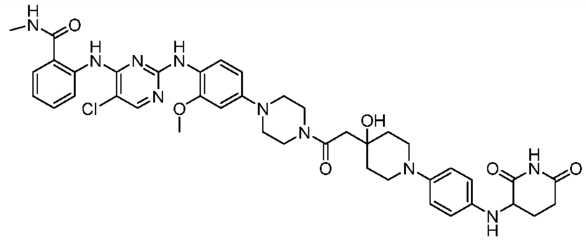 T-321 | 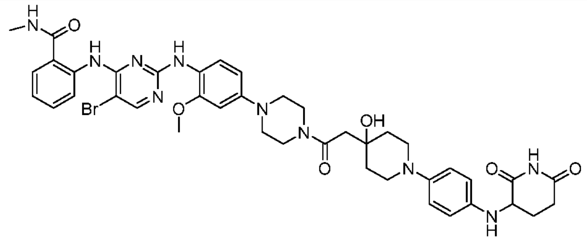 T-322 |
| 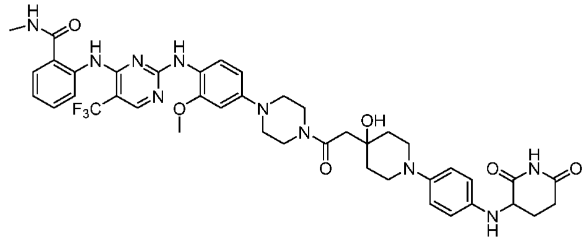 T-323 | 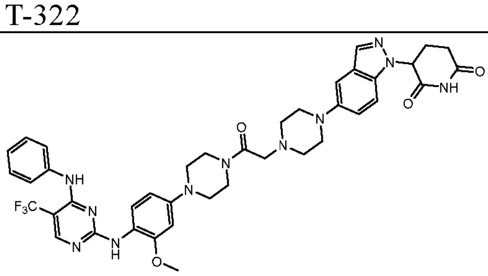 T-324 |
| 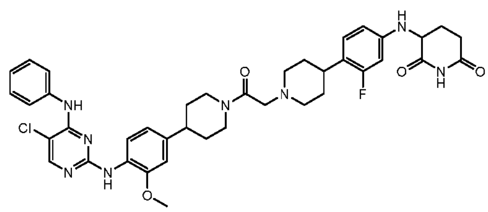 T-325 | 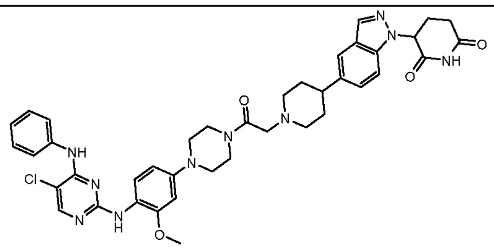 T-326 |
| 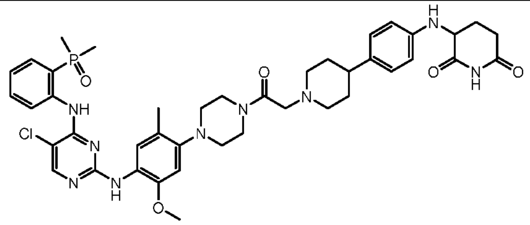 T-327 | 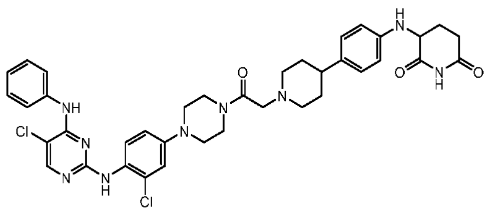 T-328 |
| 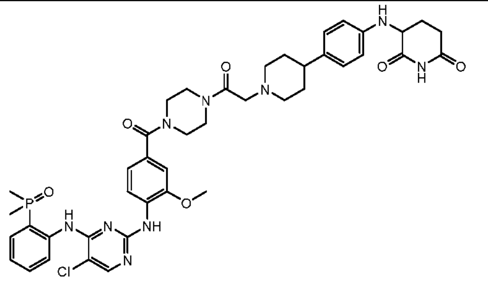 T-329 | 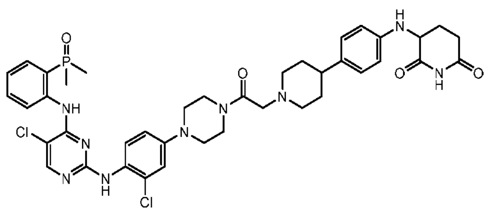 T-330 |
| 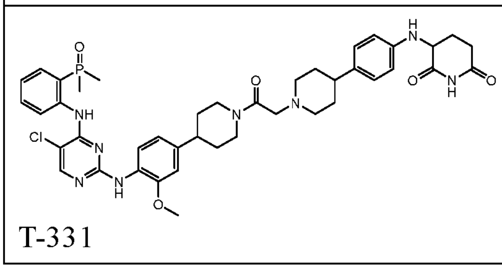 T-331 | 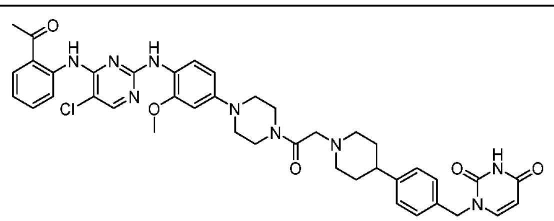 T-332 |

| | |
|---|---|
| 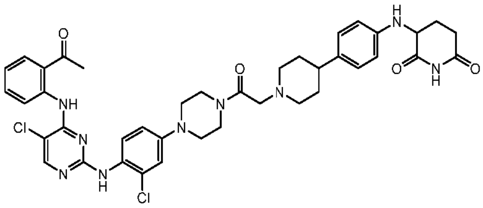 T-333 | 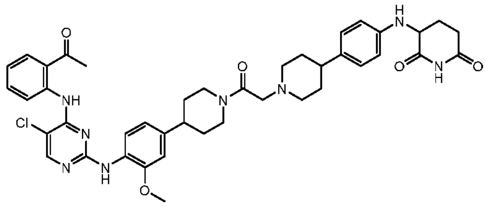 T-334 |
| 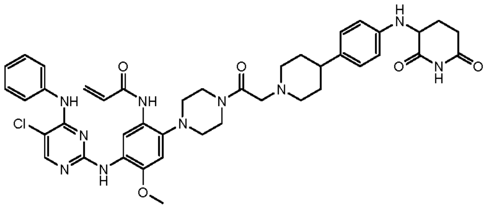 T-335 | 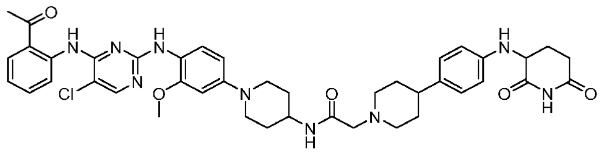 T-336 |
| 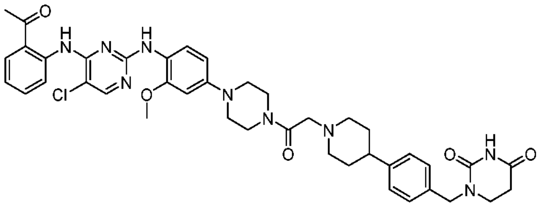 T-337 | 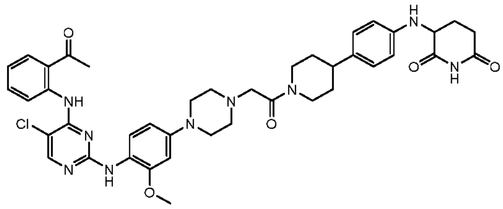 T-338 |
| 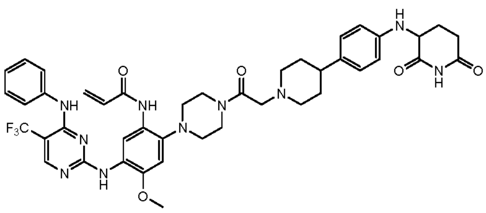 T-339 | 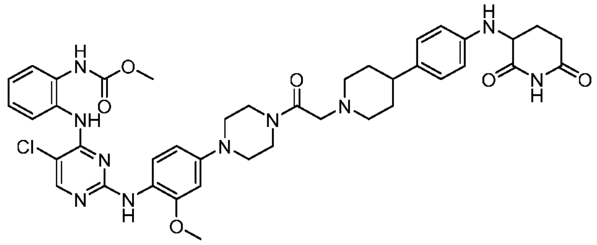 T-340 |
| 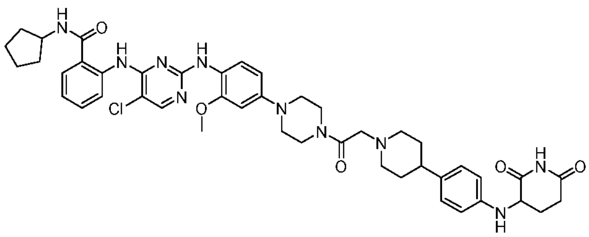 T-341 | 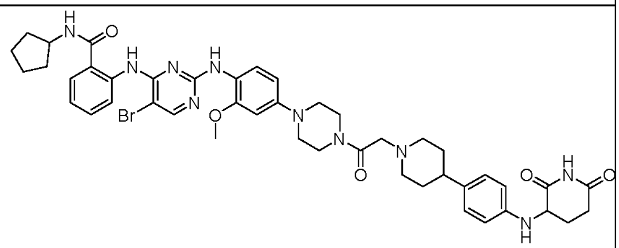 T-342 |
| 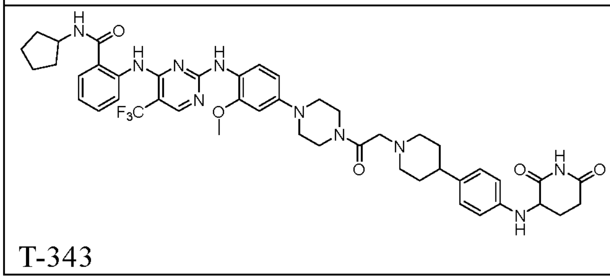 T-343 | 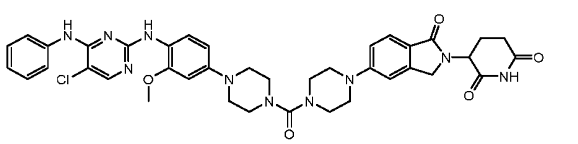 T-344 |

| | |
|---|---|
| 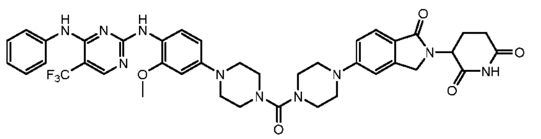<br>T-345 | 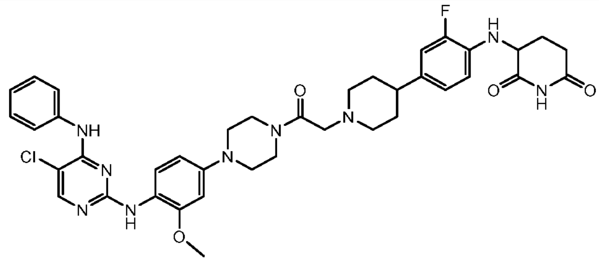<br>T-346 |
| 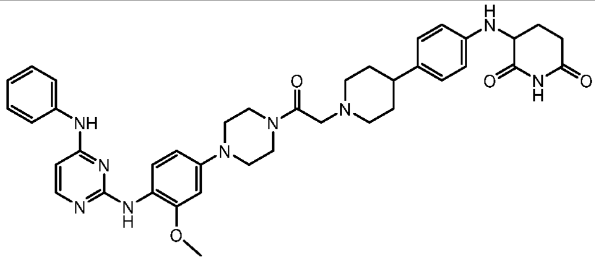<br>T-347 | 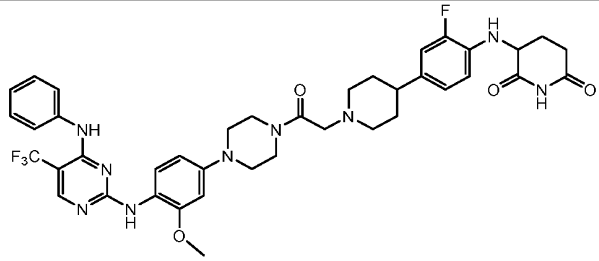<br>T-348 |
| 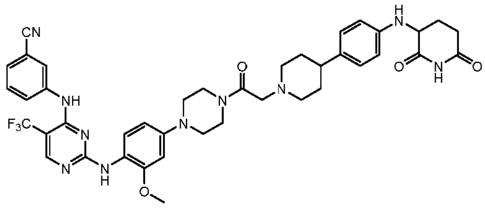<br>T-349 | 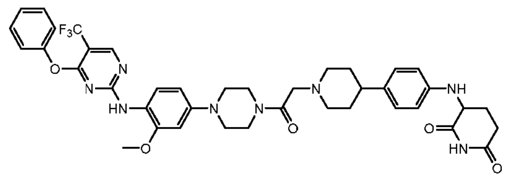<br>T-350 |
| 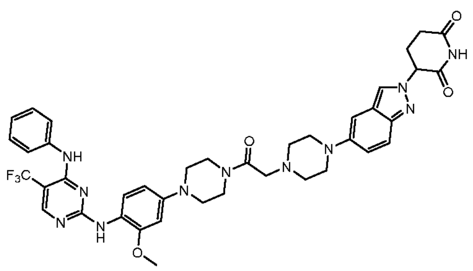<br>T-351 | 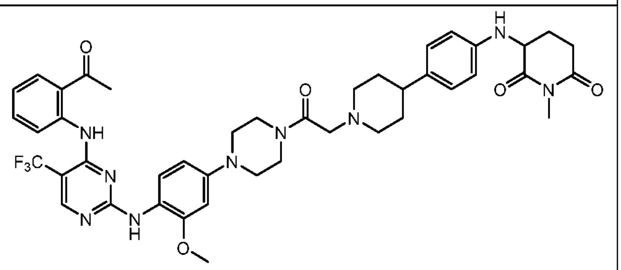<br>T-352 |
| 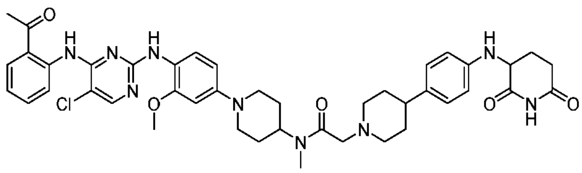<br>T-353 | 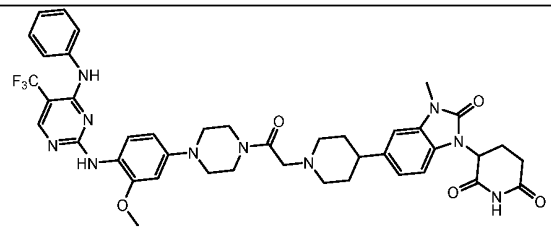<br>T-354 |
| 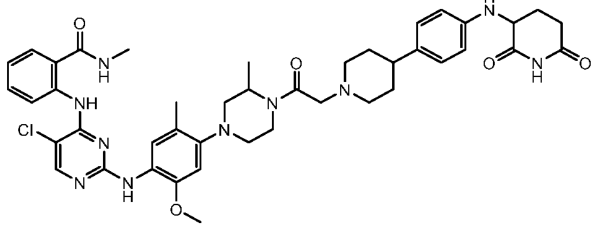<br>T-355 | 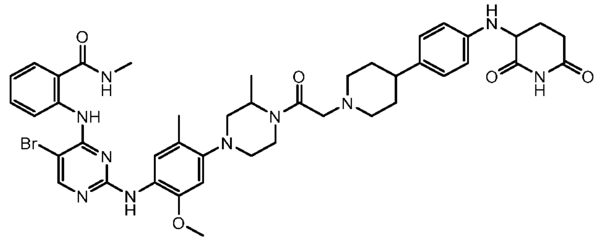<br>T-356 |

| | |
|---|---|
| 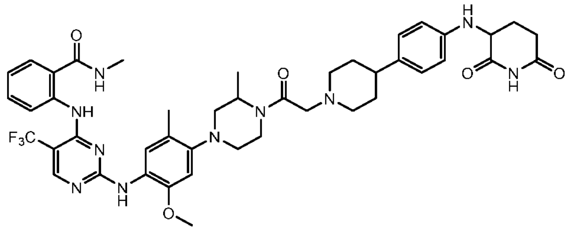 T-357 | 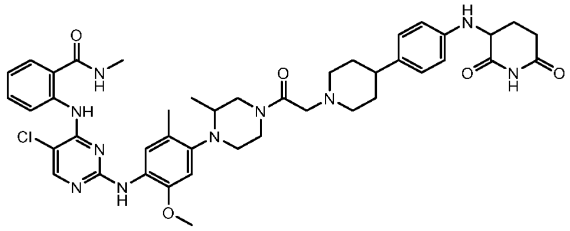 T-358 |
| 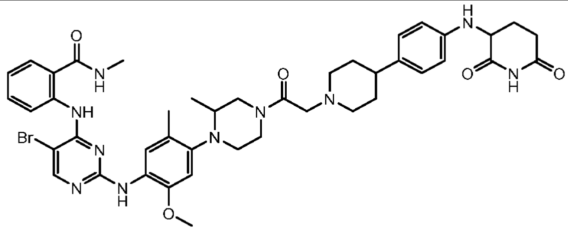 T-359 | 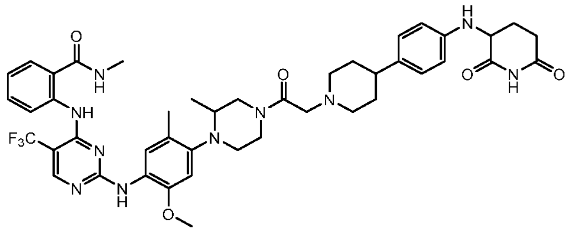 T-360 |
| 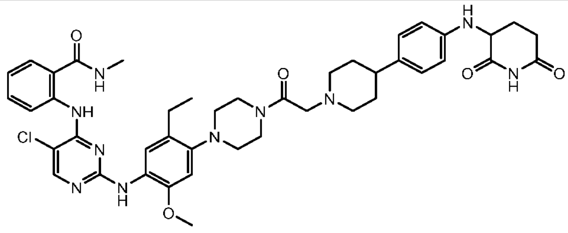 T-361 | 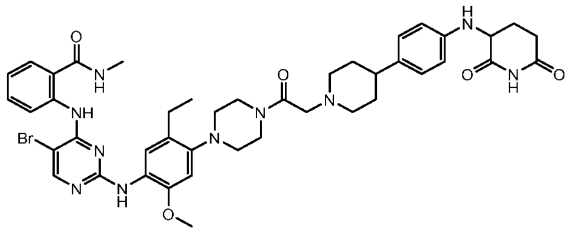 T-362 |
| 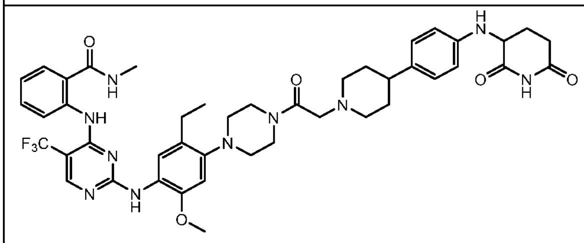 T-363 | 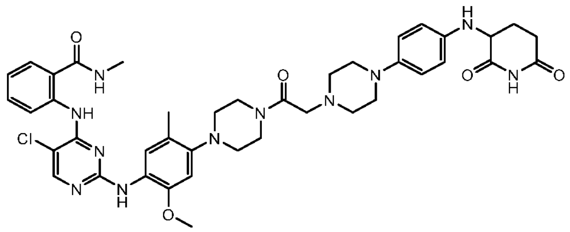 T-364 |
| 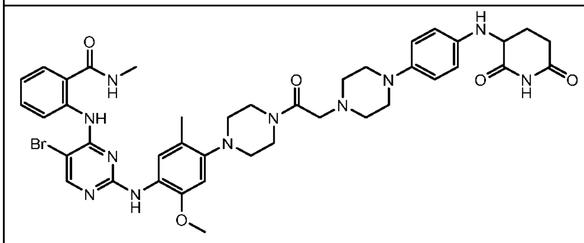 T-365 | 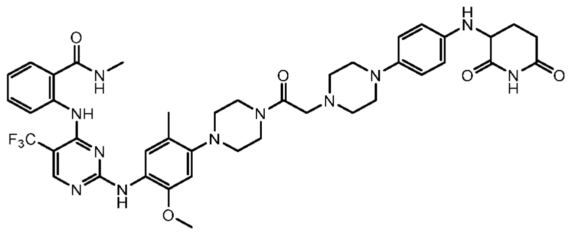 T-366 |
| 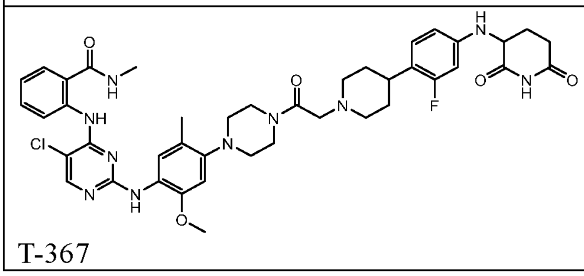 T-367 | 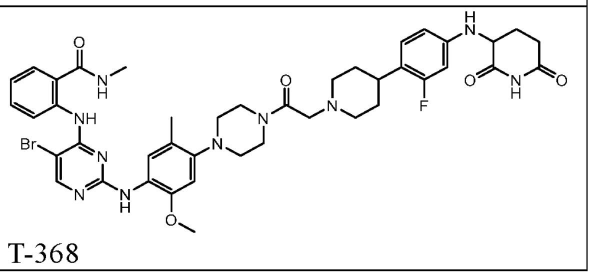 T-368 |

| | |
|---|---|
| 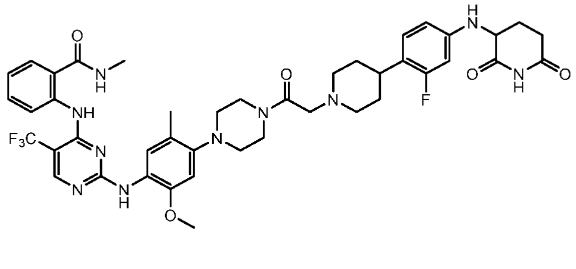 T-369 | 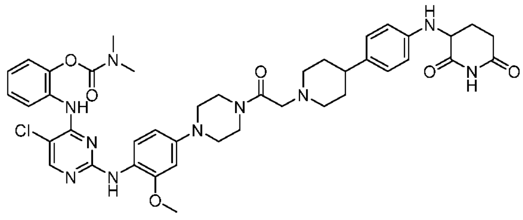 T-370 |
| 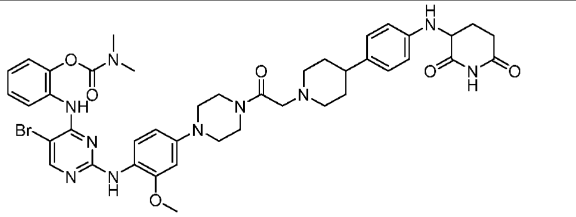 T-371 | 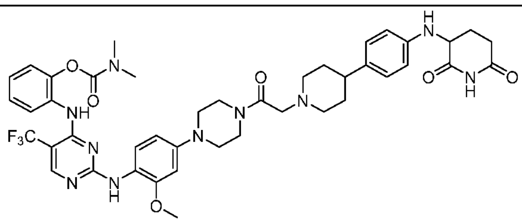 T-372 |
| 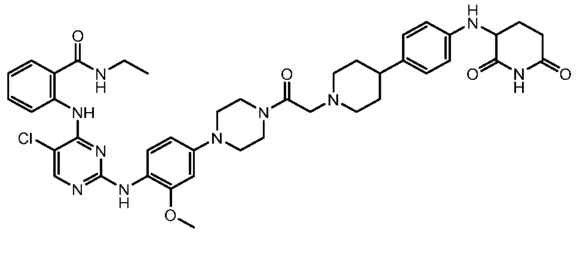 T-373 | 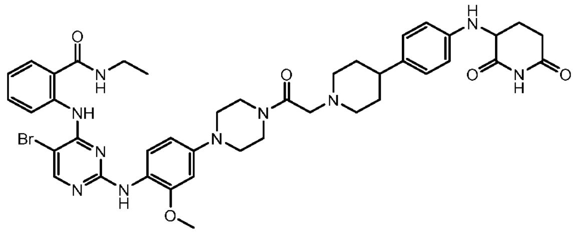 T-374 |
| 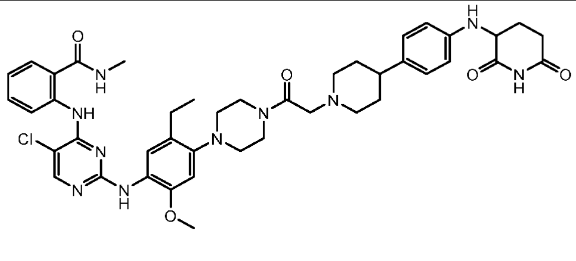 T-375 | 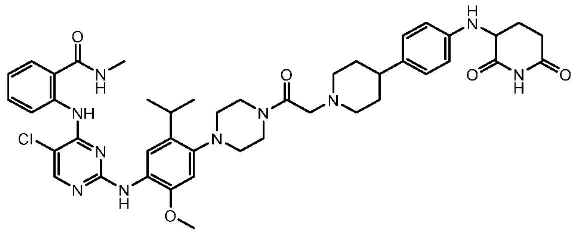 T-376 |
| 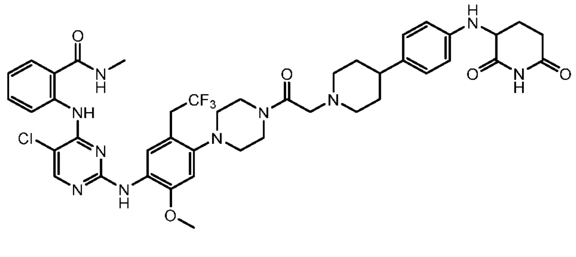 T-377 | 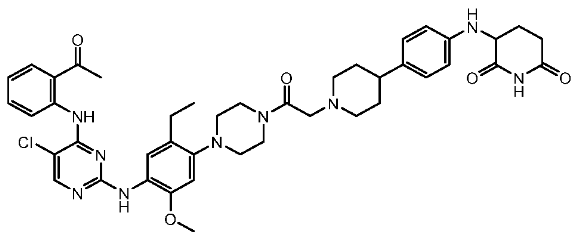 T-378 |
| 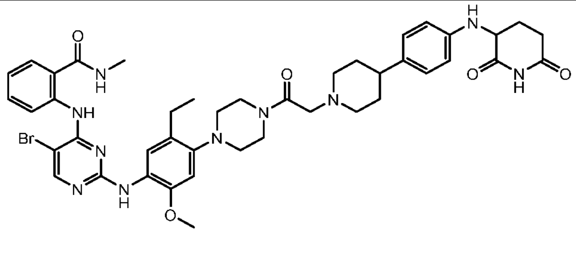 T-379 | 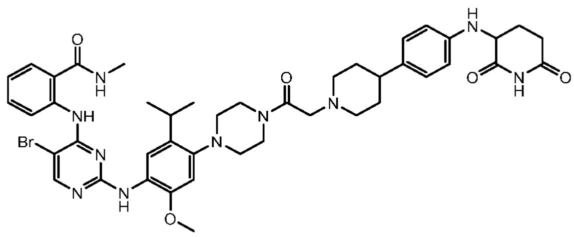 T-380 |

| | |
|---|---|
| 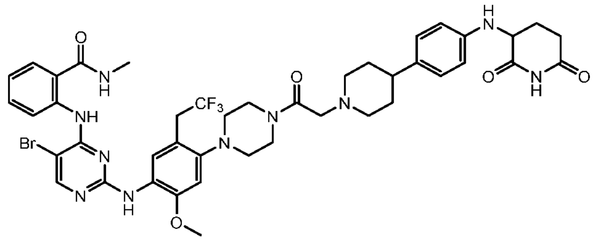 T-381 | 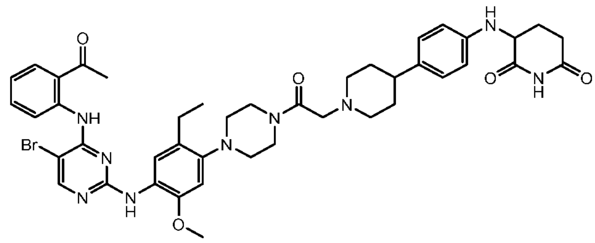 T-382 |

In another preferred embodiment, the pharmaceutically acceptable salt is an inorganic acid salt or an organic acid salt;

wherein the inorganic acid salt is selected from the group consisting of: hydrochloride, hydrobromide, hydriodate, sulfate, bisulfate, nitrate, phosphate, and acid phosphate;
the organic acid salt is selected from the group consisting of: formate, acetate, trifluoroacetate, propionate, pyruvate, glycolate, oxalate, malonate, fumarate, maleate, lactate, malate, citrate, tartrate, methanesulfonate, ethanesulfonate, benzenesulfonate, p-toluenesulfonate, salicylate, picrate, glutamate, ascorbate, camphorate, and camphorsulte.

In the second aspect of the present invention, provided is a pharmaceutical composition, comprising the compound as described in the first aspect of the present invention, and pharmaceutically acceptable carriers.
In the third aspect of the present invention, provided is a use of the compound as described in the first aspect of the present invention and a composition comprising the same in the use selected from the group consisting of:

1) Preparation of drugs for modulating EGFR kinase activity or treating EGFR-related diseases;
2) Preparation of drugs for degrading EGFR proteins;
3) Preparation of drugs for degrading an EGFR mutated protein selected from the group consisting of: DEL19, L858R, L858R/T790M, L858R/C797S, DEL19/T790M/C797S, L858R/T790M/C797S, exon20 insertion mutation, L861Q, DEL19-G724S and L858R-L792H.

In another preferred embodiment, the EGFR-related disease is selected from the group consisting of: inflammation, cancer, cardiovascular disease, infection, immune disease and metabolic disease.
In another preferred embodiment, the cancer is selected from the group consisting of: lung cancer (including lung adenocarcinoma, non-small cell lung cancer), breast cancer, prostate cancer, colorectal cancer, liver cancer, pancreatic cancer, ovarian cancer, leukemia, neuroblastoma, gastric cancer, renal cancer, esophageal cancer, uterine cancer, glioma, and head and neck cancer. A certain embodiment of the present invention relates to a compound of formula I or a pharmaceutically acceptable salt thereof as described herein for use as a drug in the therapeutic and/or prophylactic treatment of a patient suffering from cancer, in particular non-small cell lung cancer, having EGFR mutations T790M/L858R, T790M/L858R/C797S, L858R and/or L858R/C797S, comprising determining the EGFR-activating mutation status of the patient, and then administering to the patient a compound of Formula I or a pharmaceutically acceptable salt thereof as described herein.
A certain embodiment of the present invention relates to a compound of formula I or a pharmaceutically acceptable salt thereof as described herein for use as a drug in the therapeutic and/or prophylactic treatment of a patient suffering from cancer, particularly non-small cell lung cancer, having an EGFR activating mutation determined by the cobas EGFR mutation assay v2, comprising determining the EGFR activating mutation status of the patient, and then administering to the patient a compound of Formula I or a pharmaceutically acceptable salt thereof as described herein.

## Detailed Description of the Invention

The present invention provides a compound of formula I and pharmaceutically acceptable salts thereof, preparations therefor, pharmaceuticals containing the same and the manufacture thereof, and the use of the compounds in the therapeutic and/or prophylactic treatment of cancer, particularly non-small cell lung cancer.

## Terms

The following terms used in this application, including the specification and claims, have the definitions given

below unless otherwise indicated.

When the substituent is described by the conventional chemical formula written from left to right, the substituent also includes the chemically equivalent substituent obtained when writing the structural formula from right to left. For example, $-CH_2O-$ is equivalent to $-OCH_2-$.

"Alkyl (alone or as part of other groups)" means a monovalent straight or branched saturated hydrocarbon group comprising only of carbon and hydrogen atoms and containing from 1 to 12 carbon atoms. Alkyl groups are preferably C1-C6 alkyl group (i.e., containing 1, 2, 3, 4, 5 or 6 carbon atoms). Examples of alkyl include, but are not limited to, methyl, ethyl, propyl, isopropyl, isobutyl, sec-butyl, tert-butyl, pentyl, n-hexyl, octyl, dodecyl, and the like. In the present application, alkyl is also intended to encompass substituted alkyl, i.e. one or more positions in the alkyl are substituted, in particular 1-4 substituents, which may be substituted in any position. "Haloalkyl" refers to an alkyl as defined herein in which one or more of the hydrogens are substitued by the same or different halogens. Examples of haloalkyl include $-CH_2Cl$, $-CH_2CF_3$, $-CH_2CCl_3$, perfluoroalkyl (e.g., $-CF_3$), and the like.

"Alkylidene" refers to divalent groups of alkyl, such as $-CH_2-$, $-CH_2CH_2$ - and $-CH_2$ $CH_2CH_2$ -.

"Alkoxy (alone or as part of other groups)" refers to an alkyl to which an oxy group is attached, having an alkyl-O- structure, wherein the alkyl is as defined above, preferably, the alkoxy is a C1-C6 alkoxy. Alkoxy includes, but is not limited to, methoxy, ethoxy, propoxy, tert-butoxy, and the like. "Haloalkoxy" refers to a group of formula -OR, wherein R is a haloalkyl as defined herein. Examples of haloalkoxy include, but are not limited to, trifluoromethoxy, difluoromethoxy, 2,2,2-trifluoroethoxy, and the like.

"Thioalkyl" means an alkyl in which the carbon is substitued by S, S(O) or $S(O)_2$.

"Alkenyl (alone or as part of other groups)" means an aliphatic group containing at least one double bond, typically having from 2 to 20 carbon atoms. In the present application, "C2-C6 alkenyl" means an alkenyl containing 2, 3, 4, 5 or 6 carbon atoms. Examples of Alkenyl include, but are not limited to, vinyl, propenyl, butenyl, 1-methyl-2-buten-1-yl, and the like. In the present application, alkenyl includes substituted alkenyl.

The term "alkenylene" refers to an alkenyl with two connection sites. For example, "vinylidene" means the group -CH=CH-. Alkenylene may also be in an unsubstituted form or in a substituted form having one or more substituents.

"Alkynyl (alone or as part of other groups)" means a straight or branched hydrocarbon chain containing more than 2 carbon atoms and characterized by one or more triple bonds, typically having from 2 to 20 carbon atoms. In the present application, "$C_{2-6}$alkynyl" means an alkynyl having 2, 3, 4, 5 or 6 carbon atoms. Alkynyl include, but is not limited to, ethynyl, propargyl, and 3-hexynyl. One of the triple bonded carbons may optionally be the connection site for the alkynyl substituent. In the present invention, the alkynyl also includes a substituted alkynyl.

"Alkynylene" refers to an alkynyl group with two connection sites. For example, "ethynylidene" means the group: -C=C-. An alkynylidene may also be in an unsubstituted form or in a substituted form having one or more substituents.

"Aliphatic group" means a straight, branched or cyclic hydrocarbon group, including saturated and unsaturated groups such as alkyl, alkenyl and alkynyl.

"Aromatic ring system" means a monocyclic, bicyclic or polycyclic hydrocarbon ring system in which at least one ring is aromatic.

"Aryl (alone or as part of other groups)" means a monovalent aromatic ring system. Representative aryl include all-aromatic ring systems such as phenyl, naphthyl, and anthracenyl; and ring systems in which an aromatic carbon ring is fused with one or more non-aromatic carbon rings, such as indanyl, phthalimido, naphthyliminoyl, or tetrahydronaphthalenyl, and the like. In the present invention, aryl is preferably a C6-C12 aryl. In the present invention, aryl is further intended to encompass substituted aryl.

"Arylalkyl" or "aryl-alkyl" means an alkyl in which the alkyl hydrogen atom is substitued by an aryl.Arylalkyl includes groups in which one or more hydrogen atoms are substituted with an aryl group, wherein the aryl and alkyl are as defined above. Examples of "arylalkyl" or "aryl-alkyl" include benzyl, 2-phenylethyl, 3-phenylpropyl, 9-fluorenyl, diphenylmethyl and triphenylmethyl.

"Aryloxy" means -O-(aryl), wherein the aryl moiety is as defined herein.

"Heteroalkyl" means a substituted alkyl having one or more backbone chain atoms selected from atoms other than carbon, e.g., oxygen, nitrogen, sulfur, phosphorus, or combinations thereof. A range of values can be given, e.g., C1-C6 heteroalkyl refers to the number of carbons in the chain, which includes from 1 to 6 carbon atoms. For example $-CH_2OCH_2CH_3$ are referred to as "C3" heteroalkyl. The connection to the rest of the molecule can be made through the heteroatoms or carbons in the heteroalkyl chain. "Heteroalkylene" refers to an optionally substituted divalent alkyl having one or more backbone chain atoms selected from atoms other than carbon, e.g., oxygen, nitrogen, sulfur, phosphorus, or combinations thereof.

"Carbocyclic system" means monocyclic, bicyclic or polycyclic hydrocarbon ring system in which each ring is fully saturated or contains one or more unsaturated units, but none of the ring is aromatic.

"Carbocyclic group" means a monovalent group of a carbocyclic system. Examples include cycloalkyl (cyclopentyl, cyclobutyl, cyclopropyl, cyclohexyl, etc.) and cycloalkenyl (e.g., cyclopentenyl, cyclohexenyl, cyclopentadienyl, etc.).

"Cycloalkyl" refers to a monovalent saturated carbocyclic group comprising a mono- or bicyclic ring having 3-12, preferably 3-10, more preferably 3-8 ring atoms. The cycloalkyl group may optionally be substituted with one or more substituents, wherein each substituent is independently hydroxyl, alkyl, alkoxy, halogen, haloalkyl, amino, monoalkylamino or dialkylamino. Examples of cycloalkyl include, but are not limited to, cyclopropyl, cyclobutyl, cyclopentyl, cyclohexyl, cycloheptyl, and the like.

"Cycloalkoxy" refers to groups having formula -OR, wherein R is cycloalkyl as defined herein. Exemplary cycloalkyloxy include cyclopropyloxy, cyclobutyloxy, cyclopentyloxy, cyclohexyloxy, and the like. "Cycloalkylalkyl" means (cycloalkyl)-alkyl- wherein cycloalkyl and alkyl are as disclosed herein. "Cycloalkylalkyl" is bonded to the parent molecule structure through the cycloalkyl.

"Heteroaromatic ring system" means monocyclic (e.g., 5- or 6-membered), bicyclic (6-12-membered), or polycyclic system in which at least one ring is aromatic and contains at least one heteroatom (e.g., N, O, or S); and in which none of the other ring is heterocyclic (as defined below). In some embodiments, a ring that is aromatic and contains heteroatoms contains 1, 2, 3, or 4 cyclic heteroatoms in the ring. At least one ring thereof is heteroaromatic and the remaining rings may be saturated, partially unsaturated or completely unsaturated.

"Heteroaryl" refers to monocyclic (e.g., 5 or 6-membered), bicyclic (e.g., 8-10-membered) or tricyclic group having 5 to 12 ring atoms and containing at least one aromatic ring which comprises 1, 2, or 3 ring heteroatoms selected from N, O, and S, with the remaining ring atoms being C. It should be clear that the connection site of the heteroaryl should be disposed on the aromatic ring. Examples of heteroaryl groups include, but are not limited to: imidazolyl, oxazolyl, isoxazolyl, thiazolyl, isothiazolyl, oxadiazolyl, thiadiazolyl, pyrazinyl, thienyl, furanyl, pyranyl, pyridyl, pyrrolyl, pyrazolyl, pyrimidyl, quinolyl, isoquinolyl, benzo-furanyl, benzofuryl, benzothienyl, benzothianyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthyridinyl, pteridinyl, carbazolyl, azepinyl, diazepinyl, acridinyl and the like. Heteroarylidene refers to a heteroaryl with two connection sites.

"Heterocyclic system" means monocyclic, bicyclic and polycyclic systems in which at least one ring is saturated or partially unsaturated (but not aromatic) and the ring contains at least one heteroatom. The heterocyclic system may be attached to side group of any heteroatom or carbon atom, which produces a stable structure and any of the ring atoms may optionally be substituted.

"Heterocyclyl" means a monovalent group of a heterocyclic ring system, usually a stable monocyclic (e.g., 3-8 membered, i.e., 3, 4, 5, 6, 7, or 8-membered) or bicyclic (e.g., 5-12 membered, i.e., 5, 6, 7, 8, 9, 10, 11, or 12-membered) ring, or a polycyclic ring (e.g., 7-14 membered, i.e., 7, 8, 10, 11, 12, 13, or 14-membered), including fused, spiro and/or bridged ring structures that are saturated, or partially unsaturated, and which contain carbon atoms and 1, 2, 3 or 4 heteroatoms independently selected from N, O, and S. Representative heterocyclyl include the following ring systems, wherein (1) each ring is non-aromatic and at least one ring comprises heteroatom, e.g., tetrahydrofuranyl, tetrahydropyranyl, tetrahydrothienyl, pyrrolidinyl, pyrrolidono, piperidinyl, pyrrolinyl, decahydroquinolinyl, oxazolidinyl, piperazinyl, dioxanyl, dioxolane, diazepinyl, oxazepinyl, thiazepinyl, morpholinyl, and quinuclidinyl; (2) at least one ring is non-aromatic and contains heteroatoms and at least one other ring is an aromatic carbocyclic ring, e.g., 1,2,3,4-tetrahydroquinolinyl, 1,2,3,4-tetrahydroisoquinolinyl; and (3) at least one ring is non-aromatic and contains heteroatoms and at least one other ring is aromatic and contains heteroatoms, e.g., 3,4-dihydro-1H- pyrano[4, 3-c]pyridine and 1,2,3,4-tetrahydro-2,6-diazanaphthalene. Heterocyclylidene are heterocyclyls having two attachment sites. Preferably in the present invention, the heterocyclylidene is a bicyclic ring, wherein one ring is heteroaryl and is connected to the rest of the general formula through the heteroaryl. Preferably in the present invention, the heterocyclylidene is a 5-6-membered monocyclic heterocyclylidene or an 8-10-membered bicyclic heterocyclylidene.

"Heterocyclyl alkyl" means an alkyl group substituted with heterocyclyl, wherein heterocyclyl and alkyl are as defined above.

"Alkylamino group" means a group having an alkyl-NR- structure, wherein R is H, or alkyl, cycloalkyl, aryl, heteroaryl, etc., as described above.

"Cycloalkylamino group" refers to a group of formula-NRaRb, wherein Ra is H, alkyl as defined herein or cycloalkyl as defined herein, Rb is cycloalkyl as defined herein, or Ra and Rb together with the N atom they attached form a 3-to -10 membered N-containing mono- or bicyclic heterocyclyl such as tetrahydropyrrolyl. As used herein, C3-C8 cycloalkylamino group is an amino group containing 3-to- 8 carbon atoms.

In the present invention, "ester group" means a -C(O)-O-R or R-C(O)-O- structure, wherein R independently represents hydrogen, alkyl, cycloalkyl, aryl, heteroaryl, heterocyclyl, as defined above.

In the present invention, the term "amide group" refers to a group with the structure -CONRR', wherein R and R' may independently represent hydrogen, alkyl or substituted alkyl, cycloalkyl or substituted cycloalkyl, aryl or substituted aryl, heterocyclyl or substituted heterocyclyl as defined herein. R and R' may be the same or different in the dialkylamine fragment.

In the present invention, the term "sulfonamido" refers to a group with the structure $-SO_2NRR'$, wherein R and R' may independently represent hydrogen, alkyl or substituted alkyl, cycloalkyl or substituted cycloalkyl, aryl or substituted

aryl, heterocyclyl or substituted heterocycyl as defined herein. R and R' may be the same or different in the dialkylamine fragment.

"Ketonic carbonyl" means R-C(=O)-, wherein R is alkyl, cycloalkyl, etc. as described above.

When the substituent is a non-terminal substituent, it is a -diylene of the corresponding group, e.g., alkyl corresponds to alkylene, cycloalkyl corresponds to cycloalkylene, heterocyclyl corresponds to heterocyclylidene, alkoxy corresponds to alkyleneoxy, and the like.

In the present invention, each of the aforementioned alkyl, alkoxy, cycloalkyl, heteroalkyl, aryl, heteroaryl, cycloheteroalkyl, alkenyl, alkynyl, heterocyclic, heterocyclyl, and the like may be substituted or unsubstituted.

For the purposes of the present invention, the term "substituted" refers to one or more hydrogen atoms on a particular group are substituted by a particular substituent. The particular substituent is a substituent described above, or a substituent appearing in the Examples. Unless specifically stated, a particular substituted group may have a substituent selected from a particular group at any substitutable site of the group, and the substituents may be the same or different at various positions.It should be understood by those skilled in the art that the combinations of substituents contemplated by the present invention are those that are stable or chemically realizable. Exemplary substituents include, but are not limited to, one or more of the group consisting of: e.g., hydrogen, deuterium, halogen (e.g., monohalogen substituent or polyhalogen substituent, the latter being e.g., trifluoromethyl or an alkyl comprising $Cl_3$), cyano, nitro, oxo (e.g., =O), trifluoromethyl,trifluoromethoxy, cycloalkyl, alkenyl, alkynyl, heterocycle, aromatic ring, $OR_a$, $SR_a$, $S(=O)R_e$, $S(=O)_2R_e$, $P(=O)_2R_e$, $S(=O)_2OR_e$, $P(=O)_2OR_e$, $NR_bR_c$, $NR_bS(=O)_2R_e$, $NR_bP(=O)_2R_e$, $S(=O)_2NR_bR_c$, $P(=O)_2NR_bR_c$, $C(=O)OR_d$, $C(=O)R_a$, $C(=O)NR_bR_c$, $OC(=O)R_a$, $OC(=O)NR_bR_c$, $NR_bC(=O)OR_e$, $NR_dC(=O)NR_bR_c$, $NR_dS(=O)_2NR_bR_c$, $NR_dP(=O)_2NR_bR_c$, $NR_bC(=O)R_a$, and $NR_bP(=O)_2Re$, wherein $R_a$ may independently represent hydrogen, deuterium, alkyl, cycloalkyl, alkenyl, alkynyl, heterocycle, or aromatic ring, $R_b$, $R_c$, and $R_d$ may independently represent hydrogen, deuterium, alkyl, cycloalkyl, heterocycle, or aromatic ring; or $R_b$ and $R_c$ with N atom may form a heterocycle; $R_e$ may independently represent hydrogen, alkyl, cycloalkyl, alkenyl, alkynyl, heterocycle or aromatic ring. Exemplary substituents such as alkyl, cycloalkyl, alkenyl, cycloalkenyl, alkynyl, heterocycle or aromatic ring as described above can be optionally substituted. The substituents are, for example (but not limited to): halogen, hydroxyl, cyano, carboxy (-COOH), C1-C6 alkyl, C2-C6 alkenyl, C2-C6 alkynyl, C3-C8 cycloalkyl, 3-to-12-membered heterocyclyl, aryl, heteroaryl, C1-C8 aldehyde, C2-C10 acyl, C2-C10 ester group, amino, C1-C6 alkoxy, C1-C10 sulfonyl, and C1-C6 ureido, etc..

"Cyano" means -CN group.

"Nitro" means $-NO_2$.

"Hydroxyl" means -OH.

"Amino" means $-NH_2$ or RNH-, wherein R is ketonic carbonyl, sulfonyl, sulfonamido, $R_a$-C(=O)-, $R_aR_b$N-C(=O)-, etc., wherein $R_a$ and $R_b$ are alkyl, cycloalkyl, aryl, or heteroaryl, etc.

"Halogen (halogenated)" means any halogen group, e.g., -F, -Cl, -Br, or -I.

"Deuteride" means a compound in which one or more hydrogen atoms (H) are substituted by deuterium atoms (D).

For the purposes of the present invention, the term "more" refers independently to 2, 3, 4 or 5.

**Active ingredient**

As used herein, the terms "compound of the invention" or "active ingredient of the invention" are used interchangeably to refer to a compound of Formula I, or a pharmaceutically acceptable salt, a hydrate, a solvate, an isotopic compound (e.g., deuterated compound) or a prodrug thereof. The term also includes racemates, optical isomers.

The compound of formula I have the following structure:

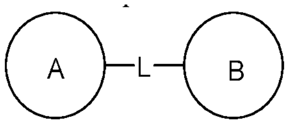

I

Rings A, ring B and L are as defined above.

Salts that may be formed by the compounds of the present invention are also within the scope of the present invention. Unless otherwise indicated, the compounds of the present invention are understood to include their salts. The term "salt", as used herein, refers to the acidic or basic salts formed with inorganic or organic acids or bases. In addition, when the compounds of the present invention contain a basic fragment, it includes, but is not limited to, pyridine or imidazole; when contain an acidic fragment, it includes, but is not limited to, carboxylic acids, the zwitterion that may be formed ("inner salts") are included within the scope of the term "salt". Pharmaceutically acceptable (i.e., non-toxic, physiologically acceptable) salts are preferred, although other salts are also useful, e.g., for isolation or purification steps

in the preparation process. The compounds of the present invention may form salts, e.g., by reacting Compound I with a certain amount of, e.g., an equivalent amount of an acid or a base, by salting out in a medium, or by freeze-drying in an aqueous solution.

The compounds of the present invention contain basic fragments, including but not limited to amine or pyridine or imidazole ring, that may form salts with organic or inorganic acids. Exemplary acids that may form salts include acetates (e.g., with acetic acid or trihaloacetic acid such as trifluoroacetic acid), adipates, alginates, ascorbates, aspartates, benzoates, benzenesulfonates, bisulfates, borates, butyrate, citrates, camphorates, camphorsulfonates, cyclopentane propionate, diethylglycolates, dodecylsulfate, ethanesulfonate, fumarate, glucoheptulose, glycerophosphate, hemisulfate, heptanoate, hexanoate, hydrochloride, hydrobromide, hydriodate, hydroxyethanesulfonate (e.g., 2-hydroxyethanesulfonate), lactate, maleate, methanesulfonate, naphthalenesulfonate (e.g., 2-naphthalenesulfonate), nicotinate, nitrate, oxalate, pectate, persulphate, phenpropionate(e.g., 3-phenpropionate), phosphate, picrate, pivalate, propionate, salicylate, succinate, sulphate (e.g. formed with sulphuric acid), sulphonate, tartrate, thiocyanate, toluenesulfonate such as p-toluenesulfonate, dodecanoate, and the like.

Some of the compounds of the present invention may contain acidic fragments, including, but not limited to, carboxylic acids, which may form salts with various organic or inorganic bases. Exemplary base-forming salts include ammonium salts, alkali metal salts such as sodium, lithium, and potassium salts, alkaline earth metal salts such as calcium and magnesium salts, and salts formed with organic bases (e.g., organic amines) such as benzathine, dicyclohexylamine, haiba amine (salt formed with N,N-bis(dehydroabietyl)ethylenediamine), N-methyl-D-glucosamine, N-methyl-D-glucosamide, tert-butylamine, and salts formed with amino acids such as arginine, lysine, etc.. Basic nitrogen-containing groups can form quaternary ammonium salt with halides such as small molecule alkyl halides (e.g., methyl, ethyl, propyl, and butyl chlorides, bromides, and iodides), dialkyl sulfates (e.g., dimethyl, diethyl, dibutyl, and dipentyl sulfates), long-chain halides (e.g., decyl, dodecyl, tetradecyl, and tetradecyl chlorides, bromides, and iodides), arylalkyl halides (e.g., benzyl and phenyl bromides), and the like.

Podrugs and solvates of the compounds of the present invention are also covered.

The term "prodrug" herein refers to a compound that undergoes a chemical transformation by metabolic or chemical process to produce a compound, salt, or solvate of the present invention in the treatment of relevant diseases. The compounds of the present invention include solvates, such as hydrates.

The compounds, salts, or solvates of the present invention, may exist in tautomeric forms (e.g., amides and imine ethers). All of these tautomers are part of the present invention.

All stereoisomers of the compounds (e.g., those asymmetric carbon atoms that may exist as a result of various substitutions), including both their enantiomeric and diastereomeric forms, are within the contemplated scope of the present invention. The independent stereoisomer of the compound of the present invention may not co-exist with other isomers (e.g., as a pure or substantially pure optical isomer with specific activity) or may also be mixtures, such as racemates, or mixtures formed of all other stereoisomers, or portions thereof. The chiral centers of the present invention are available in either the S or R configuration, as defined by the 1974 recommendations of the International Union of Pure and Applied Chemistry (IUPAC). The racemic forms may be resolved by physical methods, such as stepwise crystallization, or separation by derivatization into diastereoisomers for crystallization, or separation by chiral column chromatography. Individual optical isomers may be obtained from the racemic form by suitable methods including, but not limited to, conventional methods such as salt formation with optically active acids followed by recrystallization.

The compounds of the present invention, obtained sequentially by preparation, isolation and purification of the compounds with a weight content equal to or greater than 90%, e.g., equal to or greater than 95%, equal to or greater than 99% ("very pure" compounds) are set forth in the description. Such "very pure" compounds of the present invention are herein also included as part of the present invention.

All conformational isomers of the compounds of the present invention are covered, whether in mixture, pure or very pure form. The definition of the compounds of the present invention encompasses the two olefinic isomers, cis(Z) and trans-, as well as the cis and trans isomers of carbocycles and heterocycles.

Throughout the specification, groups and substituents may be selected to provide stable fragments and compounds.

Specific functional groups and definitions of chemical terms are detailed below. For the purposes of the present invention, the chemical elements correspond to those defined in Periodic Table of the Elements, CAS version, Handbook of Chemistry and Physics, 75th Ed. Definitions of specific functional groups are also described therein. In addition, the basic principles of organic chemistry, as well as specific functional groups and reactivity, are described in "Organic Chemistry," Thomas Sorrell, University Science Books, Sausalito: 1999, which is incorporated by reference in its entirety.

Certain compounds of the present invention may exist in specific geometric or stereoisomeric forms. The present invention covers all compounds, including their cis and trans isomers, R and S enantiomers, diastereomers, (D)-isomers, (L)-isomers, racemic mixtures and other mixtures. Additionally the asymmetric carbon atom may represent a substituent such as alkyl. All isomers, as well as mixtures thereof, are encompassed within the present invention.

For the purpose of the present invention, mixtures of isomers may contain isomers in various ratios. For example,

mixtures in which there are only two isomers can have the following combinations: 50:50, 60:40, 70:30, 80:20, 90:10, 95:5, 96:4, 97:3, 98:2, 99:1, or 100:0, with all ratios of the isomers being within the scope of the present invention. Similar ratios, which are readily understood by one of ordinary skill in the art, and ratios for mixtures of more complicated isomers, are also within the scope of the present invention.

The present invention also includes isotopically labeled compounds, equivalent to the original compounds disclosed herein. In practice, however, substitution of one or more atoms by atoms having a different atomic weight or mass number from that of the atoms usually occurs. Examples of isotopes that may be classified as isotopes of the compounds of the present invention include hydrogen, carbon, nitrogen, oxygen, phosphorus, sulphur, fluorine and chlorine isotopes such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds, or enantiomers, diastereomers, isomers, or pharmaceutically acceptable salts or solvates of the present invention which contain isotopes or other isotopic atoms of the above mentioned compounds are within the scope of the present invention. Certain isotopically labeled compounds of the present invention, e.g., the radioisotopes of $^{3}H$ and $^{14}C$ are also included and are useful in tissue distribution experiments of drugs and substrates. Tritium, i.e., $^{3}H$ and carbon-14, i.e., $^{14}C$, which are relatively easy to prepare and detect are preferred among the isotopes. In addition, heavier isotopic substitutions such as deuterium, i.e., $^{2}H$, can be preferred in some cases due to their very good metabolic stability and advantages in certain therapies, such as increased half-life in vivo or reduced dosage. Isotopically labeled compounds can be prepared in a general way by replacing the no-isotopically labeled reagent with a readily available isotopically labeled reagent, using the protocols disclosed in the Examples.

If the synthesis of a specific enantiomer of a compound of the invention is to be devised, it can be prepared by asymmetric synthesis, or derivatized with a chiral cofactor, the resulting diastereomeric mixture can be separated, and the chiral cofactor can then be removed to give the pure enantiomer. Alternatively, if the molecule contains a basic functional group, such as an amino acid, or an acidic functional group, such as carboxyl group, suitable optically active acid or base can be used to form a diastereoisomeric salt with it, which can then be separated by conventional means, such as by separation and crystallization or by chromatography, and the pure enantiomer is then obtained.

As described herein, the compounds of the present invention may be expanded by the addition of any number of substituents or functional groups. Generally, the term "substituted" whether preceded or followed by the term "optionally", includes substituents in the formulations of the present invention in the general formulas, that refer to the substitution of hydrogen radicals with specified structural substituents. When a plurality of positions in a given structure are substitued by a plurality of specific substituents, each position of the substituent may be the same or different. The term "substitution" as used herein includes all permitted substitutions in organic compounds. Broadly speaking, permitted substituents include acyclic, cyclic, branched and unbranched, carbocyclic and heterocyclic, aromatic and non-aromatic organic compounds. For the purposes of the present invention, e.g., the heteroatom nitrogen may have hydrogen substituents or any of the permitted organic compounds described above to supplement its valence. Furthermore, the present invention is not intended to limit in any way the permitted substitution of organic compounds. The present invention contemplates that combinations of substituents and variable groups in the form of stable compounds are desirable in the treatment of diseases. The term "stable" herein refers to compounds with stability that is detectable for a sufficiently long period of time to maintain the structural integrity of the compound, preferably for a sufficiently long period of time, and is used herein for the above purposes.

Metabolites of the compounds and pharmaceutically acceptable salts thereof, as well as prodrugs that can be transformed in vivo to the structures of the compounds and pharmaceutically acceptable salts thereof, are also included in the claims of this application.

In another preferred embodiment, any one of the respective groups in the compound is the corresponding group in the specific compound, respectively.

## Preparation method

Methods for preparing compounds of Formula I are described in the following schemes and examples. Raw materials and intermediates are purchased from commercial sources, prepared by known steps, or otherwise described. In some embodiments, the order of the steps in which the reaction scheme is carried out can be altered to facilitate the reaction or to avoid unwanted side reaction products.

Methods of preparing the compounds with the structure of Formula I of the present invention are described more specifically below, but these specific methods do not constitute any limitation of the present invention. The compounds of the present invention can also be conveniently made by optionally combining various synthetic methods described in this specification or known in the art, and such combinations can be readily carried out by those skilled in the field to which the present invention belongs.

Typically, in the preparative process, each reaction is usually protected under inert gas, in a suitable solvent, at 0 to 150°C, and the reaction time is usually 2-24 hours.

Preferable preparation method is as follows:

Method I:

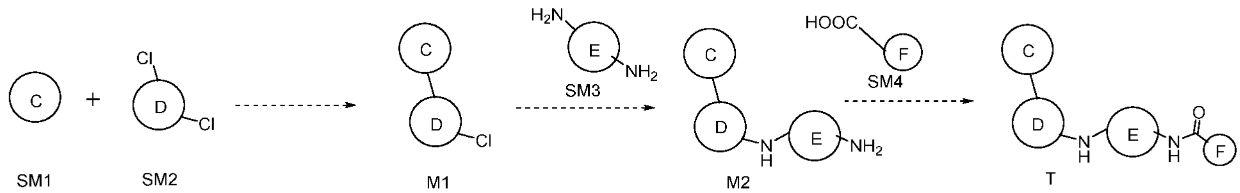


SM1 SM2 M1 M2 T

Step 1: Cooling a mixture of Compound SM2, diisopropylethylamine and n-butanol to -20°C, compound SM1 was added for reaction, then warmed up to room temperature and stirred overnight; the solvent of the reaction system was removed; the residue was added with ethyl acetate and water, and partitioned; the organic phase was taken to remove the solvent, isolated and purified to give M1;

Step 2: a mixture of compound M1, compound SM3, p-toluenesulfonic acid monohydrate and n-butanol was heated to 120 °C and reacted overnight. The solvent in the reaction was removed; the residue was added with ethyl acetate and aqueous sodium bicarbonate, and partitioned; the organic phase was taken to remove the solvent, isolated and purified to give M2;

Step 3: Compound M2 and SM4 were amidocoupled with coupling agent such as HATU, stirred overnight at room temperature, and extracted with water and ethyl acetate; The organic phase was taken to remove the solvent, isolated and purified to give compound T (i.e., compound of formula I).

Method 2:

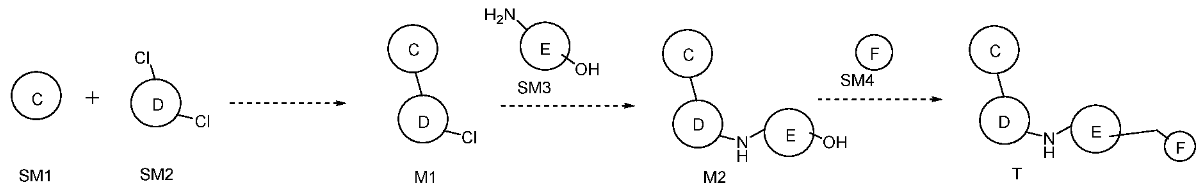


SM1 SM2 M1 M2 T

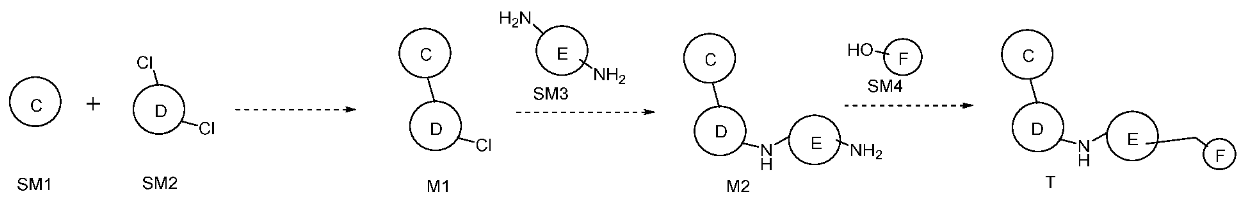


SM1 SM2 M1 M2 T

Step 1: Cooling a mixture of Compound SM2, diisopropylethylamine and n-butanol to -20°C, compound SM1 was added for reaction, then warmed up to room temperature and stirred overnight. The solvent of the reaction system was removed; the residue was added with ethyl acetate and water, and partitioned; the organic phase was taken to remove the solvent, separated and purified to give M1;

Step 2: a mixture of compound M1, compound SM3, p-toluenesulfonic acid monohydrate and n-butanol was heated to 120 °C and reacted overnight. The solvent in the reaction was removed, the residue was added with ethyl acetate and aqueous sodium bicarbonate, and partitioned, the organic phase was taken to remove the solvent, separated and purified to give M2;

Step 3: The hydroxyl group of compound M2 was oxidized to form an aldehyde, which was reductively aminated with SN4 in the presence of sodium borohydride acetate at room temperature, and after completion of the reaction, compound T was obtained by isolation and purification.

Unless otherwise indicated, all of the above starting materials are commercially available or synthesised according to reported literature.

**Pharmaceutical composition and mode of application**

The pharmaceutical composition described in the present invention are used for the prevention and/or treatment of the following diseases: inflammation, cancer, cardiovascular diseases, infections, immune diseases, metabolic diseases.

The compounds of formula I can be administered in combination with other drugs known to treat or ameliorate similar medical conditions. When administered in combination, the mode of administration and dosage of the known drug may be unchanged, while the compound of formula I is administered simultaneously or subsequently.When the compound of formula I is administered in combination with one or more other drugs, a pharmaceutical composition containing both one or more known drugs and the compound of formula I may preferably be used. Drug combination also includes administering the compound of formula I and one or more other known drugs at an overlapped time period.When the compound of formula I is administered in combination with one or more other drugs, the dose of the compound of formula I or the known drug may be lower than when they are administered alone.

Drugs or active ingredients that can be administered in combination with the compound of formula I include, but are not limited to, gefitinib, erlotinib, ectinib, lapatinib, XL647, NVP-AEE-788, ARRY-334543, vandetanib, PF00299804, cetuximab, panitumumab, pertuzumab, zalumumab, nimotuzumab, MDX-214, CDX-110, IMC-11F8, CNF2024, tamspiramycin, spiramycin, IPI-504, NVP-AUY922.

The dosage form of the pharmaceutical composition of the present invention includes (but are not limited to): injections, tablets, capsules, aerosols, suppositories, membranes, pills, topical liniments, controlled-release or sustained-release or nano-formulations.

The pharmaceutical composition of the present invention comprises a compound of the present invention or a pharmaceutically acceptable salt thereof within the safe and effective amount range, and pharmaceutically acceptable excipients or carriers. The "safe and effective amount" refers to the amount of compound that is sufficient to significantly improve the condition without causing serious side effects. Typically, the pharmaceutical composition contains 1-2000mg compound of the present invention/formulation, and more preferably, 1-1000mg compound of the present invention/formulation. Preferably, the "one dose" is a capsule or tablet.

"Pharmaceutically acceptable carrier" refers to one or more compatible solid or liquid fillers or gel substances, which are suitable for human use and must have sufficient purity and sufficiently low toxicity. "Compatibility" herein refers to the ability of components of a composition to blend with each other and with the compounds of the invention without significantly reducing the efficacy of the compounds. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, Magnesium stearate), calcium sulfate, vegetable oil (such as soybean oil, sesame oil, peanut oil, olive oil, etc.), polyols (such as propylene glycol, glycerin, mannitol, sorbitol, etc.), emulsifiers(such as Tweens®), wetting agents (such as sodium dodecyl sulfate), colorants, flavoring agents, stabilizers, antioxidants, preservatives, non-thermal raw water, etc.

The mode of administration of the compounds or pharmaceutical compositions of the present invention are not particularly limited, and representative modes of administration include, but are not limited to, oral, intratumoral, rectal, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) filler or compatibilizer, such as starch, lactose, sucrose, glucose, Mannitol and silicic acid; (b) adhesives, such as hydroxymethyl cellulose, alginate, gelatin, polyvinylpyrrolidone, sucrose and gum arabic; (c) moisturizing agents, such as glycerol; (d) disintegrating agents, such as agar, calcium carbonate, potato starch or tapioca, algic acid, some complex silicates, and sodium carbonate; I, mitigation agent, such as paraffin; (f) absorption accelerators, such as quaternary amine compounds; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. In capsules, tablets and pills, dosage forms may also contain buffers.

Solid dosage forms such as tablets, sugar pills, capsules, pills and granules may be prepared using coating and shell materials such as casing and other materials well known in the art. They may comprise an opacifying agent, and the release of the active compound in such a composition or compound may be released in a delayed manner in a part of the digestive tract. Examples of embedding components that can be employed are polymeric substances and wax substances.If necessary, the active compound may also form a microcapsule form with one or more of the excipients described above.

Liquid dosage forms for oral administration include pharmaceutically acceptable emulsions, solutions, suspensions, syrups or tinctures.In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil,

peanut oil, corn germ oil, olive oil, castor oil and sesame oil, or mixtures thereof.

In addition to these inert diluents, the composition may also contain auxiliaries such as wetting agents, emulsifiers, suspending agents, sweeteners, flavoring agents and flavors.

In addition to the active compound, the suspension may comprise suspending agents, such as ethoxylated isooctadecanol, polyoxyethylene sorbitol and dehydrated sorbitol esters, microcrystalline cellulose, methanolic aluminum, agar, and any mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

Dosage forms of the compound of the invention for topical administration include ointments, powder, patches, propellants and inhalants.The active ingredient is mixed under sterile conditions with physiologically acceptable carriers and any preservatives, buffers or propellants as may be required.

The treatment method of the present invention can be administered alone or in combination with other treatment methods or drugs.

When using the pharmaceutical composition, a safe and effective amount of the compound of the present invention is administered to mammals in need of treatment (such as humans and rats), where the dosage at the time of administration is the pharmaceutically considered effective dose, which is typically 1 to 2,000 mg per day, more preferably 50 to 1000mg per day for human of 60 kg body weight. Of course, the specific dosage should also consider the route of administration, the patient's health condition and other factors, which are within the skill range of skilled doctors.

The present invention also provides a method for preparing a pharmaceutical composition comprising the step of: mixing pharmaceutically acceptable carriers with a compound of formula I of the present invention, or a crystalline form thereof, a pharmaceutically acceptable salt, a hydrate, or a solvate thereof, thereby forming a pharmaceutical composition.

The present invention also provides a method of treatment comprising the step of: administering to a subject in need of treatment a compound of formula I of the present invention, or a crystalline form thereof, a pharmaceutically acceptable salt, hydrate, or a solvate thereof, or administering a pharmaceutical composition of the present invention, thereby inhibiting EGFR.

Compared to the prior art, the present invention has the following main advantages:

(1) The compound of the present invention have excellent inhibitory ability against one or more activating or resistant mutations of EGFR; e.g., against three specific EGFR mutation types: H1975 (T790M/L858R); HCC827 (19DEL); PC-9 (19DEL).

(2) The compound of the present invention can be used in the treatment of EGFR-sensitive mutant cancers;

(3) The compound of the present invention may be applicable to cases of secondary resistance arising from current EGFR therapy;

(4) The compound of the present invention have excellent degradation properties for EGFR proteins. For both H1975 and HCC827, more than 80% degradation can be achieved in specific advantageous examples of the present invention.

The present invention is further described below in conjunction with specific embodiments. It is to be understood that these examples are intended to illustrate the invention only and not to limit the scope of the invention. The experimental methods that do not indicate specific conditions in the following examples usually follow the conventional conditions, such as those described in Sambrook et al., Molecular Cloning: A Laboratory Manual (New York: Cold Spring Harbor Laboratory Press, 1989), or the conditions suggested by the manufacturer. Unless otherwise specified, percentages and parts are calculated by weight.

Unless otherwise defined, all professional and scientific terms used herein have the same meanings as commonly understood by those skilled in the art. In addition, any methods and materials similar or equivalent to those described can be applied to the method of the present invention. The preferred embodiments and materials described herein are for exemplary purposes only.

**Example 1**

The compound synthesized in the present invention:

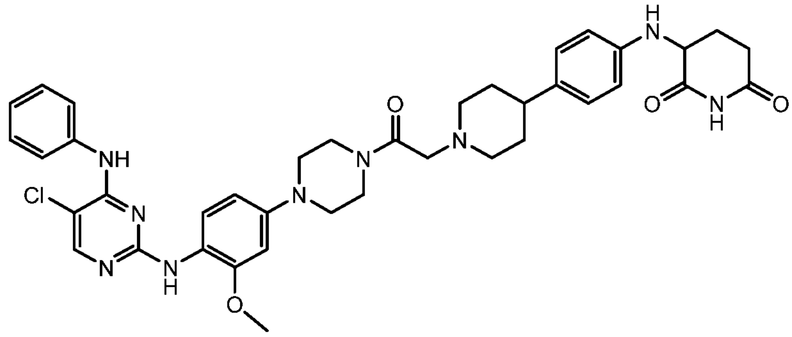

T-01

The experimental procedure is as follows:

T. Synthesis of intermediate M1

The synthesis route is as follows:

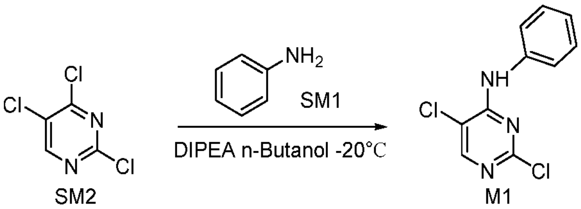


Synthesis of intermediate M1

M1:To a 100 ml single-necked flask was added 7.8 mmol of compound SM2, 3 ml of diisopropylethylamine and 30 ml of n-butanol to give a mixture; the mixture was cooled down to -20°C by a cold bath, and compound SM1 (13.8 mmol) was added dropwise, and the reaction was carried out at low temperature for 1 hour. Then the cold bath was removed and the reaction was warmed up to room temperature and stirred overnight. Then the solvent was evaporated to dryness under reduced pressure; the residue was added to 100ml of ethyl acetate (EA), and washed twice with 50 ml of water; the organic phase was evaporated to dryness, and the residue was separated by column chromatography (eluent was ethyl acetate:petroleum ether=1:30 (v/v)) to giveed the intermediate M1.

II. Synthesis of intermediate M2

The synthesis route is as follows:

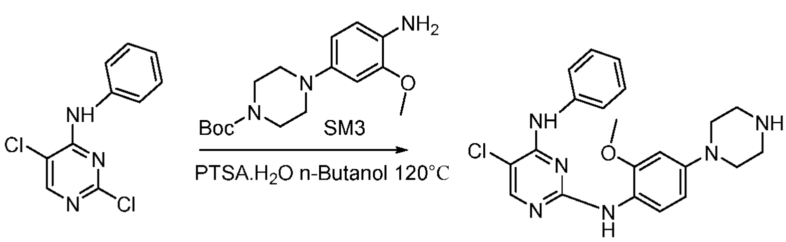


700 mg of M1, 663 mg of SM3, and 663 mg of p-toluenesulfonic acid monohydrate were dissolved in 3 ml of n-butanol in a 100 ml three-necked flask, then the mixture was heated to 110°C and reacted overnight. The reaction was monitored by TLC unitl the reaction was complete. The solvent was then removed by rotary evaporation, and the residue was added with water, extracted with ethyl acetate, dried with anhydrous sodium sulfate, spun-dried and subjected to column chromatography to give 800 mg of the intermediate M2. LC-MS [M+1]: 441.

III. Synthesis of intermediate SM4

The synthesis route is as follows:

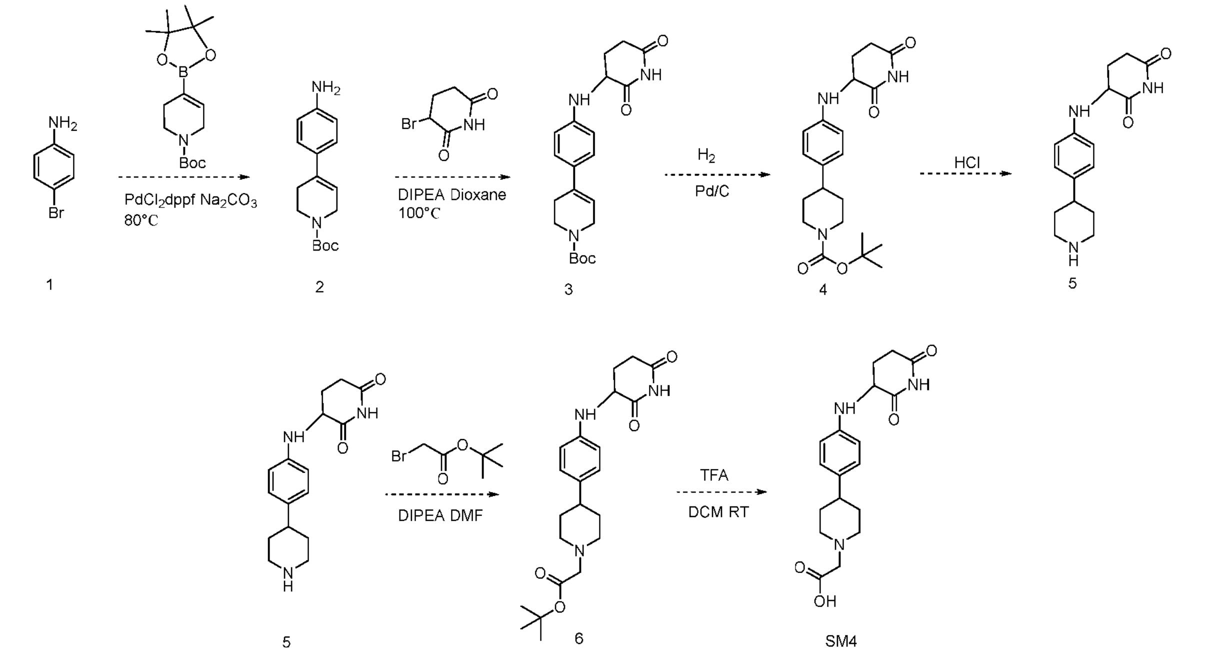


T. Synthesis of compound 2

5 g of compound 1 and 10.8 g n-Boc-1,2,5,6-tetrahydropyridin-4-boronic acid pinacol ester, 60 ml of tetrahydrofuran, 24 ml of methanol, 12 ml of water, 6.8 g of sodium carbonate, and 0.43 g of PdCl2dppf were mixed homogeneously in a 250ml three-necked flask and purged with nitrogen. The reaction was then heated to 80 °C and reacted overnight. The reaction was monitored by TLC until the reaction was complete. Then the reaction was extracted with ethyl acetate, and the organic phase was dried with anhydrous sodium sulfate, spun-dried and separated by column chromatography to give 5.8 g of compound 2. MS[M+1]:275

2. Synthesis of compound 3

5.1 g of compound 2, 7.1 g of 3-bromopiperidin-2,6-dione, and 7.2 g of N,N-diisopropylethylamine were dissolved in 51 ml of 1,4-dioxane in a 100 ml three-necked flask, and then the reaction was warmed up to 100 °C and reacted overnight. The reaction was monitored by TLC until the reaction was complete. The reaction was then extracted with dichloromethane, and the organic phase was dried with anhydrous sodium sulfate, spun-dried and subjected to column chromatography to give 3.6 g of compound 3.LC-MS [M+1]: 386.

3. Synthesis of compound 4

To an autoclave reactor was added 3.6 g of compound 2, 0.72 g of palladium carbon, and 36 ml of anhydrous ethanol, and hydrogen pressure was adjusted to 0.7 MPa and then the reaction was reacted for two days. The reaction solution was leached, and the solvent was removed by rotary evaporation to give 1.2 g of compound 4. LC-MS [M+1]: 388.

4. Synthesis of compound 5

To a 100 ml single-necked flask was added 1.2 g of compound 4, and 30 ml of methanol was added to dissolve it, then 4 M solution of dioxane hydrochloride was added dropwise under a cooling ice-water bath. After dropwise addition, the reaction was carried out at 40 °C for 2 h. The solvent was then removed by rotary evaporation to give 1.4 g of compound 5.LC-MS [M+1]: 288.

5. Synthesis of compound 6

To a 100 ml single-necked flask was added 0.8 g of compound 5, 8 ml of N,N-dimethylformamide, N,N-diisopropylethylamine, tert-butyl bromoacetate and stirred at room temperature overnight. TLC detected the completion of the reaction. The reaction solution was then extracted with ethyl acetate and water, and the organic phase was dried, filtered, and subjected to column chromatography to give 300 mg of compound 6, LC-MS [M+1]: 402.

6. Synthesis of compound SM4

To a 100 ml single-necked flask was added 100 mg of compound 6, 1 ml of dichloromethane, and 1.6 ml of trifluoroacetic acid dropwise and stirred at room temperature overnight. TLC detected the completion of the reaction, and the solvent was removed by rotary evaporation to give 150 mg of SM4, LC-MS [M-1]: 344.

IV. Synthesis of compound T-01

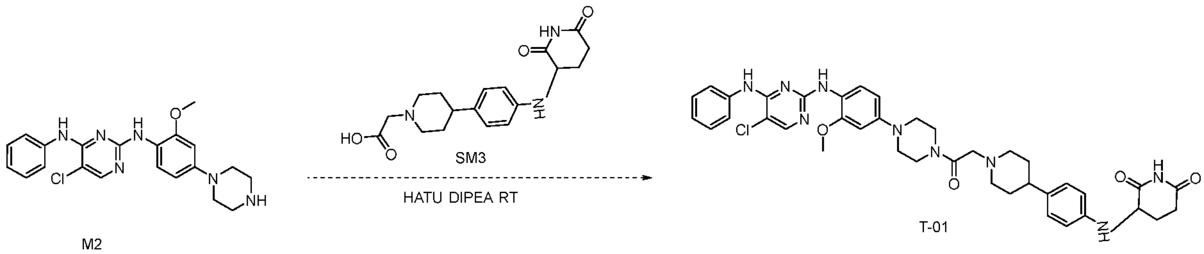


M2 SM3 T-01

The synthesis route is as follows:
Compounds M2 and SM3 were dissolved in 2ml of N,N-dimethylformamide in a 50 ml round-bottom flask and cooled down to 0°C under an ice-water bath, and then N,N-diisopropylethylamine and HATU were added under stirring. The reaction was carried out for 1 h in an ice-water bath under nitrogen protection, and then naturally warmed up to room temperature. TCL detected the completion of the reaction. The reaction was then extracted with ethyl acetate and water, and the organic phase was dried, filtered, and subjected to column chromatography to give 100 mg of compound T-01, LC-MS [M+1]: 739.

The following compounds were synthesized with reference to the method of Example 1:

| Compound No. | Structure | Characterization data: |
|---|---|---|
| T-02 | 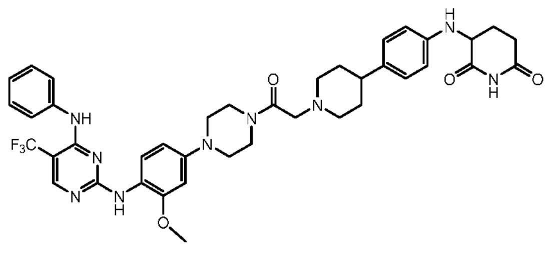 T-02 | LC-MS[M+1]:772 |
| T-03 | 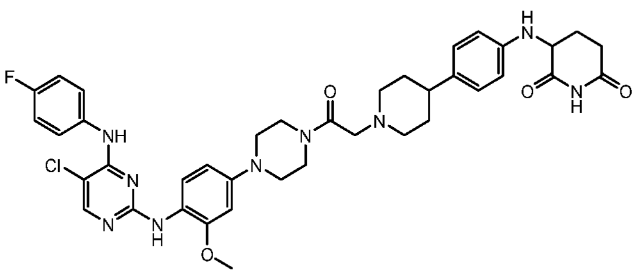 T-03 | LC-MS[M+1]:757 |

(continued)

| Compound No. | Structure | Characterization data: |
|---|---|---|
| T-04 | 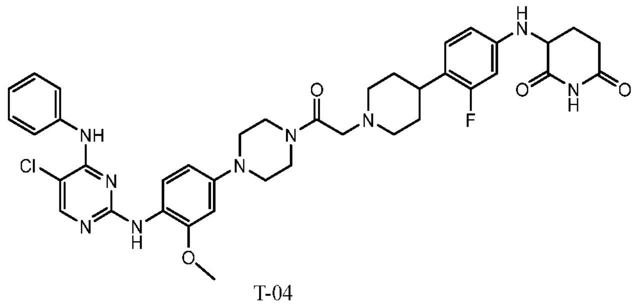 T-04 | LC-MS[M+1]:757 |
| T-08 | 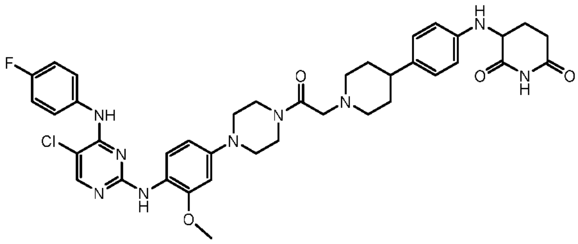 T-08 | LC-MS[M+1]:757 |
| T-09 | 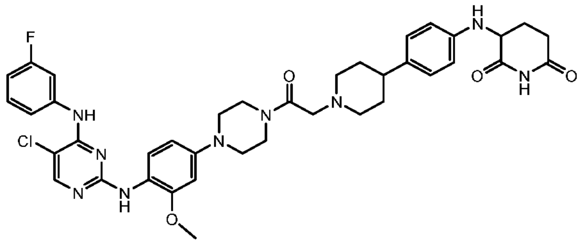 T-09 | LC-MS[M+1]:757 |
| T-10 | 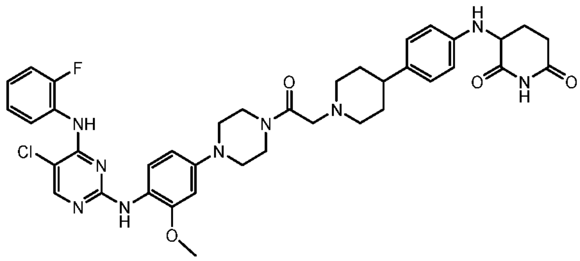 T-10 | LC-MS[M+1]:757 |
| T-11 | 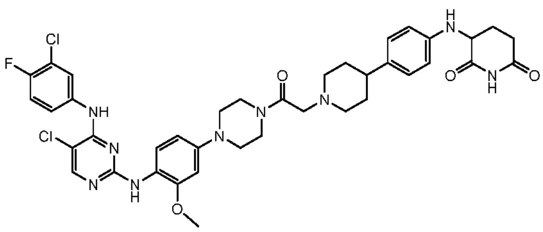 T-11 | LC-MS[M+1]:792 |

(continued)

| Compound No. | Structure | Characterization data: |
|---|---|---|
| T-12 | 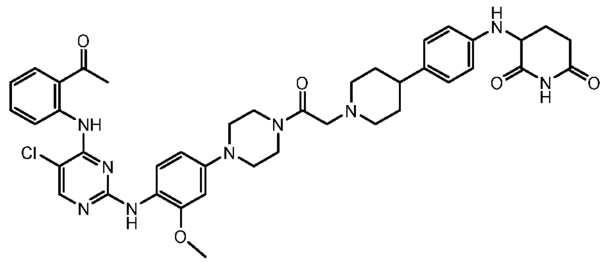 T-12 | LC-MS[M+1]:784 |
| T-13 | 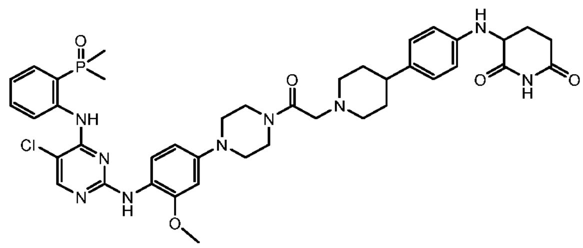 T-13 | LC-MS[M+1]:815 |
| T-46 | 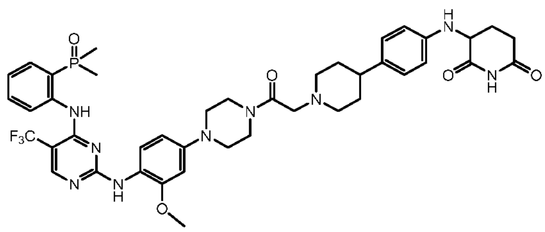 T-46 | LC-MS[M+1]:849 |
| T-47 | 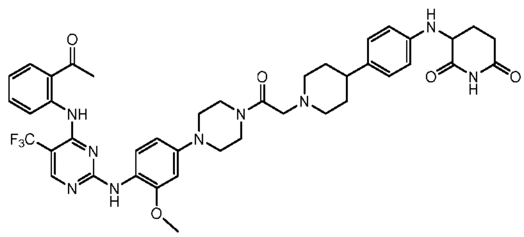 | LC-MS[M+1]:814 |
| T-48 | 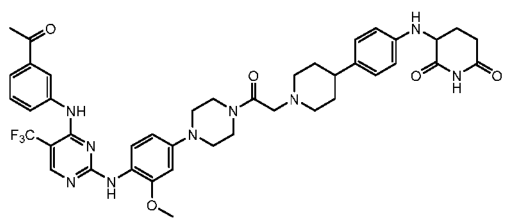 | LC-MS[M+1]:814 |

(continued)

| Compound No. | Structure | Characterization data: |
|---|---|---|
| T-49 | 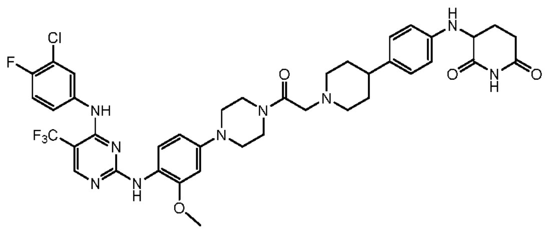 T-49 | LC-MS[M+1]:825 |
| T-51 | 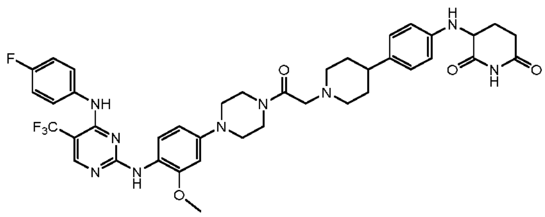 | LC-MS[M+1]:790 |
| T-52 | 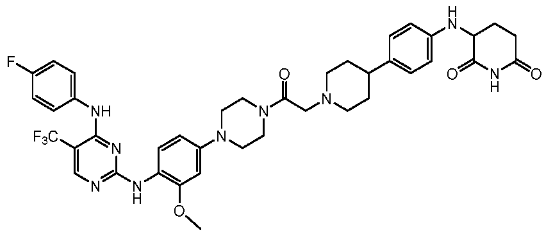 | LC-MS[M+1]:790 |
| T-82 | 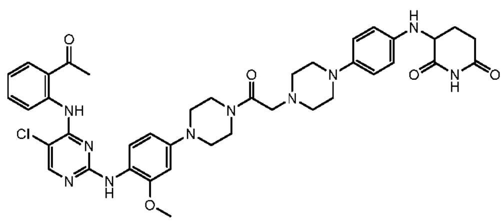 | LC-MS[M+1]:782 |
| T-84 | 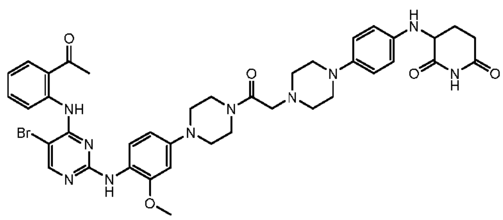 | LC-MS[M+1]:826 |
| T-131 | 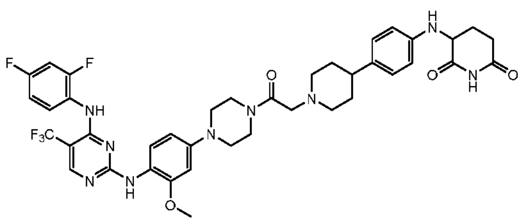 | LC-MS[M+1]:809 |

(continued)

| Compound No. | Structure | Characterization data: |
|---|---|---|
| T-132 | 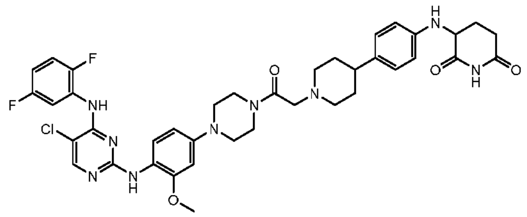 | LC-MS[M+1]:775 |
| T-133 | 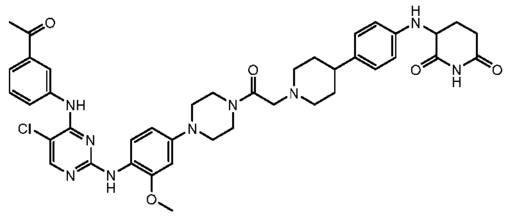 | LC-MS[M+1]:781 |
| T-134 | 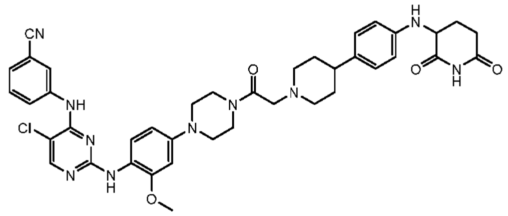 | LC-MS[M+1]:764 |
| T-135 | 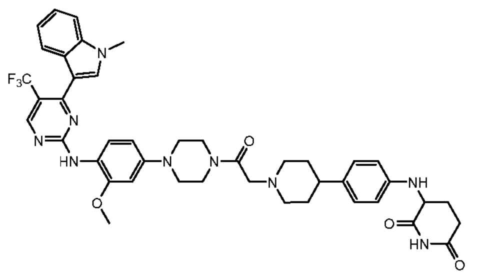 | LC-MS[M+1]:811 |
| T-136 | 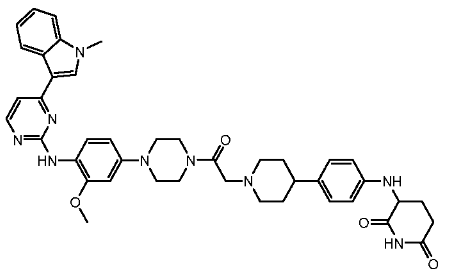 | LC-MS[M+1]:743 |
| T-137 | 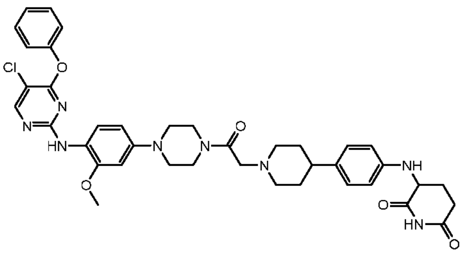 | LC-MS[M+1]:740 |

(continued)

| Compound No. | Structure | Characterization data: |
|---|---|---|
| T-138 | 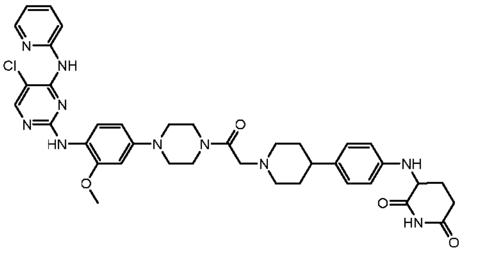 | LC-MS[M+1]:741 |
| T-139 | 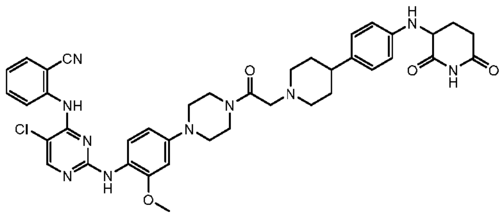 | LC-MS[M+1]:764 |
| T-181 | 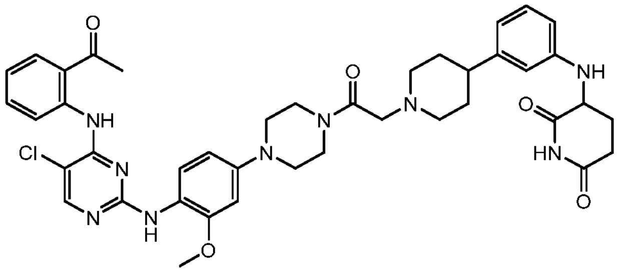 | LC-MS[M+1]:781 |
| T-187 | 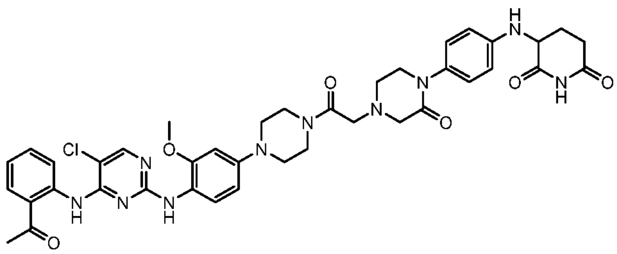 | LC-MS[M+1]:796 |
| T-190 | 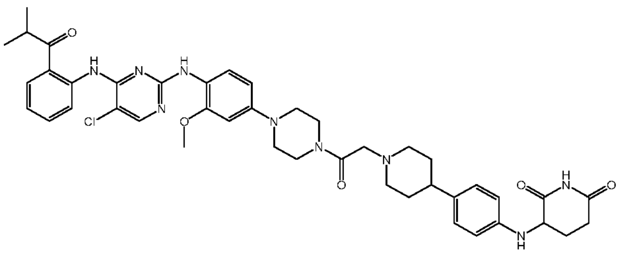 | LC-MS[M+1]:809 |
| T-193 | 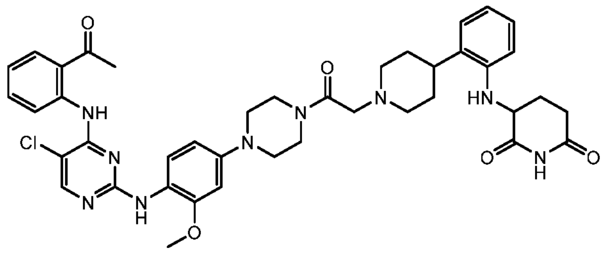 | LC-MS[M+1]:781 |

(continued)

| Compound No. | Structure | Characterization data: |
|---|---|---|
| T-194 | 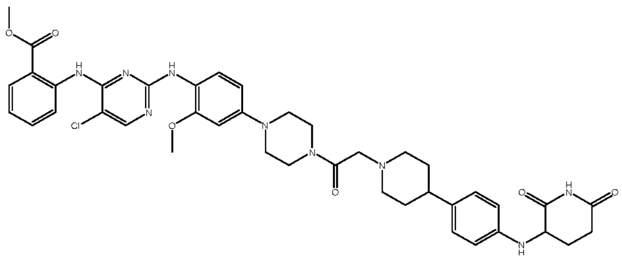 | LC-MS[M+1]:797 |
| T-200 | 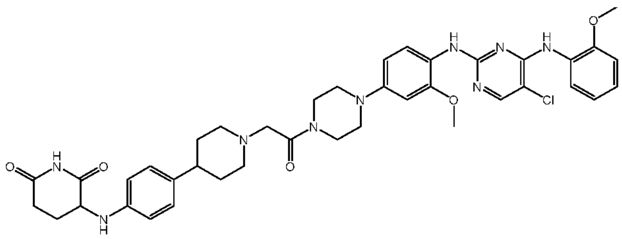 | LC-MS[M+1]:769 |
| T-338 | 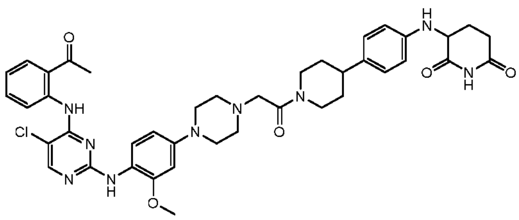 | LC-MS[M+1]:781 |
| T-346 | 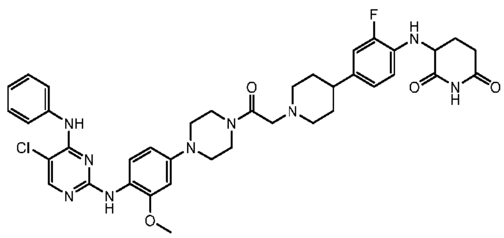 | LC-MS[M+1]:756 |
| T-347 | 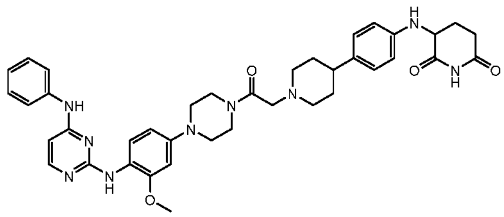 | LC-MS[M+1]:704 |
| T-348 | 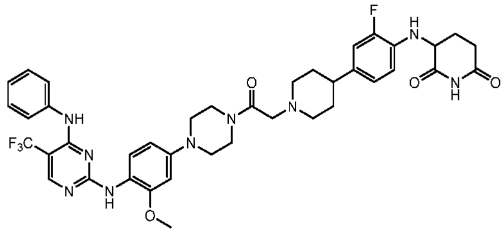 | LC-MS[M+1]:790 |

(continued)

| Compound No. | Structure | Characterization data: |
|---|---|---|
| T-349 | 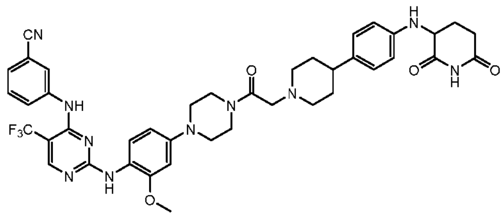 | LC-MS[M+1]:797 |
| T-350 | 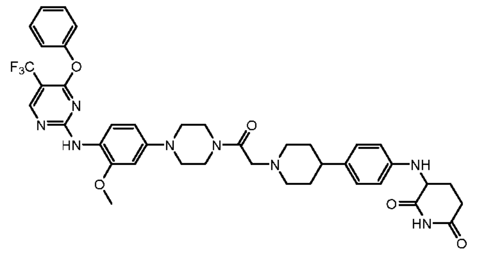 | LC-MS[M+1]:773 |

**Example 2**

The compound synthesized in the present invention:

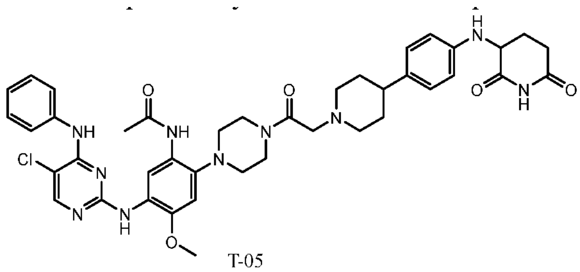

T-05

The experimental procedure is as follows:

T. Synthesis of intermediate M1

The synthesis route is as follows:

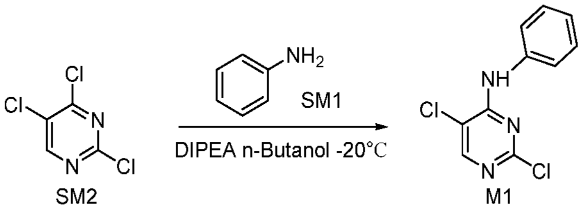


Intermediate M1 was synthesized with reference to the method of Example 1

II. Synthesis of intermediate M2

The synthesis route is as follows:

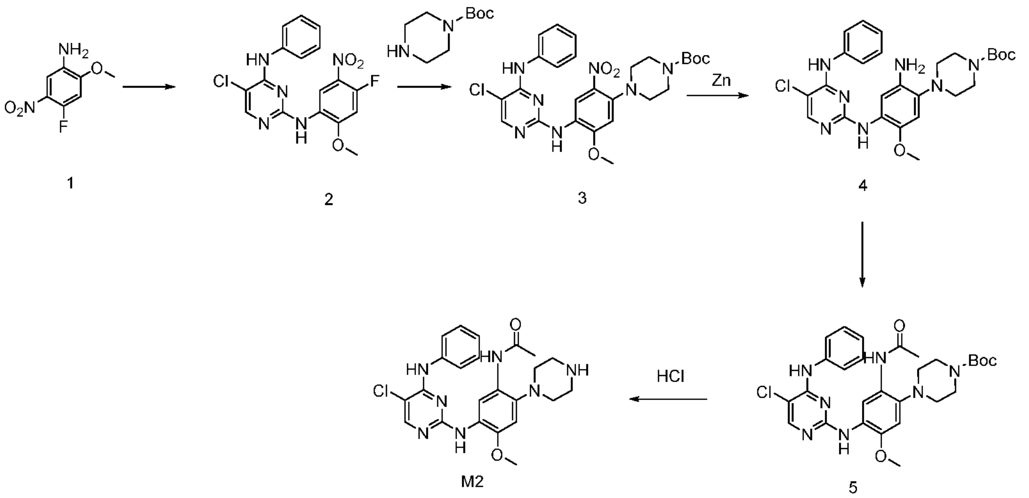


T. Synthesis of compound 2

500mg of M1, 465mg of 4-fluoro-2-methoxy-5-nitroaniline, and 475mg of p-toluenesulfonic acid monohydrate were dissolved in 3ml of n-butanol in a 100 ml single-necked flask, then heated to 110°C and reacted overnigh. TLC monitored the reaction until complete. The solvent was then removed by rotary evaporation and the residue was added water, extracted with ethyl acetate, dried with anhydrous sodium sulphate, spun-dried, and subjected to column chromatography to give 750 mg of compound 2.LC-MS [M+1]: 391.

2. Synthesis of compound 3

700 mg of compound 2, 667 mg of N-boc piperazine, and 694 mg of DIPEA were dissolved in 10.5 ml of DMAC in a 100 ml single-necked flask, warmed to 110 °C and reacted for 18 h. The solvent was then removed by rotary evaporation, and the residue was added with water, extracted with ethyl acetate, dried with anhydrous sodium sulfate, spun-dried, and subjected to column chromatography to give 1.1 g of compound 3. LC-MS [M+1]: 557.

3. Synthesis of compound 4

1.0 g of compound 3, 950 mg of ammonium chloride, and 700 mg of zinc powder were dispersed in 6 ml solvent of ethanol: water 5:1 in a 100 ml single-necked flask, heated to refluxing and reacted for 18h. The solvent was then removed by rotary evaporation, and the residue was added with water and the pH was adjusted to 7-8 with a small amount of saturated aqueous sodium bicarbonate. Then the mixture was extracted with ethyl acetate, dried with anhydrous sodium sulfate, spun-dried, and subjected to column chromatography to give 900 mg compound 4 as brown solid.LC-MS [M+1]: 527.

4. Synthesis of compound 5

200 mg of compound 4 and 0.15 ml of triethylamine were dissolved in 1.6 ml of tetrahydrofuran in a 50 ml single-necked flask, cooled to 0°C in an ice-water bath. the reaction solution was slowly added dropwise with a diluent of 46.5 mg of acetic anhydride in 0.4 ml of tetrahydrofuran, and then reacted at room temperature for 2 h. TLC detected disappearance of the raw material. The reaction was then terminated, and the reaction solution was spun-dried to give the compound 5.LC-MS [M+1]: 568.

5. Synthesis of intermediate M2

300 mg of compound 5 was dissolved in 2 ml of tetrahydrofuran in a 50 ml single-necked flask, and the reaction solution was cooled to 0 °C in an ice-water bath, and slowly added dropwise with 1ml of 4 M HCl/dioxane, and then reacted at room temperature for 2 h. TLC detected disappearance of the raw material. The reaction was then terminated, and the reaction solution was spun-dried to give M2.LC-MS [M+1]: 468.

III. Synthesis of compound T-05

The synthesis route is as follows:

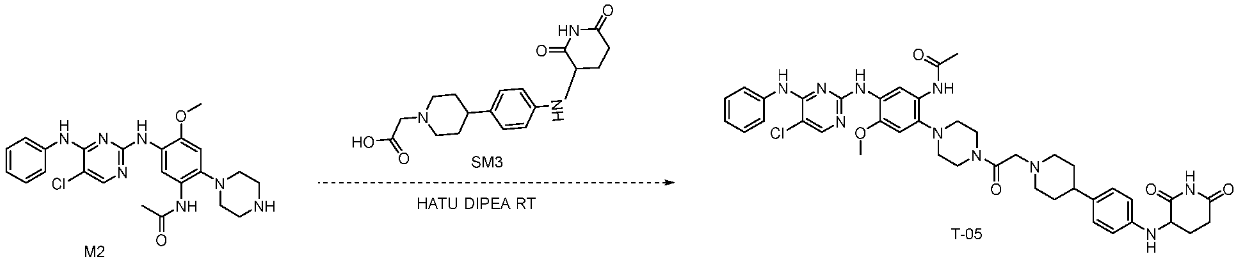

M2
T-05

Compounds M2 and SM3 were dissolved in 2ml of N,N-dimethylformamide in a 50 ml round-bottom flask and cooled down to 0°C in an ice-water bath. Then N,N-diisopropylethylamine and HATU were added under stirring, and the reaction was carried out for 1 h in an ice-water bath under nitrogen protection, then naturally warmed up to room temperature. TCL detected the completion of the reaction. The reaction solution was then extracted with ethyl acetate and water, and the organic phase was dried, filtered, and subjected to column chromatography to give 100 mg of compound T-05, LC-MS [M+1]: 796.

The following compounds were synthesized with reference to the method of Example 2:

| Compound No. | Structure | Characterization data: |
|---|---|---|
| T-05 | 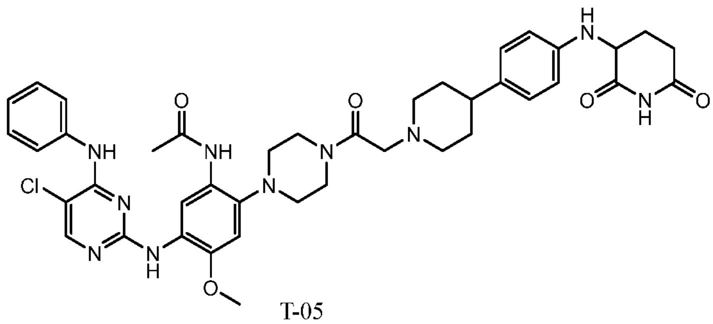 T-05 | LC-MS[M+1]:796 |
| T-06 | 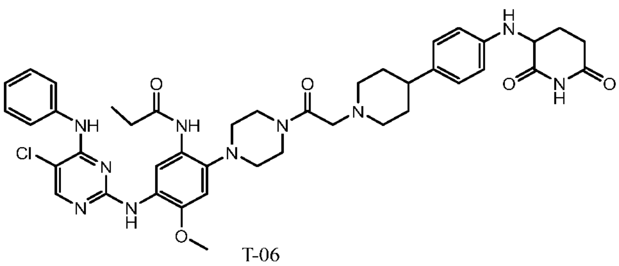 T-06 | LC-MS[M+1]:810 |
| T-55 | 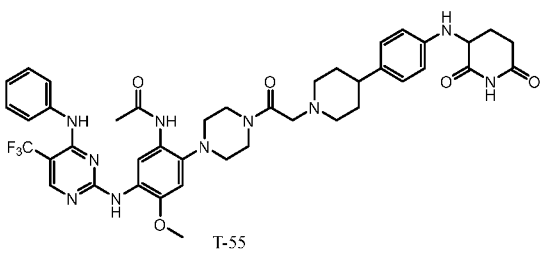 T-55 | LC-MS[M+1]:833 |

| | | |
|---|---|---|
| T-91 | 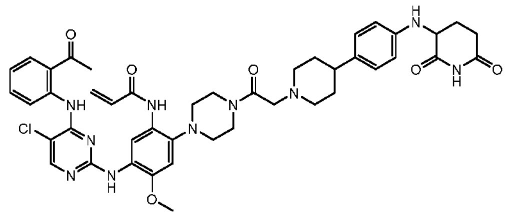 | LC-MS[M+1]:850 |
| T-180 | 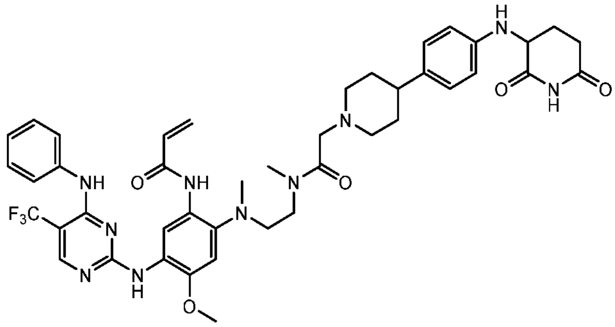 | LC-MS[M+1]:844 |
| T-335 | 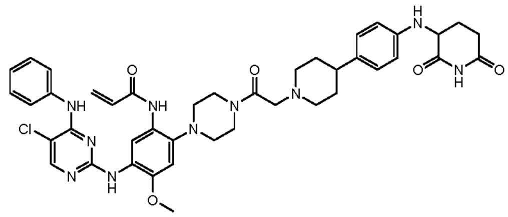 | LC-MS[M+1]:808 |
| T-339 | 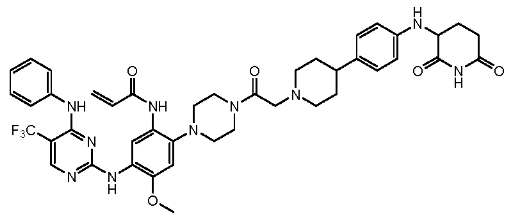 | LC-MS[M+1]:842 |

**Example** 3

The compound synthesized in the present invention:

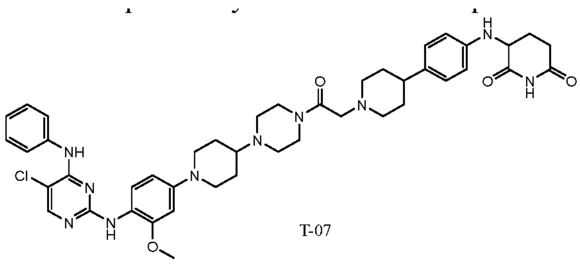

T-07

The experimental procedure is as follows:

T. Synthesis of intermediate M1

The synthesis route is as follows:

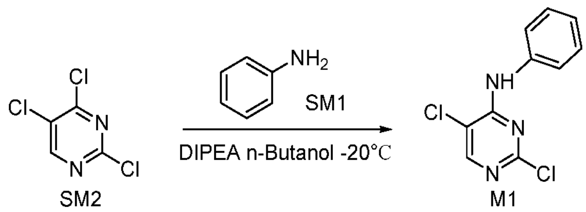


Intermediate M1 was synthesized with reference to the method of Example 1

II. Synthesis of intermediate M2

The synthesis route is as follows:

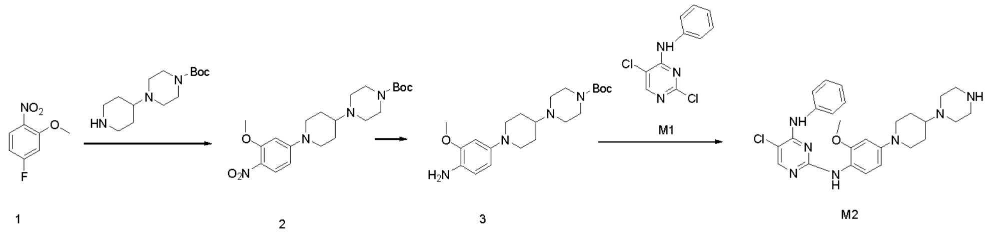


T. Synthesis of compound 2

5 g of 5-fluoro-2-nitroanisole, 11.8 g of 1-Boc-4-(piperidin-4-yl)-piperazine, and 11.3 g of dipea were dissolved in 50 ml of DMAC in a 250 ml three-necked flask, then heated to 110 °C and reacted overnight. TLC monitored the reaction until the reaction was complete. The reaction was then added with water, extracted with ethyl acetate, dried with anhydrous sodium sulphate, spun-dried and subjected to column chromatography to give 11 g of Compound 2.LC-MS [M+1]: 421.

2. Synthesis of compound 3

1.0 g of compound 3, 1.26g of ammonium chloride, and 800mg of zinc powder were dispersed in 6 ml solvent of ethanol: water 5:1 in a 100 ml single-necked flask, then heated to reflux and reacted for 18h. TLC showed disappearance of the raw material. Solvent was then removed by rotary evaporation, and the residue was added with water and the pH was adjusted to 7-8 with a small amount of saturated aqueous sodium bicarbonate. The reaction solution was extracted with ethyl acetate, dried with anhydrous sodium sulfate, spun-dried, and subjected to column chromatography to give 900 mg compound 3 as brown solid.LC-MS [M+1]: 391.

3. Synthesis of intermediate M2

500 mg of M1, 300 mg of compound 3 and 292 mg of p-toluenesulfonic acid monohydrate were dissolved in 5 ml of n-butanol in a 10 ml microwave tube, and then microwaved at 120°C for 20 min. TLC monitored the reaction until the reaction was complete. Solvent was then removed by rotary evaporation, and the residue was added with water, extracted with ethyl acetate, dried with anhydrous sodium sulfate, spun-dried and subjected to column chromatography to give 350 mg of the intermediate M2. LC-MS [M+1]: 496.

III. Synthesis of compound T-07

The synthesis route is as follows:

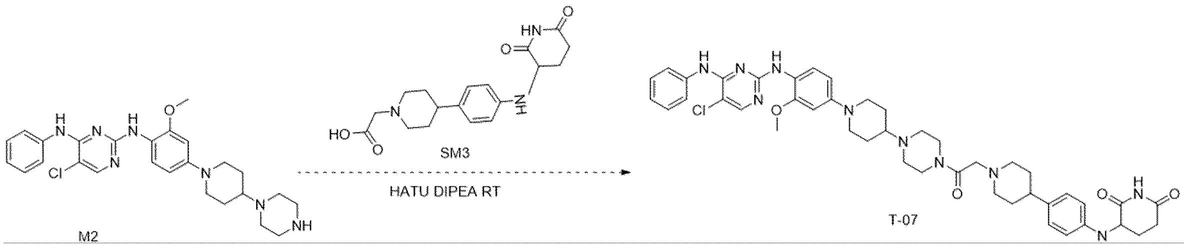


Compounds M2 and SM3 were dissolved in 2ml of N,N-dimethylformamide in a 50 ml round-bottom flask and cooled down to 0°C in an ice-water bath, and then N,N-diisopropylethylamine and HATU were added under stirring. the reaction was carried out for 1 h in an ice-water bath under nitrogen protection, and then naturally warmed up to room temperature. TCL detected the completion of the reaction. The reaction solution was then extracted with ethyl acetate and water, and the organic phase was dried, filtered, and subjected to column chromatography to give 50mg of compound T-07, LC-MS [M+1]: 822.

The following compounds were synthesized with reference to the method of Example 3:

| Compound No. | Structure | Characterization data: |
|---|---|---|
| T-07 | 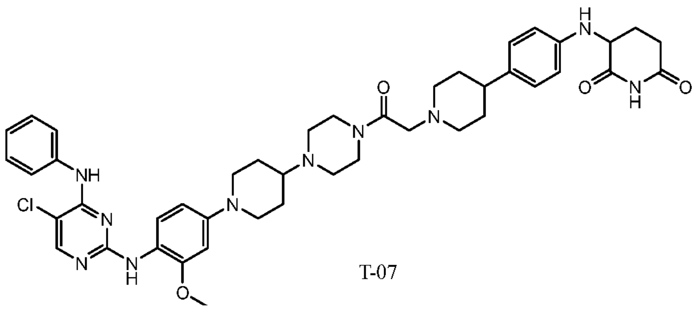 T-07 | LC-MS[M+1]:822 |
| T-53 | 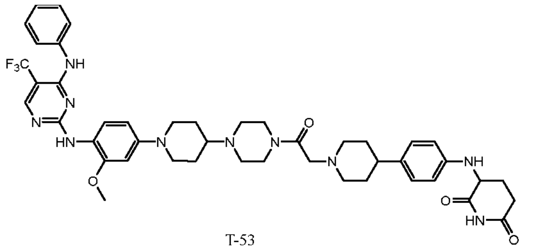 T-53 | LC-MS[M+1]:859 |
| T-57 | 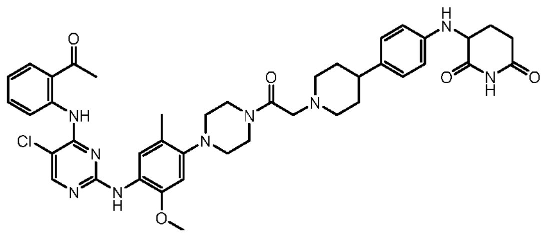 | LC-MS[M+1]:795 |
| T-58 | 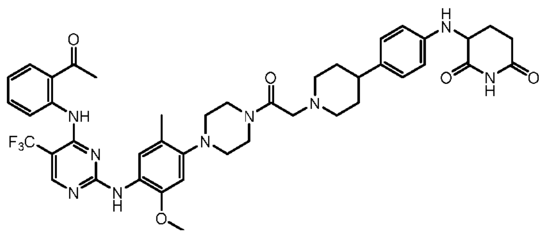 | LC-MS[M+1]:828 |

(continued)

| Compound No. | Structure | Characterization data: |
|---|---|---|
| T-59 | 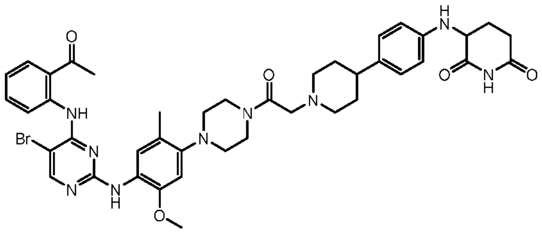 | LC-MS[M+1]:838 |
| T-60 | 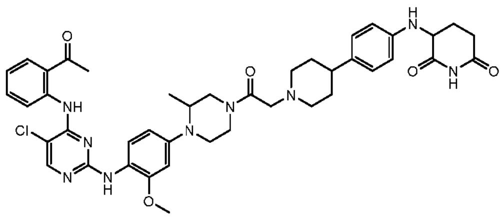 | LC-MS[M+1]:795 |
| T-63 | 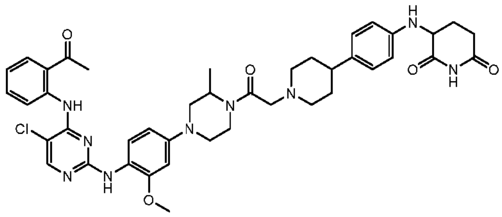 | LC-MS[M+1]:795 |
| T-121 | 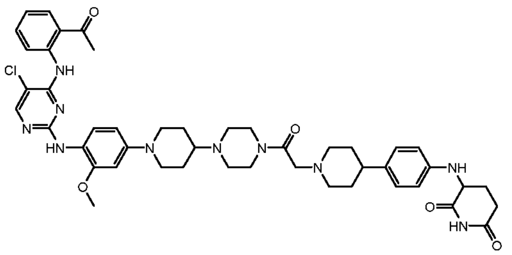 | LC-MS[M+1]:864 |
| T-123 | 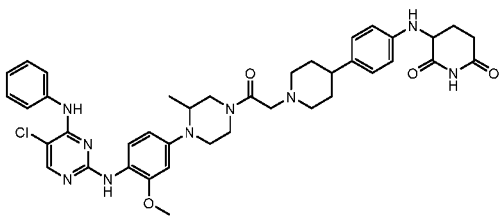 | LC-MS[M+1]:753 |
| T-124 | 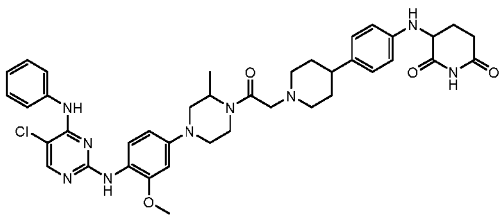 | LC-MS[M+1]:753 |

(continued)

| Compound No. | Structure | Characterization data: |
|---|---|---|
| T-125 | 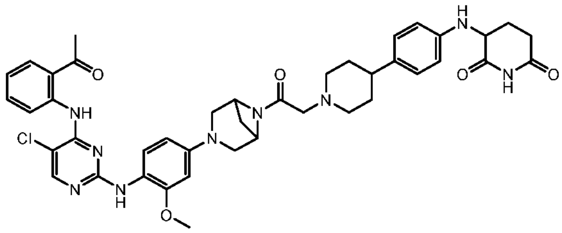 | LC-MS[M+1]:793 |
| T-127 | 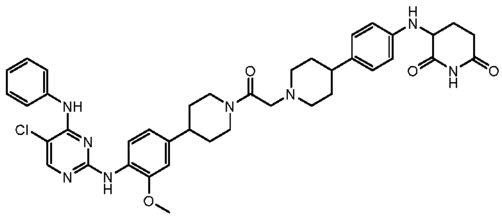 | LC-MS[M+1]:737 |
| T-165 | 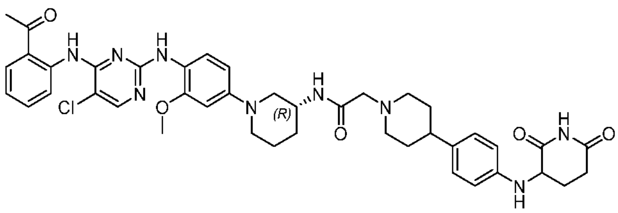 | LC-MS[M+1]:795 |
| T-166 | 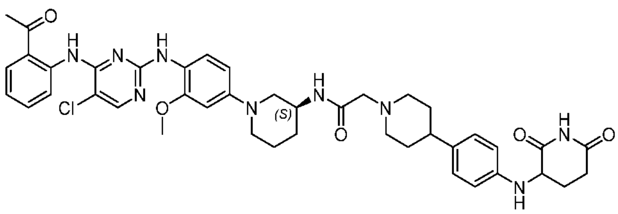 | LC-MS[M+1]:795 |
| T-167 | 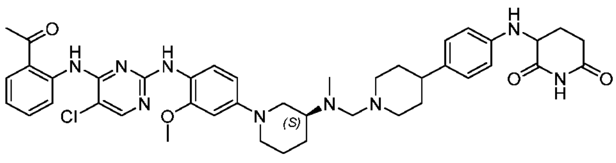 | LC-MS[M+1]:781 |
| T-168 | 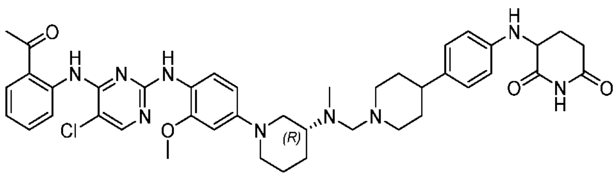 | LC-MS[M+1]:781 |

(continued)

| Compound No. | Structure | Characterization data: |
|---|---|---|
| T-169 | 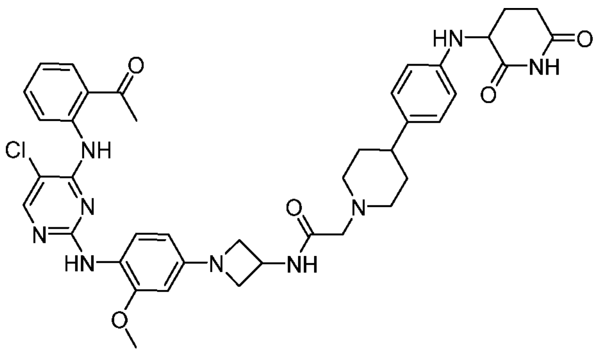 | LC-MS[M+1]:768 |
| T-170 | 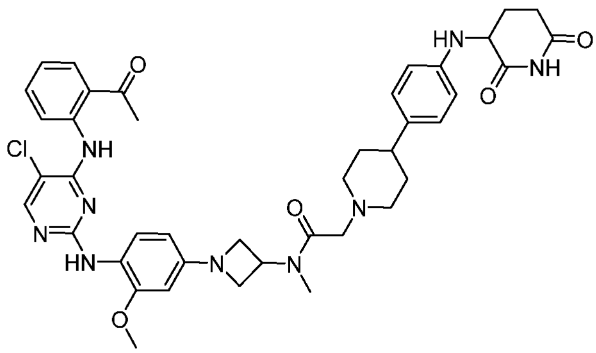 | LC-MS[M+1]:781 |
| T-171 | 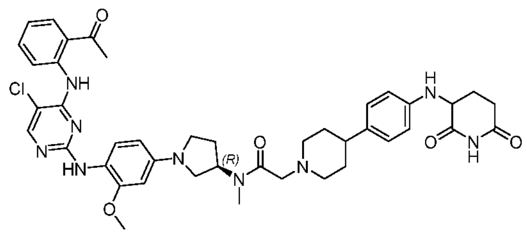 | LC-MS[M+1]:795 |
| T-172 | 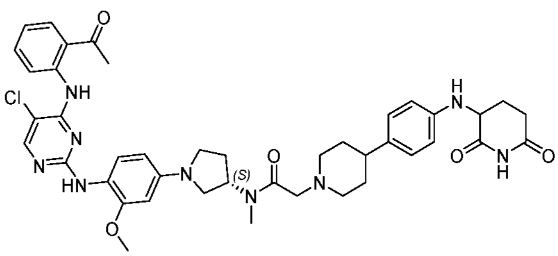 | LC-MS[M+1]:795 |
| T-173 | 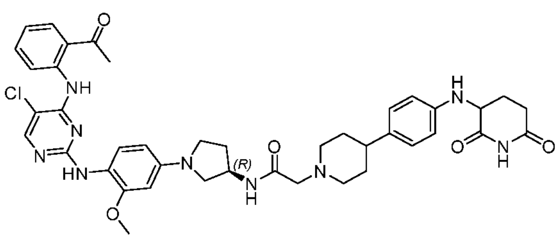 | LC-MS[M+1]:781 |

(continued)

| Compound No. | Structure | Characterization data: |
|---|---|---|
| T-174 | 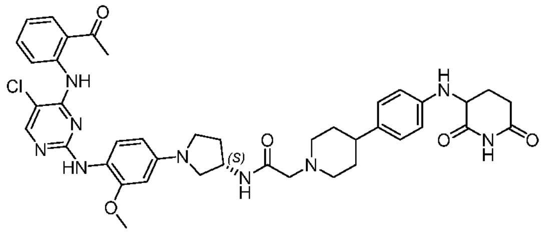 | LC-MS[M+1]:781 |
| T-175 | 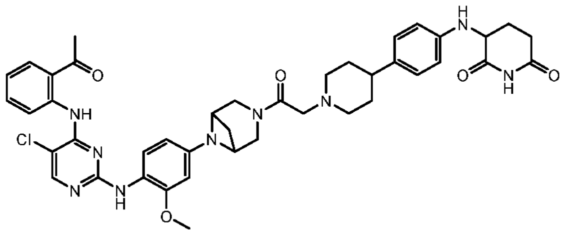 | LC-MS[M+1]:793 |
| T-176 | 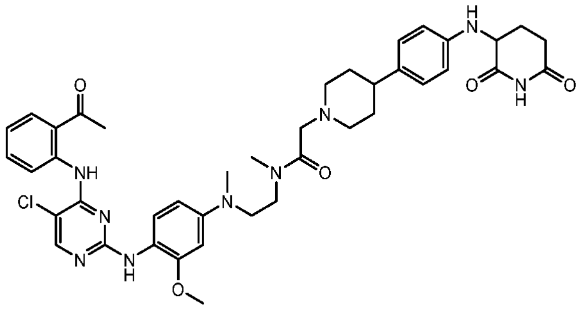 | LC-MS[M+1]:783 |
| T-177 | 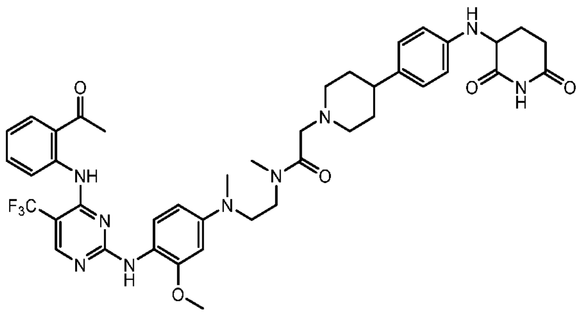 | LC-MS[M+1]:817 |
| T-179 | 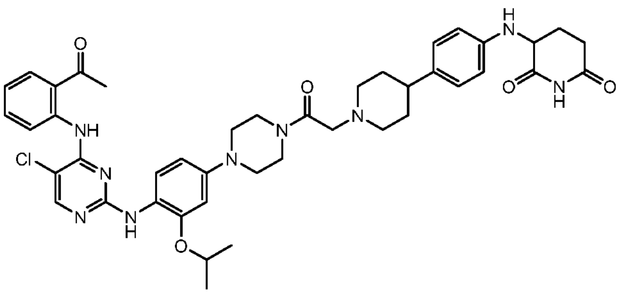 | LC-MS[M+1]:809 |

(continued)

| Compound No. | Structure | Characterization data: |
|---|---|---|
| T-188 | 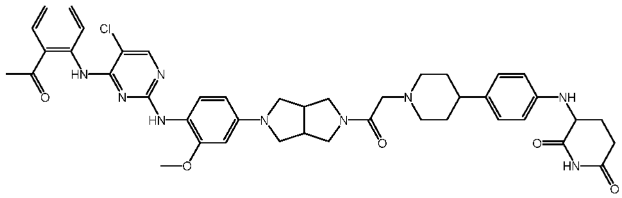 | LC-MS[M+1]:807 |
| T-189 | 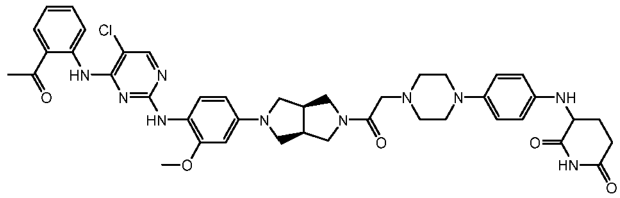 | LC-MS[M+1]:807 |
| T-191 | 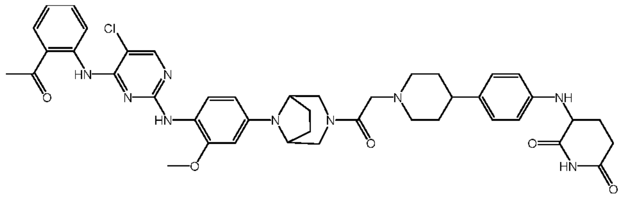 | LC-MS[M+1]:807 |
| T-192 | 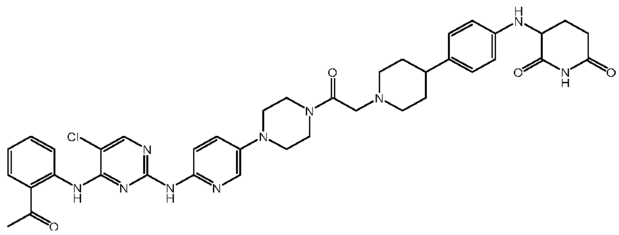 | LC-MS[M+1]:752 |
| T-203 | 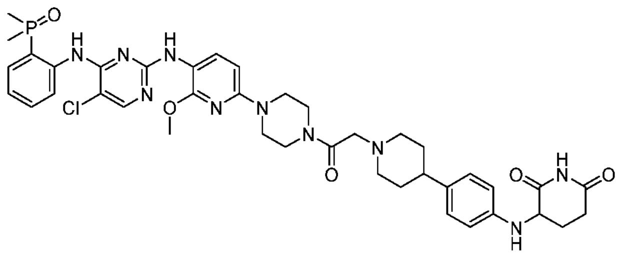 | LC-MS[M+1]:816 |
| T-208 | 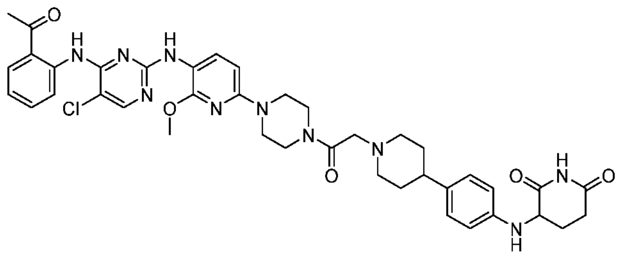 | LC-MS[M+1]:782 |
| T-214 | 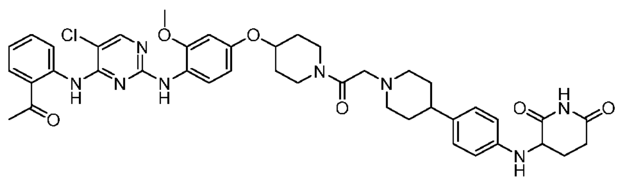 | LC-MS[M+1]:796 |

(continued)

| Compound No. | Structure | Characterization data: |
|---|---|---|
| T-224 | 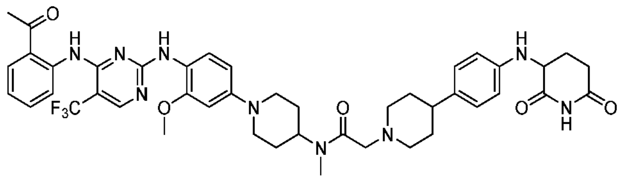 | LC-MS[M+1]:842 |
| T-239 | 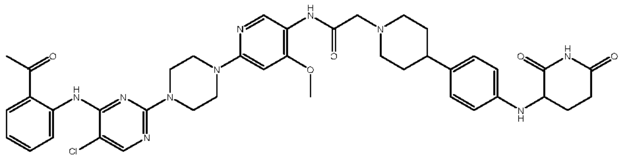 | LC-MS[M+1]:781 |
| T-244 | 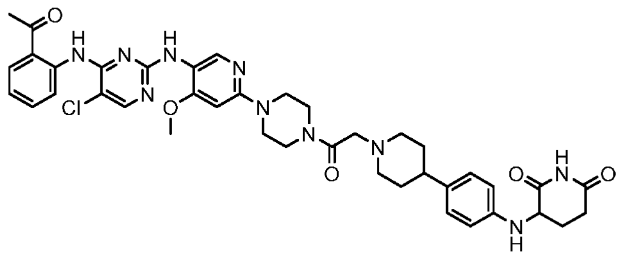 | LC-MS[M+1]:782 |
| T-258 | 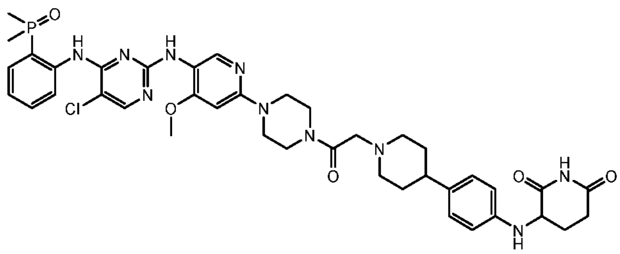 | LC-MS[M+1]:816 |
| T-267 | 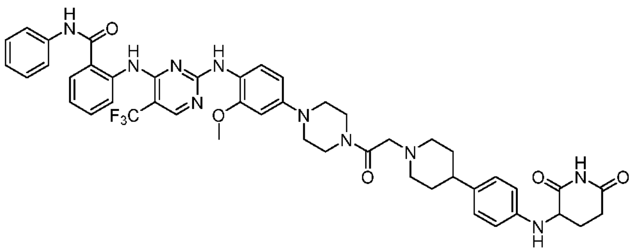 | LC-MS[M+1]:891 |
| T-327 | 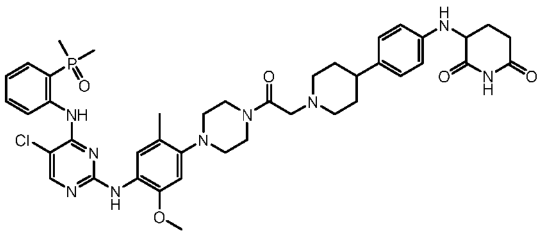 | LC-MS[M+1]:828 |
| T-328 | 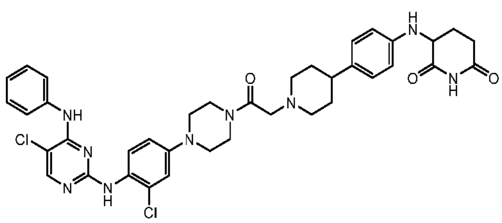 | LC-MS[M+1]:743 |

(continued)

| Compound No. | Structure | Characterization data: |
|---|---|---|
| T-330 | 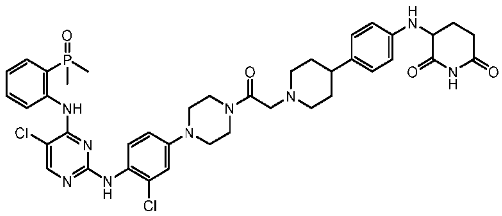 | LC-MS[M+1]:819 |
| T-331 | 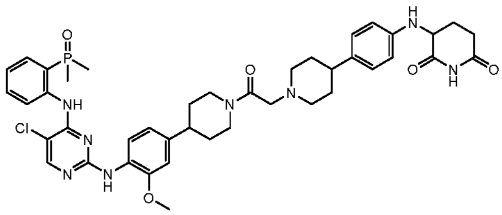 | LC-MS[M+1]:814 |
| T-333 | 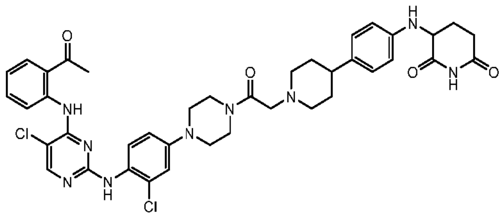 | LC-MS[M+1]:785 |
| T-334 | 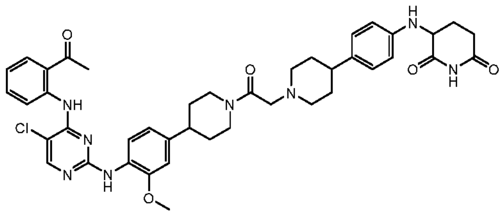 | LC-MS[M+1]:780 |
| T-336 | 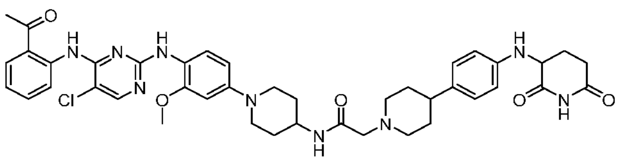 | LC-MS[M+1]:795 |
| T-353 | 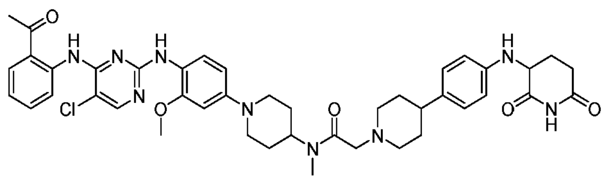 | LC-MS[M+1]:809 |

**Example 4**

The compound synthesized in the present invention:

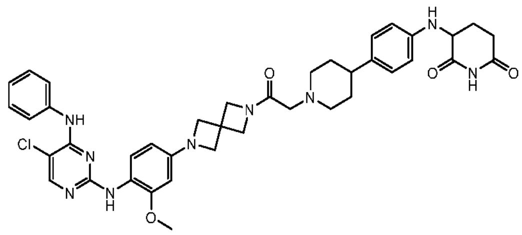

T-126

The experimental procedure is as follows:

T. Synthesis of intermediate M1

The synthesis route is as follows:

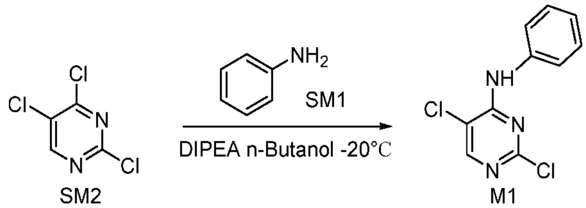


Intermediate M1 was synthesized with reference to the method of Example 1

II. Synthesis of intermediate M2

The synthesis route is as follows:

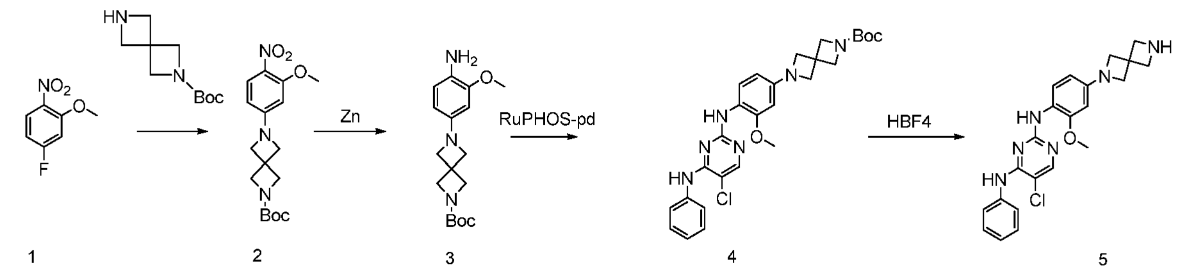


T. Synthesis of compound 2

1.0 g of 5-fluoro-2-nitroanisole, 4.26 g of tert-butyl 2,6-diazaspiro[3.3]heptan-2-carboxylate oxalate and 5 ml of DIPEA were dissolved in 10 ml of DMAC in a 100 ml three-necked flask, then heated to 110 °C and reacted overnight. TLC monitored the reaction until complete. The reaction was then added with water, extracted with ethyl acetate, dried with anhydrous sodium sulphate, spun-dried, and subjected to column chromatography to give 4.0 g of compound 2.

2. Synthesis of compound 3

4.0g of compound 3, 6.1g of ammonium chloride, and 4.5g of zinc powder were dispersed in 24ml solvent of ethanol: water 5:1 in a 100 ml single-necked flask, then heated to reflux and reacted for 18h. TLC showed disappearance of the raw material. Solvent was then removed by rotary evaporation, and the residue was added with water and pH was adjusted to 7-8 with a small amount of saturated aqueous sodium bicarbonate. The resulting solution was then extracted with ethyl acetate, dried with anhydrous sodium sulfate, spun-dried, and subjected to column chromatography to give 3.0g compound 3 as brown solid.LC-MS [M+1]: 320.

3. Synthesis of compound 4

100 mg of M1, and 150 mg of compound 3 were dissolved in 5 ml of tetrahydrofuran in a 50 ml single-necked flask, and 3.5 mg of Ruhos-Pd-G3 catalyst and 60 mg of sodium tert-butanol were added under nitrogen protection; the reaction was refluxed for 15h. TLC monitored the reaction until complete. Solvent was then removed by rotary evaporation, and the residue was added with water, extracted with ethyl acetate, dried with anhydrous sodium sulfate, spun-dried, and subjected to column chromatography to give 50mg compound 4.LC-MS [M+1]: 523.

4. Synthesis of intermediate M2

To a 50ml single-necked flask was added 50mg of compound 4, 50mg of tetrafluoroboric acid 50wt% aqueous solution, and 2ml of tetrahydrofuran, and then heated to 30 °C for 2h. LC-MS showed that the raw material was reacted completely. Solvent was then removed by rotary evaporation, and pH was adjusted to 7-8 with saturated aqueous sodium bicarbonate. The aqueous phase was extracted with ethyl acetate, and the organic phase was dried, filtrated, spun-dried, and separated by column chromatography to give 45mg intermediate M2.LC-MS [M+1]: 423.

III. Synthesis of compound T-126

The synthesis route is as follows:

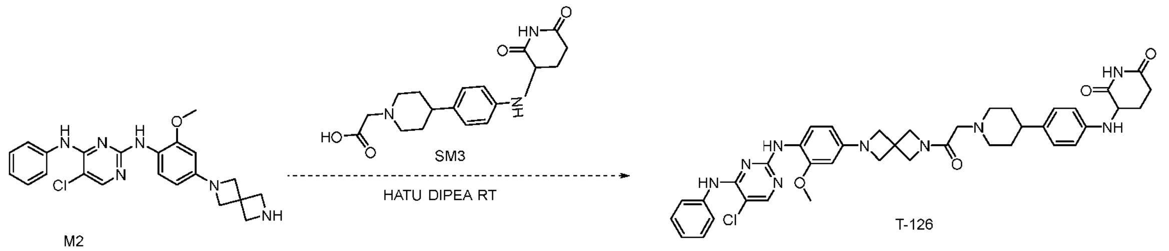

M2

T-126

Compounds M2 and SM3 were dissolved in 2ml of N,N-dimethylformamide in a 50 ml round-bottom flask and cooled down to 0°C in an ice-water bath; then N,N-diisopropylethylamine and HATU were added under stirring. The reaction was carried out for 1 h in an ice-water bath under nitrogen protection, and then naturally warmed up to room temperature. TCL detected the completion of the reaction. The reaction solution was then extracted with ethyl acetate and water, and the organic phase was dried, filtered, and subjected to column chromatography to give 50mg of compound T -126, LC-MS [M+1]: 752.

1H NMR (400 MHz, DMSO-d6) δ 10.79 (s,1H), 8.64 (s, 1H), 8.02 (s, 1H), 7.85 (s, 1H),7.66 (d, J = 8.0 Hz, 2H), 7.38 (d, J = 8.4 Hz,1H), 7.24 (t, J = 7.8 Hz, 2H), 7.05 (t, J = 7.4Hz, 1H), 6.97 (d, J = 8.3 Hz, 2H), 6.62 (d, J= 8.2 Hz, 2H), 6.12 (d, J = 2.4 Hz, 1H), 5.94(dd, J = 8.5, 2.4 Hz, 1H), 5.71 (d, J = 7.8 Hz,1H), 4.42 (s, 2H), 4.31 - 4.23 (m, 1H), 4.11(s, 2H), 3.96 (s, 3H), 3.74 (s, 3H), 2.89 (s,1H), 2.74 (ddd, J = 17.3, 11.9, 5.3 Hz, 2H),2.60 (s, 1H), 2.56 (s, 1H), 2.15 - 2.04 (m,2H), 2.00 (q, J = 7.3 Hz, 1H), 1.87 (tt, J =12.2, 6.1 Hz, 2H), 1.72 (s, 4H), 1.34 - 1.27(m, 2H), 1.16 (dd, J = 11.0, 6.5 Hz, 2H), 1.06(q, J = 7.5, 7.0 Hz, 1H), 0.85 (t, J = 6.5 Hz,1H).

The following compounds were synthesized with reference to the method of Example 4:

| Compound No. | Structure | Characterization data: |
|---|---|---|
| T-66 | 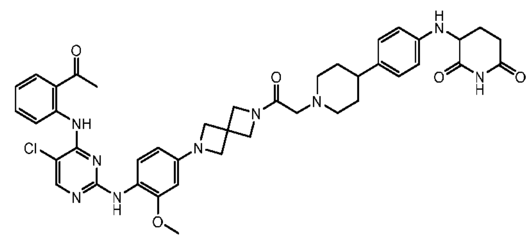 | LC-MS[M+1]:793 |

(continued)

| Compound No. | Structure | Characterization data: |
|---|---|---|
| T-126 | 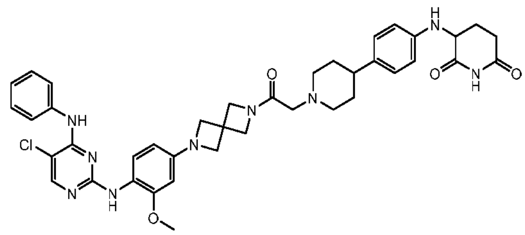 | LC-MS[M+1]:752 |

**Example 5**

The compound synthesized in the present invention:

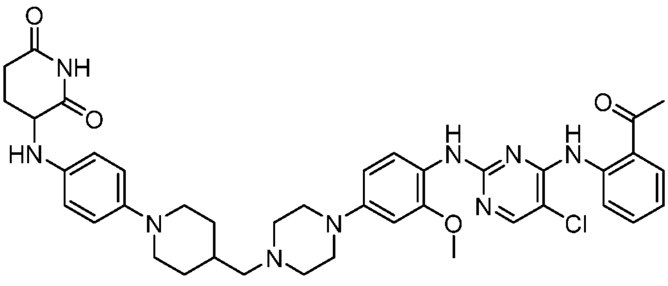

T-85

The experimental procedure is as follows:

I. The synthesis route is as follows:

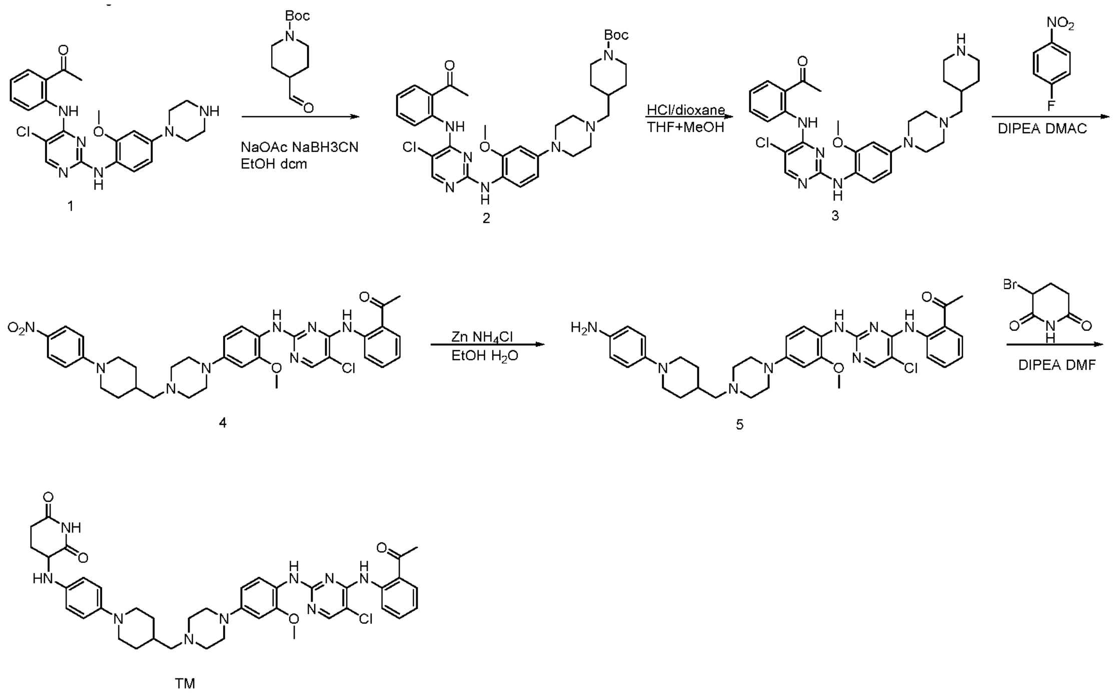

T. Synthesis of compound 2

300mg (1.0eq.) of compound 1, 164.55mg (1.2eq.) of N-Boc-4-piperidine carboxaldehyde and 158.37mg (3.0eq.) of sodium acetate were added to a mixture of 4ml of ethanol + 2ml of DCM in a 50 ml single-necked flask and stirred at room temperature for 2h. TLC monitored the reaction until complete and the reaction intermediate state was generated. Then 121.35 mg (3.0 eq.) of sodium cyanoborohydride was added and stirred at room temperature overnight. LCMS detected that the raw material was completely consumed and the target product LC-MS[M+1]:650 was generated. The reaction solution was then mixed directly with 1g of silica gel (100 mesh-200 mesh) and purified by column chromatography (EA in PE from 0% to 80%) to give the target product 2 (200mg, yellow solid).LC-MS [M+1]: 650.

2. Synthesis of compound 3

510 mg (1.0 eq.) of compound 2 was dissolved in a mixture of THF (5 ml) + MeOH (2 ml) in a 50 ml single-necked flask. HCl/dioxane (2 ml) was added, and stirred at room temperature for 4 h. LCMS monitored the reaction until complete.Tthe reaction solution was then directly evaporated and used for the next step.LC-MS [M+1]: 550.

3. Synthesis of compound 4

510 mg (1.0 eq) of compound 3, and 157.39 mg (1.2 eq) of p-fluoronitrobenzene were dissolved in 5 ml of DMAC in a 50 ml single neck flask, and refluxed at 120°C for 2h. LCMS monitored the reaction until complete. The reaction solution was then cooled to room temperature, diluted with ethyl acetate, extracted with water, dried with anhydrous sodium sulfate, spun-dried, mixed with 1g of silica gel (100 mesh-200 mesh), and purified by column chromatography (EA in PE from 0% to 50%) to give the target product 4 (220mg, yellow solid). LC-MS [M+1]: 671.

4. Synthesis of compound 5

Compound 4 (220mg,1.0eq.) was dissolved in 4 ml ethanol in a 50 ml round-bottom flask, and then 128.06mg of zinc powder (6.0eq.), ammonium chloride (174mg 10.0eq.) and water (1ml) were added. The reaction solution was refluxed overnight at 90 °C. LCMS detected the completion of the reaction. The reaction solution was then mixed directly with 500mg of silica gel (100 mesh-200 mesh) and purified by column chromatography (MeOH in DCM 0% to 20%) to give the target product 5 (220mg, yellow solid).LC-MS [M+1]: 641.

5. Synthesis of compound T-85

220mg (1.0eq) of compound 5 and 198mg (3.0eq) of 3-bromopiperidin-2,6-dione were dissolved in 4ml of DMF in a 50ml single-necked flask, and refluxed at 80°C for 12h. LCMS monitored the reaction until complete. The reaction solution was then cooled to room temperature, diluted with ethyl acetate, extracted with water, dried with anhydrous sodium sulfate, spun-dried, mixed with 500 mg of silica gel (100 mesh-200 mesh), and purified by column chromatography (MeOH in DCM from 0% to 20%) to give the target product TM (120 mg, brown solid) HPLC=89%, which was then purified by HPLC to give the final product TM (i.e., T-85) (10 mg, green solid, HPLC=95.53%) LC-MS [M+1]: 752.3.1H NMR (400 MHz, DMSO-d6) δ 11.83 (s, 1H),10.78 (s, 1H), 8.79 (s, 1H), 8.34 (s, 1H), 8.16 (s, 1H),8.09 (s, 1H), 7.42 (s, 1H), 7.35 (d, J = 8.5 Hz, 1H),7.14 (t, J = 7.6 Hz, 2H), 6.77 (d, J = 8.5 Hz, 2H), 6.68- 6.57 (m, 3H), 6.51 (dd, J = 8.9, 2.5 Hz, 1H), 5.38(d, J = 7.3 Hz, 1H), 4.60 (t, J = 5.4 Hz, 1H), 4.19 (dt,J = 11.8, 6.5 Hz, 1H), 3.75 (s, 3H), 3.53 - 3.37 (m,17H), 3.18 (s, 4H), 2.68 (s, 4H), 2.38 - 2.20 (m, 4H),2.15 - 2.06 (m, 2H), 1.81 (d, J = 12.6 Hz, 3H), 1.64(s, 2H).

The following compounds were synthesized with reference to the method of Example 5:

| Compound No. | Structure | Characterization data: |
|---|---|---|
| T-72 | 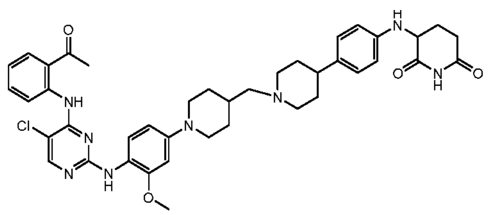 | LC-MS[M+1]:752 |

(continued)

| Compound No. | Structure | Characterization data: |
|---|---|---|
| T-178 | 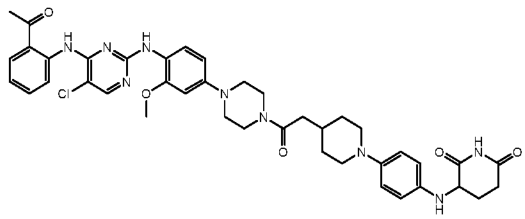 | LC-MS[M+1]:781 |
| T-225 | 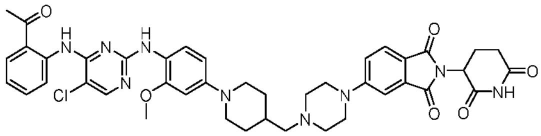 | LC-MS[M+1]:807 |
| T-226 | 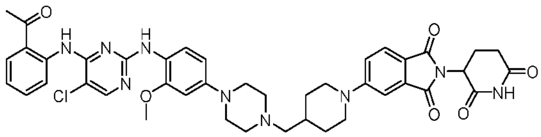 | LC-MS[M+1]:807 |
| T-240 | 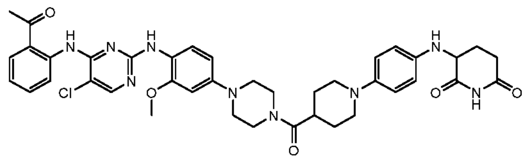 | LC-MS[M+1]:767 |

**Example 6**

The compound synthesized in the present invention:

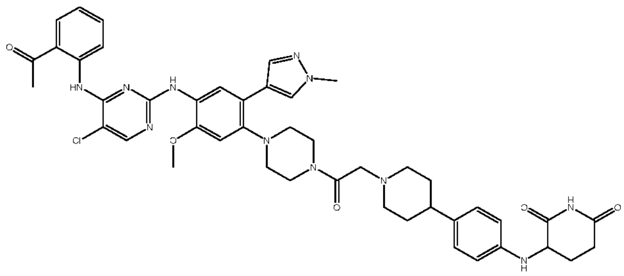

T-233

The experimental procedure is as follows:

I. The synthesis route is as follows:

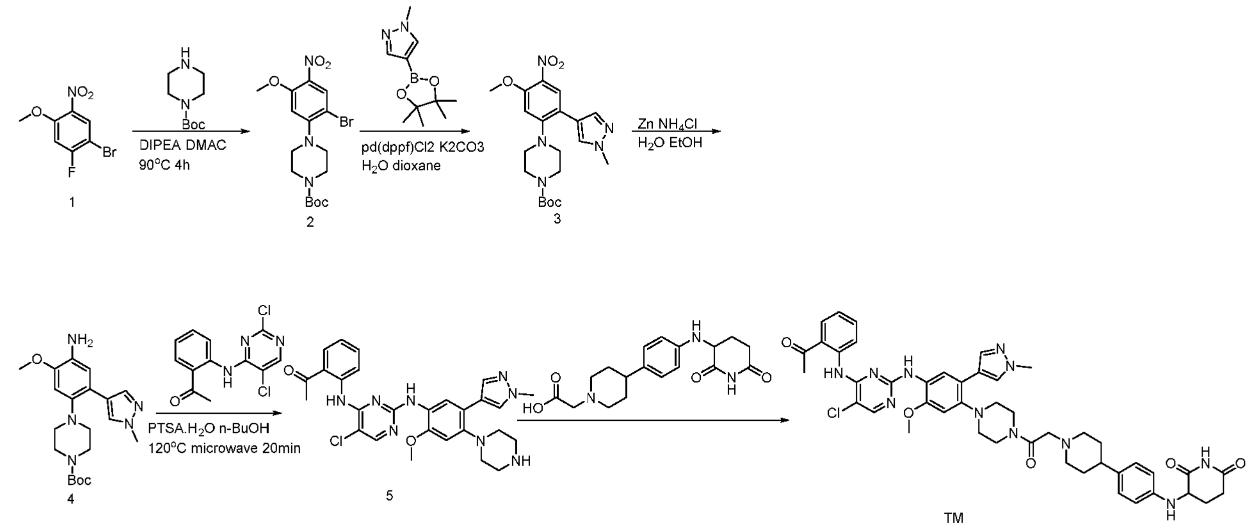


T. Synthesis of compound 2

500 mg (1.0 eq.) of compound 1, 412 mg (1.1 eq.) of N-Boc-4-piperazine, and 520 mg (2.0 eq.) of DIPEA were dissolved in DMAC in a 50 ml single-necked flask and stirred at 90°C for 4 h. LCMS monitored the reaction until complete. The reaction solution was then cooled to room temperature, diluted with EA, added with $H_2O$ and then allowed to separate into layers. The aqueous phase was extracted with EA (10 ml*3). The combined organic phases were washed with saturated saline, dried with anhydrous sodium sulfate, filtered, and the filtrate was mixed with 2g of silica gel (100 mesh-200 mesh), and purified by column chromatography (EA in PE from 0% to 45%) to give the target product 2 (630mg, yellow solid).LC-MS [M+1]: 416.27.

2. Synthesis of compound 3

Compound 2 (200 mg, 1.0 eq) and 1-methyl-4-(4,4,5,5-tetramethyl-1,3,2-dioxaborolan-2-yl)-1H-pyrazole (120 mg 1.2 eq) were dissolved in dioxane in a 50 ml single-necked flask, potassium carbonate (133 mg 2.0 eq), Pd(dppf)$Cl_2$ (7 mg 0.02 eq) and $H_2O$ (0.5 ml) were added, and the mixture was then displaced with $N_2$ for three times, and stirred at 100 °C for 12 h under nitrogen protection. LCMS detected the completion of the reaction. The reaction solution was then cooled to room temperature, diluted with EA, added with $H_2O$ and partitioned statically, and the aqueous phase was extracted with EA (5 ml*3). The combined organic phases were washed with saturated saline, dried with anhydrous sodium sulfate, and filtered; the filtrate was mixed with 600 mg of silica gel (100 mesh-200 mesh), and purified by column chromatography (EA in PE from 0% to 70%) to give the target product 3 (202 mg, yellow solid).LC-MS [M+1]: 418.2.

3. Synthesis of compound 4

Compound 3 (202mg,1.0eq) was dissolved in 2ml ethanol in a 50ml round-bottom flask, and then zinc powder (154mg 6.0eq.), ammonium chloride (315mg 10.0eq.) and water (0.4ml) were added. The reaction solution was refluxed overnight at 90 °C. LCMS detected the completion of the reaction. The reaction solution was then mixed directly with 500mg of silica gel (100 mesh-200 mesh) and purified by column chromatography (EA in PE from 0% to 75%) to give the target product 4 (127mg, yellow oil).LC-MS [M+1]: 388.2.

6. Synthesis of compound 5

Compound 4 (127 mg, 1.0 eq) and 1-(2-((2,5-dichloropyrimidin-4-yl)amino)phenyl)ethan-1-one (92.2 mg 1.0 eq) was dissolved in 2 ml of n-butanol in a 50 ml microwave tube, and p-toluenesulfonic acid monohydrate (75 mg, 1.2 eq) was added. The reaction was microwaved at 120°C for 20 min. LCMS detected the completion of the reaction. The reaction solution was then adjusted with saturated aqueous sodium bicarbonate to pH>7, and extracted with DCM; the organic phase was washed with saturated saline and dried with anhydrous sodium sulfate, then mixed with 500mg of silica gel (100 mesh-200 mesh) and purified by column chromatography (MeOH in DCM from 0% to 20%) to give the target product 5 (100mg, brown solid).LC-MS [M+1]: 533.03.

T. Synthesis of compound TM

compound 1 (50 mg, 1.0eq) and 2-(4-(4-((2,6-dioxopiperidin-3-yl)amino)phenyl)piperidin-1- yl)acetic acid (57 mg, 1.2eq purifity68%) were dissolved in 2 ml of N,N-dimethylformamide in a 50 ml round-bottom flask and cooled down to 0°C in an ice-water bath, and N,N-diisopropylethylamine (24mg, 2.0eq) and HATU (43mg, 1.2eq) were added under stirring. The reaction was carried out in an ice-water bath under nitrogen protection for 1 h, and then naturally warmed up to room temperature. TCL detected the completion of the reaction. The reaction was then extracted with ethyl acetate and water, and the organic phase was dried, filtered, and subjected to column chromatography to give 50 mg of compound TM (i.e. T-233), LC-MS [M+1]: 860.$^1$H NMR (400 MHz, Chloroform-d) $\delta$ 11.97 (s, 1H), 8.85 (d, $J$ = 8.3 Hz, 1H), 8.27 (s, 1H), 8.16 (s, 1H), 8.13 - 8.03 (m, 1H), 7.98 (s, 1H), 7.95 - 7.86 (m, 1H), 7.53 (d, $J$ = 7.6 Hz, 1H), 7.38 (s, 2H), 7.08 (d, $J$ = 8.0 Hz, 2H), 6.97 (s, 2H), 6.76 - 6.54 (m, 3H), 4.66 (s, 1H), 4.05 (d, $J$ = 12.6 Hz, 1H), 3.88 (d, $J$ = 20.4 Hz, 6H), 3.73 (s, 4H), 3.28 (s, 2H), 3.03 (d, $J$ = 10.8 Hz, 2H), 2.98 - 2.87 (m, 4H), 2.85 (d, $J$ = 3.8 Hz, 1H), 2.81 - 2.74 (m, 1H), 2.72 (s, 3H), 2.55 (d, $J$ = 13.0 Hz, 1H), 2.44 (s, 1H), 2.28 (s, 2H), 1.80 (s, 2H).

**Example 7**

The compound synthesized in the present invention:

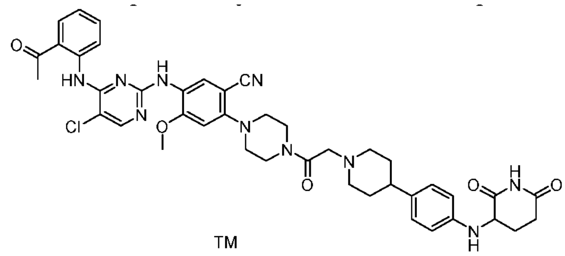

TM

T-282

The experimental procedure is as follows:

I. The synthesis route is as follows:

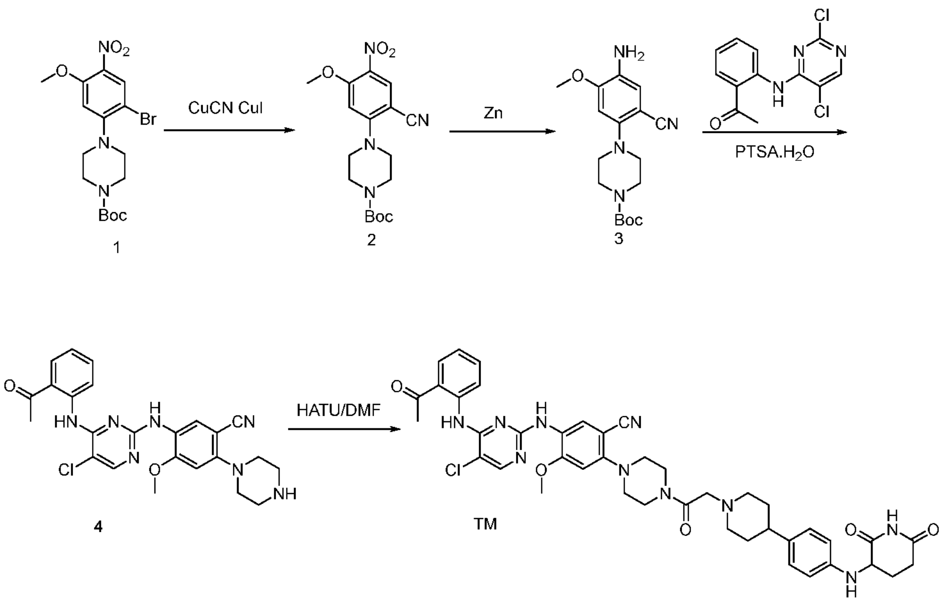


1 2 3

4 TM

T. Synthesis of compound 2

230 mg (1.0 eq.) of compound 1, cuprous cyanide (64.37 mg 1.3 eq.), and cuprous iodide (137 mg 1.3 eq.) were added to 2.3 ml of DMF in a 50 ml three-necked flask and stirred at 150°C for 4 h. LCMS detected the completion of the reaction. The reaction solution was then cooled to room temperature, filtered, and the filtrate was diluted with EA,

added with $H_2O$ and partitioned. The aqueous phase was extracted with EA (10 ml*3). The combined organic phases were washed with saturated saline, dried with anhydrous sodium sulfate, and filtered; the filtrate was mixed with 600 mg of silica gel (100 mesh-200 mesh), and purified by column chromatography (EA in PE from 0% to 40%) to give the target product 2 (140mg, yellow solid). LC-MS [M+1]: 362.

2. Synthesis of compound 3

Compound 2 (140mg,1.0eq) was dissolved in 2ml of ethanol in a 50ml round-bottom flask, then zinc powder (123mg 6.0eq.), ammonium chloride (251mg, 10.0eq.) and water (0.4ml) were added. The reaction solution was refluxed at 90°C for 4 h. LCMS detected the completion of the reaction. The reaction solution was then mixed directly with 500mg of silica gel (100 mesh-200 mesh) and purified by column chromatography (EA in PE from 0% to 30%) to give the target product 3 (30mg, white solid).LC-MS [M+1]: 332.

3. Synthesis of compound 4

Compound 4 (30mg, 1.0eq), 1-(2-((2,5-dichloropyrimidin-4-yl)amino)phenyl)ethan-1-one (26mg 1.0eq) were dissolved in 1ml of n-butanol in a 10 ml microwave tube, p-toluenesulfonic acid monohydrate (21mg 1.2eq) was added, and the mixture was microwaved at 120°C for 20 min. LCMS detected the completion of the reaction. The reaction solution was then adjusted with saturated aqueous sodium bicarbonate to pH>7, and extracted with DCM; the organic phase was washed with saturated saline and dried with anhydrous sodium sulfate, then mixed with 200mg of silica gel (100 mesh-200 mesh), and purified by column chromatography (MeOH in DCM 0% to 10%) to give the target product 4 (17mg, yellow solid).LC-MS [M+1]: 477.

4. Synthesis of compound TM

compound 1 (20 mg, 1.0eq) and 2-(4-(4-((2,6-dioxopiperidin-3-yl)amino)phenyl)piperidin-1- yl)acetic acid (30mg, 1.2eq purifity68%) were dissolved in 2 ml of N,N- dimethylformamide in a 50 ml round-bottom flask, and cooled down to 0°C in an ice-water bath. N,N-diisopropylethylamine (15mg,2.0eq) and HATU (18mg 1.2eq) were added under stirring, and the reaction was carried out in an ice-water bath under nitrogen protection for 1 h, and then naturally warmed up to room temperature. TLC monitored the reaction until complete. The reaction was then extracted with ethyl acetate and water, and the organic phase was dried, filtered, and subjected to column chromatography to give 15 mg of compound TM (i.e. T-282), LC-MS [M+1]: 805.

Example 8

The compound synthesized in the present invention:

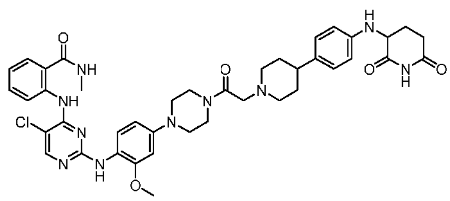

T-184

The experimental procedure is as follows:

T. Synthesis of intermediate M1

The synthesis route is as follows:

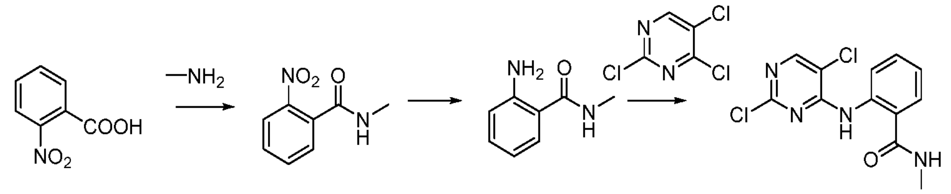

SM1 SM2 SM3 M1

1.

1. Synthesis of compound SM2

5g of o-nitrobenzoic acid, and 19g of dipea were dissolved in 25ml of DMF in a 250 ml three-necked flask, and the mixture was displaced with $N_2$ for 3 times, and then cooled to 0°C in an ice bath. 19g of dipea, 3g of 30% methanol solution of methylamine and 13.6g HATU were added in sequence and reacted overnight. TLC monitored the reaction until complete. The reaction was then added with water, and extracted with ethyl acetate; the organic phase was washed with saturated NaCl solution, dried with anhydrous sodium sulfate, spun-dried, and subjected to column chromatography to give 6g of crude compound 2. LC-MS [M+1]: 181.

2. Synthesis of compound SM3

6.0g of compound SM2, 14.7g of ammonium chloride, and 10.8g of zinc powder were dispersed in 55ml solvent of ethanol: water 5:1 in a 250ml three-necked flask, then heated to reflux and reacted for 18h. TLC showed disappearance of the raw material. Solvent was then removed by rotary evaporation, and the residue was added with water and pH was adjusted to 7-8 with a small amount of saturated aqueous sodium bicarbonate. The resulting solution was then extracted with ethyl acetate, dried with anhydrous sodium sulfate, spun-dried, and subjected to column chromatography to give 3.3g solid of compound SM3. LC-MS [M+1]: 151.

3. Synthesis of intermediate M1

950 mg of SM3, 1.16 g of 2,4,5-trichloropyrimidine and 4 g of dipea were dissolved in 10 ml of isopropanol in a 100 ml three-necked flask, and reacted at 80°C overnight. TLC monitored the reaction until complete. Solvent was then removed by rotary evaporation, and the residue was extracted with water and ethyl acetate; the organic phase was washed with saturated NaCl solution, dried with anhydrous sodium sulfate, spun-dried, and subjected to column chromatography to give 570 mg of the intermediate M1. LC-MS [M+1]: 342.

II. Synthesis of compound T-184

The synthesis route is as follows:

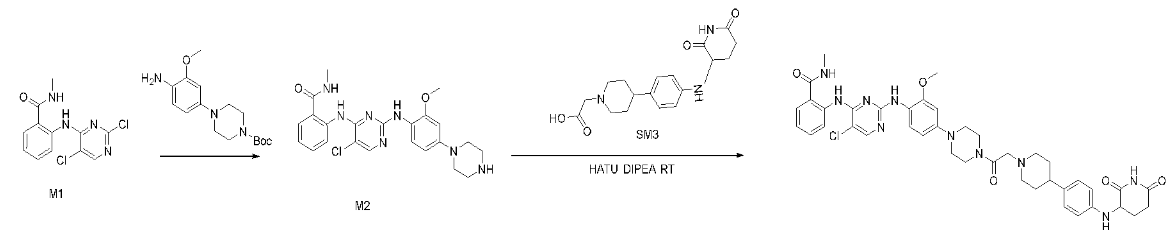


M1 M2

T. Synthesis of compound M2

200 mg of M1, 190 mg of tert-butyl 4-(4-amino -3-methoxyphenyl)piperazin -1-carboxylate and 135mg of p-toluenesulfonic acid monohydrate were dissolved in 3ml of isopropanol in a 10 ml microwave tube, and the mixture was microwaved at 120 °C for 20 min. TLC monitored the reaction until complete. The reaction solution was then transferred to a 125 ml separatory funnel, added with water, and extracted with ethyl acetate; the organic phase was washed with saturated NaCl solution, dried with anhydrous sodium sulfate, spun-dried, and subjected to column chromatography to give 130 mg of intermediate M2. LC-MS [M+1]: 512.

2. Synthesis of compound T-184

130 mg of compound M2 and 147 mg of 60% SM3 were dissolved in 4 ml of N, N-dimethylformamide in a 50 ml round-bottom flask, and cooled down to 0°C in an ice-water bath. 164 mg of N, N-diisopropylethylamine and 116 mg of HATU were added under stirring. The reaction was carried out in an ice-water bath under nitrogen protection for 1 h, and then naturally warmed up to room temperature. TLC detected the completion of the reaction. The reaction solution was then extracted with ethyl acetate and water; the organic phase was dried, filtered, and subjected to column chromatography to give 50 mg of compound T-184, LC-MS [M+1]: 796.1H NMR (400 MHz, DMSO-d6) δ 11.61 (s, 1H), 10.79 (s, 1H), 8.73 (d, J = 4.9 Hz, 1H), 8.60 (s, 1H), 8.19 (s, 1H), 8.11 (s, 1H), 7.69 (d, J = 7.9 Hz, 1H), 7.44 (d, J = 8.5 Hz, 1H), 7.32 (s, 1H), 7.02 (t, J = 7.6 Hz, 1H), 6.95 (d, J = 8.1 Hz, 2H), 6.71 (d, J = 2.4 Hz, 1H), 6.62 - 6.50 (m, 4H), 5.67 (d, J = 7.5 Hz, 1H), 4.26 (dq, J = 12.1, 5.9, 5.3 Hz, 1H), 3.78 (s, 4H), 3.63 (s, 2H), 3.22 (s, 3H), 3.12 (s, 2H), 2.94 (s, 3H), 2.79 (d, J = 4.4 Hz, 3H), 2.75 - 2.66 (m, 2H), 2.58 (d, J = 4.4 Hz, 1H), 2.33 (s, 2H), 2.13 - 2.05 (m, 3H), 1.84 (dd, J = 12.1, 4.4 Hz, 2H), 1.70 (s, 3H), 1.60 (s, 3H).

The following compounds were synthesized with reference to the method of Example 8:

| Compound No. | Structure | Characterization data: |
|---|---|---|
| T-185 | 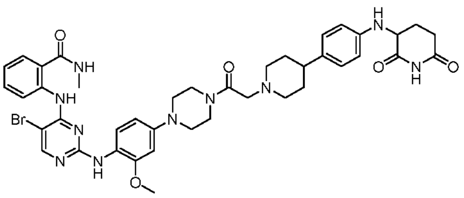 | LC-MS[M+1]:841 |
| T-198 | 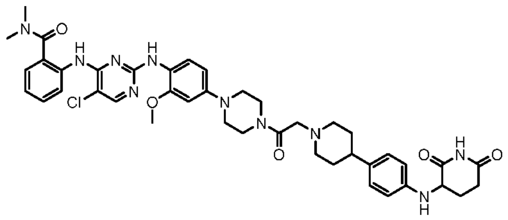 | LC-MS[M+1]:810 |
| T-241 | 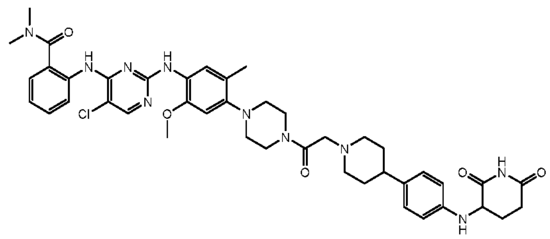 | LC-MS[M+1]:824 |
| T-186 | 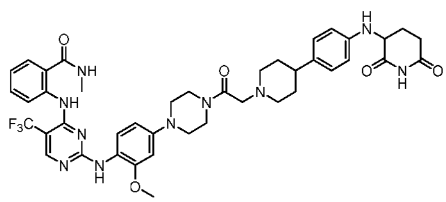 | LC-MS[M+1]:829 |
| T-253 | 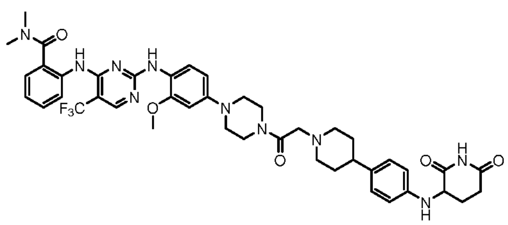 | LC-MS[M+1]:843 |

(continued)

| Compound No. | Structure | Characterization data: |
|---|---|---|
| T-254 | 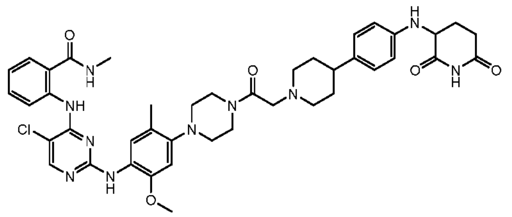 | LC-MS[M+1]:809 |
| T-259 | 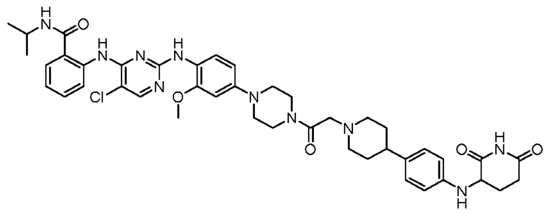 | LC-MS[M+1]:823 |
| T-341 | 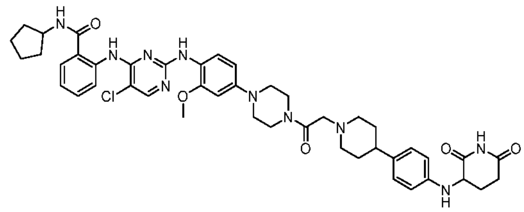 | LC-MS[M+1]:849 |

### Example 9

The compound synthesized in the present invention:

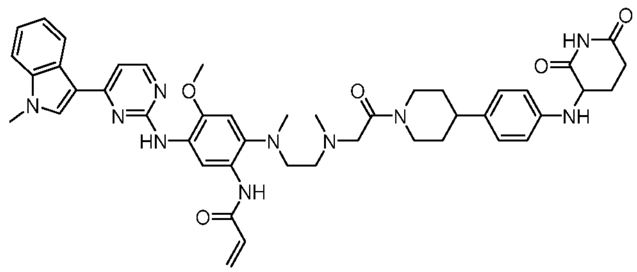

T-237

The synthesis route is as follows:

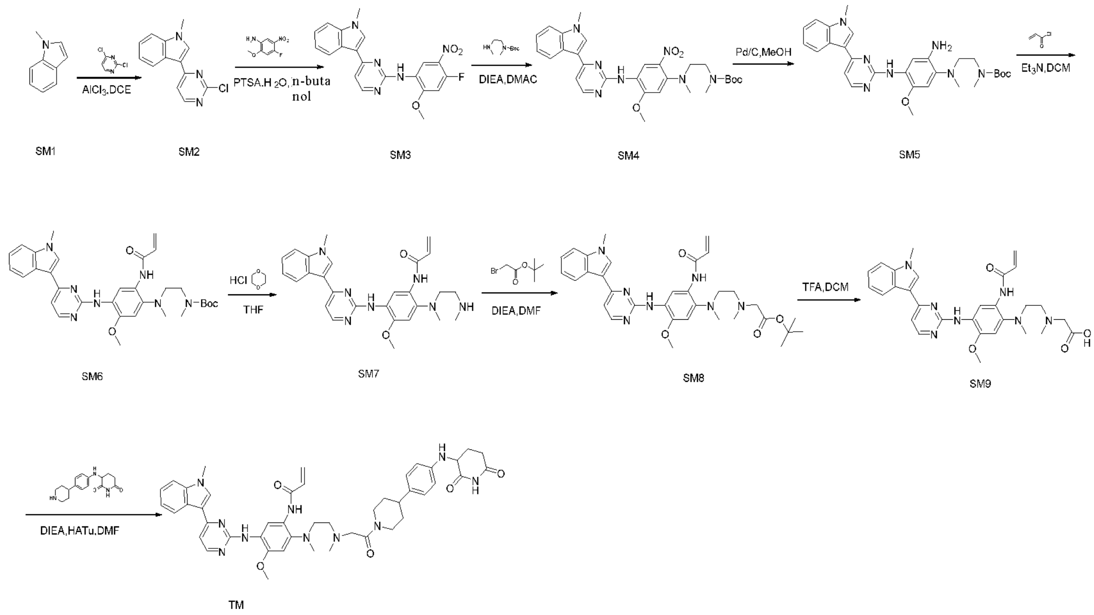


SM1 SM2 SM3 SM4 SM5

SM6 SM7 SM8 SM9

TM

T. Synthesis of SM2

To a 50 ml single-necked flask was add 1.36g of 2,4-dichloropyrimidine, 691 mg of $AlCl_3$ and 10 ml of DCE, replaced with nitrogen, and heated to 80°C for 30 min. Then 400 mg N-methylindole was added and reacted overnight. TLC detected the completion of the reaction. The reaction solution was then added with water, extracted with DCM, dried with anhydrous sodium sulfate, and mixed with silica gel and subjected to column chromatography to give 700 mg of SM2. LC- MS[M+1]:244.

2. Synthesis of SM3

400 mg of SM2, 306.2 mg of 4-fluoro-2-methoxy-5-nitroaniline and 376.2 mg of p-toluenesulfonic acid monohydrate were dissolved in 4 ml of n-butanol in a 10 ml microwave tube, and the mixture was microwaved at 120°C for 20 min. TLC monitored the the completion of the reaction. The reaction solution was then adjusted to be basic with saturated sodium bicarbonate, added with methylene chloride, and filtrated; the filter cake was dried to give 439 mg of intermediate SM3. LC-MS [M+1]: 394.

3. Synthesis of SM4

339 mg of SM3 and 195 mg of methyl (2-(methylamino)ethyl)amine were dissolved in 5 ml of DMAC in a 50 ml single-necked flask, and 332 mg of DIEA was added. The reaction was replaced with nitrogen, heated to 120°C and reacted overnight. TLC monitored the reaction until complete. The reaction solution was then add with water, extracted with EA, dried with anhydrous sodium sulfate, and mixed with silica gel and subjected to column chromatography to give 417 mg of SM4. LC- MS[M+1]:562.

4. Synthesis of SM5

417 mg of SM4 was dissolved in 5 ml of methanol and 2 ml of THF in a 50 ml single-necked flask, and 41.7 mg of Pd/C was added. The reaction was displaced with hydrogen and reacted at room temperature for 4 h. TLC monitored the reaction until complete. The reaction solution was then filtered, and mixed with silica gel and subjected to column chromatography to give 360 mg of SM5. LC- MS[M+1]:532.

5. Synthesis of SM6

360mg of SM5 was dissolved in 5ml dichloromethane in a 50ml single-necked flask, and the reaction was replaced with nitrogen, and cooled to 0°C. 102.7mg of triethylamine and 67.5mg of acryloyl chloride were added, and the reaction was restored to room temperature and reacted overnight. TLC monitored that most of the raw material had been reacted. The reaction solution was then added with water, extracted with DCM, dried with anhydrous sodium

sulfate, and mixed with silica gel and subjected to column chromatography to give 263 mg SM6. LC- MS[M+1]:586.

6. Synthesis of SM7

263 mg of SM6 was dissolved in 3 ml of dichloromethane in a 50 ml single-necked flask, and the mixture was displaced with nitrogen, and cooled to 0°C. 3 ml of trifluoroacetic acid was added, and the reaction was restored to room temperature and reacted for 30 min. LC-MS monitored the reaction until complete. The reaction solution was then spun-dried directly to give a theoretical yield of 218 mg of SM7. LC- MS[M+1]:486.

7. Synthesis of SM8

150 mg of SM7 was dissolved in 2 ml of DMF in a 50 ml single-necked flask, and the mixture was replaced with nitrogen, and cool to 0°C. 200 mg of DIEA and 66.3 mg of tert-butyl bromoacetate was added, and the reaction was restored to room temperature and reacted overnight. TLC monitored the reaction until complete. The reaction solution was then added with water, extracted with EA, dried with anhydrous sodium sulfate, and mixed with silica gel and subjected to column chromatography to give151 mg of SM8. LC-MS[M+1]:600.

8. Synthesis of SM9

150 mg of SM8 was dissolved in 2 ml of dichloromethane in a 50 ml single-necked flask, and the mixture was displaceed with nitrogen, and cooled to 0 °C. 2 ml of trifluoroacetic acid was added, and the reaction was restored to room temperature and reacted overnight. LC-MS monitored the reaction until complete. The reaction solution was then directly spun-dried to give a theoretical yield of 136 mg of SM9. LC- MS[M+1]:544.

8. Synthesis of TM

60 mg of SM9, 36 mg of 2,6-piperidinedione, and 3-[[4-(4-piperidinyl)phenyl]amino] were dissolved in 3 ml of DMF in a 50 ml single-necked flask, 99.8 mg of DIEA was added, and the mixture was replaced with nitrogen, and cooled to 0 °C. 54.6 mg of HATu was added, and the reaction was restored to room temperature and reacted for 3 h. TLC monitored the reaction until complete. The reaction solution was then added with water, extracted with EA, dried with anhydrous sodium sulfate, mixed with silica gel subjected to column chromatography, and then subjected to thin-layer chromatography to give 63mg of TM (i.e. T-237). LC- MS[M+1]:813.

1H NMR (400 MHz, Chloroformd) δ 9.78 (s, 1H), 9.37 (s, 1H), 8.97 (s, 1H), 8.31 (d, J = 5.3 Hz, 1H), 8.00 (d, J = 7.6 Hz, 1H), 7.84 (d, J = 19.1 Hz, 1H), 7.66 (s, 1H), 7.36 - 7.28 (m, 1H), 7.22 (s, 1H), 7.14 (d, J = 5.3 Hz, 2H), 6.87 (d, J = 7.8 Hz, 2H), 6.71 (s, 1H), 6.44 (dd, J = 33.4, 11.0 Hz, 4H), 5.73 - 5.61 (m, 1H), 4.62 (d, J = 13.1 Hz, 1H), 4.52 (s, 1H), 3.89 (s, 5H), 3.82 (s, 3H), 2.98 (d, J = 15.8 Hz, 4H), 2.75 (d, J = 18.4 Hz, 2H), 2.62 (s, 5H), 2.60 - 2.09 (m, 8H), 1.78 (d, J = 28.7 Hz, 4H).

The following compounds were synthesized with reference to the method of Example 9:

| Compound No. | Structure | Characterization data: |
|---|---|---|
| T-219 | 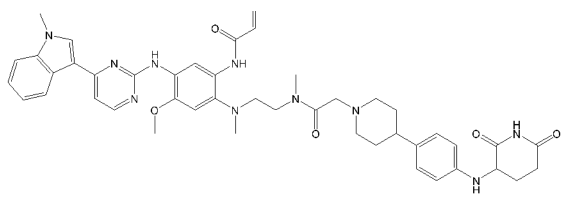 | LC-MS[M+1]:813 |

## Example 10

The compound synthesized in the present invention:

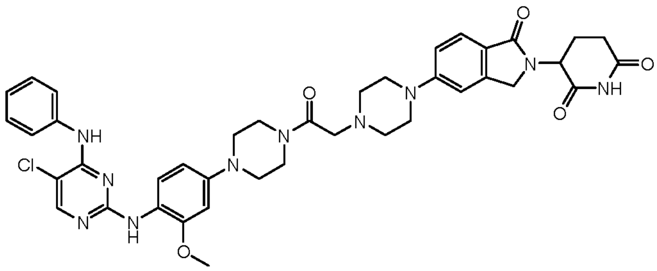

T-15

The synthesis route is as follows:

Synthesis of intermediate SM1

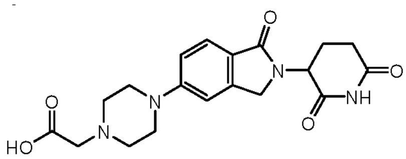

SM1

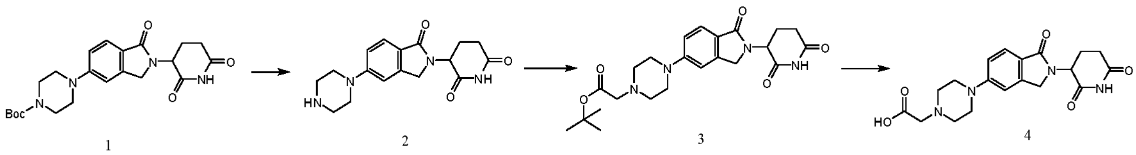

T. Synthesis of compound 2

To a 100 ml single-necked flask was added 120mg of compound 1, and 30 ml of methanol was added to dissolve it; then 4 M solution of dioxane hydrochloride was added dropwise under a cooling ice-water bath. After dropwise addition, the reaction was carried out at 40 °C for 2 h. The solvent was then removed by rotary evaporation to give 140mg of compound 2.LC-MS [M+1]: 329.

2. Synthesis of compound 3

To a 100 ml single-necked flask was added 80mg of compound 2, 8 ml of N,N-dimethylformamide, N,N-diisopropylethylamine, and tert-butyl bromoacetate and then stirred at room temperature overnight. TLC detected the completion of the reaction. The reaction solution was then extracted with ethyl acetate and water, and the organic phase was dried, filtered, and subjected to column chromatography to give 30mg of compound 3, LC-MS [M+1]: 443.

3. Synthesis of compound SM1

To a 100 ml single-necked flask were added 100 mg of compound 6, and 1 ml of dichloromethane. 1.6 ml of trifluoroacetic acid was added dropwise and stirred at room temperature overnight. TLC detected the completion of the reaction. The reaction solution was thenrotarily evaporated to remove solvent to give 150 mg of compound 4 (i.e., SM1), LC-MS [M-1]: 385.

4. Synthesis of compound T -15

The synthesis route is as follows:

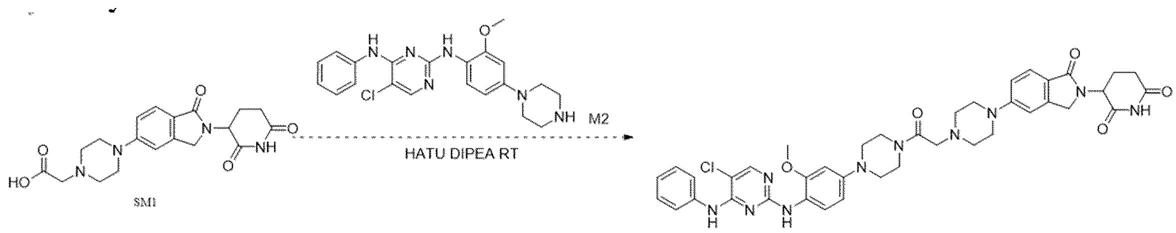


Compounds M2 and SM1 were dissolved in 2ml N,N-dimethylformamide in a 50 ml round-bottom flask, and then cooled down to 0°C in an ice-water bath. Then N,N-diisopropylethylamine and HATU were added under stirring, and the reaction was carried out for 1 h in an ice-water bath under nitrogen protection, and then naturally warmed up to room temperature. TLC detected the completion of the reaction. The reaction solution was then extracted with ethyl acetate and water, and the organic phase was dried, filtered, and subjected to column chromatography to give 100 mg of compound T-15, LC-MS [M+1]: 780.

The following compounds were synthesized with reference to the method of Example 10:

| Compound No. | Structure | Characterization data: |
|---|---|---|
| T-14 | 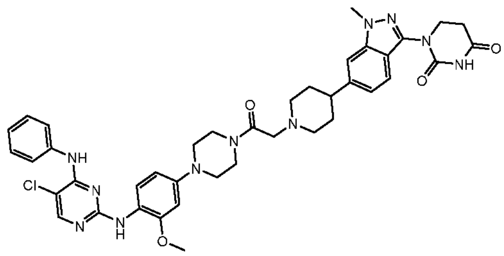 | LC-MS[M+1]:779 |
| T-45 | 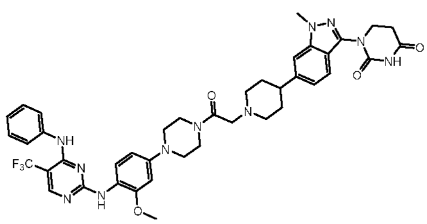 | LC-MS[M+1]:812 |
| T-101 | 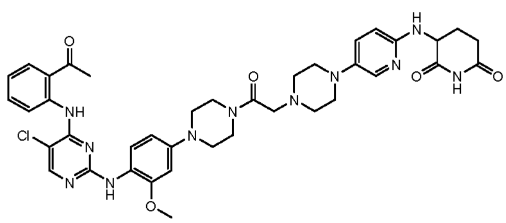 | LC-MS[M+1]:783 |
| T-109 | 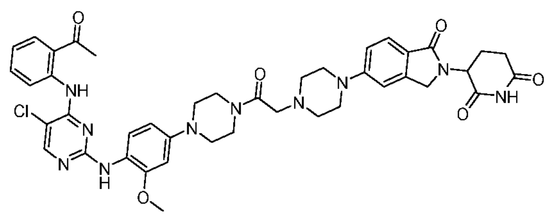 | LC-MS[M+1]:821 |
| T-197 | 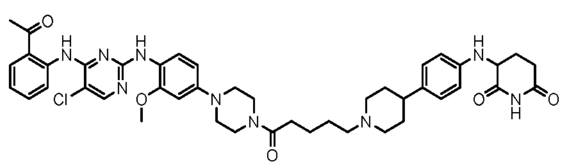 | LC-MS[M+1]:823 |

(continued)

| Compound No. | Structure | Characterization data: |
|---|---|---|
| T-201 | 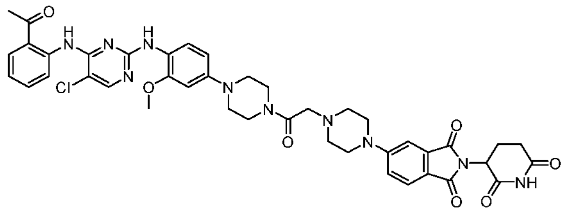 | LC-MS[M+1]:836 |
| T-204 | 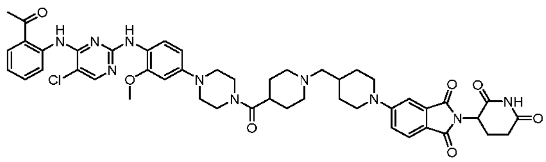 | LC-MS[M+1]:918 |
| T-205 | 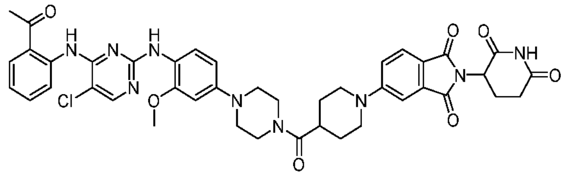 | LC-MS[M+1]:821 |
| T-207 | 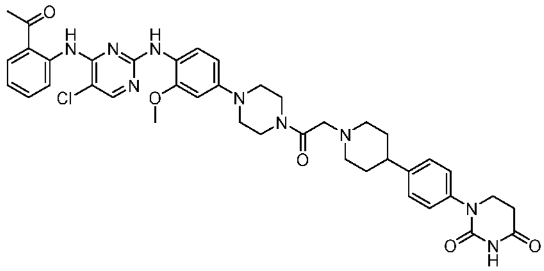 | LC-MS[M+1]:767 |
| T-212 | 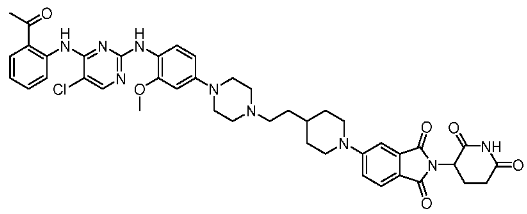 | LC-MS[M+1]:821 |
| T-215 | 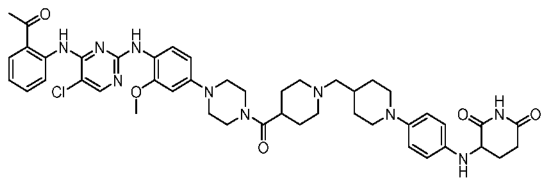 | LC-MS[M+1]:863 |
| T-216 | 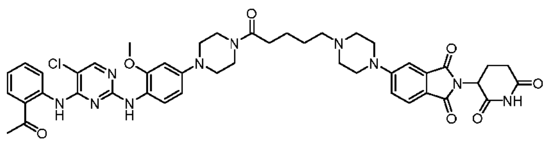 | LC-MS[M+1]:877 |

(continued)

| Compound No. | Structure | Characterization data: |
|---|---|---|
| T-217 | 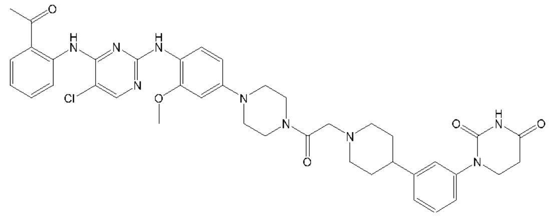 | LC-MS[M+1]:767 |
| T-221 | 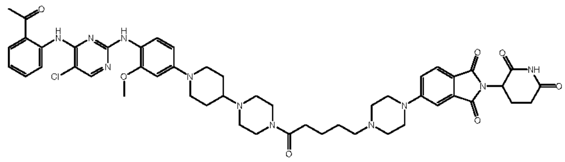 | LC-MS[M+1]:961 |
| T-228 | 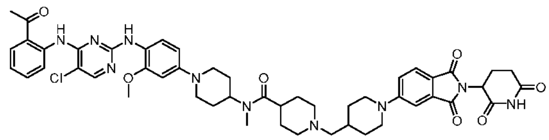 | LC-MS[M+1]:945 |
| T-230 | 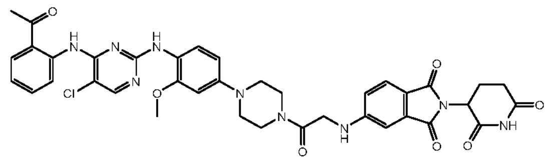 | LC-MS[M+1]:766 |
| T-231 | 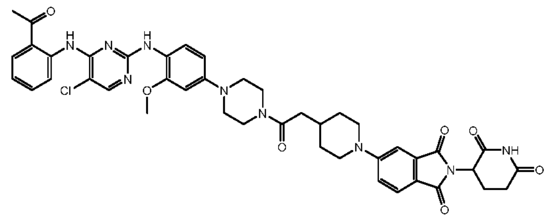 | LC-MS[M+1]:834 |
| T-232 | 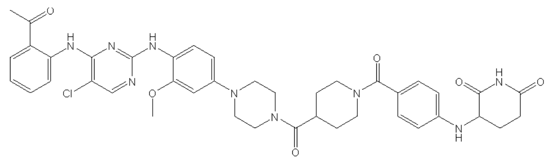 | LC-MS[M+1]:794 |
| T-242 | 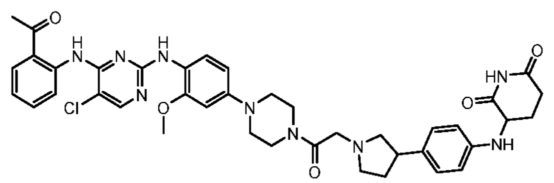 | LC-MS[M+1]:766 |

(continued)

| Compound No. | Structure | Characterization data: |
|---|---|---|
| T-275 | 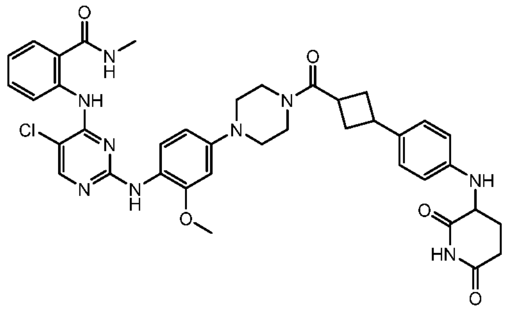 | LC-MS[M+1]:752 |
| T-324 | 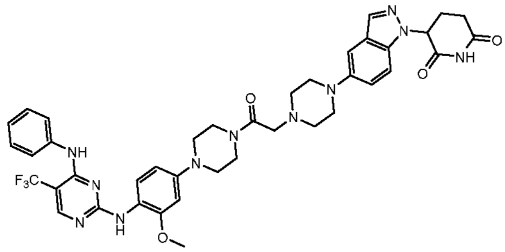 | LC-MS[M+1]:798 |
| T-326 | 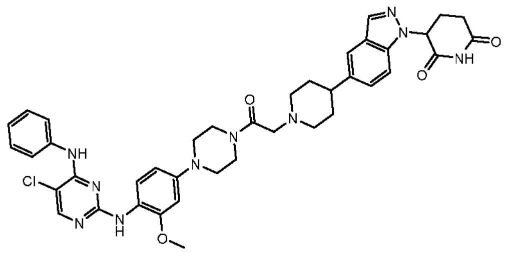 | LC-MS[M+1]:763 |
| T-332 | 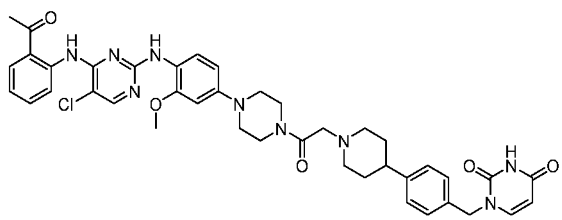 | LC-MS[M+1]:779 |
| T-337 | 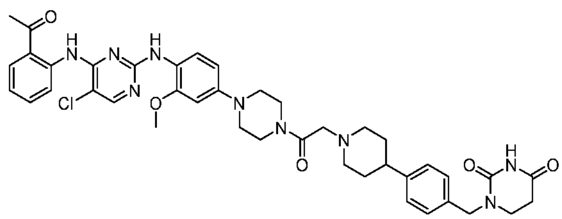 | LC-MS[M+1]:781 |
| T-351 | 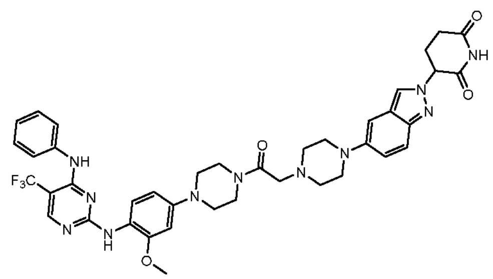 | LC-MS[M+1]:798 |

**Example** 11

The compound synthesized in the present invention:

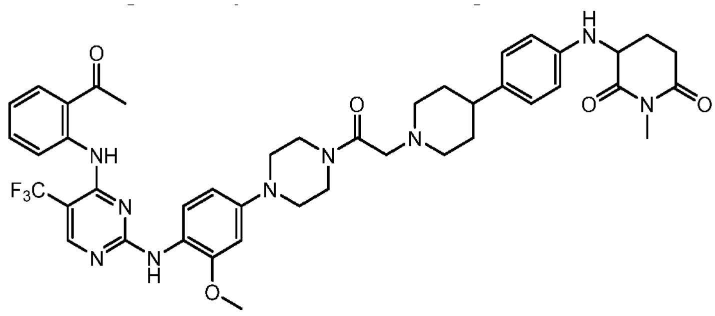

T-352

The synthesis route is as follows:

Synthesis of intermediate SM1

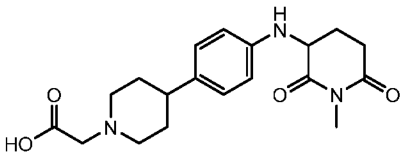

The synthesis route is as follows:

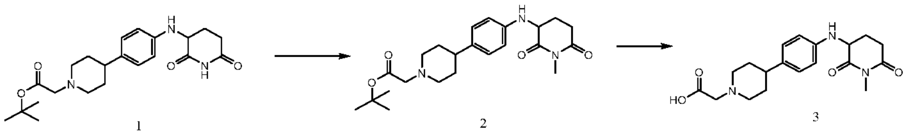

T. Synthesis of compound 2

200mg of compound 1 was dissolved in 4ml of DMF in a 50ml 3-necked flask under stirring, and cooled down to 0-5°C. 30mg of sodium hydride was slowly added, and the mixture was kept at 0°C for 30 minutes under stirring, and then added with 0.034ml of iodomethane. The temperature was kept for 30 minutes under stirring. TLC detected the disappearance of raw material and the generation of new spot. The reaction was then separated and purified by column chromatography to give 30mg of compound 2, LC-MS:[M+H]:416.

2. Synthesis of compound 3

To a 50ml single-necked flask was added 30mg of compound 2, and 1ml of dichloromethane, and 0.5ml of trifluoroacetic acid was added dropwise and then the mixture was stirred at room temperature overnight. TLC detected the completion of the reaction. The reaction solution was rotarily evaporated to remove solvent to give 20mg of compound 3 (i.e., SM1), LC-MS [M-1]: 360.

Synthesis of compound T-352

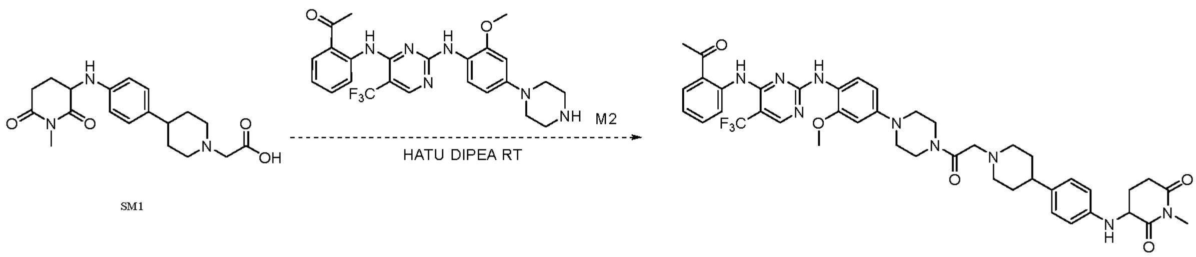


M2

SM1

Compounds M2 and SM1 were dissolved in 2m1 N,N-dimethylformamide in a 50 ml round-bottom flask, and cooled down to 0°C in an ice-water bath. Then N,N-diisopropylethylamine and HATU were added under stirring, and the reaction was carried out for 1 h in an ice-water bath under nitrogen protection, and then naturally warmed up to room temperature. TLC detected the completion of the reaction. The reaction solution was then extracted with ethyl acetate and water, and the organic phase was dried, filtered, and subjected to column chromatography to give 16mg of compound T-352, LC-MS [M+1]: 828.HNMR:1H NMR (400 MHz, DMSO-d6) δ 8.44 (s,1H), 8.33 (s, 1H), 8.28 (s, 1H), 7.50 (s, 2H),7.38 (d, J = 8.5 Hz, 1H), 7.26 (d, J = 7.9 Hz,2H), 7.09 (t, J = 7.5 Hz, 1H), 6.98 (d, J = 8.0Hz, 2H), 6.72 - 6.58 (m, 3H), 6.38 (s, 1H),5.80 (d, J = 7.7 Hz, 1H), 4.35 (ddd, J = 11.9,7.7, 4.9 Hz, 1H), 3.77 (s, 3H), 3.66 (s, 4H),3.16 (d, J = 30.3 Hz, 5H), 2.99 (s, 3H), 2.91 -2.65 (m, 3H), 2.09 (dt, J = 12.9, 4.6 Hz, 1H),1.99 (s, 1H), 1.89 (ddt, J = 24.1, 12.2, 6.5 Hz,5H), 1.32 - 1.20 (m, 3H).

**Example 12**

The compound synthesized in the present invention:

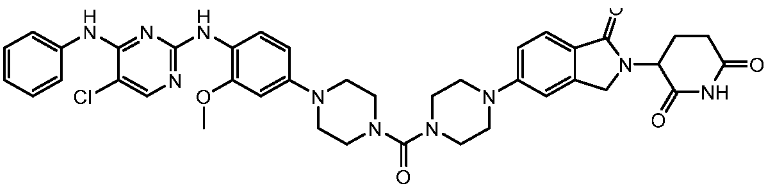

T-344

The synthesis route is as follows:

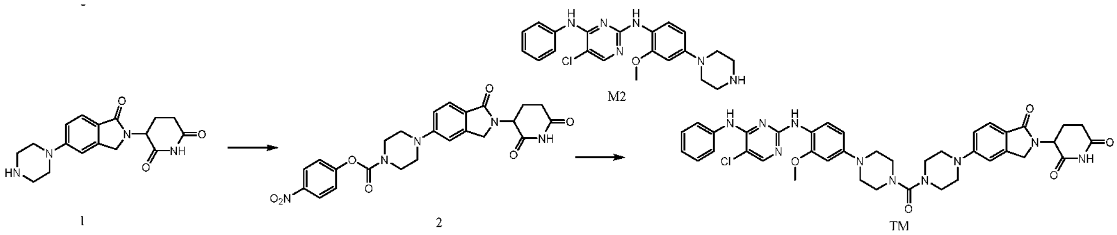

M2

1

2

TM

Synthesis of compound 2

230 mg of compound 1 was dissolved in 3 ml of 1,2-dichloroethane in a 50 ml single-necked flask, 0.3 ml of triethylamine and 150 mg of p-nitrophenyl chloroformate were added, and the mixture was reacted at room temperature for 3 h. TLC detected the completion of the reaction. The reaction was then purified by column chromatography to give 250 mg of compound 2.

Synthesis of compound T-344

110 mg of compound 2 was dissolved in 0.2 ml of DMF in a 10 ml reaction tube, then 0.1 ml of triethylamine and 100 mg of intermediate M2 were added, and the reaction was reacted at 80°C overnight. After isolation and purification, 10mg of compound T-344 was obtained.LC-MS [M+1]: 766.1H NMR (400 MHz, DMSO-d6) δ 8.76 (s, 0H), 8.12 (d, J =15.5 Hz, 1H), 7.92 (s, 1H), 7.73 - 7.54 (m, 1H), 7.32 (dd, J= 8.5, 7.3 Hz, 1H), 7.18 - 7.08 (m, 1H), 6.72 (d, J = 2.6 Hz,1H), 6.47 (dd, J = 8.8, 2.6 Hz, 1H), 3.83 (s, 2H), 3.69 - 3.54(m, 2H), 3.25 - 3.09 (m, 2H), 2.56 (p, J = 1.8 Hz, 2H),

1.29(d, J = 5.7 Hz, 1H).

The following compounds were synthesized with reference to the method of Example 12:

| Compound No. | Structure | Characterization data: |
|---|---|---|
| T-79 | 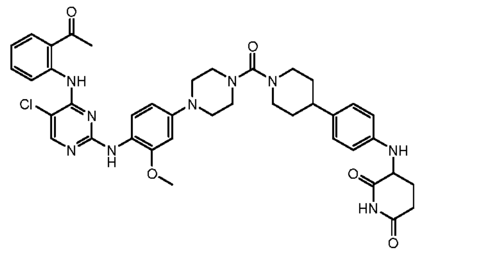 | LC-MS[M+1]:767 |
| T-345 | 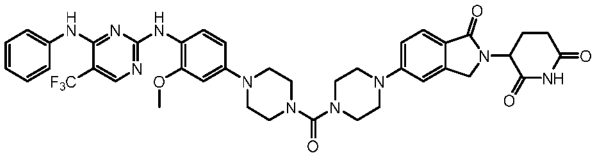 | LC-MS[M+1]:799 |

### Example 13

The compound synthesized in the present invention:

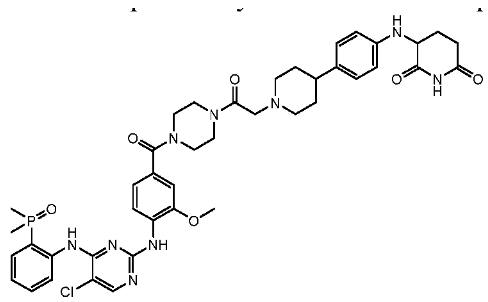

T-329

The synthesis route is as follows:

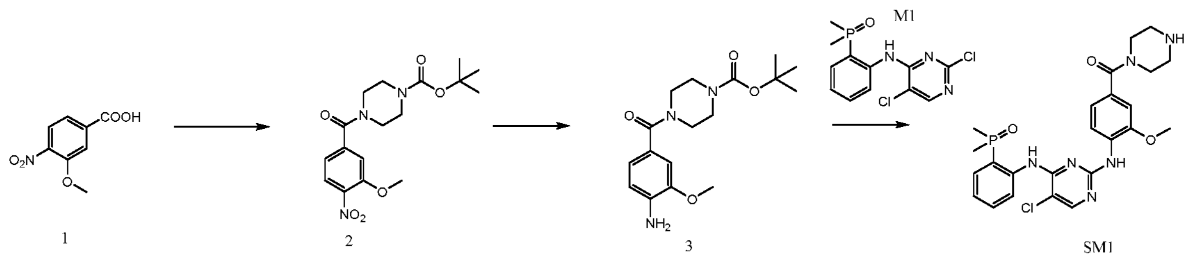

1 2 3 M1 SM1

Synthesis of intermediate SM1

Synthesis of compound 2

1.0 g of compound 1 was dissolved in 10 ml of DMF in a 50 ml single-necked flask, 1.13g of N-Boc piperazine and 3.28 g of DIEA were added, and the mixture was cooled down 0°C, and 2.31 g of HATU was added. The temperature was naturally warmed to room temperature and the reaction was stirred for 2 h. TLC detected the completion of the reaction. The reaction was then quenched with water, and extracted with ethyl acetate. The organic phases were combined and subjected to column chromatography to give 2.63 g of compound 2.

Synthesis of compound 3

To a 50 ml single-necked flask was added 2.63 g of compound 2, 5 ml of methanol, 5 ml of tetrahydrofuran, and 263 mg of palladium-carbon catalyst, and the mixture was replaced with hydrogen three times via a hydrogen balloon and then sealed and reacted overnight. TLC detected the completion of the reaction. The reaction was then leached to remove palladium-carbon, and the reaction solution was spun-dried to give 2.56 g of compound 3.

Synthesis of intermediate SM1

500 mg of compound 3, 471.6 mg of intermediate M1, and 284 mg of p-toluenesulfonic acid monohydrate were dissolved in 5 ml of n-butanol in a 10 ml microwave tube, and the mixture was microwaved at 120 °C for 20 min. TLC monitored the reaction until complete. The reaction solution was then adjusted to be basic using saturated sodium bicarbonate, added with methylene chloride, and filtrated; the filter cake was dried to give 150mg of intermediate SM1.LC-MS [M+1]: 516.

Synthesis of compound T-329

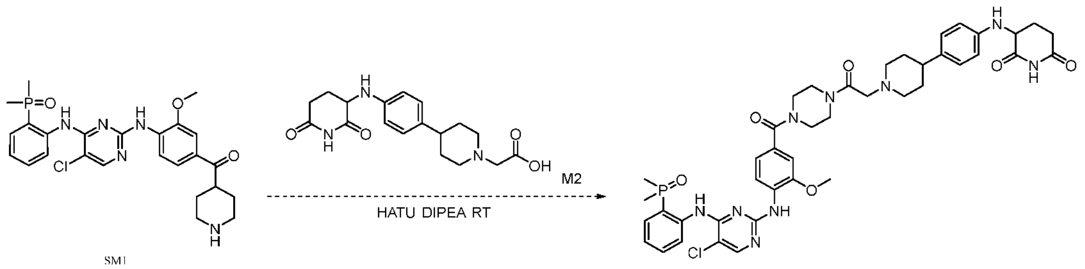


SM1

150 mg of compound SM1, and 378 mg of M2 were dissolved in 3 ml of NN-dimethylformamide in a 50 ml round-bottom flask, and cooled down to 0 °C in an ice-water bath. N,N-diisopropylethylamine and HATU were added under stirring. The reaction was then carried out for 1 h in an ice-water bath under nitrogen protection, and then naturally warmed up to room temperature. TLC detected the completion of the reaction. The reaction solution was then extracted with ethyl acetate and water; the organic phase was dried, filtered, and subjected to column chromatography to give 24mg of compound T-329, LC-MS [M+1]:843.

The following compounds were synthesized with reference to the method of Example 13:

| Compound No. | Structure | Characterization data: |
|---|---|---|
| T-130 | 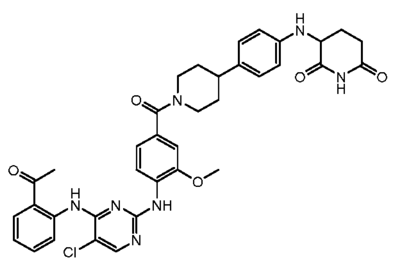 | LC-MS[M+1]:683 |
| T-229 | 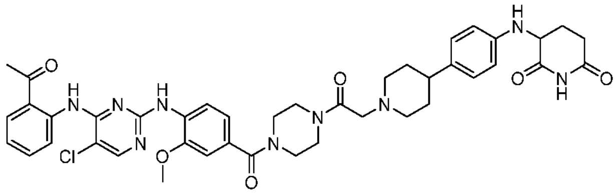 | LC-MS[M+1]:809 |

**Example 14**

The compound synthesized in the present invention:

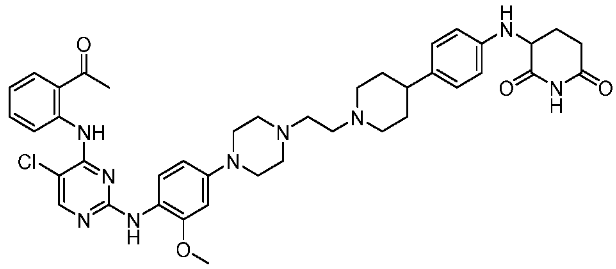

T-182

The synthesis route is as follows:

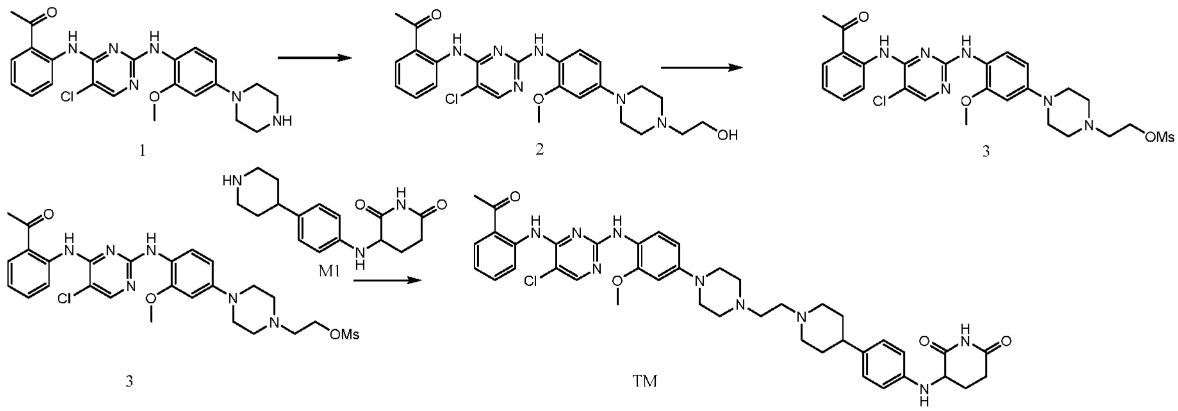

1 2 3

3 M1 TM

Synthesis of compound 2

To a reaction flask was added 200 mg of compound 1, 70 mg of 2-bromoethanol and 0.6 ml of DIEA , and dissolved with 10 ml of acetonitrile. The mixture was then heated to 70 °C and reacted for 3h under nitrogen protection. LC-MS showed the disappearance of raw material. M + H: 498. Then the reaction solution was purified by column chromatography to give 300 mg of compound 2.

Synthesis of compound 3

To a solution of 300 mg of compound 2 in 5 ml DMF was added 0.15 ml of triethylamine under an ice water bath, and 63 mg of methanesulfonyl chloride was added slowly dropwise, and then the mixture was stirred at room temperature for 4 hours. TLC detected the disappearance of most raw materials. The reaction solution was then separated by column chromatography to give 150 mg of compound 3.

Synthesis of compound T-182

To a solution of 130 mg of compound 3 in 5 ml of acetonitrile was added 0.5 ml of DIEA, followed by the addition of 80 mg of M2. Then the mixture was heated to 70°C and reacted overnight under nitrogen protection, then purified bycolumn chromatography to give 15 mg of compound T-182.1H NMR (400 MHz, DMSO-d6) δ11.87 (s, 1H), 10.84 (s, 1H), 8.82(s, 1H), 8.39 (s, 1H), 8.27 - 7.87(m, 2H), 7.42 (d, J = 28.1 Hz, 2H),7.18 (s, 1H), 7.00 (s, 2H), 6.64 (t, J= 29.8 Hz, 4H), 5.75 (s, 1H), 4.32(s, 1H), 3.79 (s, 3H), 3.23 (s, 5H),2.93 (s, 2H), 2.74 (q, J = 24.7, 23.0Hz, 10H), 2.14 (s, 2H), 1.98 - 1.65(m, 5H), 1.27 (s, 3H)

The following compounds were synthesized with reference to the method of Example 14:

| Compound No. | Structure | Characterization data: |
| --- | --- | --- |
| T-183 | 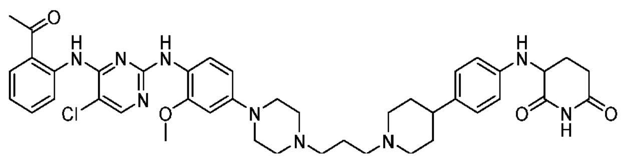 | LC-MS[M+1]:781 |

**Example 15**

The compound synthesized in the present invention:

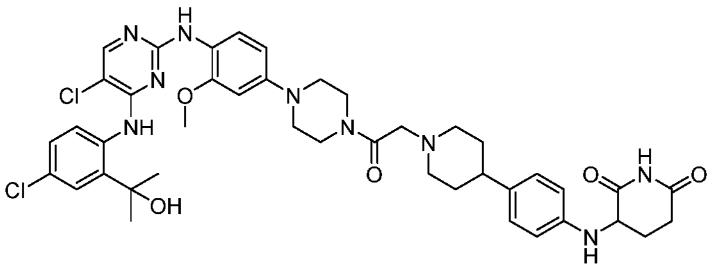

T-243

The experimental procedure is as follows:

T. Synthesis of intermediate M1

The synthesis route is as follows:

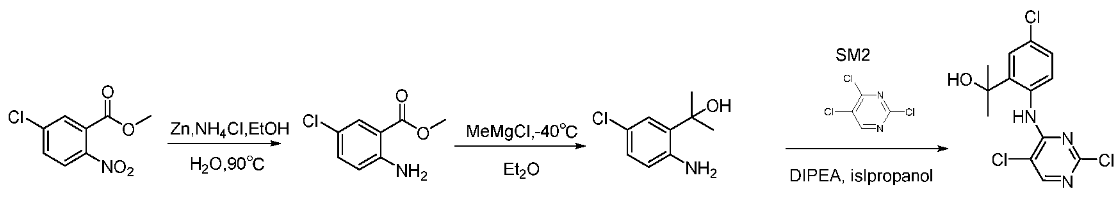


SM1 2 3 M1

T. Synthesis of compound 2

2 g of methyl 5-chloro-2-nitrobenzoate, 3.62 g of Zn powder, and 5 g of NH4Cl were dissolved in 20 ml of EtOH and 4 ml of EtOH in a 100 ml single-necked flask, then heated to 90 °C and reacted overnight. TLC monitored the reaction until complete. The reaction solution was then spun-dried directly, and mixed with silica gel and subjected to column chromatography to give 445 mg of Compound 2.LC-MS [M+1]: 186.

2. Synthesis of compound 3

To a 100 ml three-necked flask was added 8 ml of MeMgCl, and then cooled to -40 °C. A solution of 445 mg of compound 2 in 15 mL of Et2O was added dropwise, and the reaction was restored to room temperature and reacted for 1 h. TLC showed the disappearance of raw material. The reaction was then quenched by a small amount of saturated aqueous sodium bicarbonate, extracted with ethyl acetate, dried with anhydrous sodium sulphate, spun-dried and subjected to column chromatography to give 378 mg of compound 3. LC-MS [M+1]: 186.

3. Synthesis of intermediate M1

278 mg of compound 3, 276 mg of SM2, and 290 mg of DIPEA were dissolved in 4 ml of isopropanol in a 100 ml single-necked flask, and then reacted overnight at 40 °C. TLC monitored the reaction until complete. The reaction

was then quenched by a small amount of saturated aqueous sodium bicarbonate, extracted with ethyl acetate, dried with anhydrous sodium sulphate, spun-dried, and subjected to column chromatography to give 437 mg of the intermediate M1.LC-MS [M+1]: 332.

II. Synthesis of intermediate M2

The synthesis route is as follows:

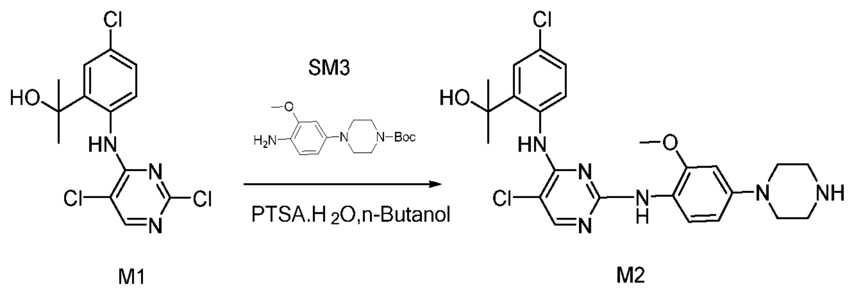


144 mg of M1, 147 mg of 4-(N-Boc-piperazine-1-yl)-2-methoxyaniline and 99 mg of p-toluenesulfonic acid monohydrate were dissolved in 2 ml of n-butanol in a 10 ml microwave tube, and the mixture was microwaved at 120°C for 20 min. TLC monitored the reaction until complete. The reaction was then quenched by a small amount of saturated aqueous sodium bicarbonate. The reaction solution was added with water, extracted with ethyl acetate, dried with anhydrous sodium sulfate, spun-dried, and subjected to column chromatography to give 150 mg of intermediate M2. LC-MS [M+1]: 503.

III. Synthesis of compound T-243

The synthesis route is as follows:

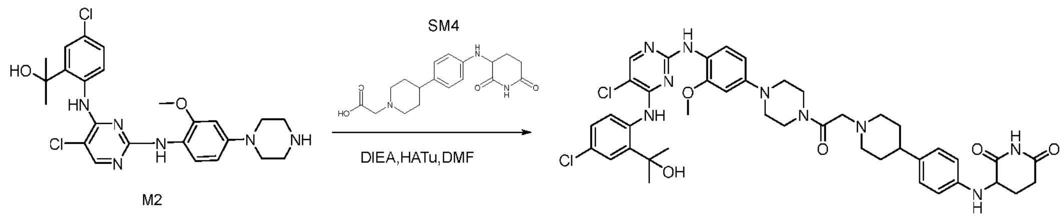


150 mg of compound M2, and 114 mg of SM4 was dissolved in 3 ml of N,N-dimethylformamide in a 50 ml round-bottom flask, and cooled down to 0 °C in an ice water bath. Then N,N-diisopropylethylamine and HATU were added under stirring and the mixture was reacted for 1 h in an ice water bath under nitrogen protection. The reaction was then naturally warmed up to room temperature and reacted for 4 h. TLC detected the completion of the reaction. The reaction solution was then extracted with dichloromethane and water; the organic phase was dried and subjected to column chromatography to give 33 mg of compound T-243, LC-MS [M+1]: 831.1HNMR (400 MHz, DMSO-d6) δ 10.77 (s, 1H), 10.45 (d, J = 6.3 Hz, 1H), 8.23 (d, J = 8.7 Hz, 1H), 8.16 - 7.98 (m, 2H), 7.43 (d, J = 8.6 Hz, 1H), 7.24 (d, J = 2.4 Hz, 1H), 7.14 (d, J = 8.8 Hz, 1H), 6.94 (d, J = 8.3 Hz, 2H), 6.69 (d, J = 2.5 Hz, 1H), 6.59 (d, J= 8.2 Hz, 2H), 6.52 (dd, J = 8.7, 2.5 Hz, 1H), 6.31 (d, J = 8.1 Hz, 1H), 5.65 (d, J = 7.5 Hz, 1H), 4.25 (dt, J = 11.4, 5.8 Hz, 1H), 3.79 - 3.76 (m, 3H), 3.63 (s, 1H), 3.51 (d, J = 0.9 Hz, 2H), 3.32 (s, 6H), 3.21 (s, 4H), 3.12 (s, 2H), 2.93 (d, J = 10.8 Hz, 2H), 2.08 (d, J = 12.2 Hz, 2H), 1.85 (dq, J = 19.5, 7.4, 6.2 Hz, 1H), 1.70 (d, J = 12.0 Hz, 2H), 1.51 (s, 7H).

The following compounds were synthesized with reference to the method of Example 15:

| Compound No. | Structure | Characterization data: |
|---|---|---|
| T-196 | 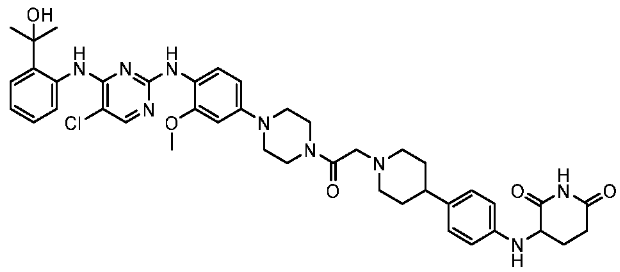 | LC-MS[M+1]:797 |
| T-199 | 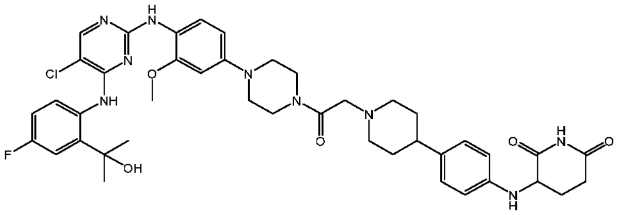 | LC-MS[M+1]:815 |

**Example 16**

The compound synthesized in the present invention:

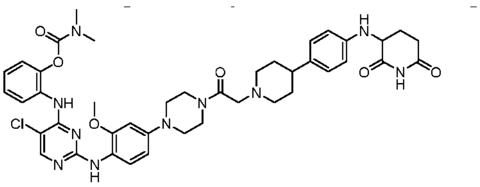

T-370

The experimental procedure is as follows:

T. Synthesis of intermediate M1

The synthesis route is as follows:

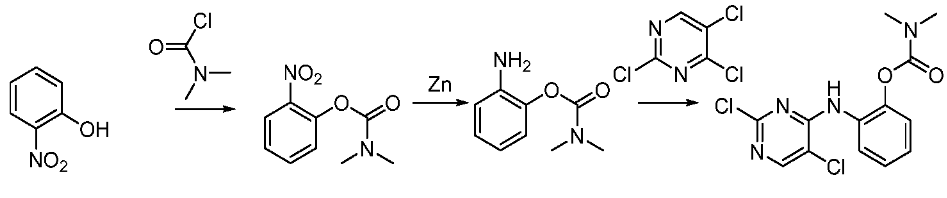


1. Synthesis of compound SM2

1 g of *o*-nitrophenol and 7 g of cesium carbonate were dissolved in 10 ml of acetone in a 100 ml three-necked flask, displaced with $N_2$ three times, and cooled to 0 °C in an ice bath. 928 mg of dimethylcarbamoyl chloride was added dropwise, and then the reaction was naturally warmed and reacted for 48h. TLC monitored the reaction until complete. The reaction was then added water, and extracted with ethyl acetate; the organic phase was washed with saturated NaCl solution, dried with anhydrous sodium sulfate, spun-dried, and subjected to column chromatography to give 6 g of crude compound SM2.LC-MS [M+1]: 211.

2. Synthesis of compound SM3

900 mg of compound SM2, 2.2 g of ammonium chloride and 1.7 g of zinc powder were dispersed in 12 ml solvent of ethanol: water 5:1 in a 100 ml three-necked flask, then heated to reflux and reacted for 18h. TLC showed the disappearance of raw material. Solvent was then removed by rotary evaporation, and the residue was added with water and pH was adjusted to 7-8 with a small amount of saturated aqueous sodium bicarbonate, and then the resulting solution was extracted with ethyl acetate, dried with anhydrous sodium sulfate, spun-dried, and subjected to column chromatography to give 500mg of compound SM3, as wine red oil. LC-MS [M+1]: 181.

3. Synthesis of intermediate M1

500 mg of SM3, 286 mg of 2,4,5-trichloropyrimidine and 403.2 mg of DIPEA were dissolved in 5 ml of n-butanol in a 100 ml three-necked flask, and reacted overnight at room temperature. TLC monitored the reaction until complete. Solvent was then removed by rotary evaporation, and the residue was extracted with water and ethyl acetate; the organic phase was washed with saturated NaCl solution, dried with anhydrous sodium sulfate, spun-dried, and subjected to column chromatography to give 260mg of the intermediate M1.LC-MS [M+1]:327.

II. Synthesis of compound T-370

The synthesis route is as follows:

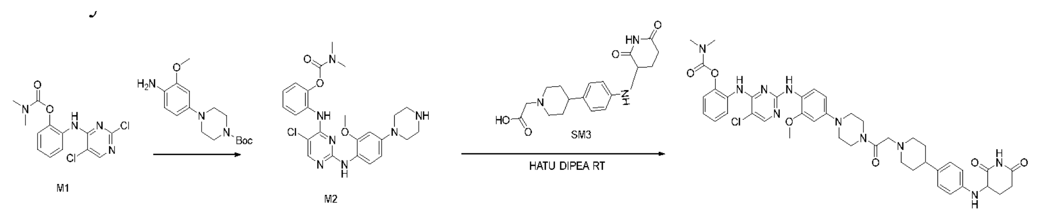


T. Synthesis of compound M2

150mg of M1, 124 mg of tert-butyl 4-(4-amino -3-methoxyphenyl)piperazine -1-carboxylate and 105mg of p-toluenesulfonic acid monohydrate were dissolved in 2ml of n-butanol in a 10 ml microwave tube, and the mixture was microwaved at 120 °C for 20 min. TLC monitored the reaction until complete. The reaction solution was then transferred to a 125 ml separatory funnel, added with water, and extracted with ethyl acetate; the organic phase was washed with saturated NaCl solution, dried with anhydrous sodium sulfate, spun-dried, and subjected to column chromatography to give 130 mg of intermediate M2.LC-MS [M+1]: 498.

2. Synthesis of compound T-152

130 mg of compound M2 and 225mg of 40% SM3 were dissolved in 3 ml of N, N-dimethylformamide in a 50 ml round-bottom flask, and cooled down to 0°C in an ice-water bath. 168 mg of N,N-diisopropylethylamine and 119 mg of HATU were added under stirring, and the mixture was reacted for 1 h in an ice-water bath under nitrogen protection. Then the reaction was naturally warmed up to room temperature. TLC detected the completion of the reaction. The reaction solution was then extracted with ethyl acetate and water; the organic phase was dried, filtered, and subjected to column chromatography to give 100mg of compound T-152, LC-MS [M+1]: 825.1H NMR (400 MHz, DMSO-d6) $\delta$ 10.79 (s, 1H), 8.41 (s, 1H), 8.08 (s, 1H), 7.65 (dt, J = 10.4, 3.4 Hz, 3H), 7.28 - 7.15 (m, 3H), 6.95 (d, J = 8.1 Hz, 2H), 6.69 - 6.55 (m, 3H), 6.37 (d, J = 8.8 Hz, 1H), 5.68 (d, J = 7.4 Hz, 1H), 4.26 (dt, J = 12.1, 6.6 Hz, 1H), 3.79 (s, 3H), 3.72 (s, 2H), 3.62 (s, 2H), 3.33 (s, 2H), 3.15 (d, J = 6.0 Hz, 2H), 3.06 (s, 2H), 2.81 (d, J = 3.1 Hz, 6H), 2.65 - 2.52 (m, 4H), 2.33 (q, J = 1.9 Hz, 1H), 2.16 - 2.03 (m, 2H), 1.85 (qd, J = 12.2, 4.6 Hz, 2H), 1.72 (s, 2H), 1.61 (s, 2H).

**Example 17**

The compound synthesized in the present invention:

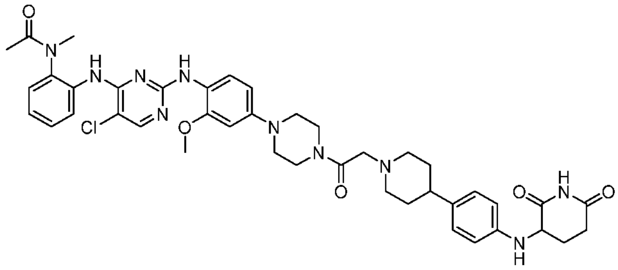

T-206

The experimental procedure is as follows:

Synthesis of intermediate SM5

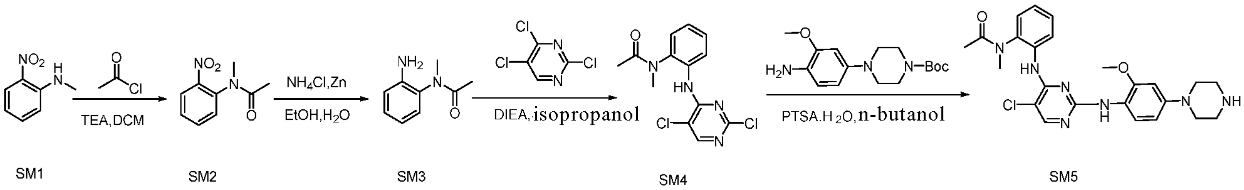


T. Synthesis of SM2

500mg of N-methyl-2-nitroaniline and 1g of TEA were dissolved in 5ml of dichloromethane in a 50ml single-necked flask, replaced with nitrogen, and cooled to 0°C. 5.2g of acetyl chloride was added, and the mixture was restored to room temperature and reacted for 2h. TLC monitored the reaction until complete. the reaction solution was then added with water, extracted with EA, dried with anhydrous sodium sulfate, and mixed with silica gel and subjected to column chromatography to give 613mg SM2.LC- MS[M+1]:195.

2. Synthesis of SM3

790mg of SM2, 2.16g of ammonium chloride, and 1.59g of zinc powder were dispersed in 6 ml solvent of ethanol: water 5:1 in a 50ml single-necked flask, then heated to reflux and reacted for 18h. TLC monitored the disappearance of raw material. Solvent was then removed by rotary evaporation, the residue was added with water and pH was adjusted to 7-8 with a small amount of saturated aqueous sodium bicarbonate. Then the resulting solution was extracted with ethyl acetate, dried with anhydrous sodium sulfate, spun-dried, and subjected to column chromatography to give 412mg SM3.LC-MS [M+1]: 165.

3. Synthesis of SM4

To a 50ml single-necked flask was added 150mg of SM3, 168mg of 2,4,5-trichloropyrimidine, 177mg of DIEA and 3ml of isopropanol, replaced with nitrogen, heated to 100°C and reacted overnight. TLC monitored the reaction until complete. The reaction solution was then added with water, extracted with EA, dried with anhydrous sodium sulfate, and mixed with silica gel and subjected to column chromatography to give 98mg of SM4.LC- MS[M+1]:311.

4. Synthesis of SM5

98 mg of SM4, 97 mg of intermediate and 72 mg of p-toluenesulfonic acid monohydrate were dissolved in 2 ml of n-butanol in a 10 ml microwave tube, and the mixture was microwaved at 120 °C for 20 min. TLC monitored the reaction until complete. The reaction solution was then adjusted to be basic using saturated sodium bicarbonate, extracted with dichloromethane, dried with anhydrous sodium sulfate, and mixed with silica gel and subjected to column chromatography to give 94 mg of SM5. LC- MS[M+1]:482.

5. Synthesis of TM

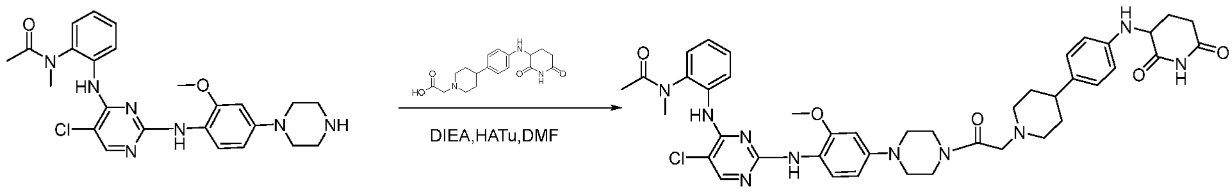


SM5

TM

94mg of SM5, and 135mg of intermediate 2 were dissolved in 2 ml of DMF in a 50 ml single-necked flask, and 126 mg DIEA was added. The mixture was replaced with nitrogen, cooled to 0 °C, and then added with 97mg of HATu. The reaction was restored to room temperature and reacted for 3 h. TLC monitored the reaction until complete. The reaction solution was then added water, extracted with EA, dried with anhydrous sodium sulfate, mixed swith silica gel and subjected to column chromatography, and then purified by thin layer chromatography to give 82mg of TM (i.e. T-206).LC- MS[M+1]:809.

1H NMR (400 MHz, DMSO-d6) δ 10.78 (s, 1H), 8.36 (s, 1H), 8.06 (d, J = 4.5 Hz, 1H), 7.81 - 7.66 (m, 2H), 7.43 (d, J = 8.6 Hz, 1H), 7.37 (d, J = 7.7 Hz, 2H), 7.28 (t, J = 7.4 Hz, 1H), 6.95 (d, J = 8.1 Hz, 2H), 6.68 - 6.55 (m, 3H), 6.32 (d, J = 8.9 Hz, 1H), 5.66 (d, J = 7.5 Hz, 1H), 4.25 (ddd, J= 11.7, 7.5, 4.8 Hz, 1H), 3.75 (d, J = 10.6 Hz, 5H), 3.61 (s, 2H), 3.22 (s, 2H), 3.14 (s, 2H), 3.05 (s, 2H), 2.99 (s, 3H), 2.94 (s, 2H), 2.80 - 2.68 (m, 2H), 2.33 (s, 1H), 2.25 - 2.03 (m, 4H), 1.92 - 1.79 (m, 2H), 1.71 (s, 5H), 1.58 (d, J = 12.7 Hz, 2H).

17: The following compounds were synthesized with reference to the method of Example

| Compound No. | Structure | Characterization data: |
|---|---|---|
| T-195 | 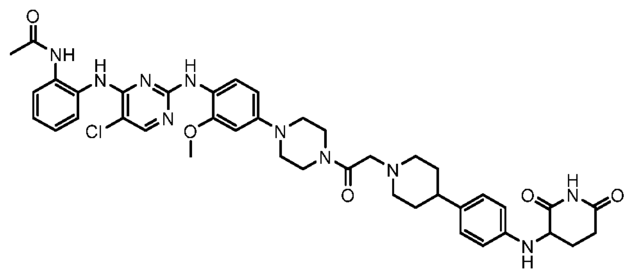 | LC-MS[M+1]:796 |
| T-202 | 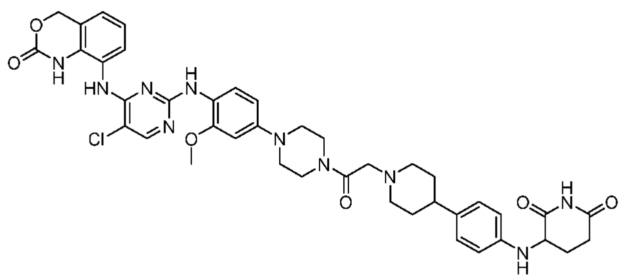 | LC-MS[M+1]:810 |
| T-211 | 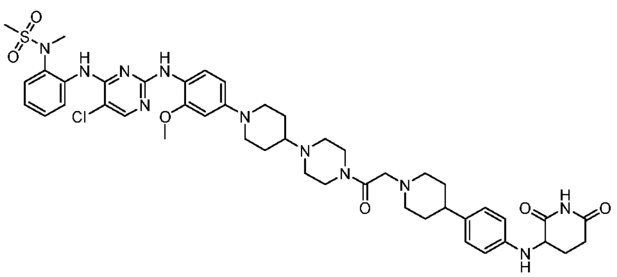 | LC-MS[M+1]:929 |

(continued)

| Compound No. | Structure | Characterization data: |
|---|---|---|
| T-218 | 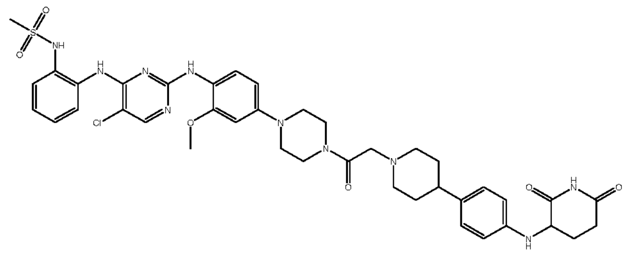 | LC-MS[M+1]:832 |
| T-220 | 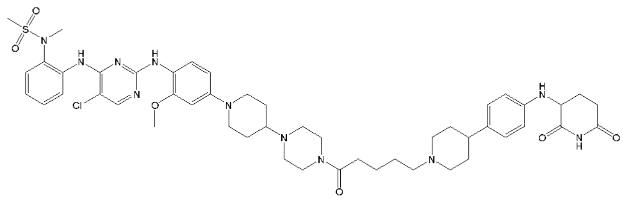 | LC-MS[M+1]:970 |
| T-222 | 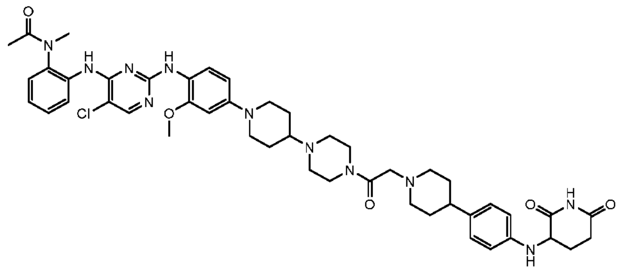 | LC-MS[M+1]:893 |
| T-227 | 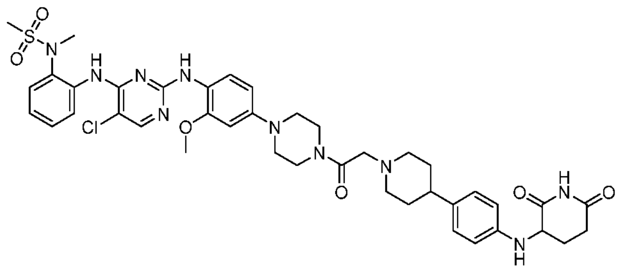 | LC-MS[M+1]:846 |
| T-340 | 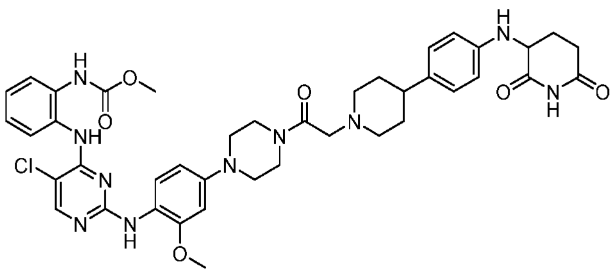 | LC-MS[M+1]:812 |

**Example 18**

The compound synthesized in the present invention:

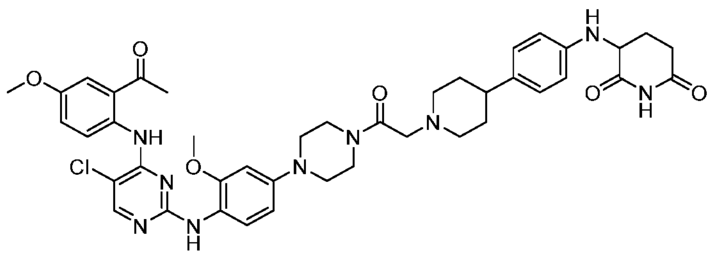

T-249

The experimental procedure is as follows:

T. Synthesis of intermediate M1

The synthesis route is as follows:

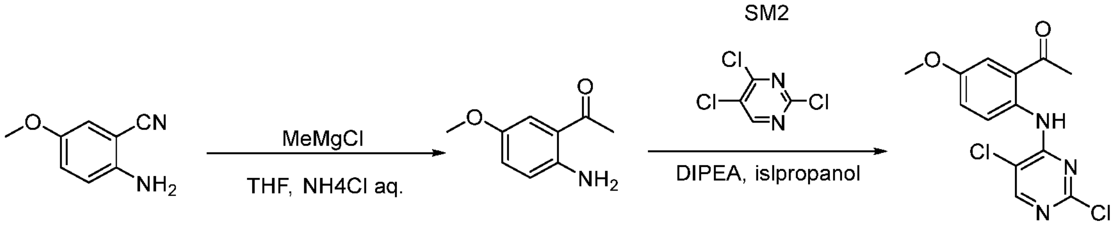


SM1 2 M1

T. Synthesis of compound 2

To a 100 ml three-necked flask was added 4 ml of ultra-dry THF, and 2.25 mL of MeMgCl was injected after $N_2$ gas exchanges for three times. A solution of 200 mg of 2-amino-5-methoxybenzonitrile in 2 ml of ultra-dry THF was slowly injected into the flask under an ice bath at 0 °C, and then the mixture was naturally warmed up to room temperature and reacted overnight. The reaction was then cooled to 0 °C in an ice bath, 20 ml of saturated aqueous NH4Cl was added dropwise, and then the reaction was restored to room temperature under high speed stirring for 30 min. TLC monitored the reaction until new spot was generated. The reaction solution was then added with water, extracted with dichloromethane, dried with anhydrous sodium sulfate, and subjected to column chromatography to give 26mg of compound 2.LC-MS [M+1]: 166.

2. Synthesis of intermediate M1

26mg of compound 2, 32 mg of SM2, and 31 mg of DIPEA were dissolved in 2 ml of isopropanol in a 50ml single-necked flask, and then reacted overnight at 80°C. TLC monitored the reaction until complete. The reaction solution was then quenched by a small amount of saturated aqueous sodium bicarbonate, extracted with ethyl acetate, dried with anhydrous sodium sulphate, spun-dried, and subjected to column chromatography to give 25mg of the intermediate M1.LC-MS [M+1]: 312.

II. Synthesis of intermediate M2

The synthesis route is as follows:

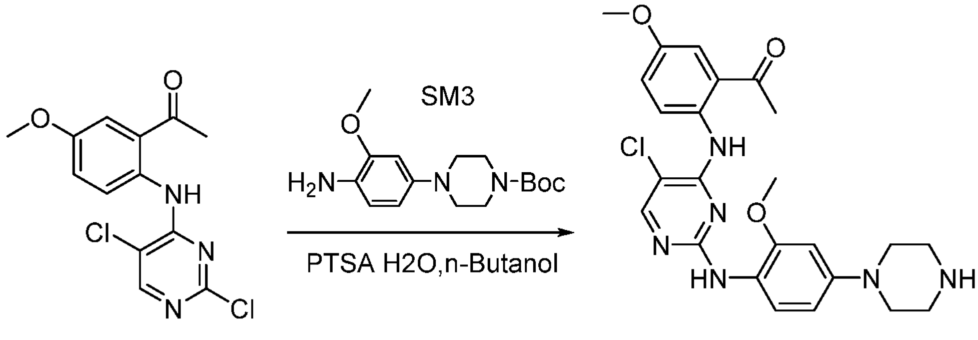


M1 M2

25 mg of M1, 28 mg of 4-(N-Boc-piperazine-1-yl)-2-methoxyaniline and 19 mg of p-toluenesulfonic acid monohydrate were dissolved in 2 ml of n-butanol in a 10 ml microwave tube, and the mixture was microwaved at 120°C for 20 min. TLC monitored the reaction until complete. The reaction was then quenched by a small amount of saturated aqueous sodium bicarbonate. The reaction solution was added with water, extracted with ethyl acetate, dried with anhydrous sodium sulfate, spun-dried, and subjected to column chromatography to give 27mg of intermediate M2.LC-MS [M+1]: 483.

III. Synthesis of compound T-249

The synthesis route is as follows:

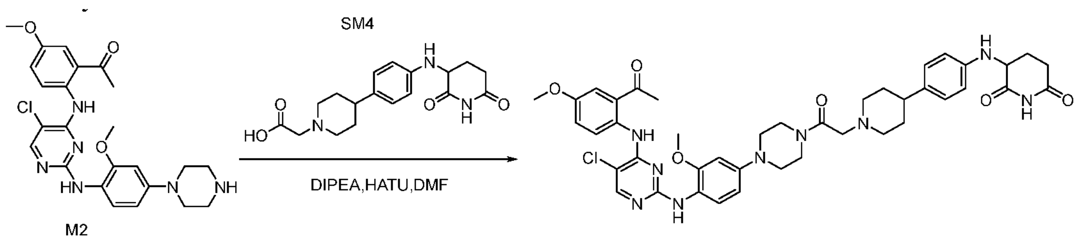


M2

27 mg of compound M2, and 32 mg of SM4 was dissolved in 2 ml of N,N-dimethylformamide in a 50 ml round-bottom flask, and cooled down to 0 °C in an ice water bath. Then N,N-diisopropylethylamine and HATU were added under stirring and reacted for 1 h in an ice water bath under nitrogen protection. The reaction was then naturally warmed up to room temperature and reacted for 4 h. TLC detected the completion of the reaction. The reaction solution was then extracted with dichloromethane and water; the organic phase was dried and subjected to column chromatography to give 13mg of compound T-249, LC-MS [M+1]: 810.1H NMR (400 MHz, DMSO-d6) δ 11.42 (s, 1H), 10.84 (s, 1H), 8.68 (s, 1H), 8.28 (s, 1H), 8.18 (s, 1H), 7.58 (d, J = 3.0 Hz, 1H), 7.46 (d, J = 8.6 Hz, 1H), 7.09 (d, J = 9.3 Hz, 1H), 7.01 (d, J = 8.1 Hz, 2H), 6.78 (d, J = 2.5 Hz, 1H), 6.70 - 6.57 (m, 3H), 5.73 (d, J = 7.6 Hz, 1H), 4.32 (dt, J = 12.1, 6.4 Hz, 1H), 3.87 (s, 3H), 3.83 (s, 3H), 3.70 (s, 2H), 3.57 (s, 1H), 3.30 (s, 2H), 3.21 (s, 2H), 3.04 (s, 2H), 2.74 (s, 3H), 2.65 (d, J = 4.4 Hz, 1H), 2.16 (dt, J = 13.0, 4.5 Hz, 2H), 1.92 (tt, J = 12.0, 5.9 Hz, 2H), 1.79 (s, 2H), 1.68 (s, 2H), 1.54 (s, 1H), 1.40 (d, J = 4.2 Hz, 1H), 1.24 (t, J = 7.2 Hz, 1H).

**Example 19**

Compounds synthesized in reference:

T. Referring to WO 2021/127561 A1(R1) of C4 Company

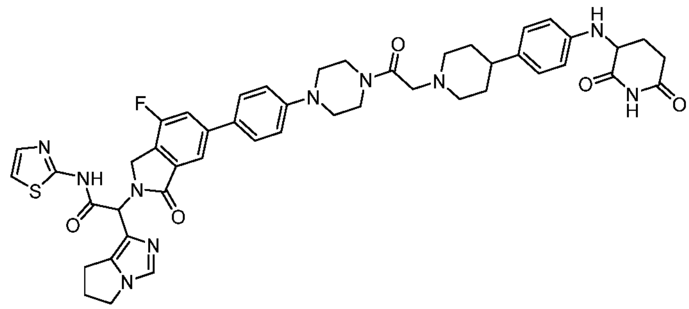

Comparative Compound 1 (R1, Example 5) Referring to WO2022/012622 A1(R2) of BeiGene

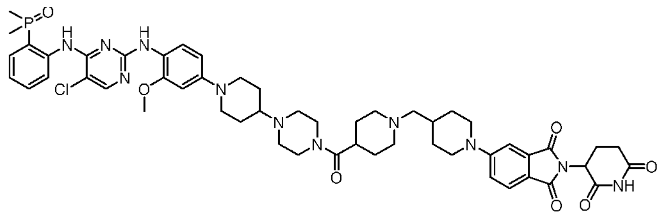

Comparative Compound 2 (R2, Example 113)

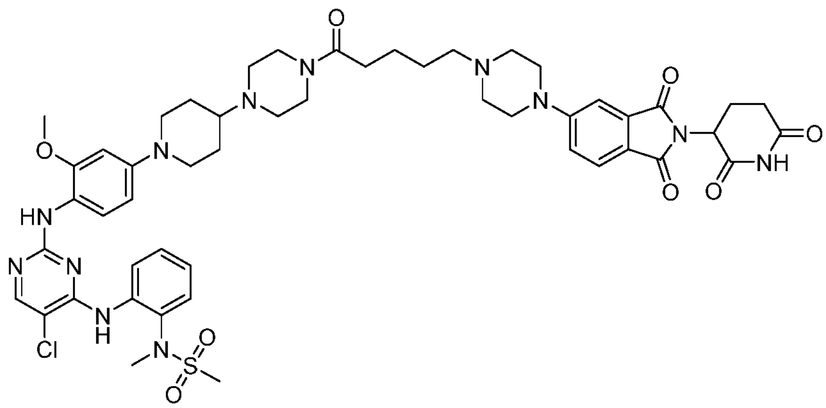

Comparative Compound 3 (R2, Example 128)

**Test of compounds for cell growth inhibitory activity.**

**Test example 1 Cell antiproliferative assay**

2.1 Experimental steps:

Experimental materials and equipment:
H1975: with EGFR: L858R/T790M double mutation; PC-9 with EGFR exon 19 deletion, HCC827 with exon 19 deletion mutation. CellCounting-Lite2.0, Trypsin EDTA, 37°C $CO_2$ incubator, cell counter, EnVision Serial No.1050454. II. Experimental preparation:

1. 96-well plate spreading

(A) Trypsin EDTA was used to digest log-phase cells, medium was added to terminate the reaction, and a cell suspension was made by blowing and mixing with a pipette.
(B) Cell concentration was determined using Vi-cell, and a suspension of 15,000-25,000 cells per milliliter was made

depending on the purpose of the experiment and the characteristics of the cells.

(C) After the cell suspension was prepared, it was gently mixed and 100 microliters per well was added so that the density of cells to be tested was 1500-2500 per well.

2. Compound treatment

Compound dilution

A) About 2 mg of compound was weighed, and the volume of DMSO required was calculated according to the formula: compound mass (mg)*compound purity (%)/compound molecular weight* 1000

(B) The inoculated cell culture plates were incubated in an incubator and the compounds with concentration gradient were added after about 24 hours.

(C) 10Mm of compound stock solution was diluted to 50Mm with medium, and 50Mm of compound solution was added sequentially to the second column of the deep-well plate, and 375M1 of medium containing 0.5% DMSO was added to the third to eleventh columns.

D) Gradient dilution: 125 μl of solution was pipetted from the second column and added to the third column, after mixing; another 125 μl of solution was pipetted from the third column and added to the fourth column, and the operation was repeated until the tenth column.

(E) 25 μl of compound was pipetted from the deep-well plate into a 96-well culture plate using a multichannel pipette, and each compound was repeated three times on the 96-well plate. A maximum concentration of 10,000 nm with concentration gradient of 1:4 was eventually formed on the 96-well plate.

3. Addition of CTG and readings

(A) 96-well plates were incubated in an incubator for 72 h. The effect of the compounds was observed under an inverted microscope.

(B) 25 μl of CTG solution was added to each well, the plate was put on shaker for 10 minutes and the OD value of each well was read.

4. Data analysis

The cell viability rate (% Cell Viability) was calculated using the following formula:

$$\%\text{Cell Viability}=100\%\times(\text{Lum_Sample- Lum_LC})/(\text{Lum_HC-Lum_LC})$$

Lum_HC: Cell reading of 0.1% DMSO control

Lum_Sample: Cell reading with compound added

Lum_LC: Reading of Blank media

$IC_{50}$ values were obtained by curve fitting with GraphPad Prism 8 software.

As shown in Table 2, where AA≤10nM; 10nM<A≤100nM; 100nM<B<1000nM; C≥1000nM.

Table 2

| Compound | H1975 (nM) | PC-9 (nM) | HCC827 (nM) |
|---|---|---|---|
| T-01 | A | B | B |
| T-02 | A | B | C |
| T-04 | A | B | C |
| T-09 | A | C | C |
| T-12 | A | B | B |

(continued)

| Compound | H1975 (nM) | PC-9 (nM) | HCC827 (nM) |
|---|---|---|---|
| T-13 | A | B | B |
| T-14 | A | C | C |
| T-46 | A | C | B |
| T-47 | A | B | A |
| T-51 | A | C | C |
| T-52 | A | C | B |
| T-57 | A | B | B |
| T-58 | A | B | B |
| T-59 | A | B | B |
| T-60 | A | B | B |
| T-63 | A | B | B |
| T-66 | A | C | B |
| T-69 | B | B | B |
| T-82 | B | C | C |
| T-84 | A | B | B |
| T-91 | A | B | A |
| T-100 | B | B | B |
| T-101 | B | B | B |
| T-109 | B | C | C |
| T-121 | B | C | B |
| T-122 | B | B | B |
| T-124 | B | C | C |
| T-125 | B | C | B |
| T-132 | B | C | B |
| T-134 | A | C | C |
| T-135 | B | C | B |
| T-139 | B | C | B |
| T-140 | A | B | B |
| T-152 | B | C | C |
| T-163 | A | B | B |
| T-166 | B | C | C |
| T-167 | B | C | C |
| T-168 | B | C | C |
| T-169 | B | B | B |
| T-170 | B | C | C |
| T-171 | A | C | B |
| T-172 | B | C | A |
| T-173 | B | C | B |

(continued)

| Compound | H1975 (nM) | PC-9 (nM) | HCC827 (nM) |
|---|---|---|---|
| T-174 | B | C | C |
| T-179 | B | C | C |
| T-180 | B | C | C |
| T-181 | B | B | C |
| T-182 | A | B | B |
| T-183 | B | - | - |
| T-184 | AA | B | A |
| T-185 | AA | B | A |
| T-188 | A | B | B |
| T-189 | A | B | B |
| T-190 | A | B | B |
| T-191 | A | B | B |
| T-193 | B | B | B |
| T-194 | B | - | - |
| T-195 | A | B | B |
| T-196 | A | B | B |
| T-197 | B | - | - |
| T-198 | AA | B | B |
| T-199 | A | B | B |
| T-200 | A | B | B |
| T-201 | B | - | - |
| T-203 | A | B | A |
| T-204 | B | - | - |
| T-208 | B | - | - |
| T-211 | A | B | A |
| T-212 | B | C | B |
| T-214 | B | C | B |
| T-218 | A | B | A |
| T-220 | A | B | B |
| T-221 | B | C | C |
| T-224 | A | B | B |
| T-225 | B | C | C |
| T-226 | B | C | B |
| T-227 | AA | A | A |
| T-228 | A | - | - |
| T-230 | B | - | - |
| T-231 | B | C | A |
| T-233 | A | B | A |

(continued)

| Compound | H1975 (nM) | PC-9 (nM) | HCC827 (nM) |
|---|---|---|---|
| T-237 | C | B | B |
| T-238 | B | - | - |
| T-239 | C | C | B |
| T-240 | B | B | B |
| T-241 | AA | B | B |
| T-242 | B | B | B |
| T-243 | AA | A | A |
| T-244 | A | A | A |
| T-246 | AA | A | A |
| T-249 | AA | A | A |
| T-254 | AA | A | A |
| T-255 | AA | A | A |
| T-259 | A | B | B |
| T-267 | B | B | B |
| T-268 | A | A | A |
| T-272 | AA | A | A |
| T-282 | A | A | A |
| T-283 | AA | A | A |
| T-286 | A | A | A |
| T-289 | A | A | A |
| T-292 | AA | A | A |
| T-295 | AA | A | A |
| T-300 | A | A | A |
| T-303 | A | A | A |
| T-313 | AA | A | A |
| T-326 | B | - | - |
| T-327 | A | B | B |
| T-328 | B | B | B |
| T-330 | B | B | B |
| T-331 | B | B | C |
| T-332 | B | B | B |
| T-333 | B | B | B |
| T-334 | B | B | B |
| T-335 | B | B | B |
| T-336 | B | B | B |
| T-337 | B | B | B |
| T-338 | B | B | B |
| T-339 | B | B | B |

(continued)

| Compound | H1975 (nM) | PC-9 (nM) | HCC827 (nM) |
|---|---|---|---|
| T-340 | B | - | - |
| T-341 | A | C | B |
| T-342 | A | B | A |
| T-343 | A | A | A |
| T-353 | A | B | B |
| T-355 | A | A | A |
| T-356 | A | A | A |
| T-357 | A | A | A |
| T-359 | A | A | A |
| T-361 | A | - | - |
| T-362 | A | - | - |
| T-363 | A | - | - |
| T-364 | A | - | - |
| T-367 | A | A | A |
| T-368 | A | A | A |
| T-369 | A | A | A |
| T-374 | A | - | - |
| T-375 | A | - | - |
| T-377 | A | - | - |
| T-378 | A | - | - |
| T-381 | A | - | - |
| Comparative Compound 1 | B | C | C |
| Comparative Compound 2 | B | C | B |
| Comparative Compound 3 | B | B | A |

As can be seen from Table 2, the compounds of the present invention showed very good inhibitory effects on H1975 (human lung adenocarcinoma cells), PC-9 (human lung cancer cells) and HCC827 (human non-small cell lung cancer cells).

Table 3

| Compound | H1975 (nM) | PC-9 (nM) | HCC827 (nM) |
|---|---|---|---|
| T-12 | 34.33 | 324.1 | 110.3 |
| T-184 | 7.72 | 121.9 | 14.21 |
| Comparative Compound 1 | 548.2 | >5000 | >5000 |
| Comparative Compound 2 | 186.1 | 1944 | 220.6 |
| Comparative Compound 3 | 386.3 | 681.6 | 72.45 |

As can be seen from Table 3, the compounds T-12 and T-184 of the present invention have a better inhibitory effect on H1975 (human lung adenocarcinoma cells), PC-9 (human lung cancer cells), and HCC827 (human non-small cell lung cancer cells) than the compounds of C4 Company and BeiGene.

The biological activity of our compounds was also tested on EGFR non-classical mutant cell lines with mutations,

such as exon20 insertion mutation, L861Q, DEL19-G724S, L858R-L792H, etc., and the compounds of the present invention also showed very good inhibitory effect.

**Test example 2** EGFR PROTAC in cell western assay

1. Experimental Materials

HCC827 cell culture medium was purchased from Corning. Cell line HCC827 was purchased from BeNa Culture Collection; EGF Receptor (D38B1) XP Rabbit mAb antibody and β-Actin (8H10D10) Mouse mAb antibody were from CST. IRDye 800CW, IRDye 680RD, and Intercept blocking solution (PBS) were purchased from Licor. Triton-100 was purchased from sigma, and 96-well black cell culture imaging microtiter plates were purchased from Agilent.

2. Experimental method

1)HCC827 cells were cell counted and cells were inoculated into 96-well black cell culture imaging microtiter plates at a density of 40,000 cells per well, with 100 μl per well. The plate was incubated overnight in a $CO_2$ incubator.
2)Day1: A gradient dilution of the compound to be tested was added to the cells of the culture plate (with a starting concentration of 3 Mm, 9 concentrations, a dilution ratio of 1:3, 2 duplicate wells), with a final concentration of DMSO being 0.5%, and a blank control of DMSO without compound. The plate was placed in the cell culture incubator for a final 16 hours of incubation.
3) The culture medium was removed from the 96 wells with a pipette and the residual medium was washed with 200 ml of PBS per well on a shaker for 5 min. After the removal of PBS, cells were quickly fixed by adding 150 Ul of fresh fixation buffer (3.7% paraformaldehyde) and incubated for 20 min at room temperature.
4) The fixation buffer was removed and 200 M1 of Triton elution buffer(0.3% Triton-100) was added to wash for four times on a shaker for 5 minutes each time.
5)150 Ul LI-COR Odyssey blocking solution was added and the cells were incubated for 1.5 hours at room temperature with slow shaking on a shaker.
6) To block the samples, a primary antibody against EGFR and Actin were prepared using Antibody Dilution Buffer at a ratio of 1:300. The blocking solution was poured out and the diluted primary antibody was added (at a total volume of 50 μl/per well). The cell plate was covered and incubated at 4°C overnight.
7) The cell plate was washed four times with PBS+Triton X-100 for 5 minutes each time.
8) Fluorescently labeled secondary antibodies IRDye® 680RD and IRDye® 800CW were diluted with Antibody Dilution Buffer (with a ratio of 1:600, at a total volume of 50 μl/well) and then incubated for one hour at room temperature, protected from light.
9) The cell plate was washed four times with PBS+Triton X-100 for 5 minutes each time.

3. Calculation

After pipetting the PBS thoroughly, the plate was inverted and imaged on a Burroughs imager. A multiple exposure program was selected, IRDye 680RD Blot was chosen for the first channel, and IRDye 800CW Blot was chosen for the second channel. The images were processed using the software Image Studio Lite Ver5.2 to analyze the signal value of each well, and the signal values of EGFR and Actin could be displayed by circling each well. The blank group was the DMSO group, EGFR/Actin was calculated as the EGFR value of the corresponding sample wells, and the degradation rate = 100-100*EGFR signal/DMSO signal. The $DC_{50}$ and $D_{max}$ of the degraded EGFR proteins were calculated by fitting curves using the Prism software, 3-parameter method.

As shown in Table 4, for DC50, AA≤10nM; 10nM<A≤100nM; 100nM<B<1000nM; C≥1000nM; for Dmax, 70%≤A≤100%; 50%≤B<70%; C<50%; NA is not tested.

Table 4

| Compound | HCC827 | |
|---|---|---|
| | $DC_{50}$ (nM) | $D_{max}$(%) |
| T-12 | A | A |
| T-13 | AA | A |

(continued)

| Compound | HCC827 | |
|---|---|---|
| | $DC_{50}$ (nM) | $D_{max}$(%) |
| T-47 | A | B |
| T-57 | A | B |
| T-60 | A | B |
| T-63 | B | B |
| T-66 | A | C |
| T-84 | A | A |
| T-91 | A | B |
| T-101 | A | B |
| T-121 | B | B |
| T-125 | A | C |
| T-130 | B | C |
| T-131 | A | C |
| T-163 | B | B |
| T-169 | C | C |
| T-172 | A | C |
| T-182 | A | B |
| T-184 | A | A |
| T-185 | AA | A |
| T-190 | C | B |
| T-191 | B | B |
| T-198 | AA | B |
| T-203 | A | B |
| T-211 | A | B |
| T-220 | A | C |
| T-221 | B | C |
| T-224 | A | C |
| T-227 | A | B |
| T-228 | B | C |
| T-233 | A | B |
| T-246 | AA | A |
| T-254 | A | A |
| T-333 | A | C |
| T-334 | B | C |
| T-335 | B | B |
| T-336 | A | C |
| Comparative Compound 1 | C | C |
| Comparative Compound 2 | A | B |

(continued)

| Compound | HCC827 | |
|---|---|---|
| | $DC_{50}$ (nM) | $D_{max}$(%) |
| Comparative Compound 3 | A | B |

Table 5

| Compound | HCC827 | |
|---|---|---|
| | $DC_{50}$ (nM) | Dmax(%) |
| T-12 | 15.56 | 70.5 |
| T-184 | 10.18 | 70.8 |
| Comparative Compound 2 | 22.28 | 64.7 |
| Comparative Compound 3 | 15.22 | 56.4 |

As can be seen from Table 5, All compounds of the present invention exhibited better degradation effect on HCC827 than the control compounds.

All documents referred to in the present invention are incorporated by reference herein as if each document is individually incorporated by reference. Further, it should be understood that upon reading the above teaching of the present invention, various modifications or alternations may be made to the present invention by those skilled in the art, and those equivalents also fall within the scope defined by the appended claims of the present application.

## Claims

1. A compound, wherein the compound is a compound of Formula I, or a pharmaceutically acceptable salt thereof, a stereoisomer, a tautomer, a hydrate, a solvate, an isotonic comnound. or a prodrug thereof.

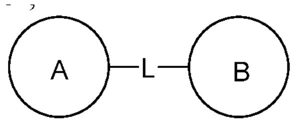

I

wherein,

L is selected from the group consisting of:

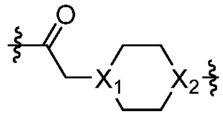
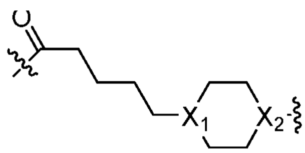
,

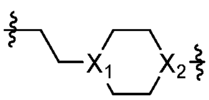
,
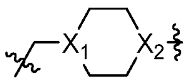
,
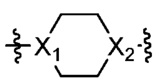
,
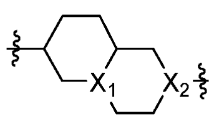
,
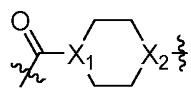
,

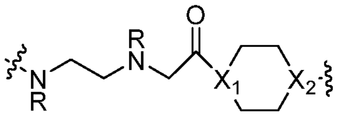, 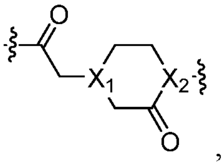, 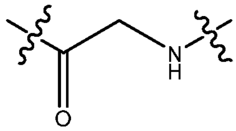, 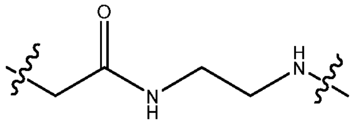,

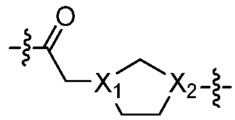, 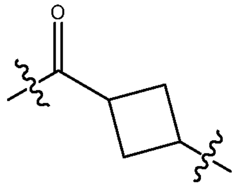, 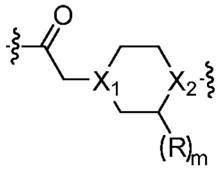 and 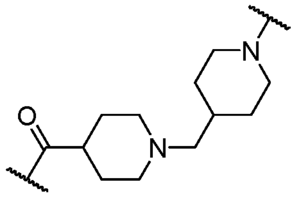;

A is selected from the group consisting of:

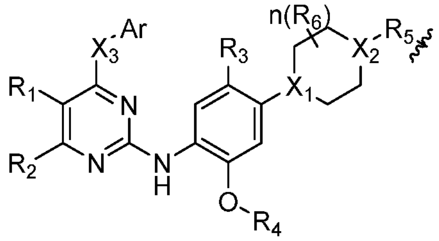,

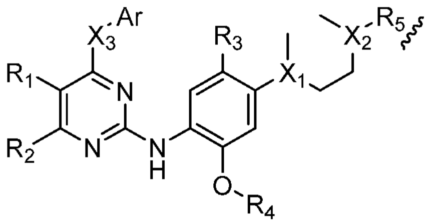, 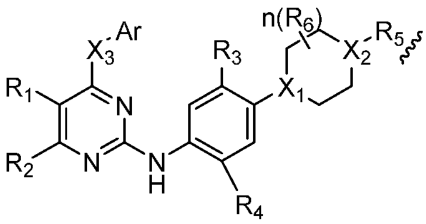, 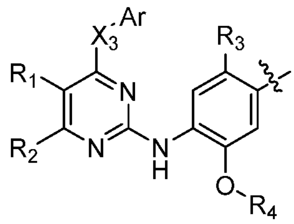,

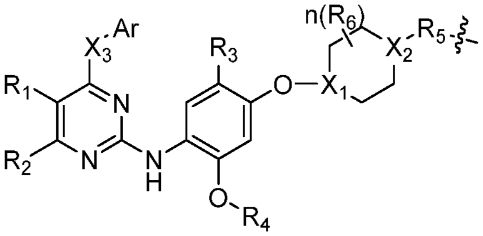, 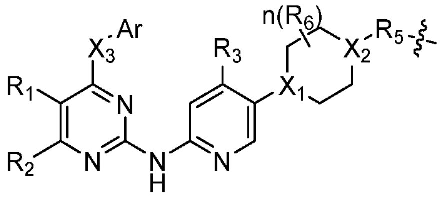,

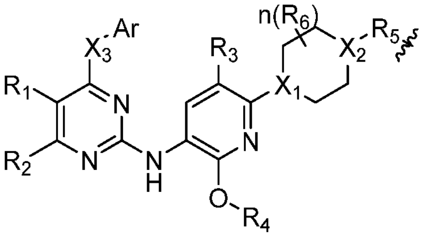, 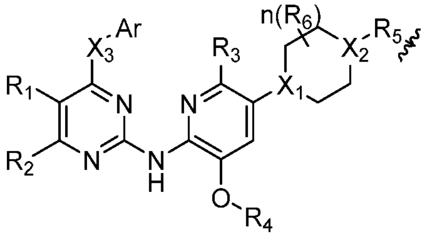,

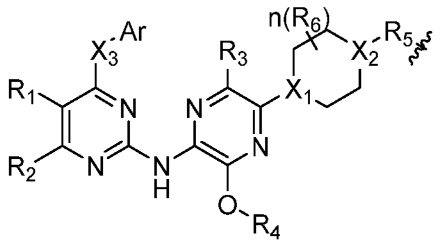

,

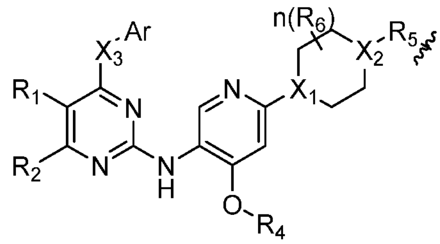

,

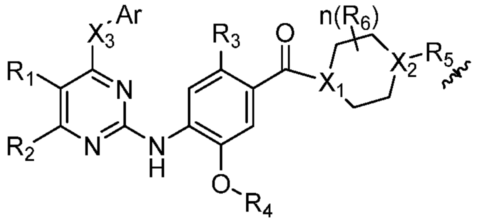

,

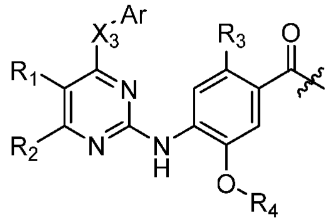

,

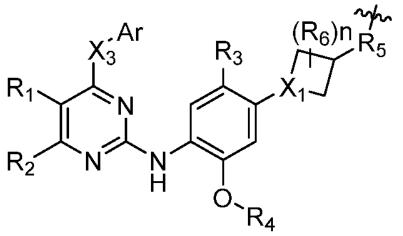

,

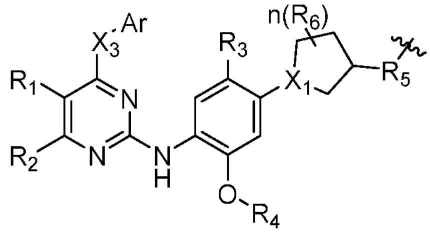

,

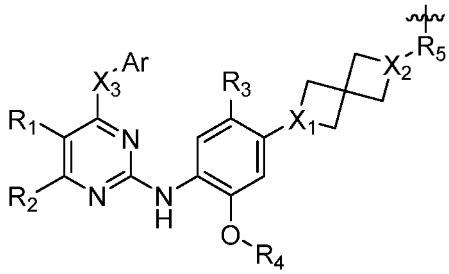

,

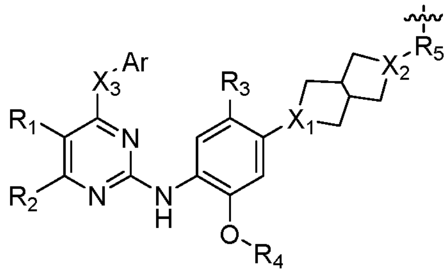

,

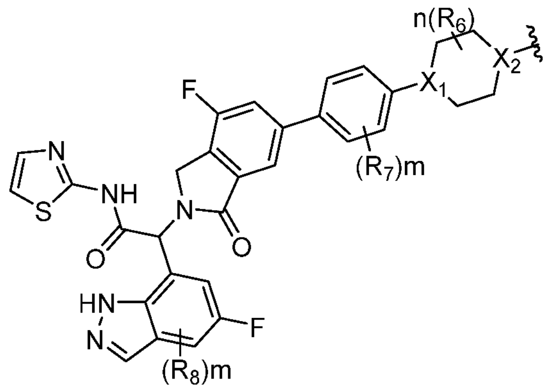

,

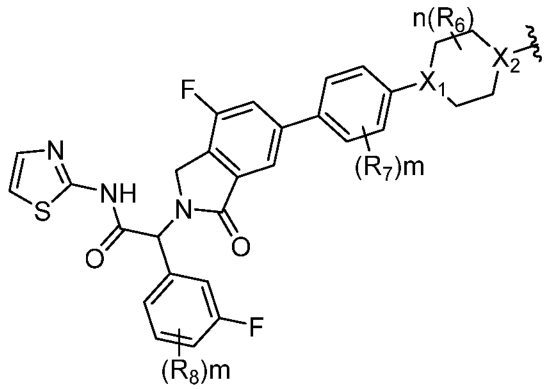

,

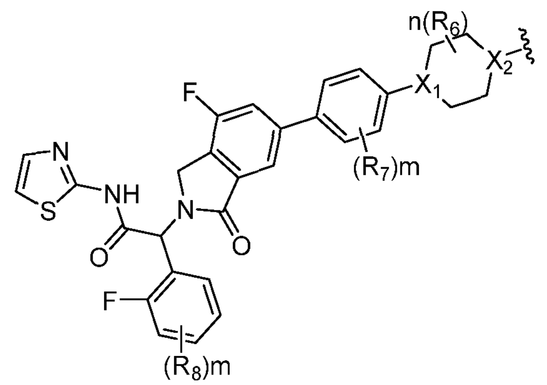

,

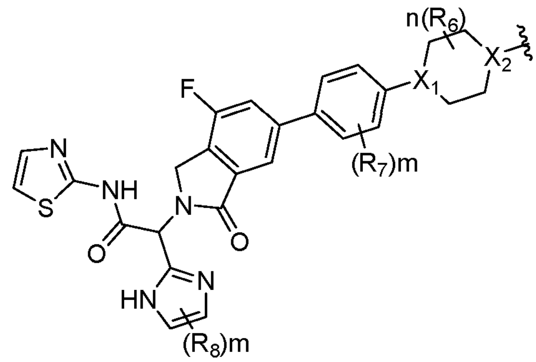

,

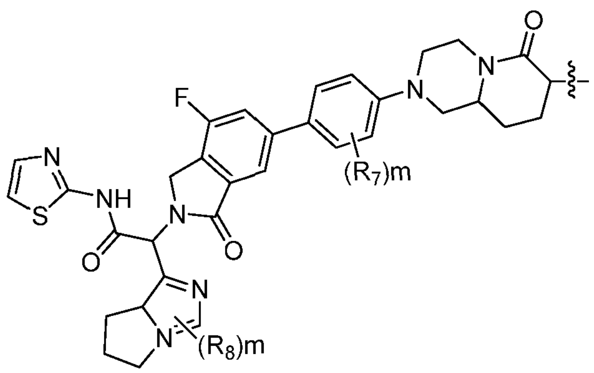

,

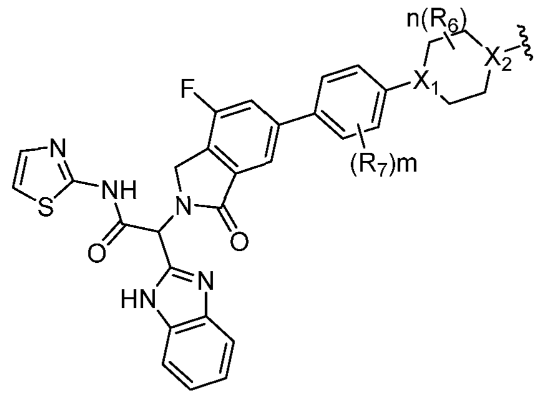

,

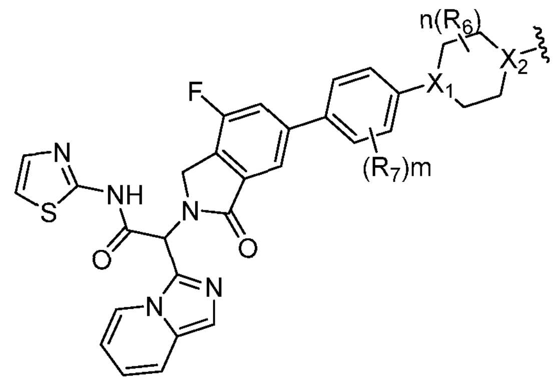

,

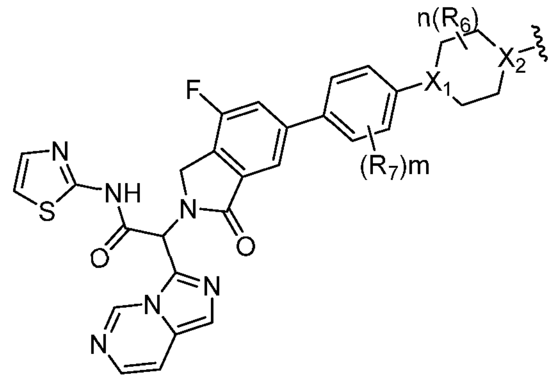

and

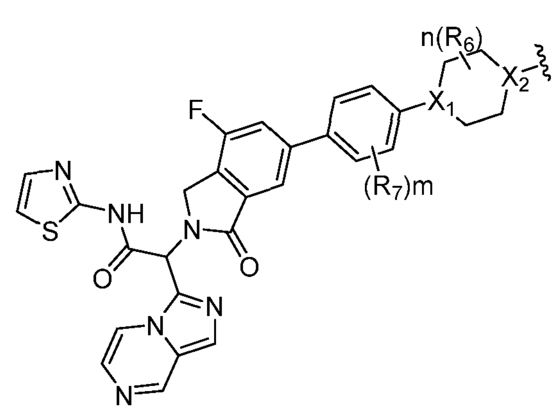

;

B is selected from the group consisting of:

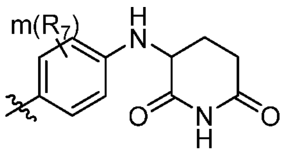, 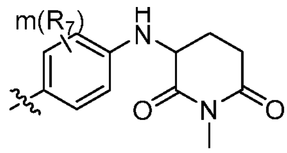,

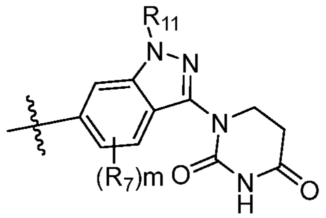, 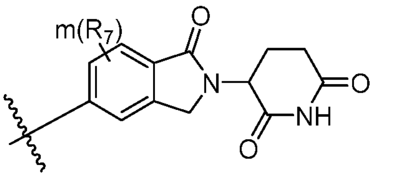, 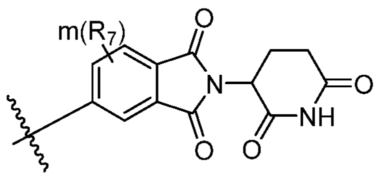,

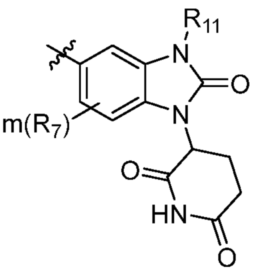, 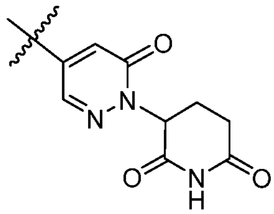, 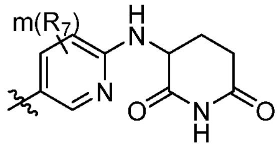, 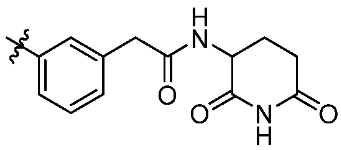,

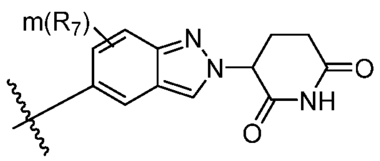, 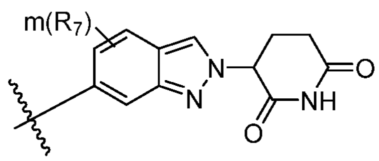, 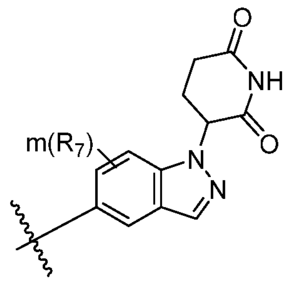,

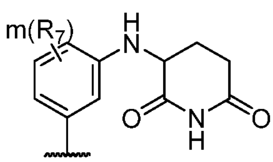, 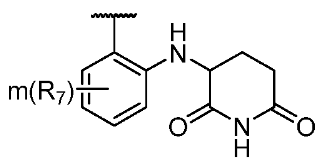, 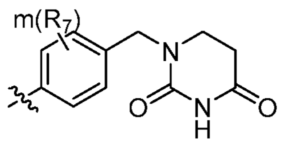,

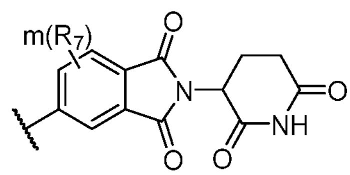 and 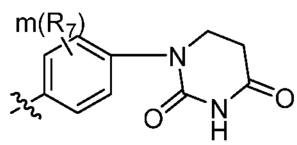;

in each formula:

each $X_1$ and $X_2$ are independently selected from the group consisting of: CR and N;
each $X_3$ is independently selected from the group consisting of: NH, O and none;

each Ar is independently selected from the group consisting of: phenyl or substituted phenyl, heteroaryl or substituted heteroaryl, and

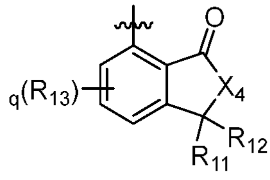;

$X_4$ is selected from the group consisting of: $CR_{11}R_{12}$, O and NR; the substituted phenyl, and substituted heteroaryl have one or more (e.g., 2, 3, or 4) substituents selected from the group consisting of: halogen, $C_{1-6}$alkyl, $C_{1-6}$haloalkyl, hydroxy-substituted $C_{1-6}$alkyl, $C_{3-6}$cycloalkyl, $C_{1-6}$alkylamino, $C_{1-6}$alkoxy, $C_{1-6}$haloalkoxy, $C_{3-6}$halocycloalkyl, cyano, oxo, $-NR_9C(O)R_{10}$, $-OC(O)NR_9R_{10}$, $-NR_9C(O)OR_{10}$, $-C(O)NR_9R_{10}$, methanesulfonyl, $-NR_9$-methanesulfonyl,

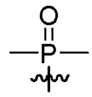,

$-CO-C_{1-6}$alkyl, $-C(=O)O-C_{1-6}$alkyl, $-CO-C_{3-6}$cycloalkyl, $-CO-C_{1-6}$haloalkyl, and $-CO-C_{3-6}$halocycloalkyl;
each $R_1$ is independently selected from the group consisting of: hydrogen, halogen, $C_{1-6}$haloalkyl and cyano;
each $R_2$, and $R_3$ are independently selected from the group consisting of: H, halogen, $C_{1-6}$alkyl, $C_{3-6}$cycloalkyl, $C_{1-6}$alkylamino, $C_{1-6}$alkoxy, $C_{1-6}$haloalkoxy, $C_{1-6}$haloalkyl, cyano, $C_{3-6}$halocycloalkyl, $-NR_9C(O)R_{10}$, $-C(O)NR_9R_{10}$, methanesulfonyl,

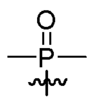,

and unsubstituted or $C_{1-6}$alkyl-substituted 5-10-membered heteroaryl containing 1, 2 or 3 heteroatoms selected from N, O and S;
each $R_4$ is independently selected from the group consisting of $C_{1-6}$alkyl, $C_{1-6}$haloalkyl, $C_{3-6}$cycloalkyl, and $C_{3-6}$halocycloalkyl;
each $R_5$ is independently absent, $C_{1-6}$alkyl, NR,

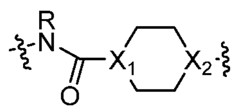, 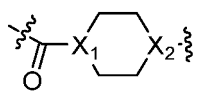

and

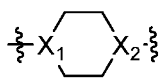;

each $R_6$, $R_7$, and $R_8$ are independently selected from the group consisting of: H, halogen, $C_{1-6}$alkyl, $C_{3-6}$cycloalkyl, $C_{1-6}$alkylamino, $C_{1-6}$alkoxy, $C_{1-6}$haloalkoxy, $C_{1-6}$haloalkyl, cyano, $C_{3-6}$halocycloalkyl-$NR_9C(O)R_{10}$, $-C(O)NR_9R_{10}$, methanesulfonyl,

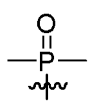, 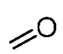

and hydroxy, or $R_6$ together with the attached ring form a 3- to 6-membered ring;

each $R_9$ is independently selected from the group consisting of: H, $C_{1-6}$alkyl, $C_{6-10}$ aryl, $C_{1-6}$haloalkyl, $C_{3-6}$cycloalkyl, and $C_{3-6}$halocycloalkyl;

each $R_{10}$ is independently selected from the group consisting of: H, $C_{1-6}$alkyl, $C_{2-6}$alkenyl, $C_{2-6}$alkynyl, $C_{3-6}$cycloalkyl, $C_{1-6}$alkoxy, $C_{1-6}$haloalkoxy, $C_{1-6}$haloalkyl, $C_{3-6}$halocycloalkyl, methylsulfonyl and

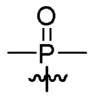;

each m, n, and q are independently selected from the group consisting of: 0, 1, 2, 3, 4 and 5;

each $R_{11}$, and $R_{12}$ are independently selected from the group consisting of: H, $C_{1-6}$alkyl, $C_{1-6}$haloalkyl, $C_{3-6}$cycloalkyl, and $C_{3-6}$halocycloalkyl; or $R_{11}$ and $R_{12}$ together with the attached moiety form a 5-to -7 membered ring;

each $R_{13}$ is independently selected from the group consisting of: H, halogen, $C_{1-6}$alkyl, $C_{1-6}$haloalkyl, $C_{3-6}$cycloalkyl and $C_{3-6}$halocycloalkyl; and

each R is independently selected from the group consisting of: H, $C_{1-6}$alkyl, hydroxyl, halogen and $C_{1-6}$haloalkyl.

**2.** The compound according to claim 1, wherein,
L is selected from the group consisting of:

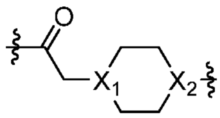, 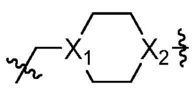

and

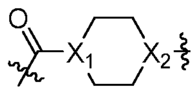;

$X_1$ and $X_2$ are as defined in claim 1.

**3.** The compound according to claim 1, wherein, Ar is selected from the group consisting of:

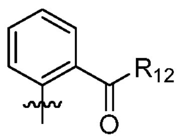, 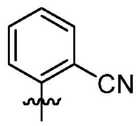, 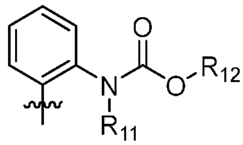, 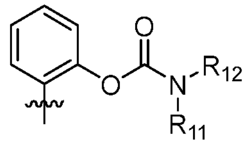, 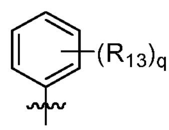,

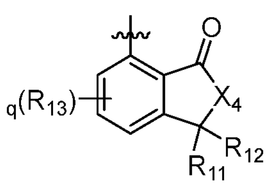 and 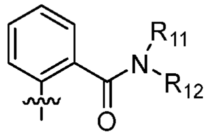;

wherein $X_4$ is selected from the group consisting of:$CR_{11}R_{12}$, O and NR;

each $R_{11}$ is independently selected from the group consisting of: H, $C_{1-6}$alkyl, $C_{1-6}$haloalkyl, $C_{3-6}$cycloalkyl, and $C_{3-6}$halocycloalkyl;

each $R_{12}$ is independently selected from the group consisting of: H, $C_{1-6}$alkyl, $C_{1-6}$haloalkyl, $C_{3-6}$cycloalkyl, $C_{3-6}$halocycloalkyl; or $R_{11}$ and $R_{12}$ together with attached moiety form a 5-to -7 membered ring;

each $R_{13}$ is independently halogen; and
q is selected from the group consisting of: 0, 1, 2, 3, 4 and 5;
R is as defined in claim 1.

**4.** The compound according to claim 1, wherein
A is selected from the group consisting of:

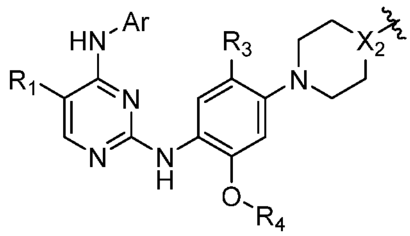

,

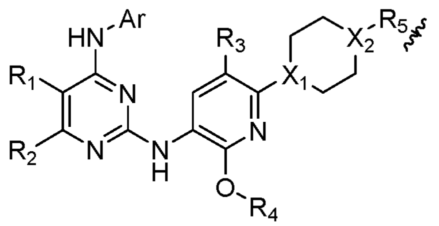

,

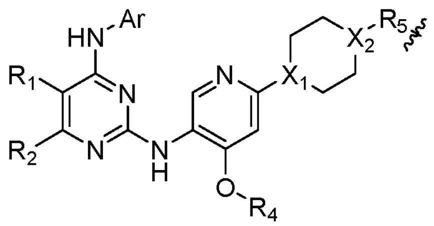

,

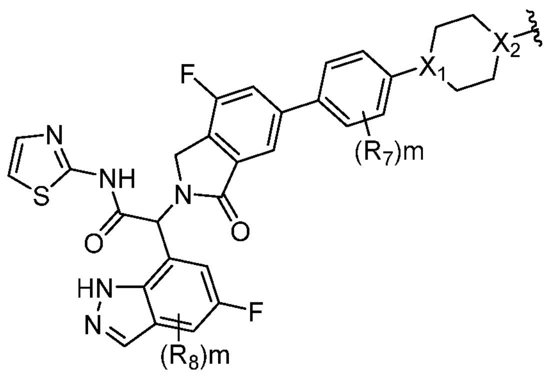

,

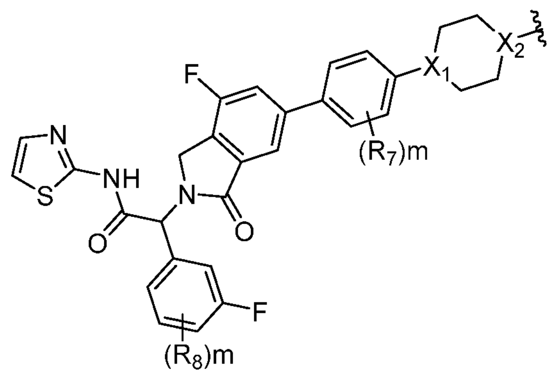

,

and

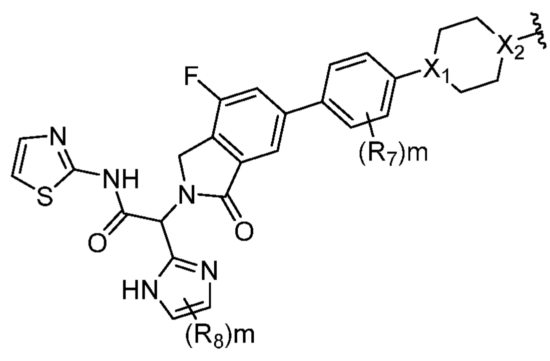

;

wherein $R_1$ is selected from the group consisting of: chlorine, trifluoromethyl and bromine;
Ar is selected from the group consisting of:

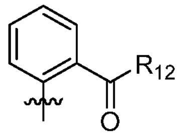, 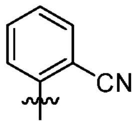, 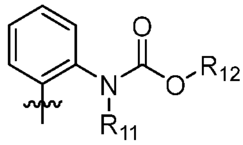,

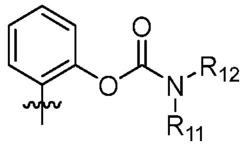, 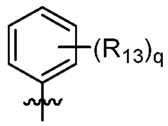, 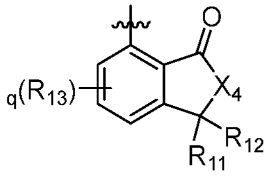 and 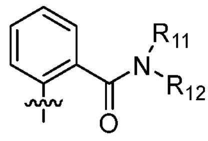;

each $R_3$ is independently selected from the group consisting of: H, halogen, $C_{1-6}$alkyl, $C_{1-6}$haloalkyl, cyano, and $-NR_9C(O)R_{10}$ ;
each $R_{12}$ is independently selected from the group consisting of: H, $C_{1-6}$alkyl, $C_{1-6}$haloalkyl, $C_{3-6}$cycloalkyl, and $C_{3-6}$halocycloalkyl.
each $R_{13}$ is independently halogen;
q is selected from the group consisting of: 0, 1, 2, 3, 4 and 5; and
$X_4$, $R_2$, $R_4$, $R_7$, $R_8$, $R_9$, $R_{10}$, $R_{11}$, $R_5$, $X_1$, $X_2$, and m are as defined in claim 1.

**5.** The compound according to claim 1, wherein B is selected from the group consisting of:

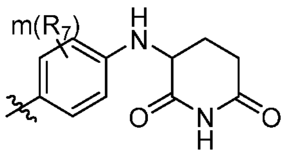, 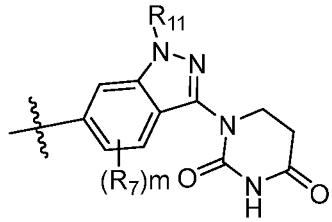, 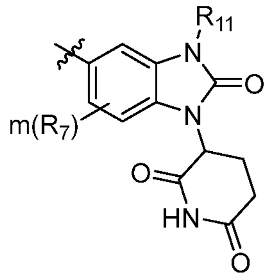

and

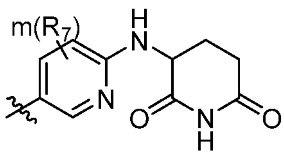,

wherein $R_7$, $R_{11}$, and m are as defined in claim 1.

**6.** The compound according to claim 1, wherein the compound is selected from the group consisting of:

| | |
|---|---|
| 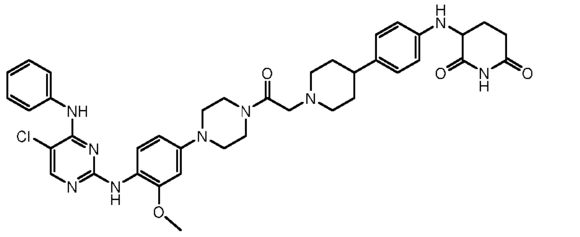 T-01 | 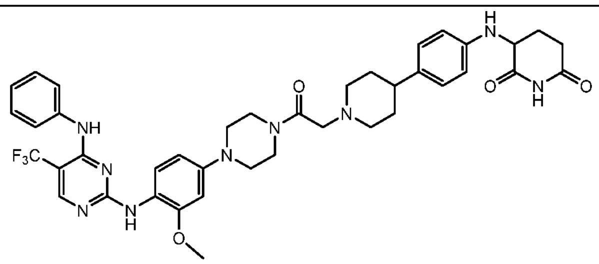 T-02 |
| 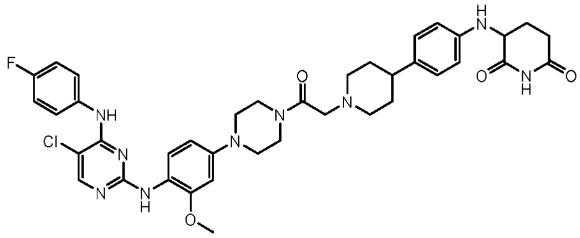 T-03 | 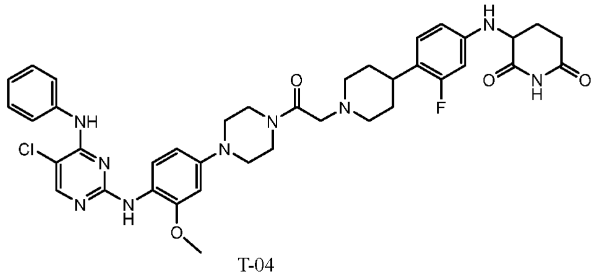 T-04 |

| | |
|---|---|
| 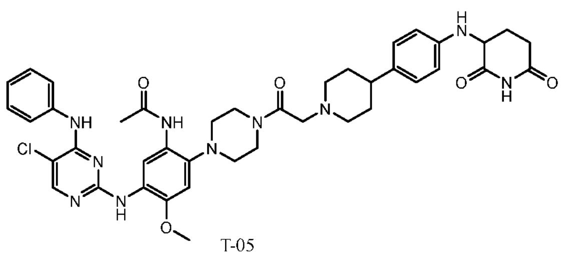 T-05 | 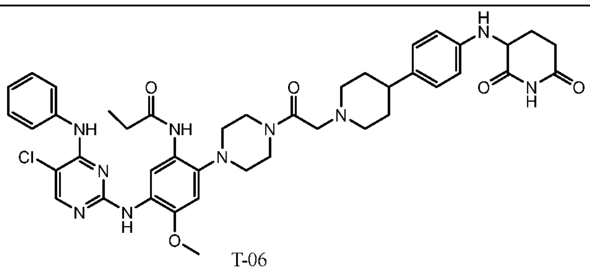 T-06 |
| 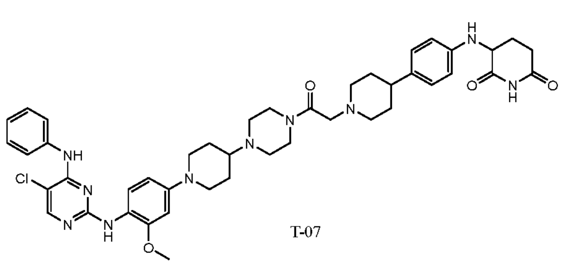 T-07 | 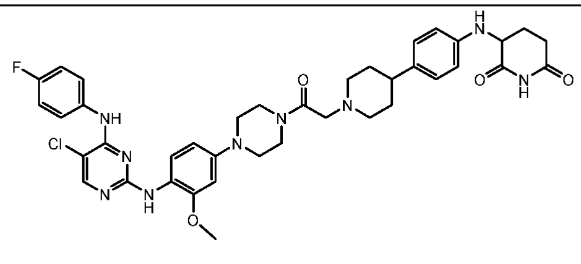 T-08 |
| 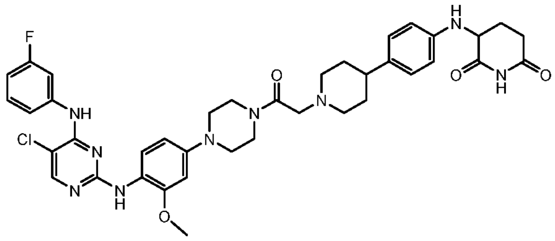 T-09 | 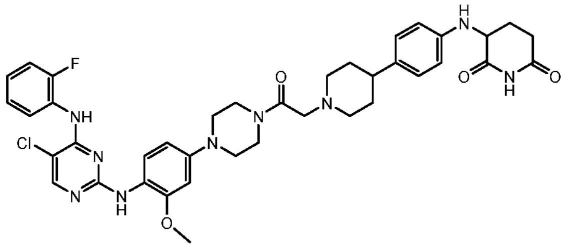 T-10 |
| 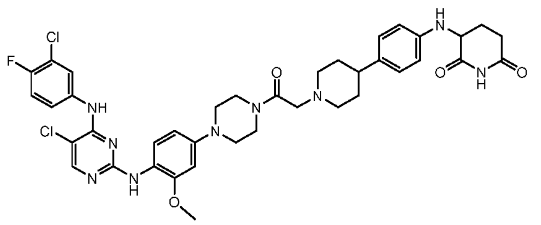 T-11 | 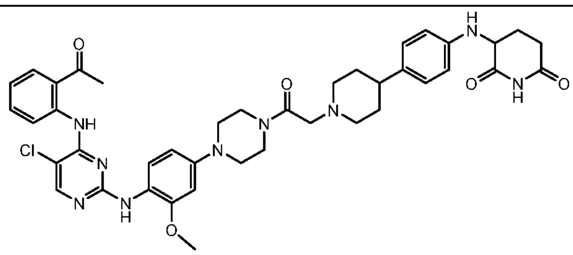 T-12 |
| 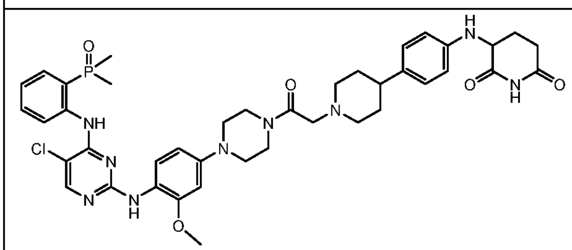 T-13 | 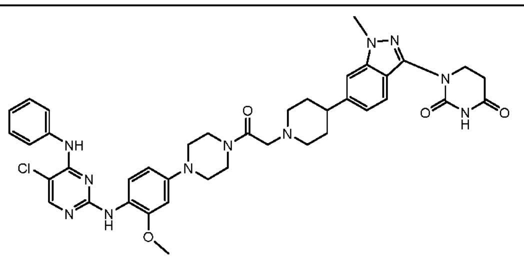 T-14 |
| 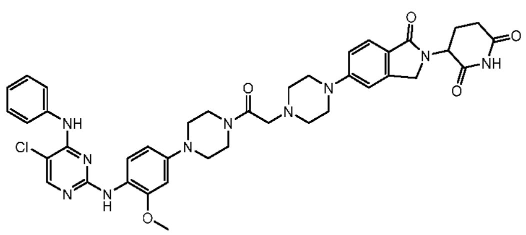 T-15 | 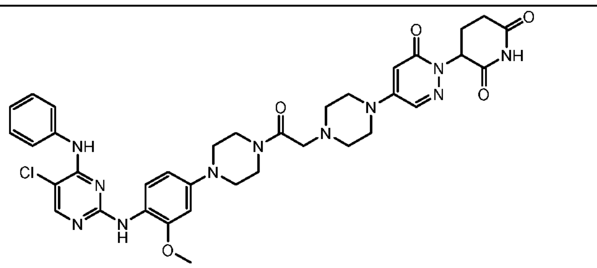 T-16 |

| | |
|---|---|
| 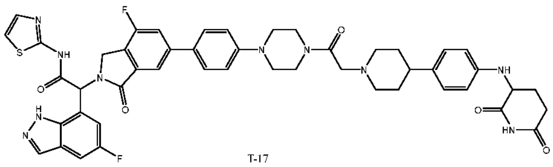<br>T-17 | 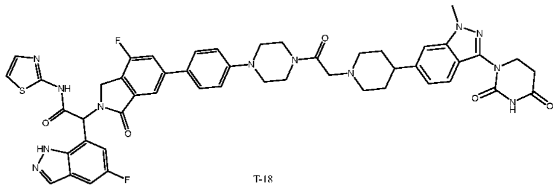<br>T-18 |
| 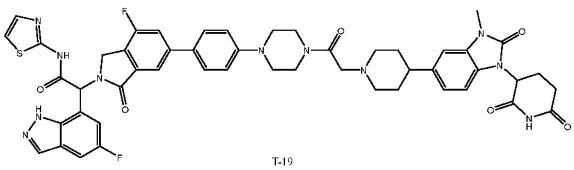<br>T-19 | 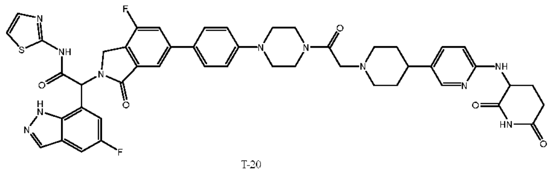<br>T-20 |
| 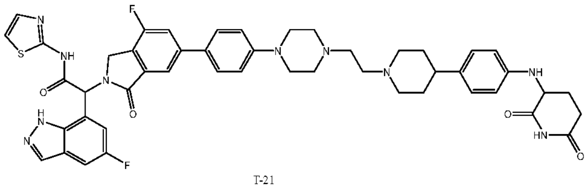<br>T-21 | 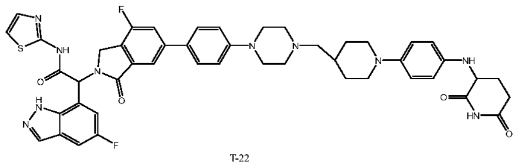<br>T-22 |
| 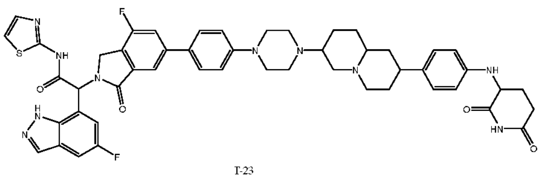<br>T-23 | 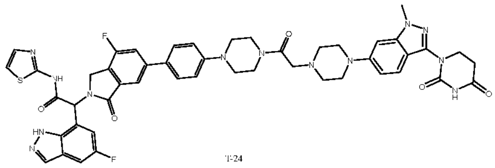<br>T-24 |
| 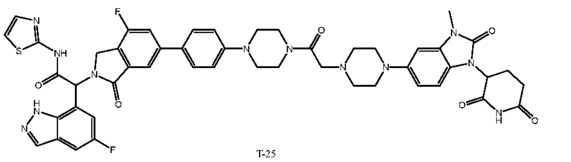<br>T-25 | 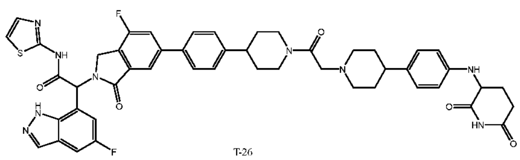<br>T-26 |
| 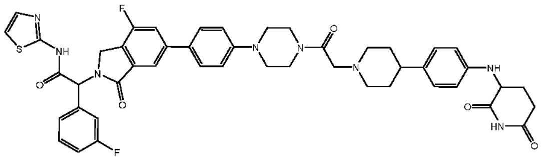<br>T-27 | 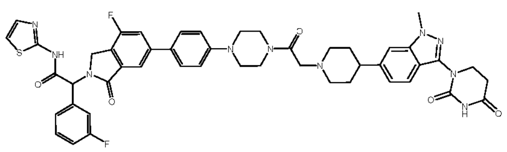<br>T-28 |
| 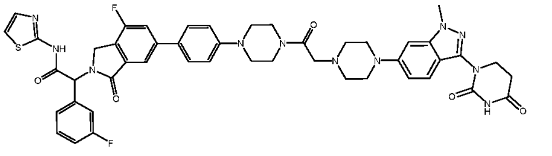<br>T-29 | 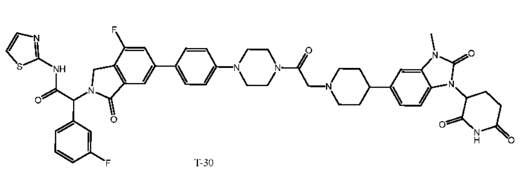<br>T-30 |

| | |
|---|---|
| 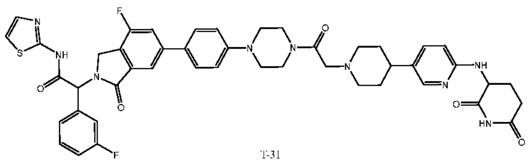 T-31 | 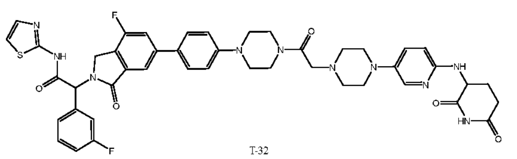 T-32 |
| 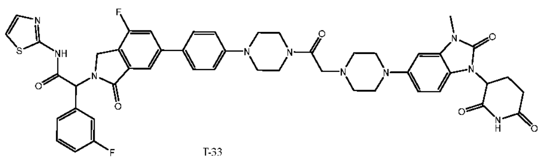 T-33 | 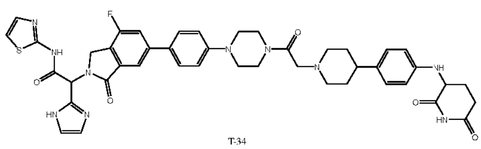 T-34 |
| 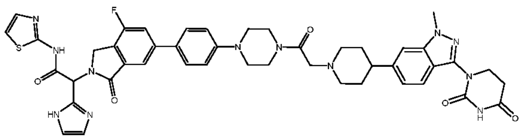 T-35 | 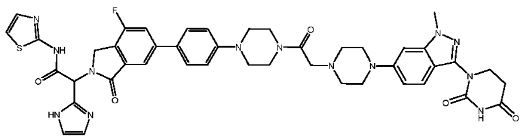 T-36 |
| 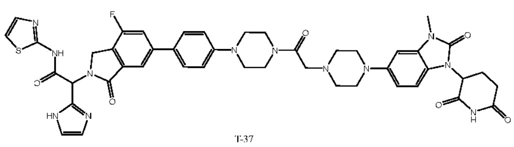 T-37 | 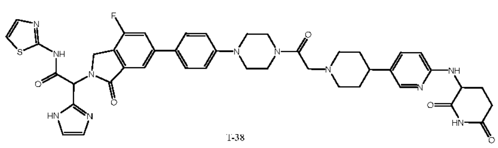 T-38 |
| 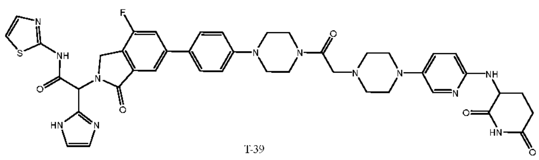 T-39 | 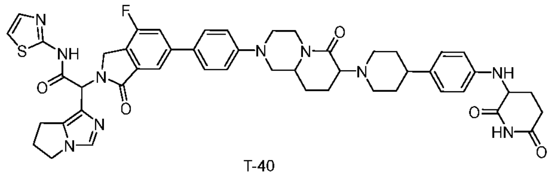 T-40 |
| 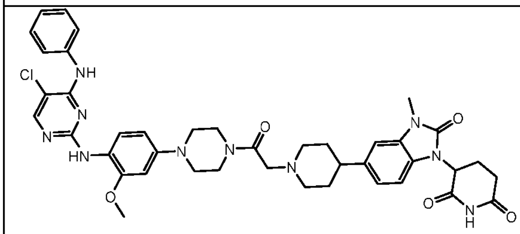 T-41 | 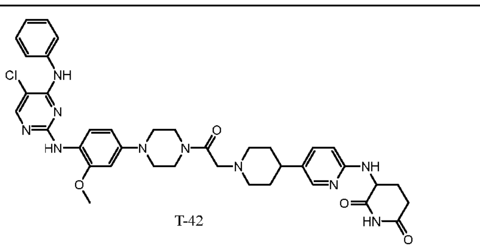 T-42 |
| 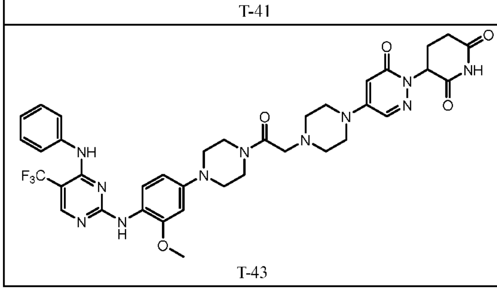 T-43 | 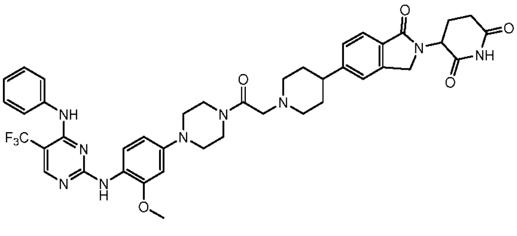 T-44 |

| | |
|---|---|
| 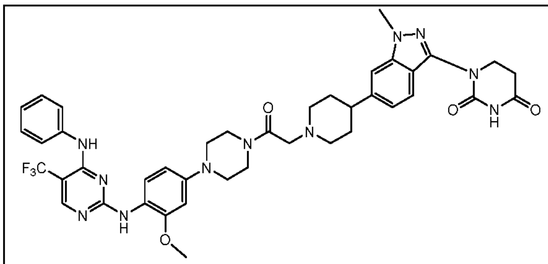 T-45 | 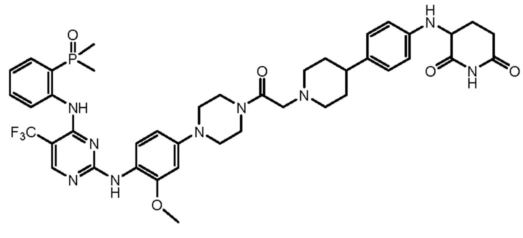 T-46 |
| 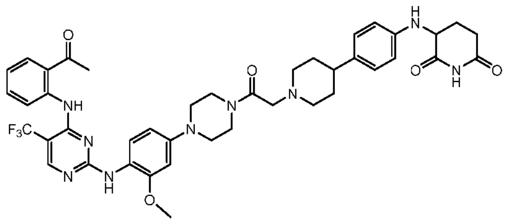 T-47 | 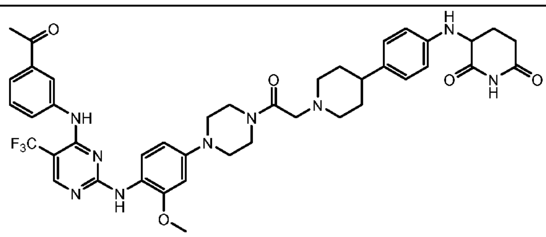 T-48 |
| 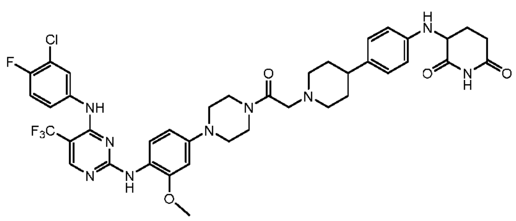 T-49 | 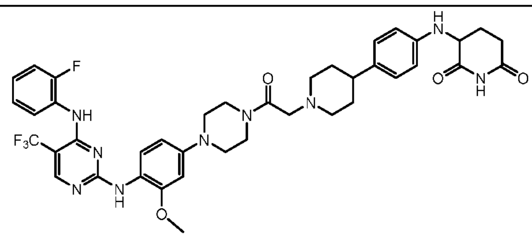 T-50 |
| 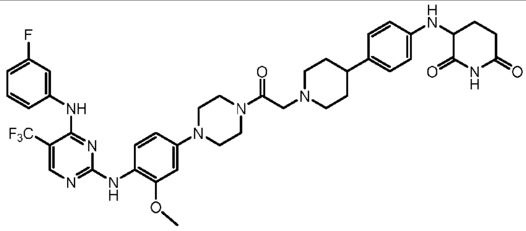 T-51 | 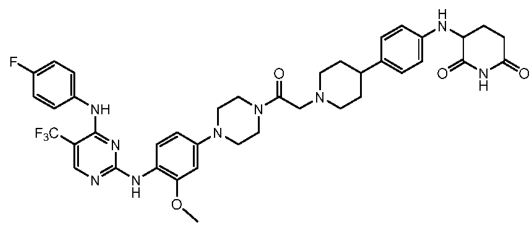 T-52 |
| 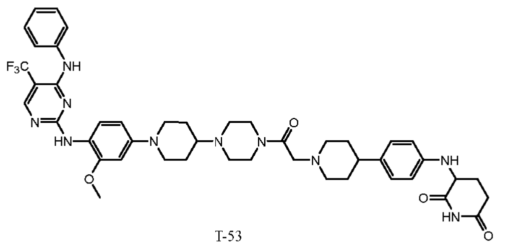 T-53 | 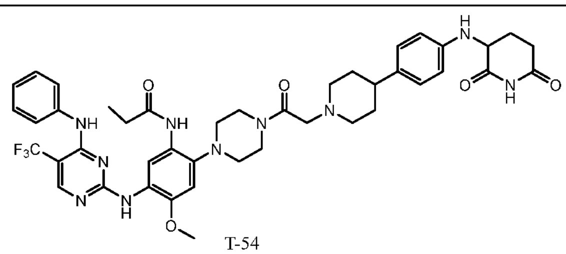 T-54 |
| 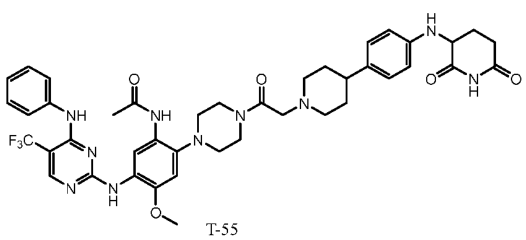 T-55 | 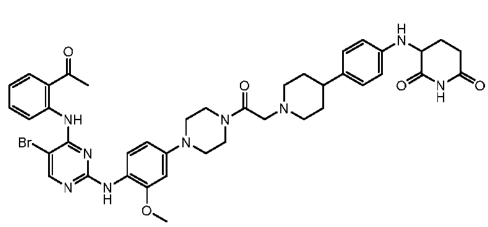 T-56 |

| | |
|---|---|
| 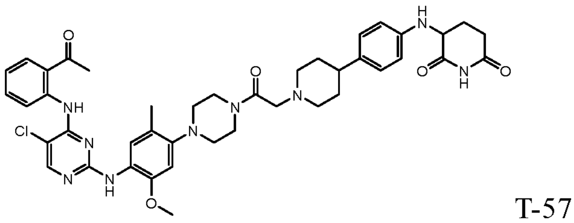 T-57 | 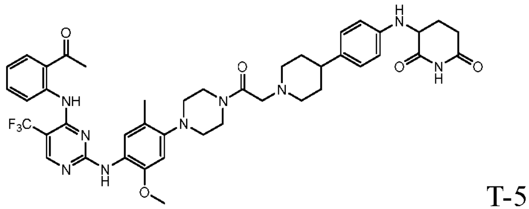 T-58 |
| 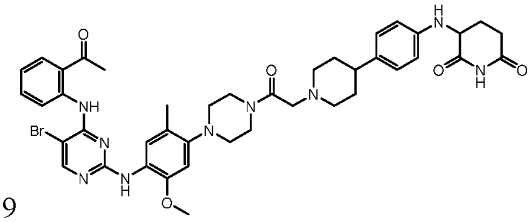 T-59 | 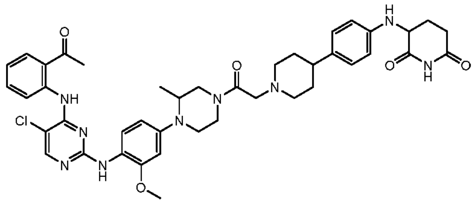 T-60 |
| 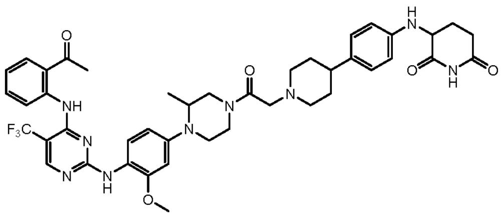 T-61 | 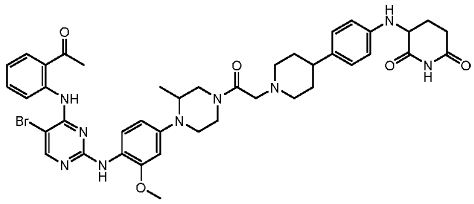 T-62 |
| 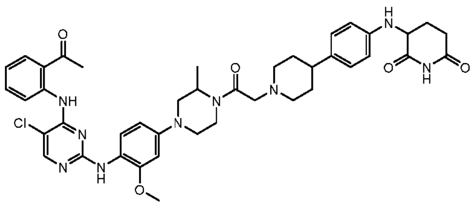 T-63 | 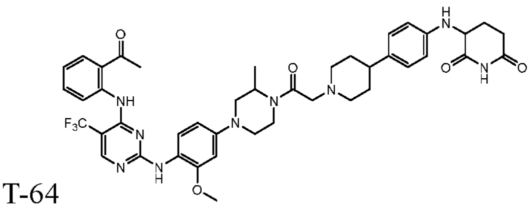 T-64 |
| 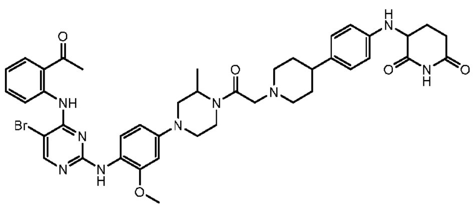 T-65 | 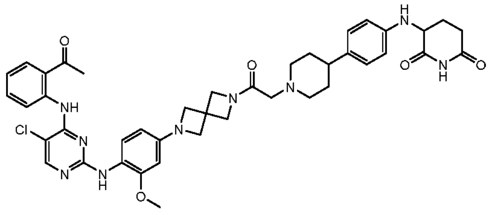 T-66 |
| 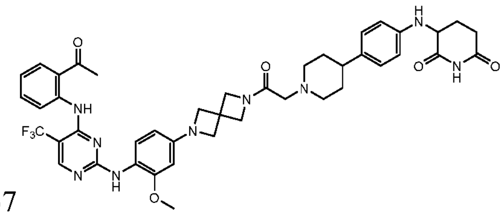 T-67 | 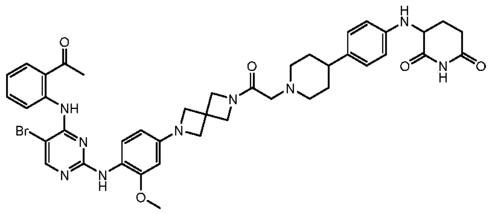 T-68 |
| 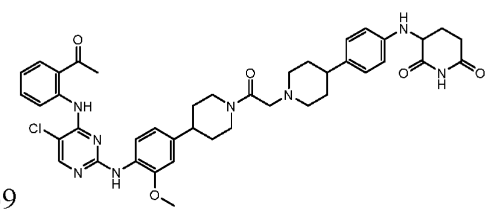 T-69 | 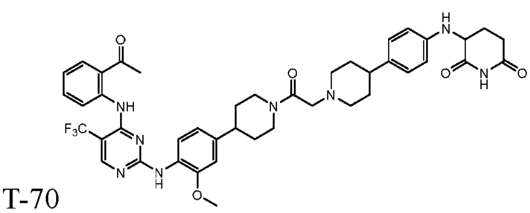 T-70 |

| | |
|---|---|
| 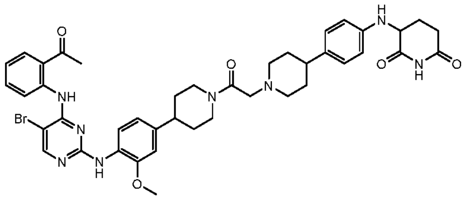 T-71 | 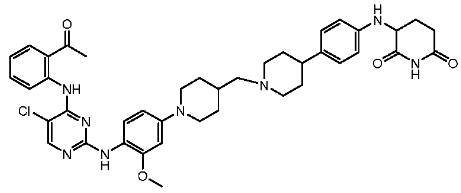 T-72 |
| 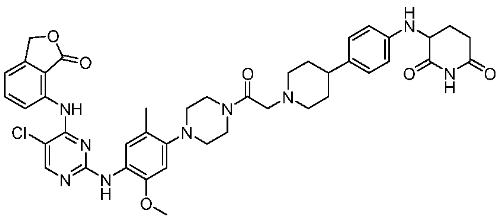 T-73 | 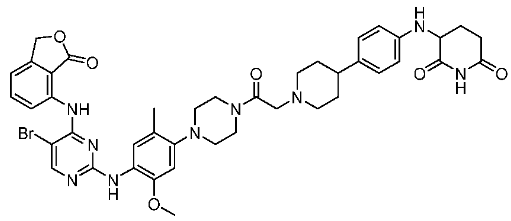 T-74 |
| 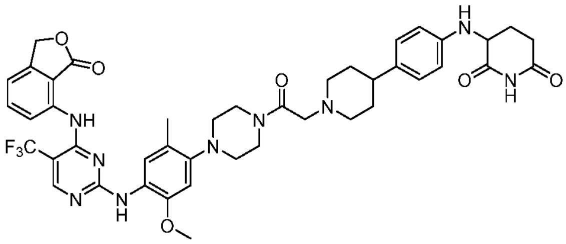 T-75 | 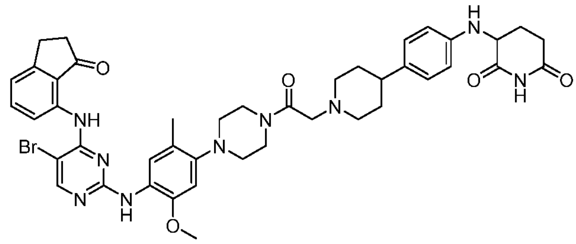 T-76 |
| 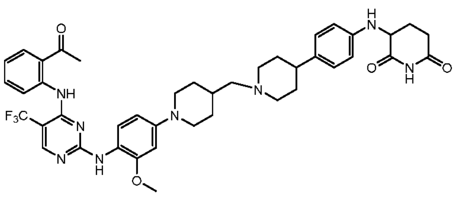 T-77 | 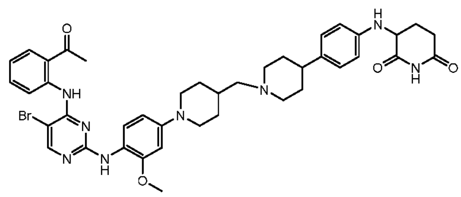 T-78 |
| 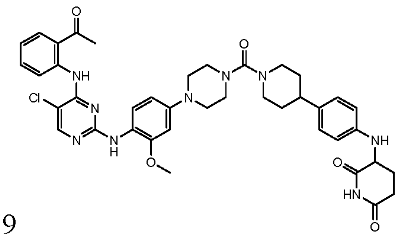 T-79 | 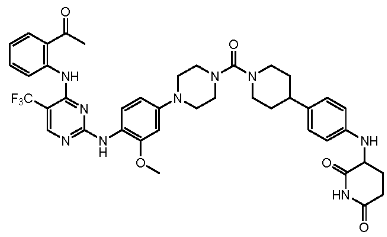 T-80 |
| 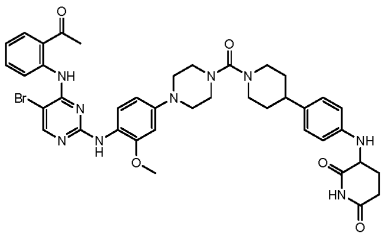 T-81 | 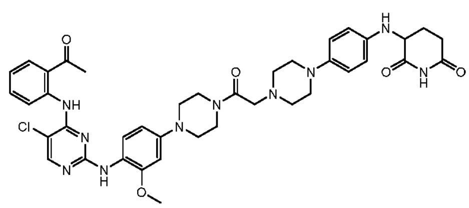 T-82 |
| 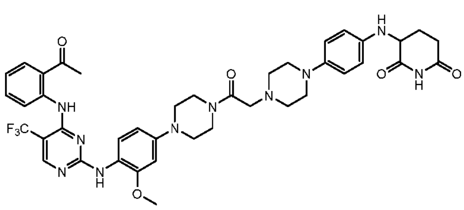 T-83 | 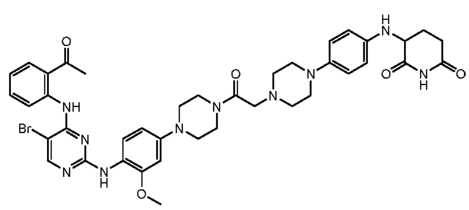 T-84 |

| | |
|---|---|
| 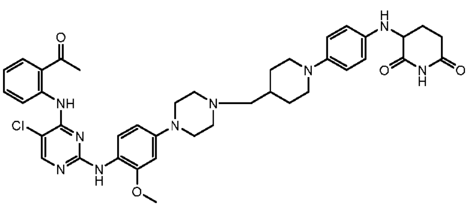 T-85 | 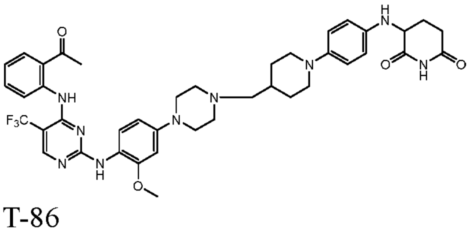 T-86 |
| 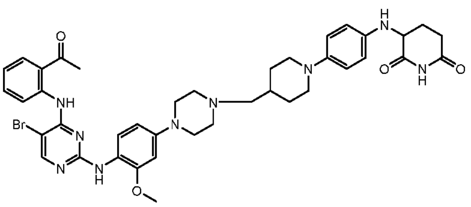 T-87 | 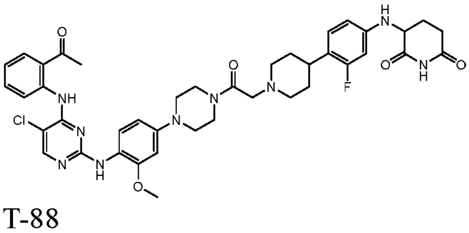 T-88 |
| 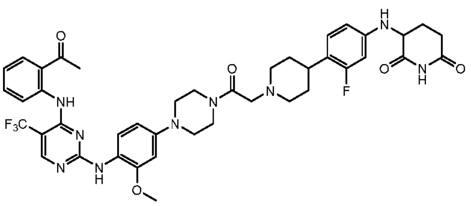 T-89 | 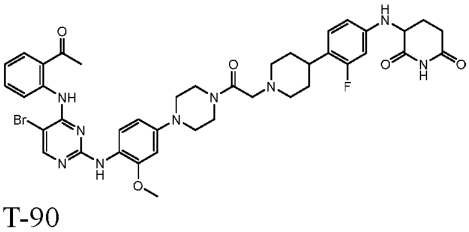 T-90 |
| 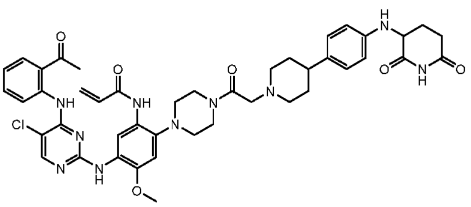 T-91 | 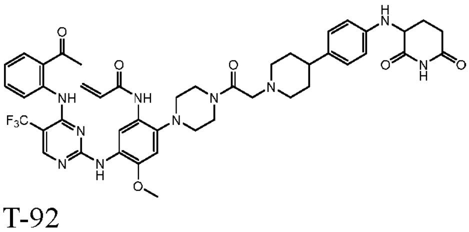 T-92 |
| 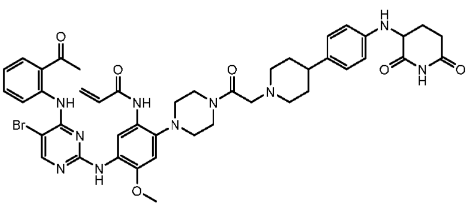 T-93 | 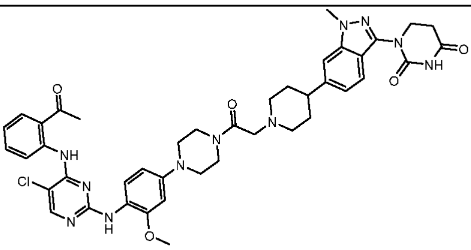 T-94 |
| 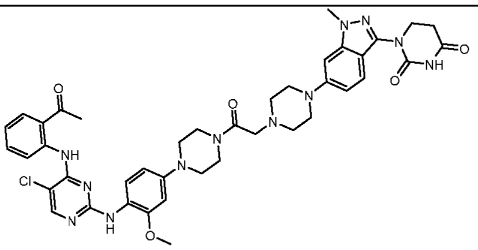 T-95 | 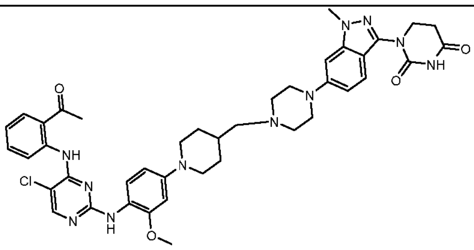 T-96 |
| 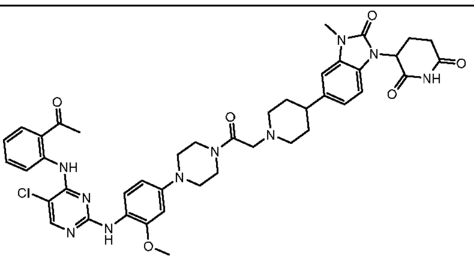 T-97 | 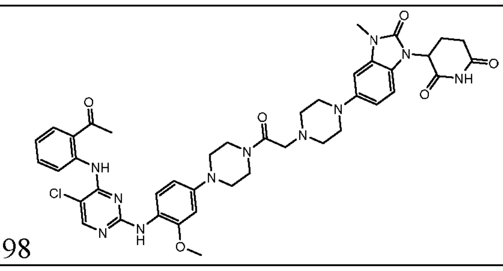 T-98 |

| | |
|---|---|
| 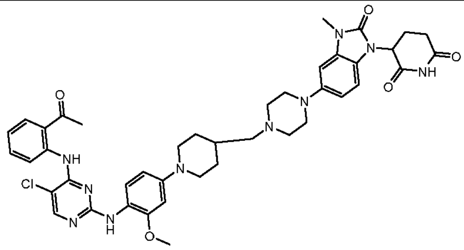 T-99 | 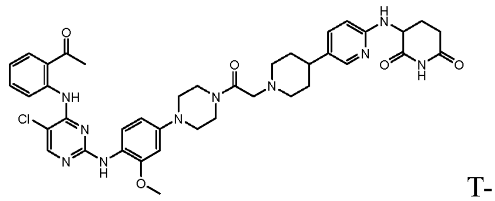 T-100 |
| 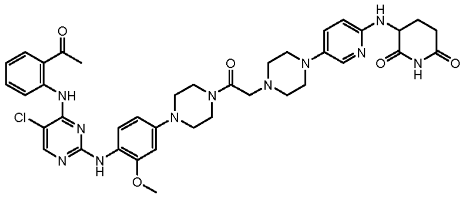 T-101 | 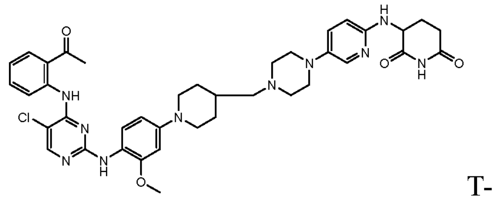 T-102 |
| 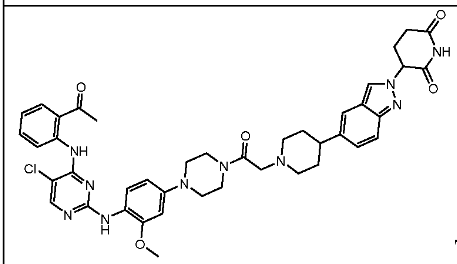 T-103 | 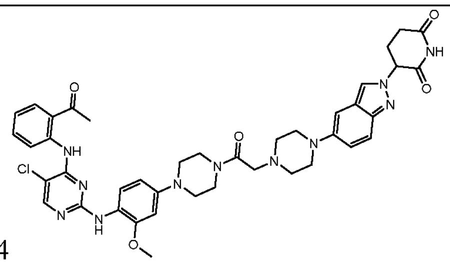 T-104 |
| 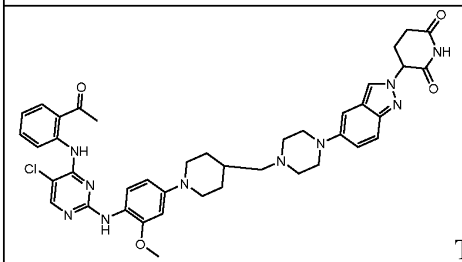 T-105 | 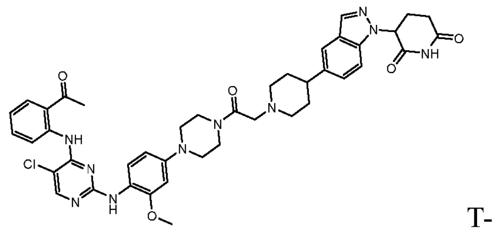 T-106 |
| 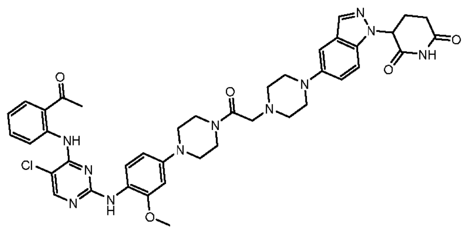 T-107 | 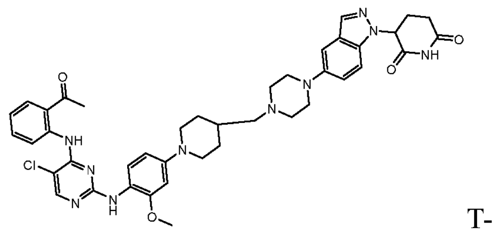 T-108 |
| 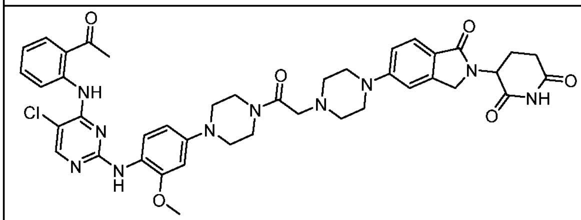 T-109 | 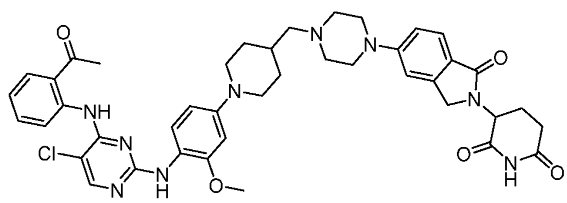 T-110 |
| 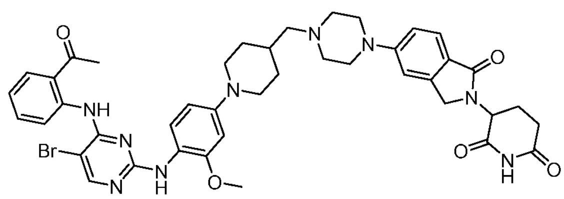 T-111 | 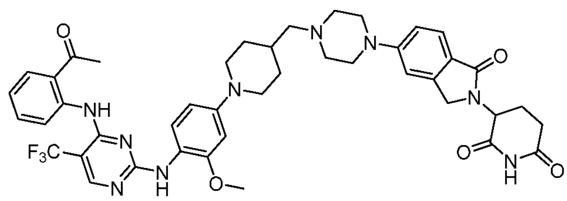 T-112 |

| | |
|---|---|
| 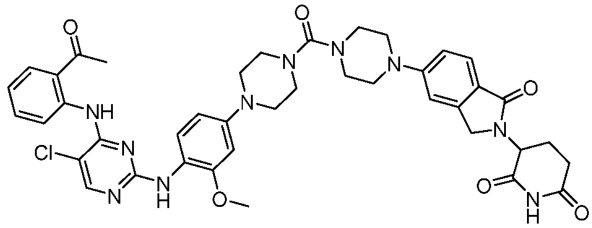 T-113 | 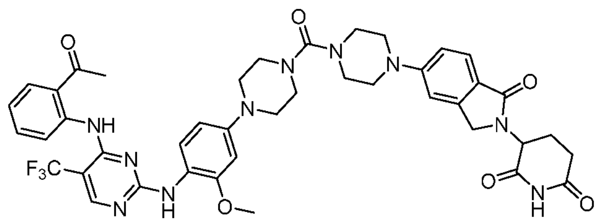 T-114 |
| 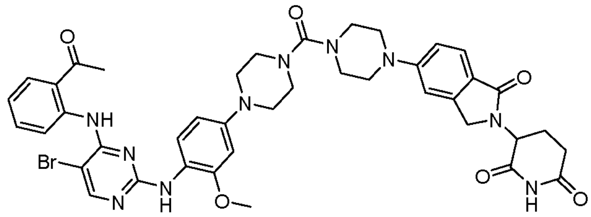 T-115 | 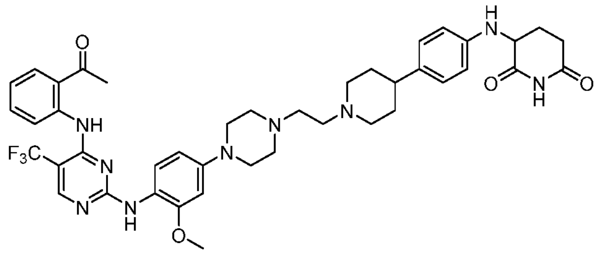 T-116 |
| 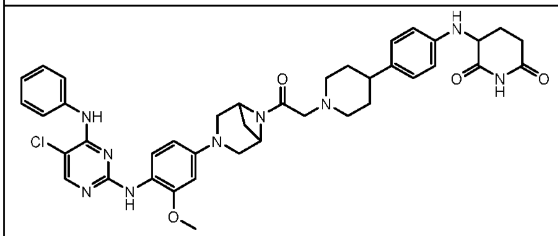 T-117 | 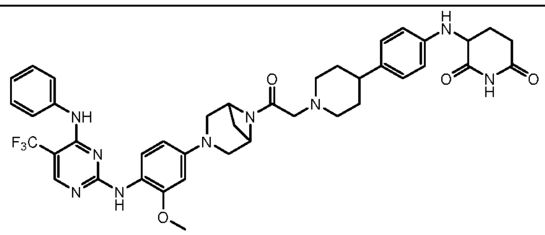 T-118 |
| 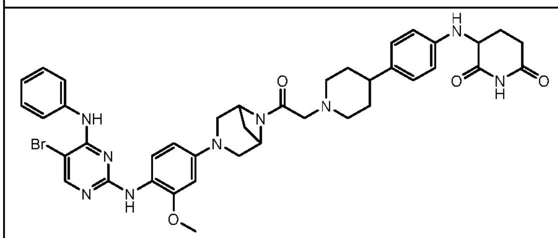 T-119 | 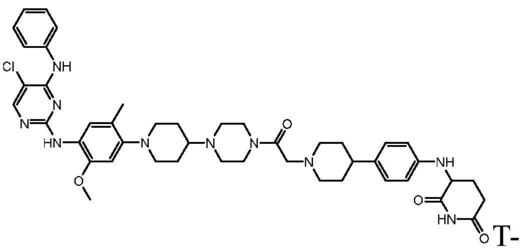 T-120 |
| 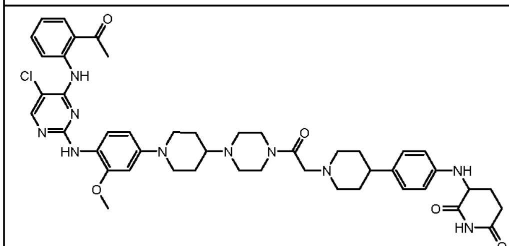 T-121 | 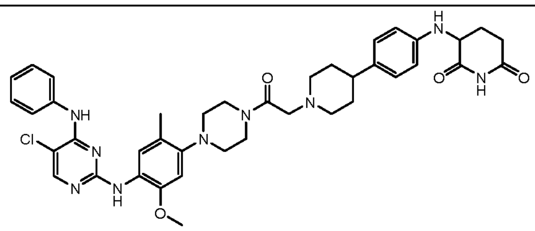 T-122 |
| 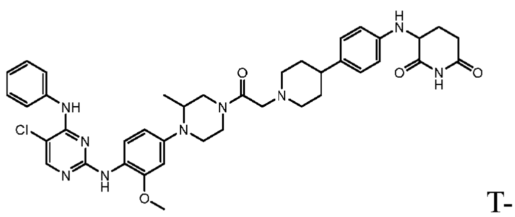 T-123 | 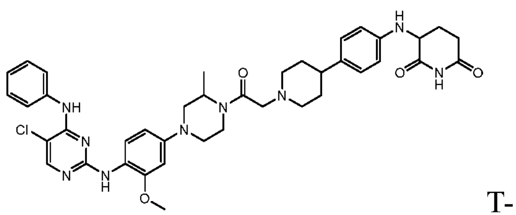 T-124 |

| | |
|---|---|
| 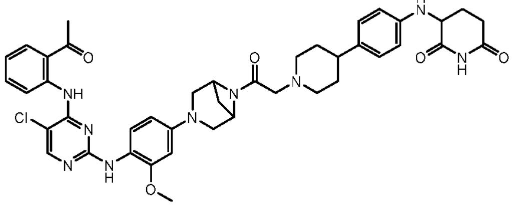 T-125 | 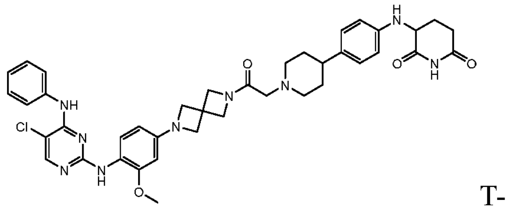 T-126 |
| 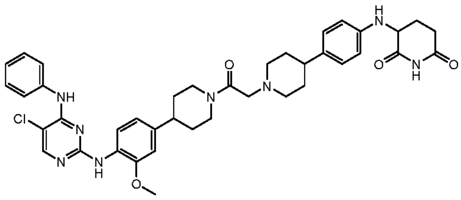 T-127 | 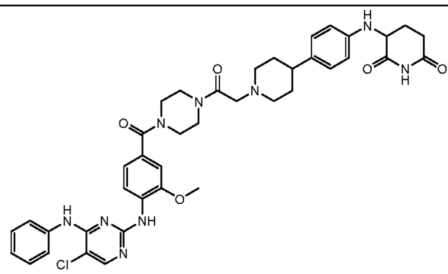 T-128 |
| 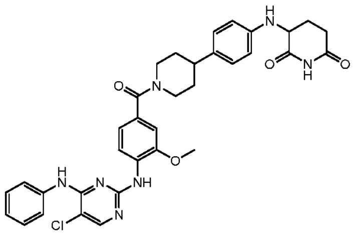 T-129 | 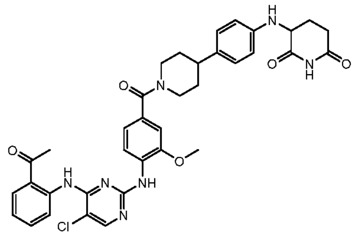 T-130 |
| 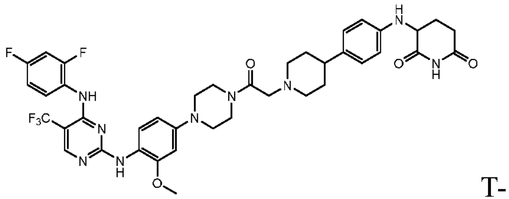 T-131 | 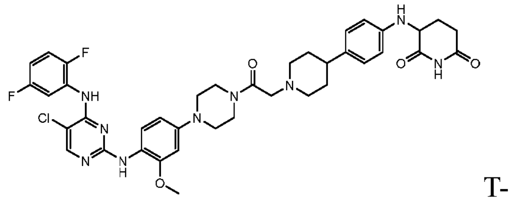 T-132 |
| 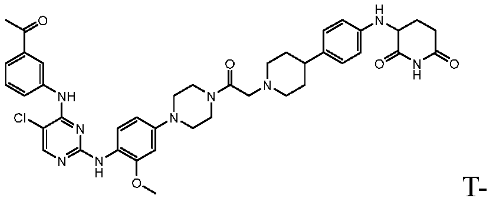 T-133 | 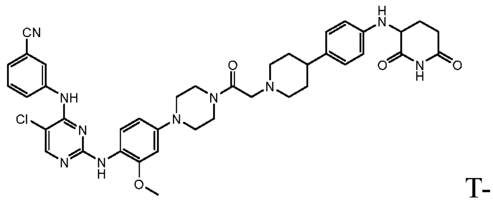 T-134 |
| 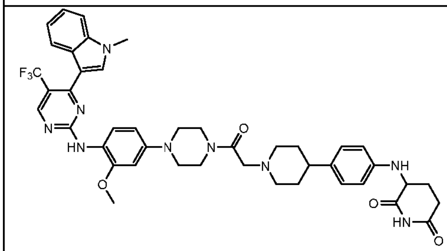 T-135 | 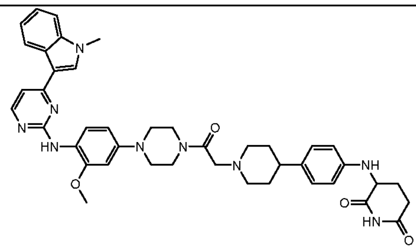 T-136 |
| 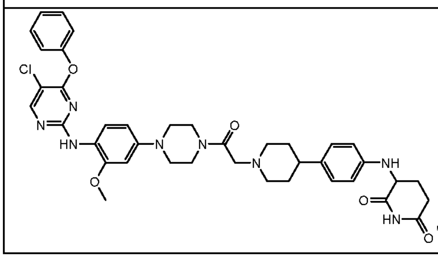 T-137 | 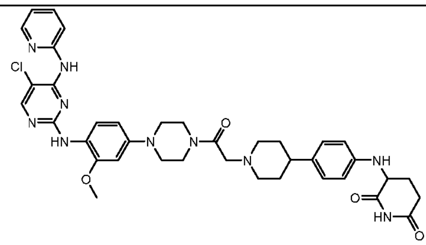 T-138 |

| | |
|---|---|
| 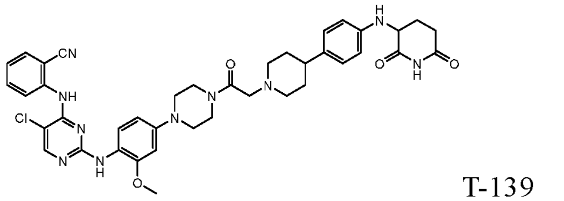 T-139 | 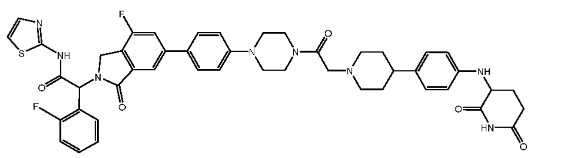 T-140 |
| 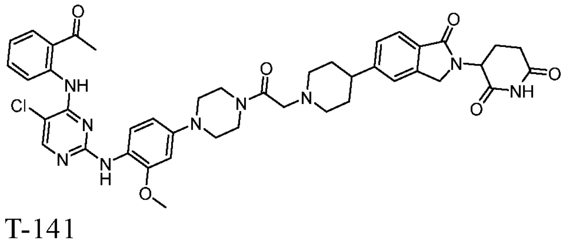 T-141 | 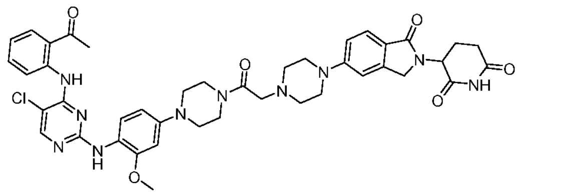 T-142 |
| 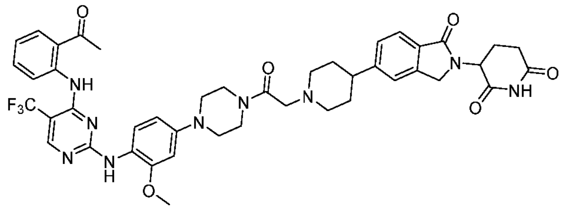 T-143 | 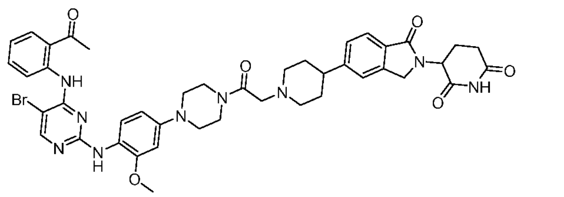 T-144 |
| 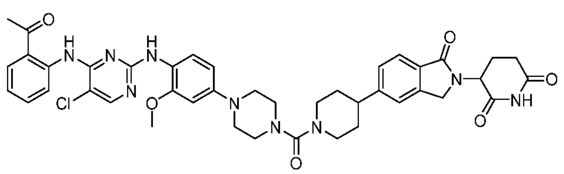 T-145 | 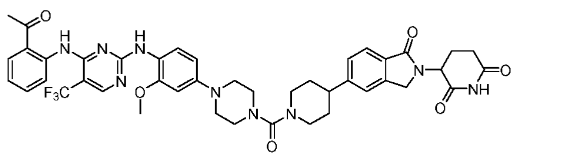 T-146 |
| 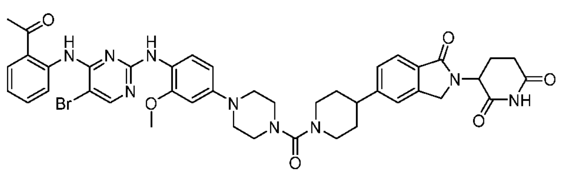 T-147 | 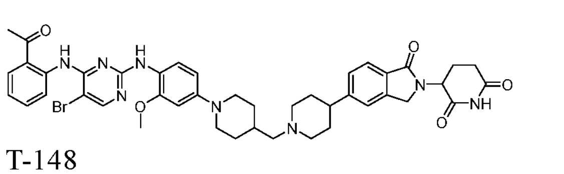 T-148 |
| 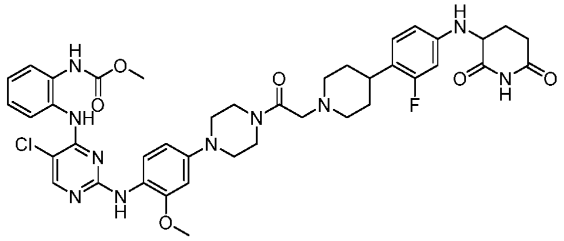 T-149 | 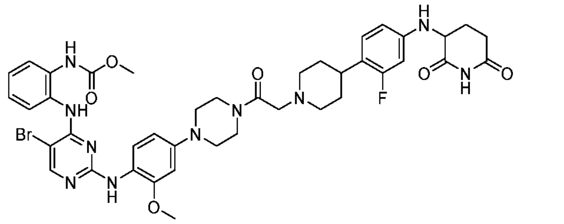 T-150 |
| 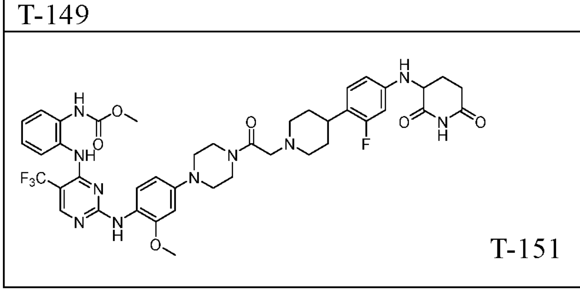 T-151 | 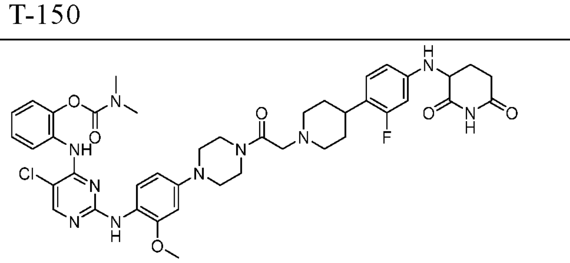 T-152 |

| | |
|---|---|
| 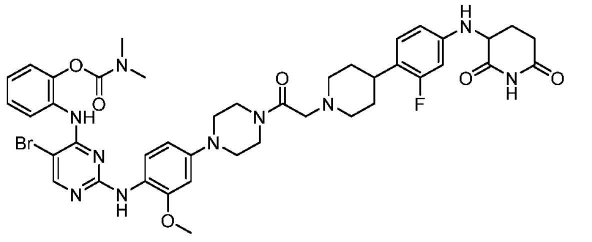 T-153 | 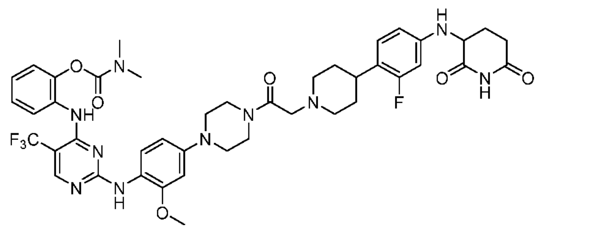 T-154 |
| 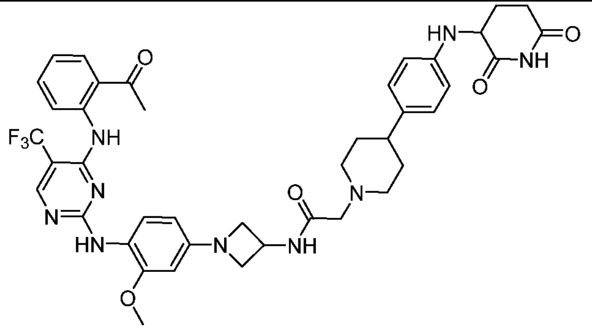 T-155 | 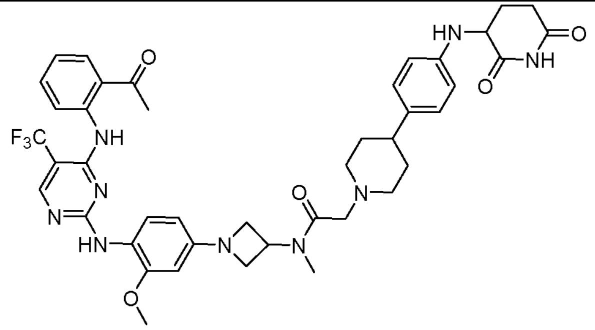 T-156 |
| 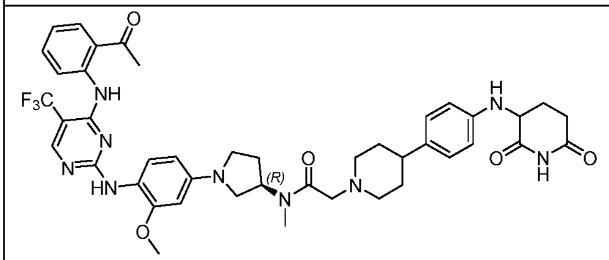 T-157 | 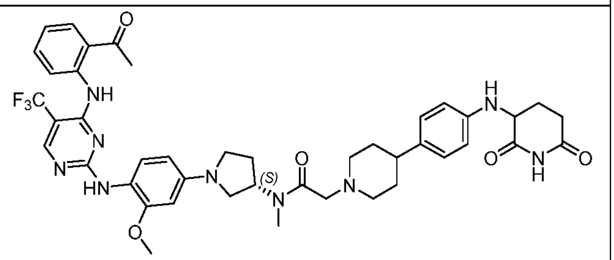 T-158 |
| 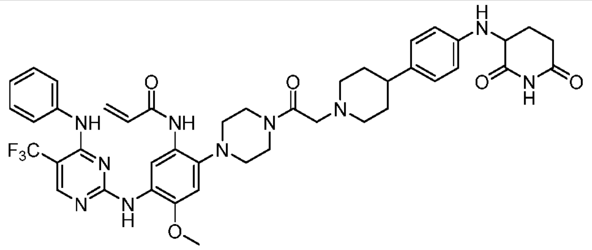 T-159 | 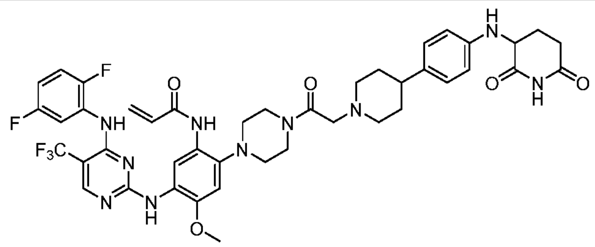 T-160 |
| 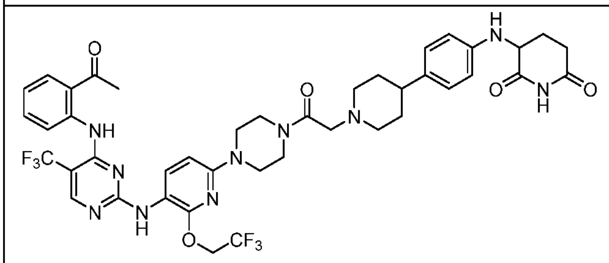 T-161 | 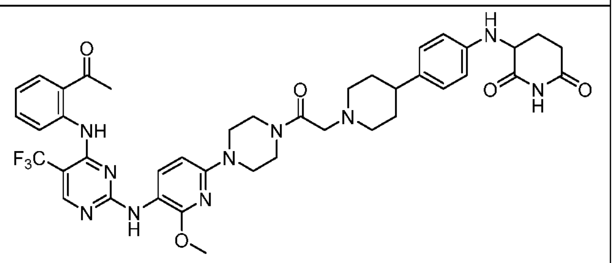 T-162 |
| 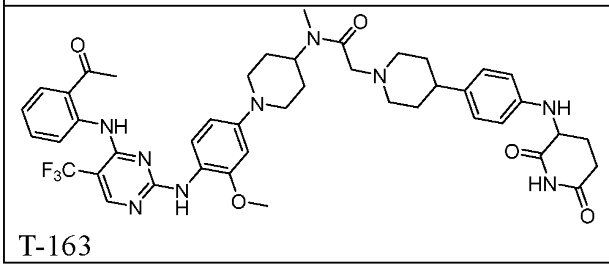 T-163 | 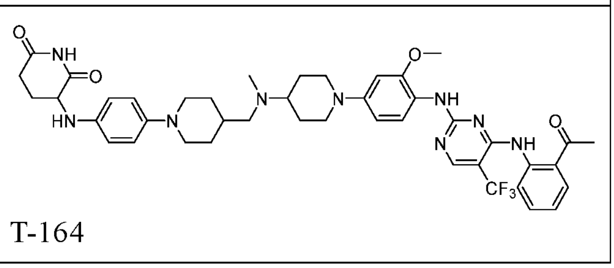 T-164 |

| | |
|---|---|
| 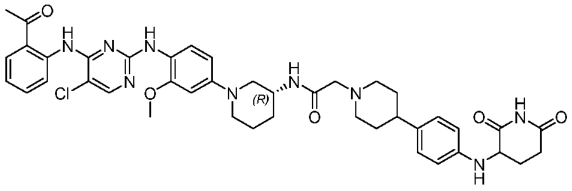 T-165 | 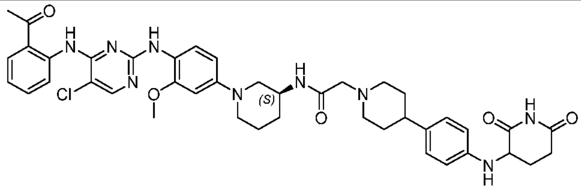 T-166 |
| 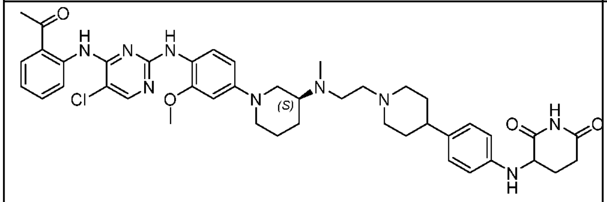 T-167 | 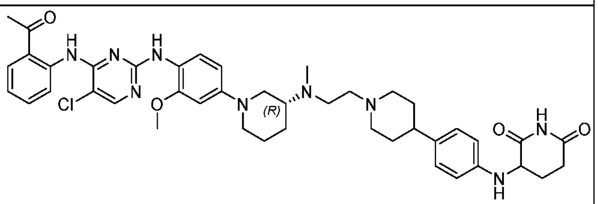 T-168 |
| 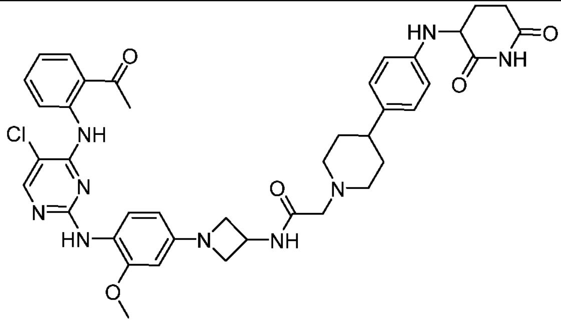 T-169 | 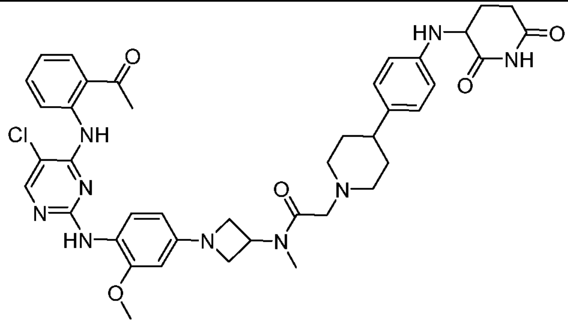 T-170 |
| 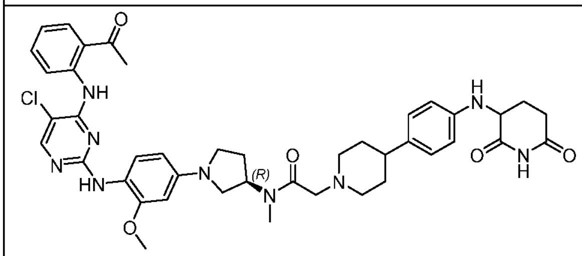 T-171 | 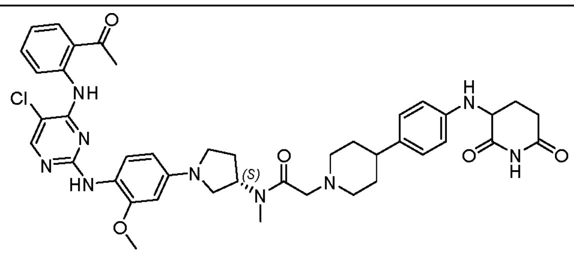 T-172 |
| 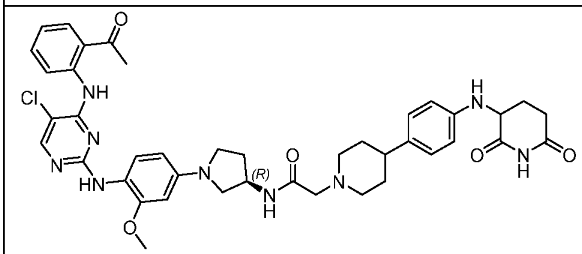 T-173 | 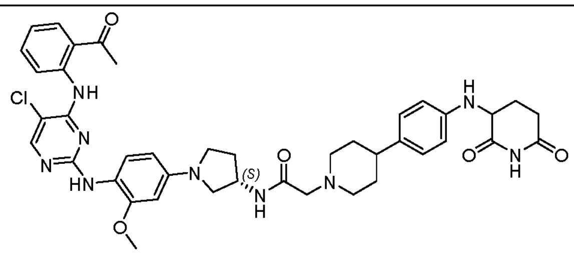 T-174 |
| 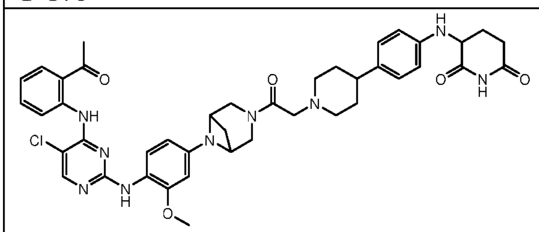 T-175 | 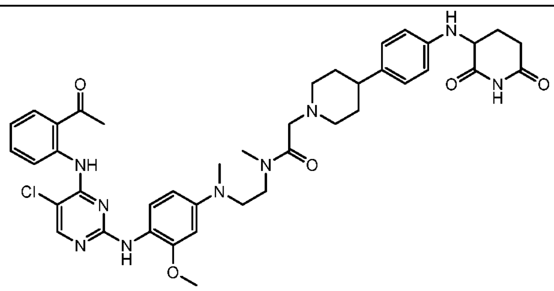 T-176 |

| | |
|---|---|
| 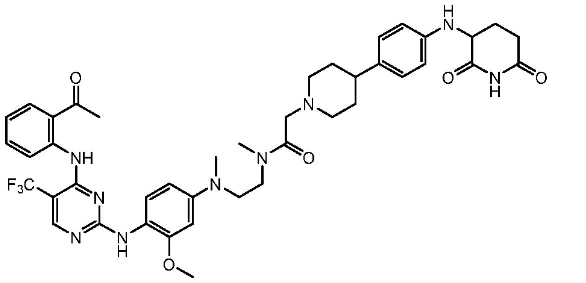 T-177 | 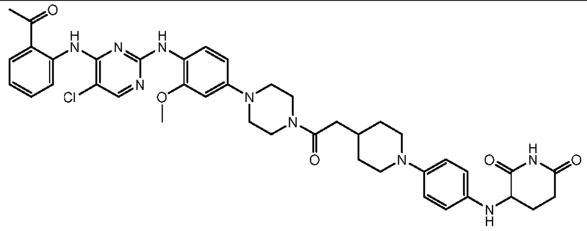 T-178 |
| 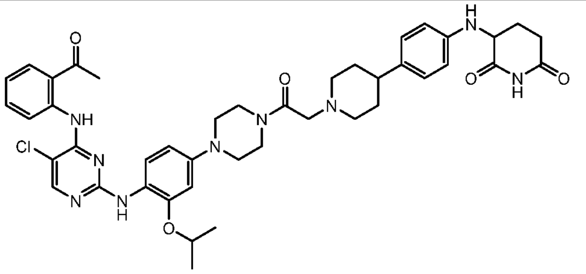 T-179 | 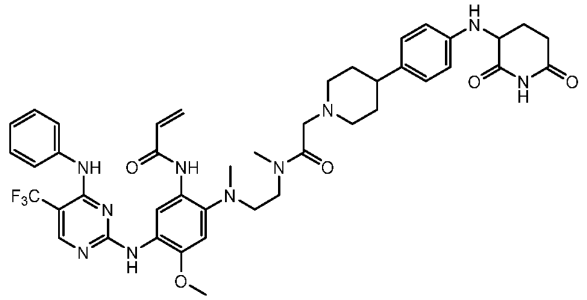 T-180 |
| 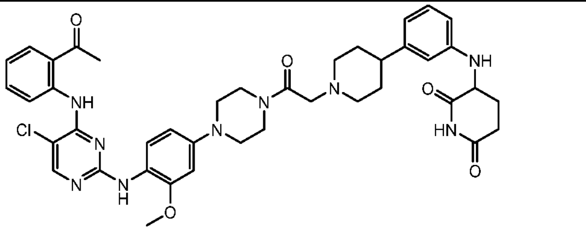 T-181 | 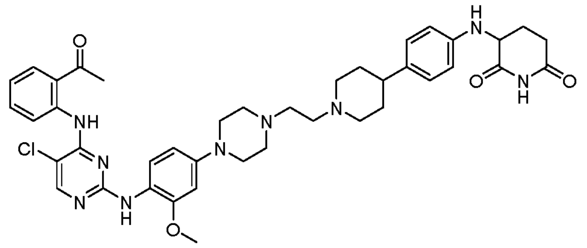 T-182 |
| 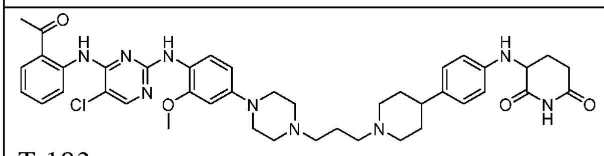 T-183 | 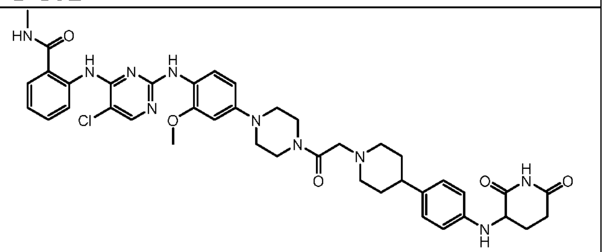 T-184 |
| 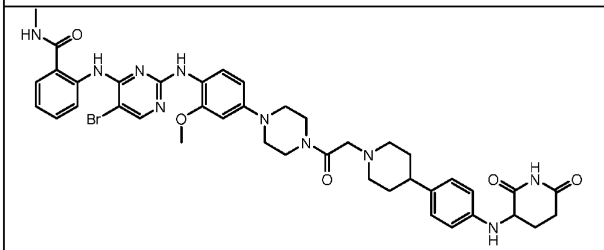 T-185 | 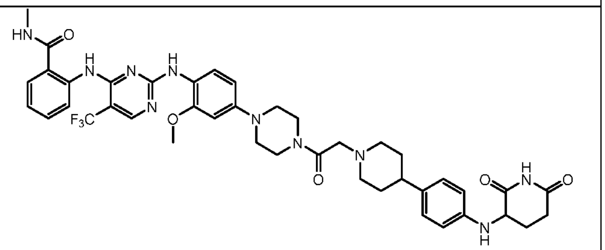 T-186 |
| 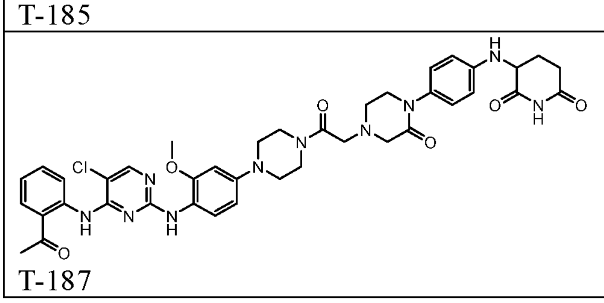 T-187 | 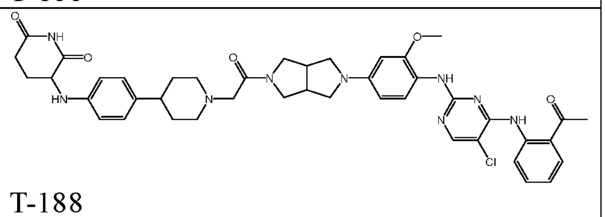 T-188 |

| | |
|---|---|
| 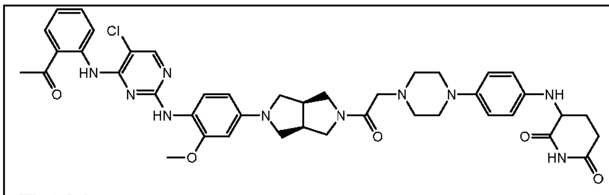 T-189 | 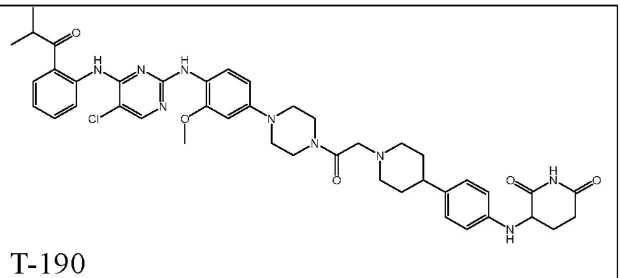 T-190 |
| 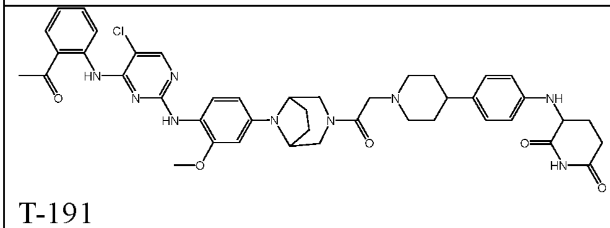 T-191 | 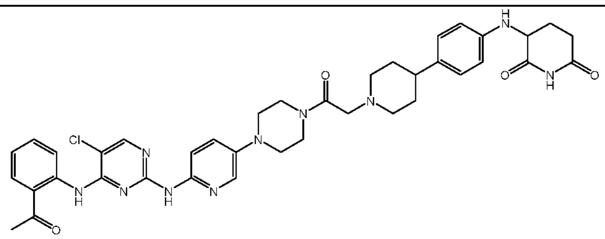 T-192 |
| 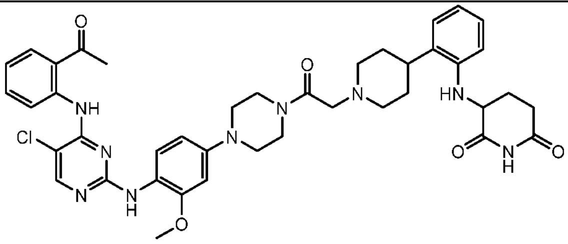 T-193 | 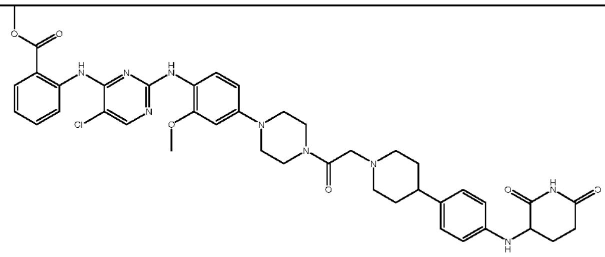 T-194 |
| 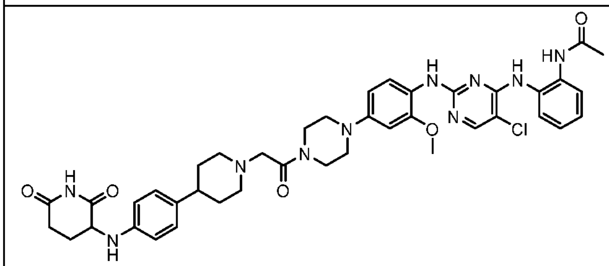 T-195 | 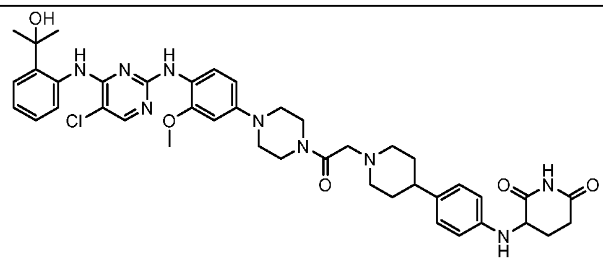 T-196 |
| 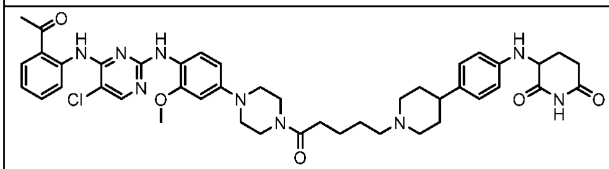 T-197 | 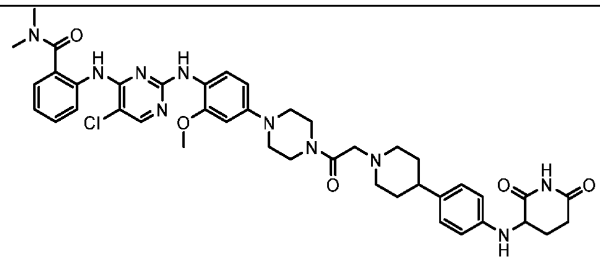 T-198 |
| 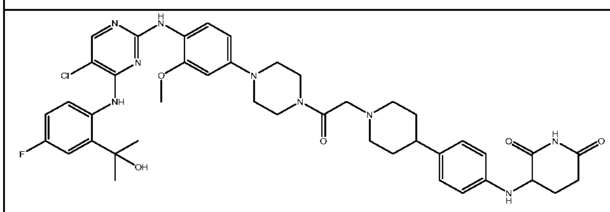 T-199 | 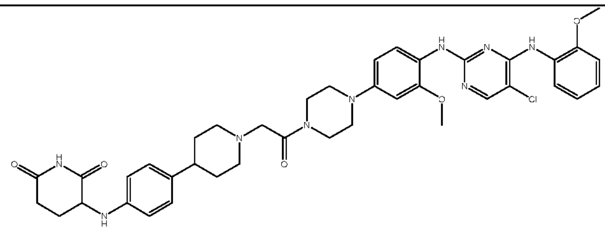 T-200 |

| | |
|---|---|
| 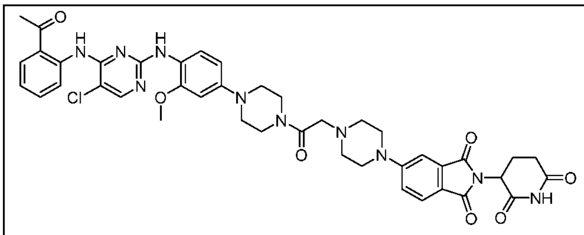 T-201 | 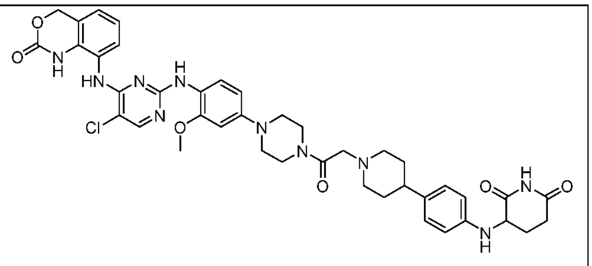 T-202 |
| 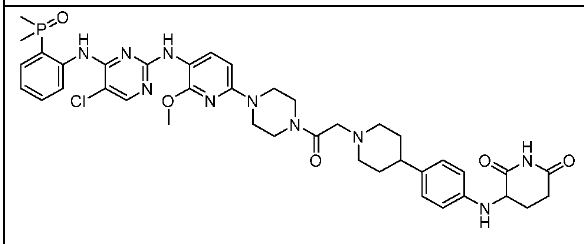 T-203 | 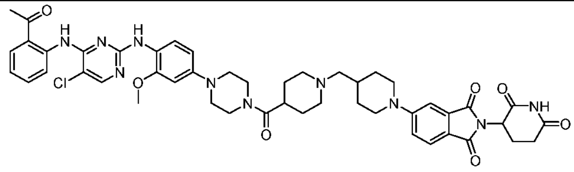 T-204 |
| 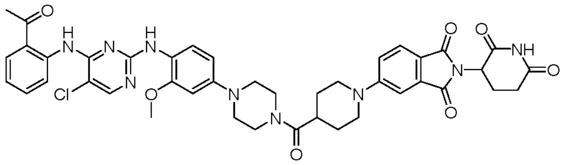 T-205 | 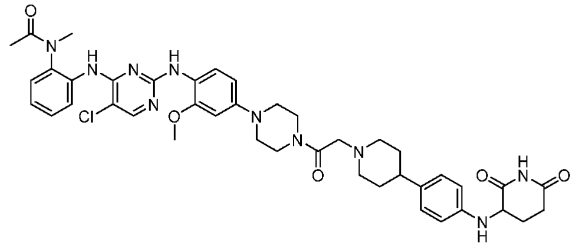 T-206 |
| 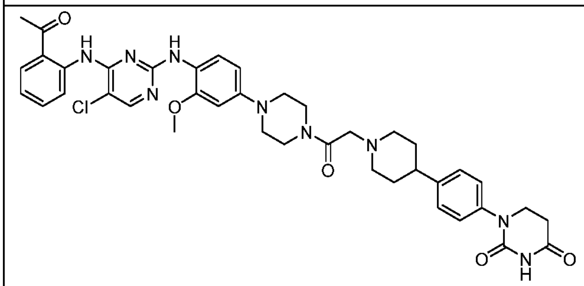 T-207 | 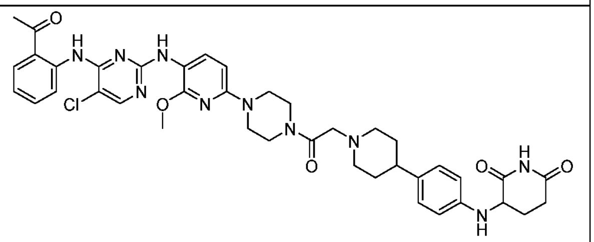 T-208 |
| 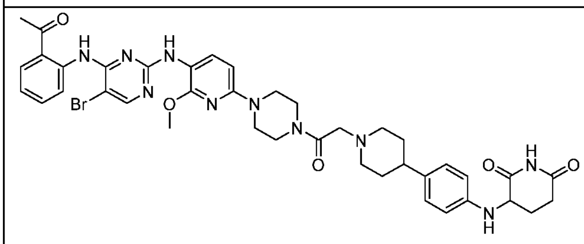 T-209 | 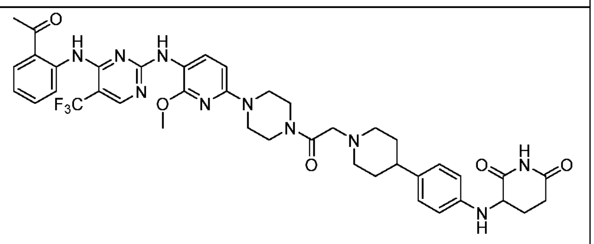 T-210 |
| 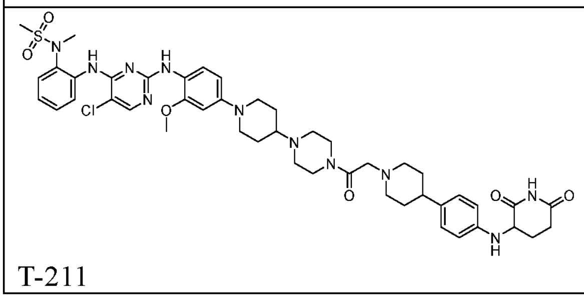 T-211 | 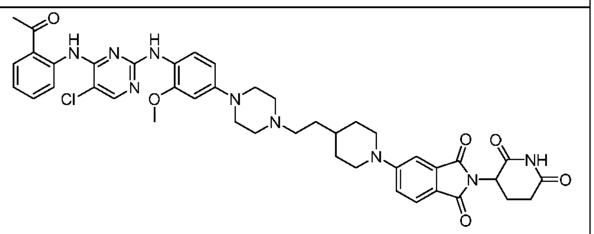 T-212 |

| | |
|---|---|
| 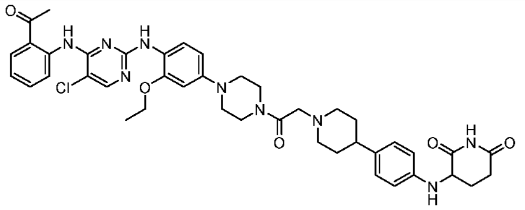 T-213 | 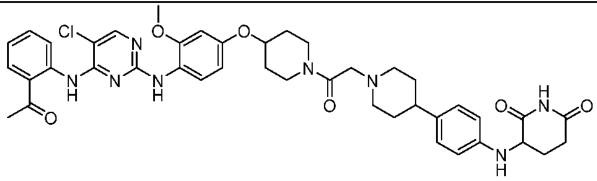 T-214 |
| 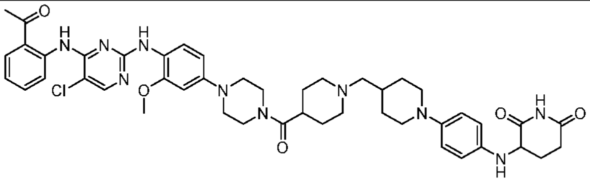 T-215 | 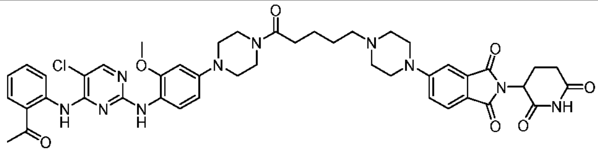 T-216 |
| 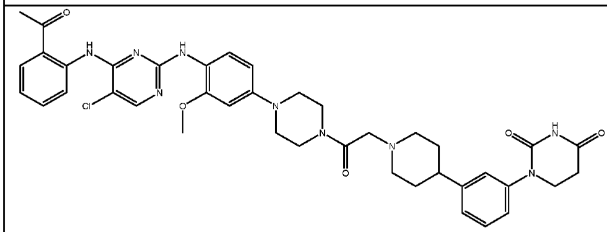 T-217 | 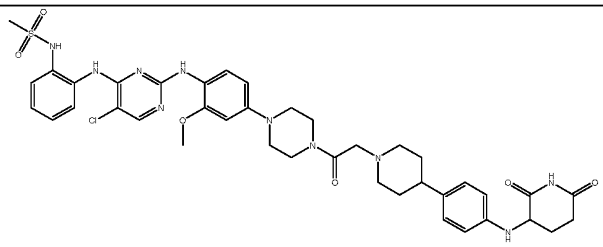 T-218 |
| 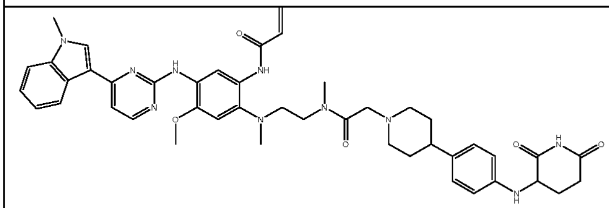 T-219 | 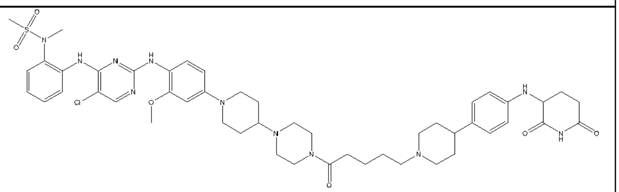 T-220 |
| 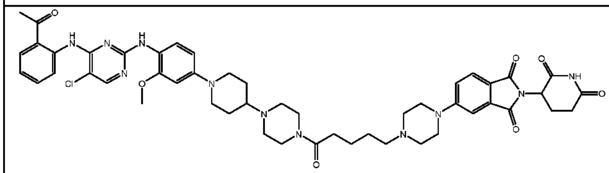 T-221 | 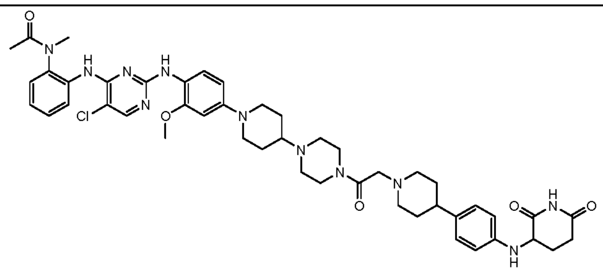 T-222 |
| 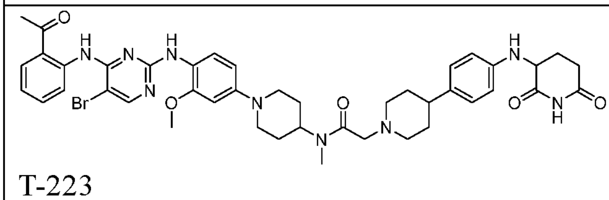 T-223 | 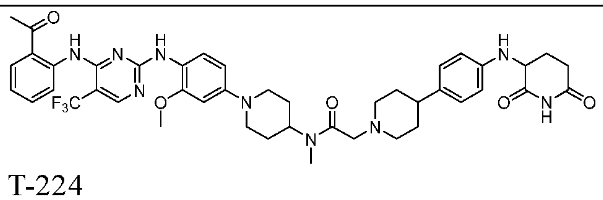 T-224 |

| | |
|---|---|
| 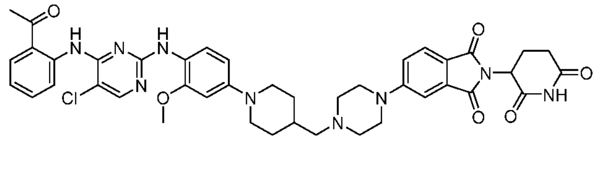 T-225 | 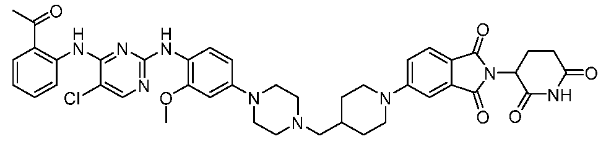 T-226 |
| 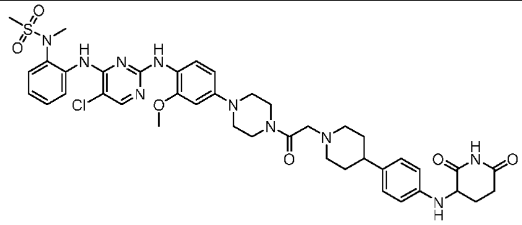 T-227 | 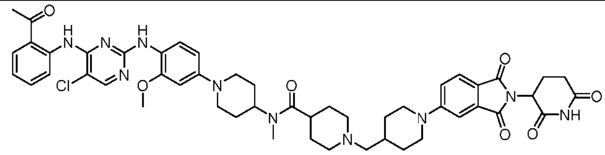 T-228 |
| 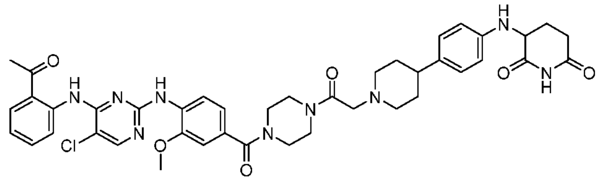 T-229 | 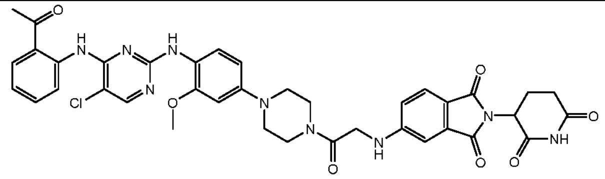 T-230 |
| 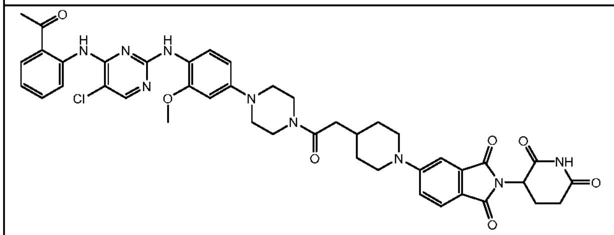 T-231 | 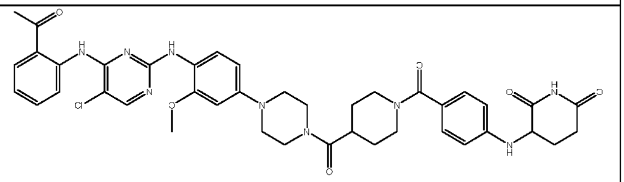 T-232 |
| 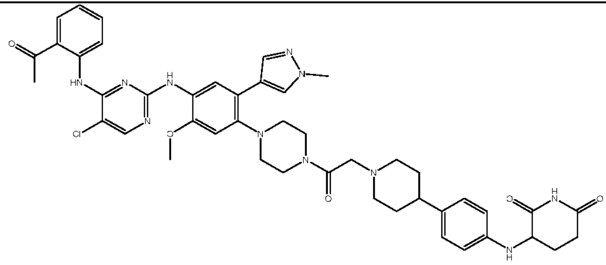 T-233 | 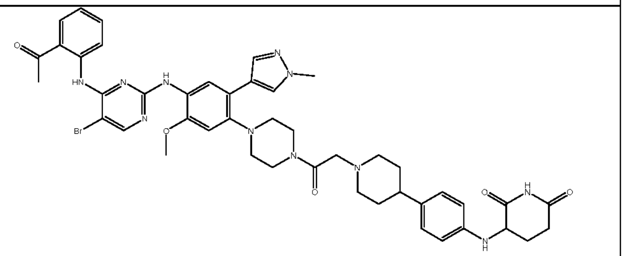 T-234 |
| 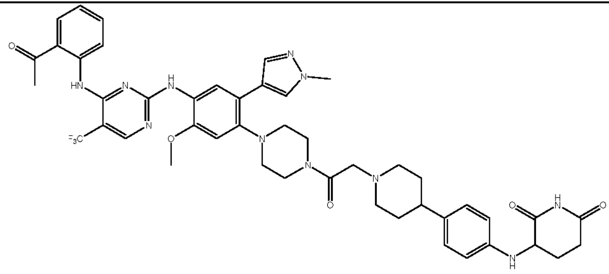 T-235 | 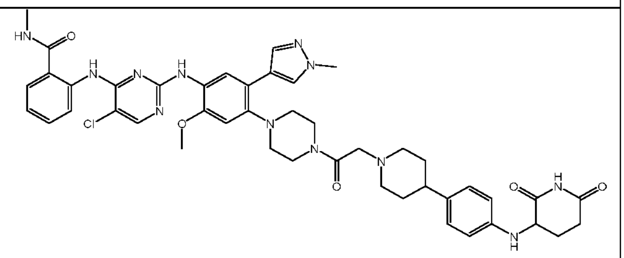 T-236 |

| | |
|---|---|
| 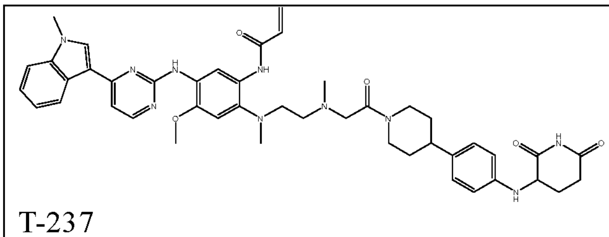 T-237 | 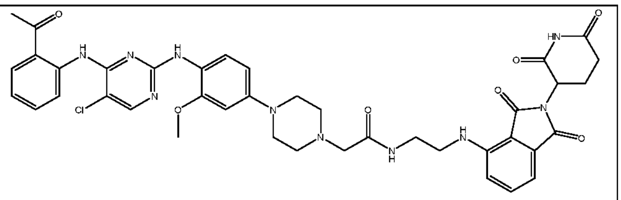 T-238 |
| 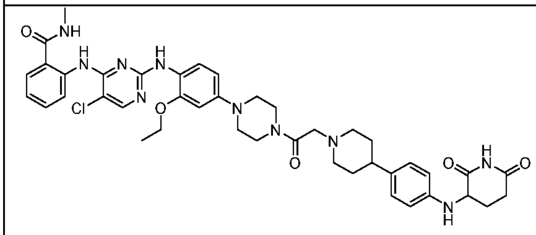 T-239 | 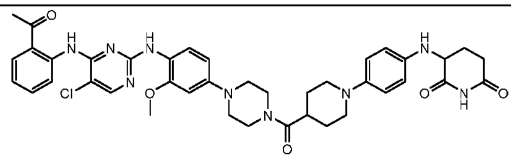 T-240 |
| 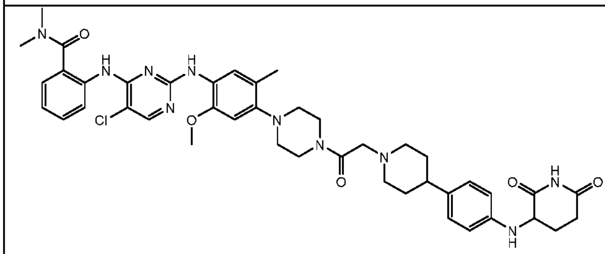 T-241 | 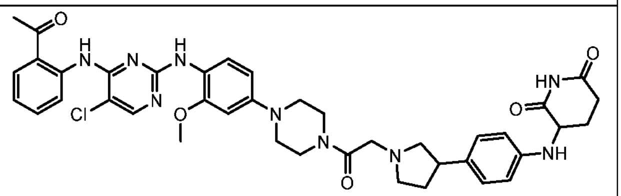 T-242 |
| 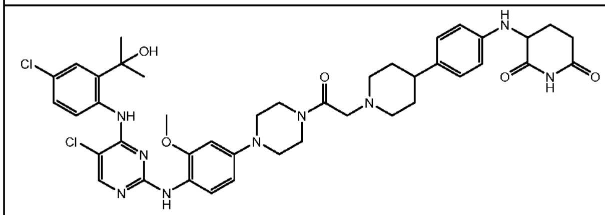 T-243 | 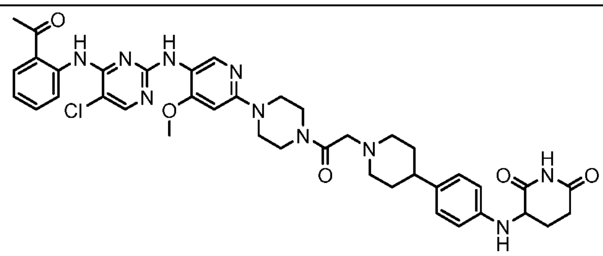 T-244 |
| 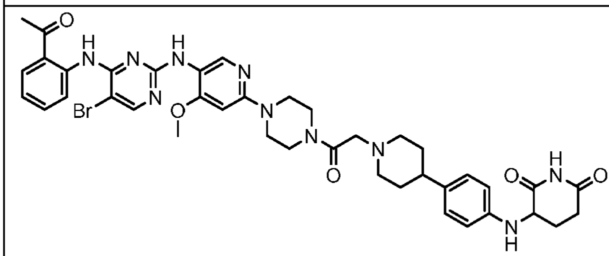 T-245 | 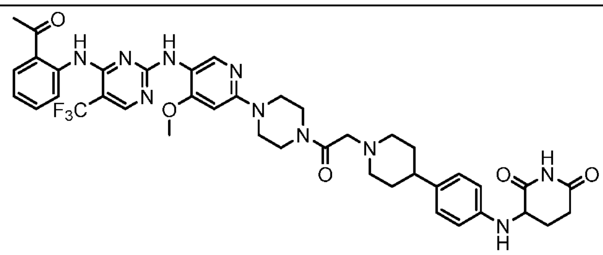 T-246 |
| 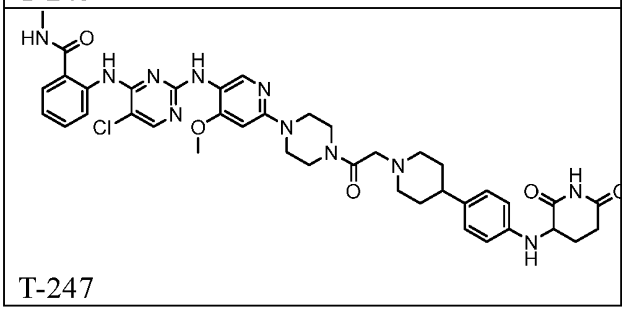 T-247 | 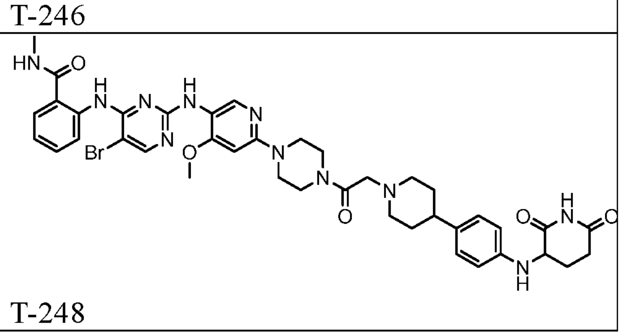 T-248 |

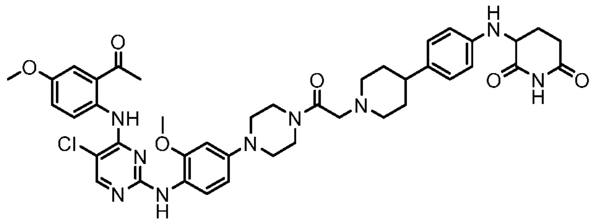
T-249

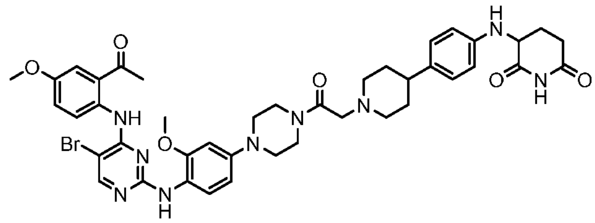
T-250

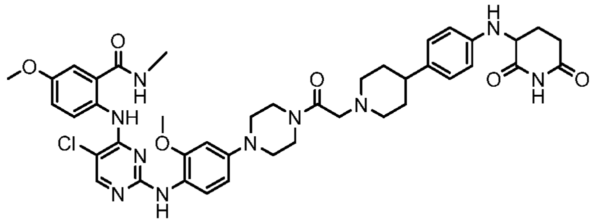
T-251

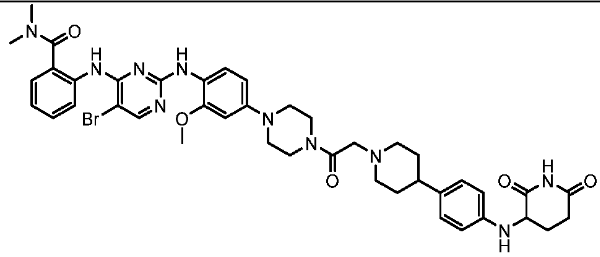
T-252

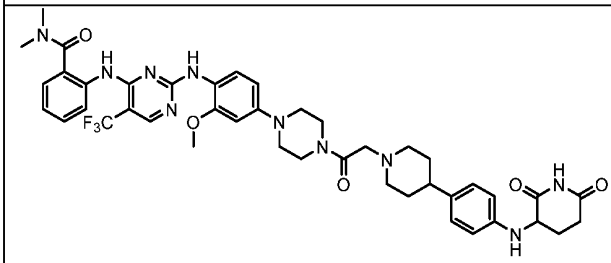
T-253

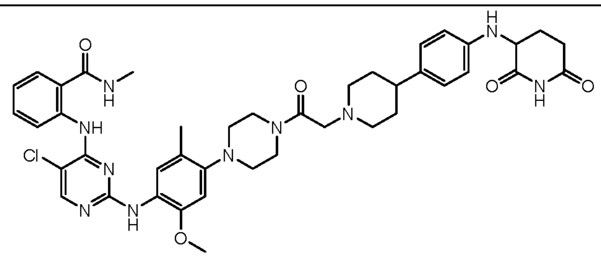
T-254

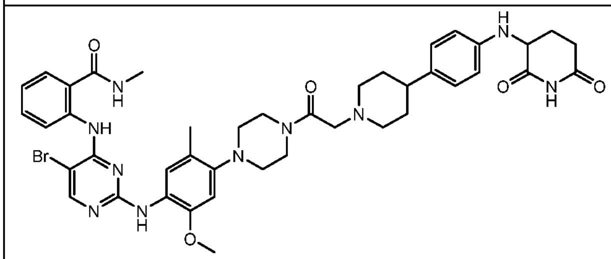
T-255

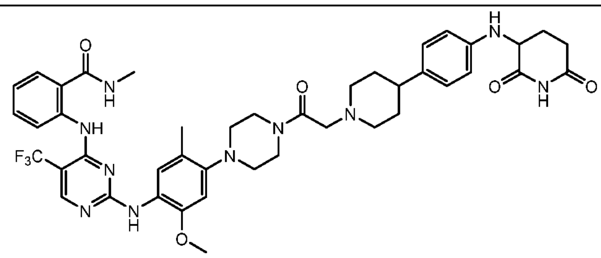
T-256

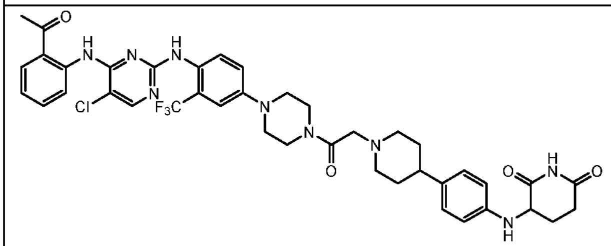
T-257

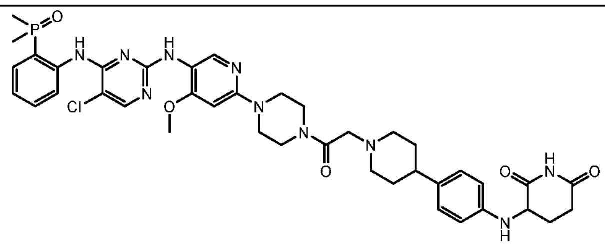
T-258

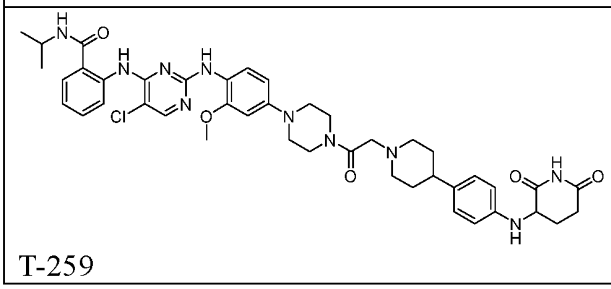
T-259

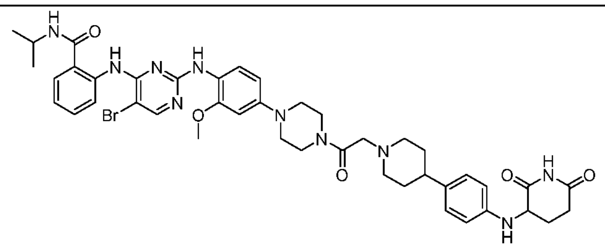
T-260

| | |
|---|---|
| 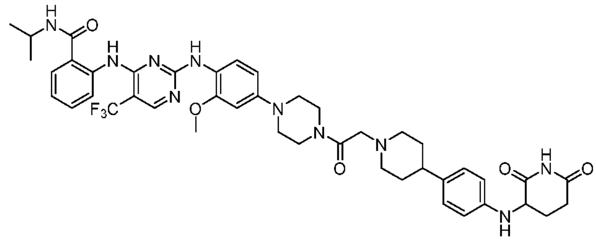 T-261 | 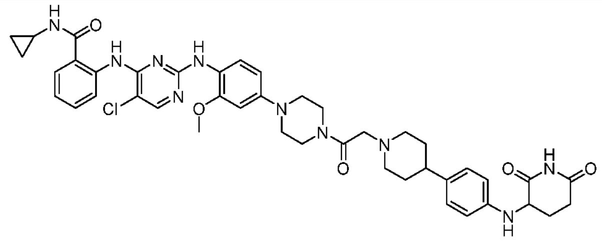 T-262 |
| 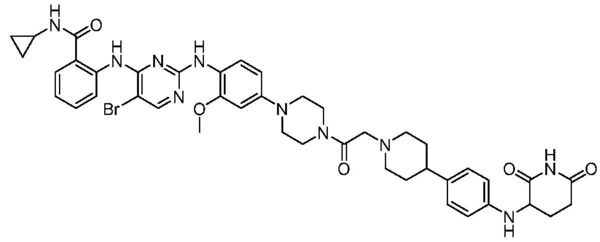 T-263 | 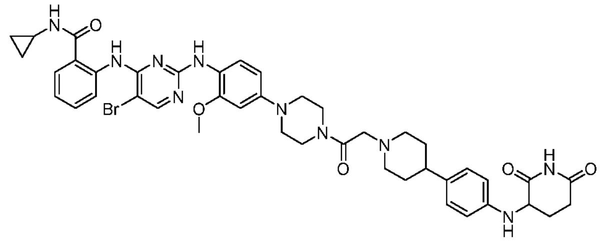 T-264 |
| 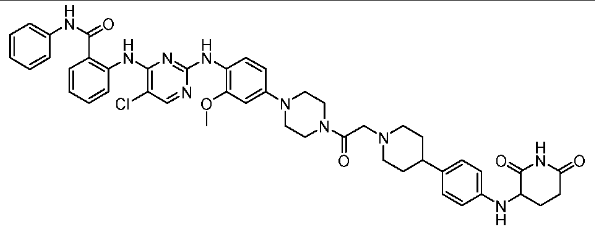 T-265 | 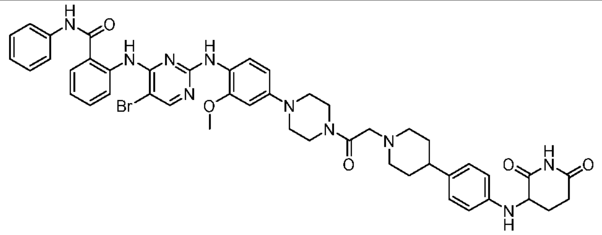 T-266 |
| 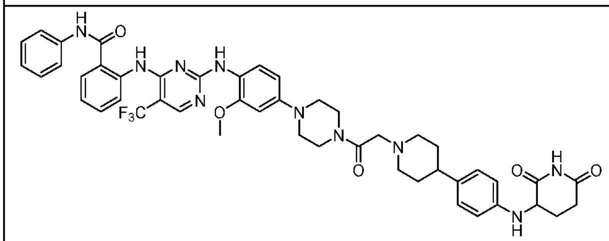 T-267 | 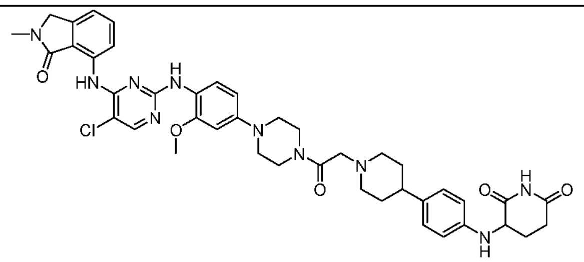 T-268 |
| 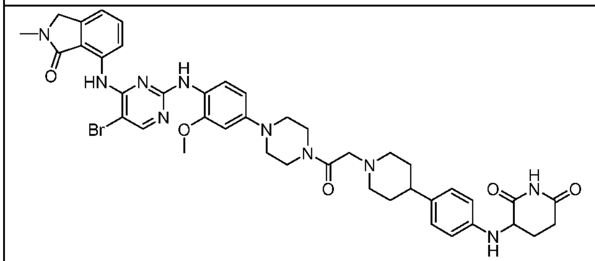 T-269 | 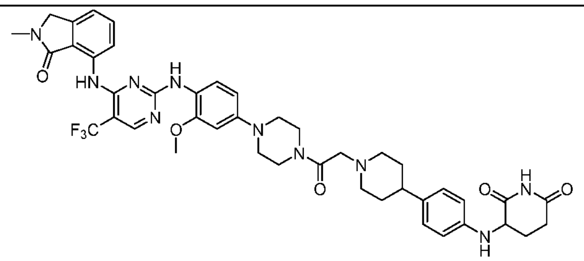 T-270 |
| 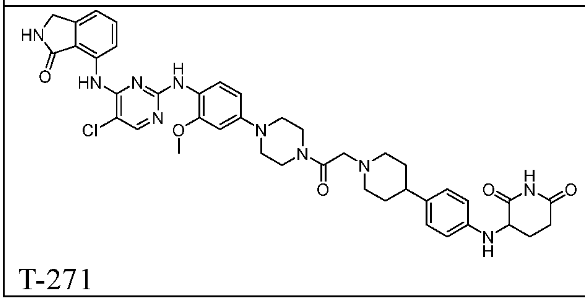 T-271 | 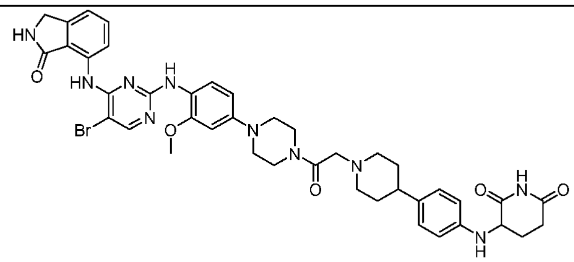 T-272 |

| | |
|---|---|
| 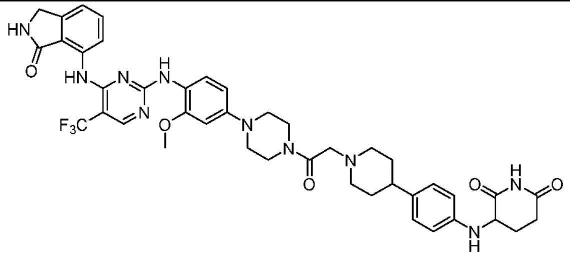 T-273 | 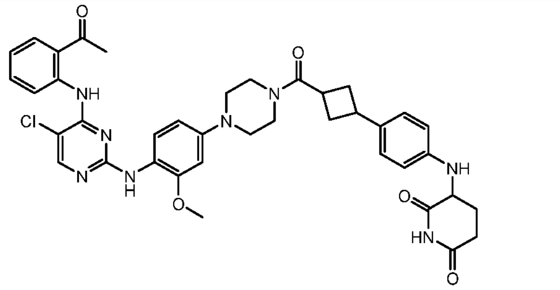 T-274 |
| 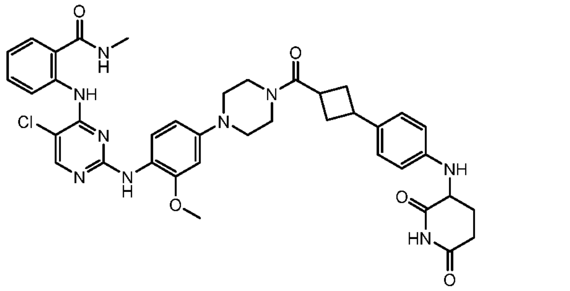 T-275 | 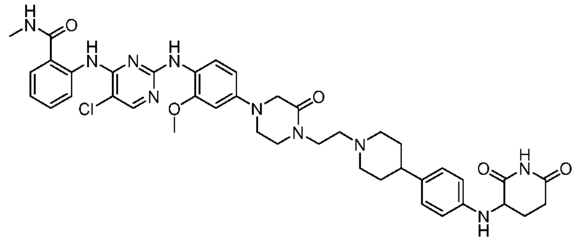 T-276 |
| 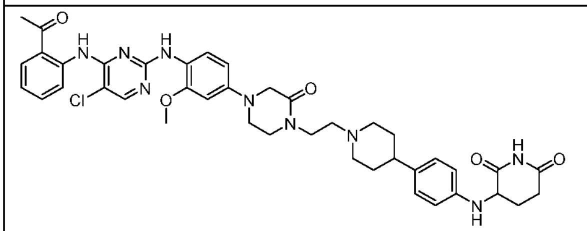 T-277 | 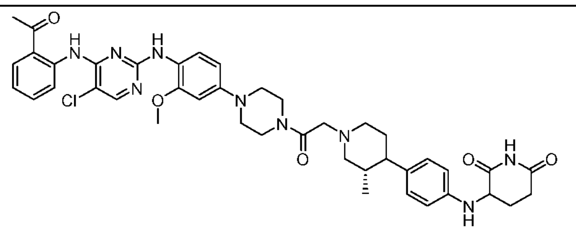 T-278 |
| 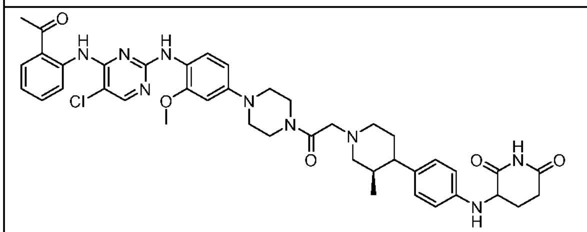 T-279 | 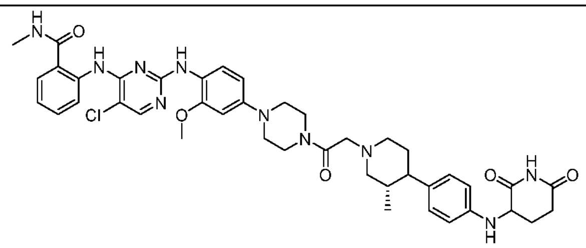 T-280 |
| 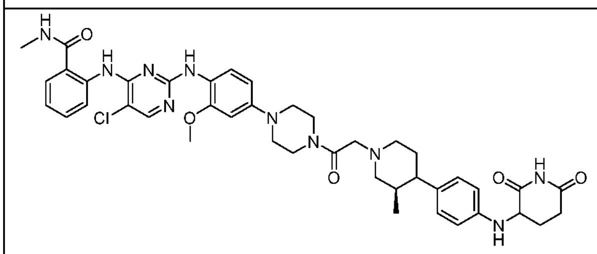 T-281 | 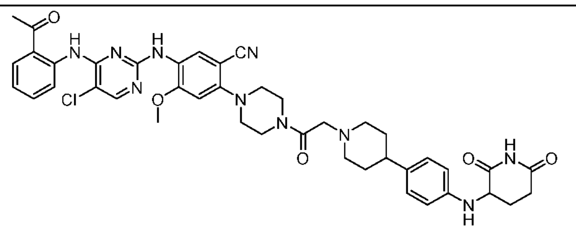 T-282 |
| 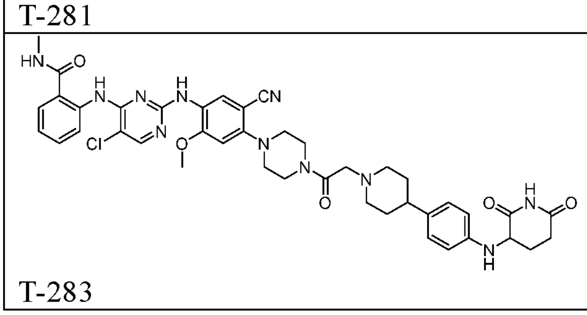 T-283 | 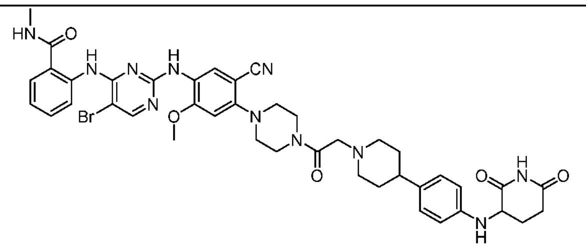 T-284 |

| | |
|---|---|
| 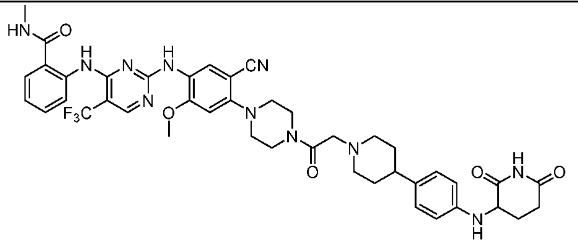 T-285 | 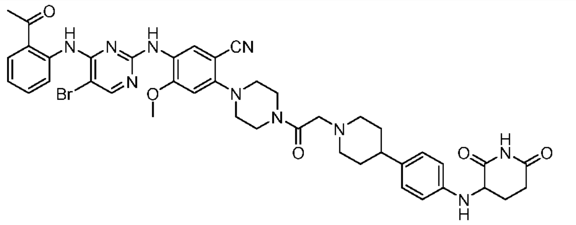 T-286 |
| 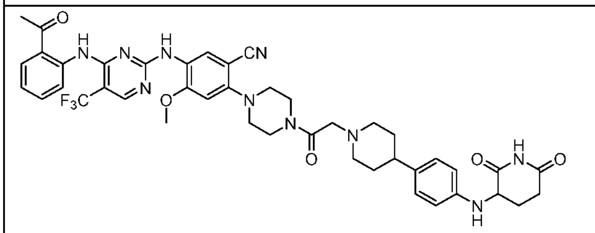 T-287 | 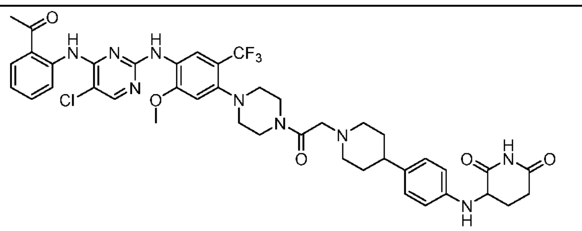 T-288 |
| 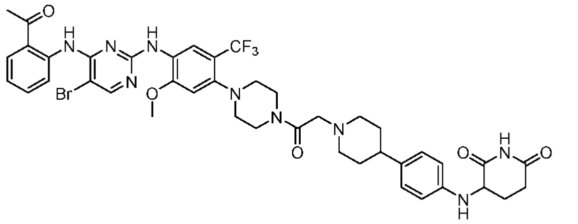 T-289 | 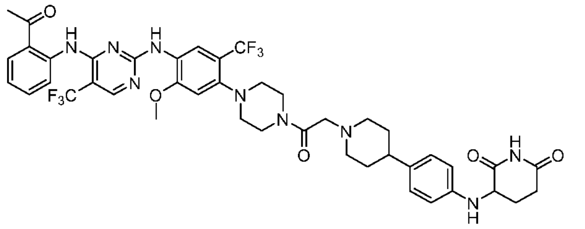 T-290 |
| 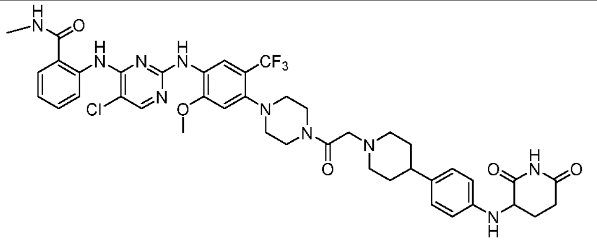 T-291 | 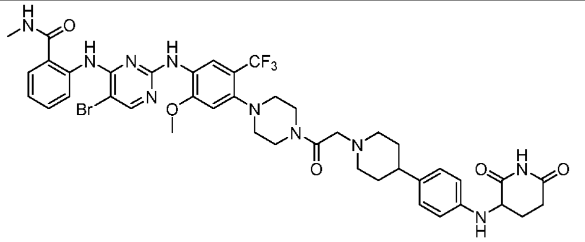 T-292 |
| 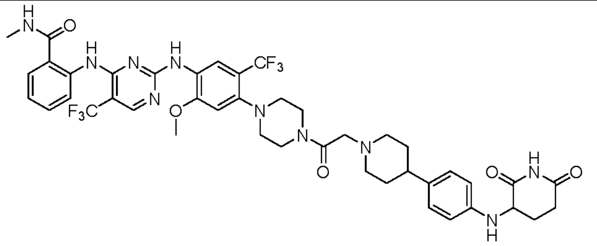 T-293 | 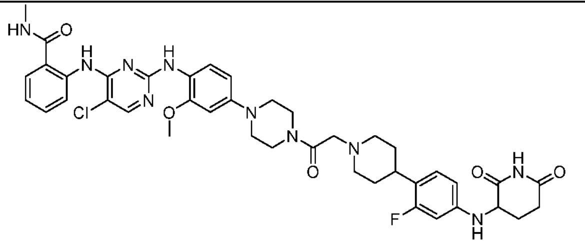 T-294 |
| 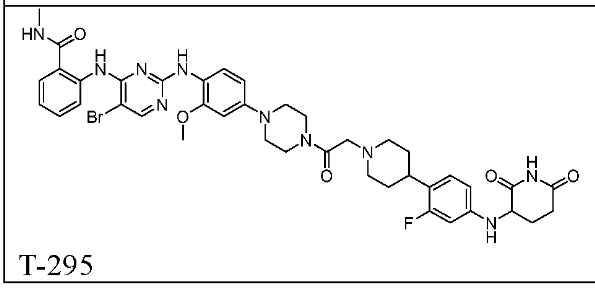 T-295 | 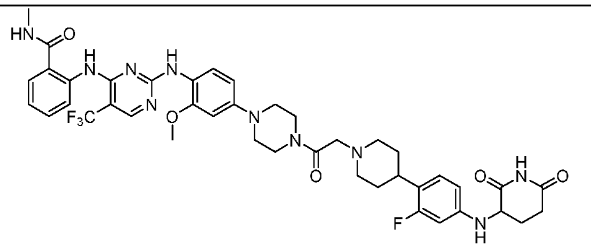 T-296 |

| | |
|---|---|
| 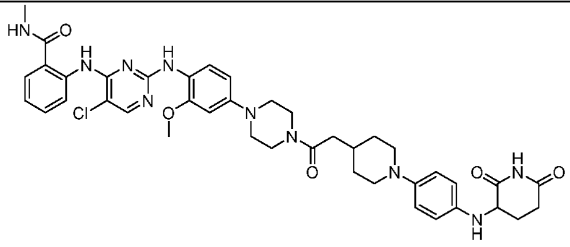 T-297 | 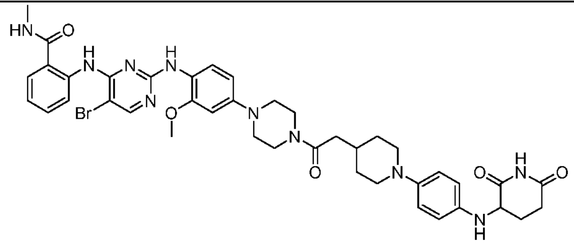 T-298 |
| 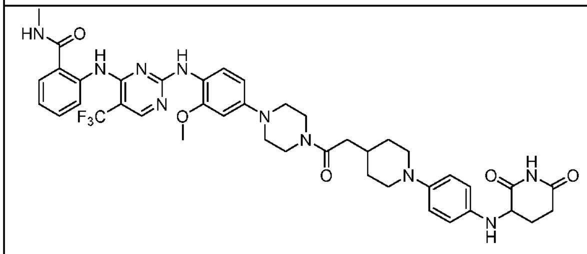 T-299 | 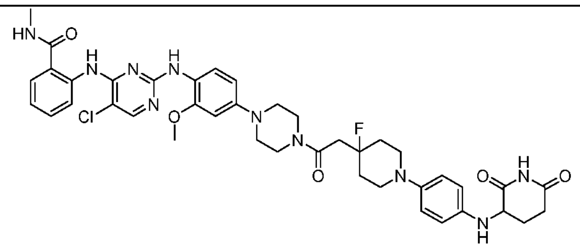 T-300 |
| 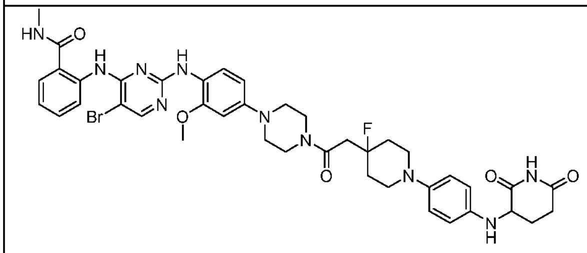 T-301 | 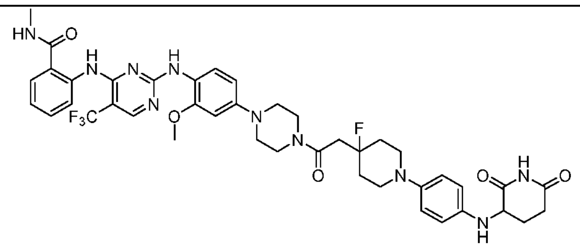 T-302 |
| 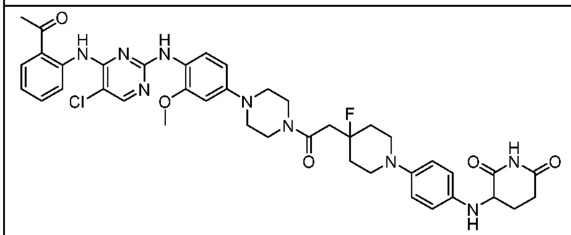 T-303 | 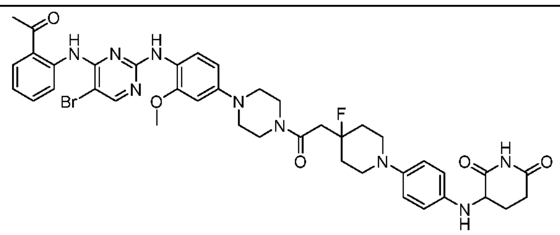 T-304 |
| 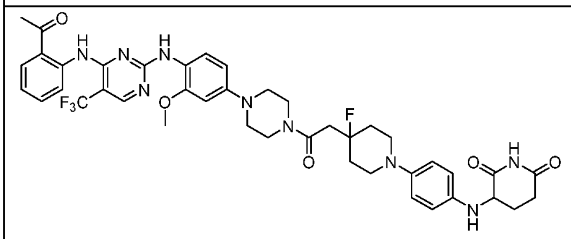 T-305 | 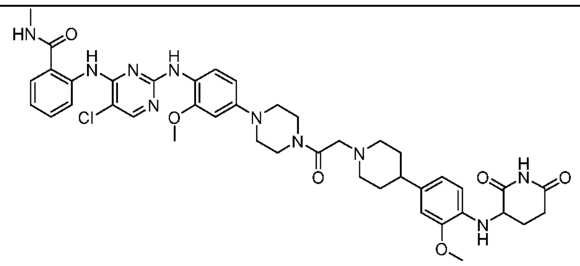 T-306 |
| 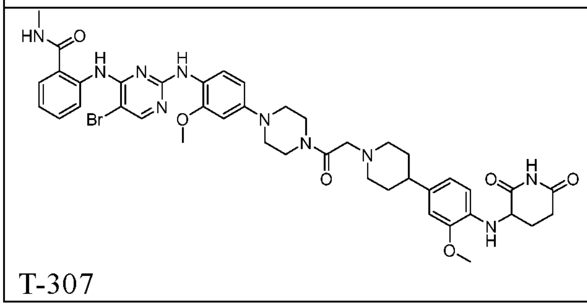 T-307 | 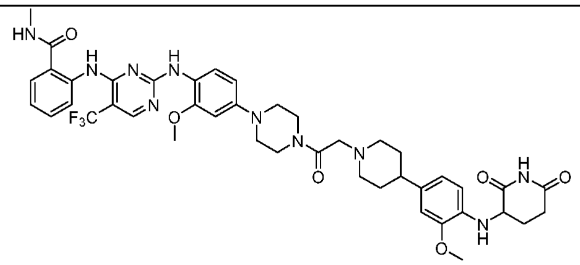 T-308 |

| | |
|---|---|
| 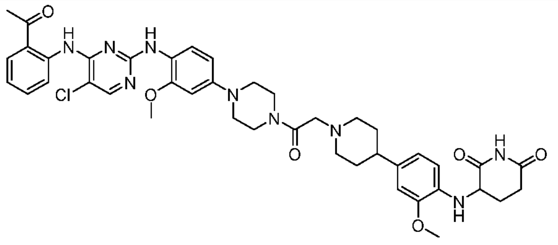<br>T-309 | 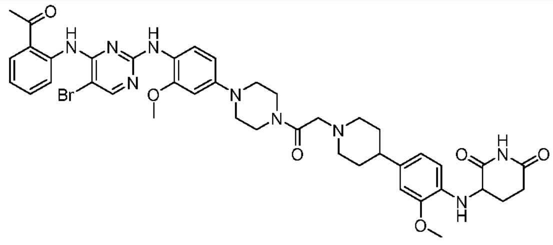<br>T-310 |
| 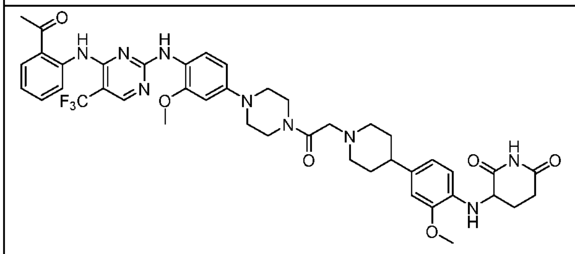<br>T-311 | 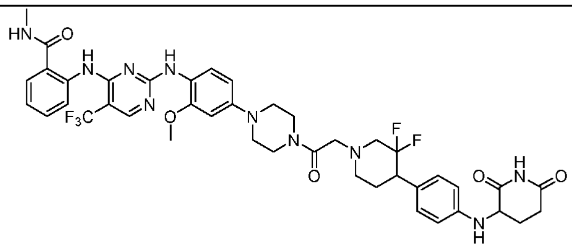<br>T-312 |
| 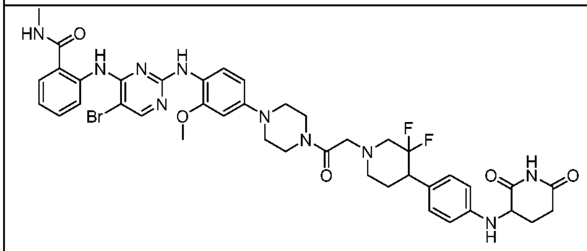<br>T-313 | 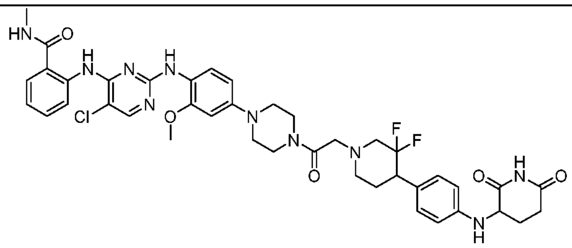<br>T-314 |
| 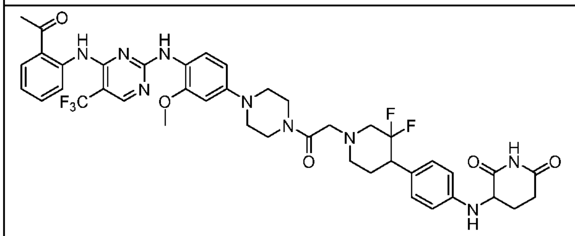<br>T-315 | 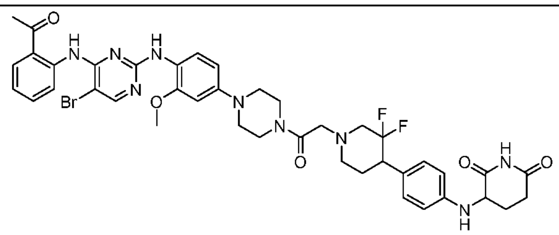<br>T-316 |
| 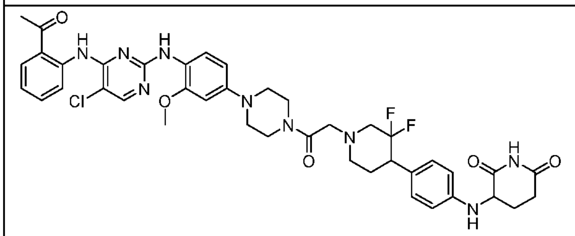<br>T-317 | 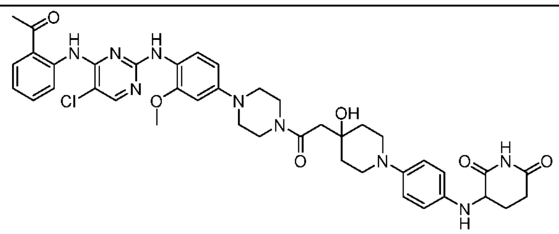<br>T-318 |
| 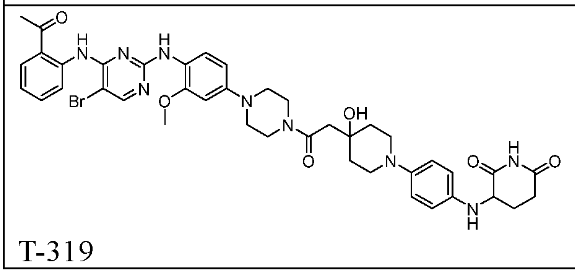<br>T-319 | 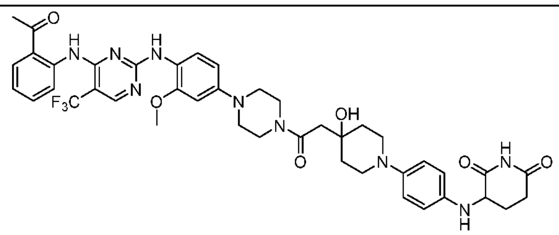<br>T-320 |

| | |
|---|---|
| 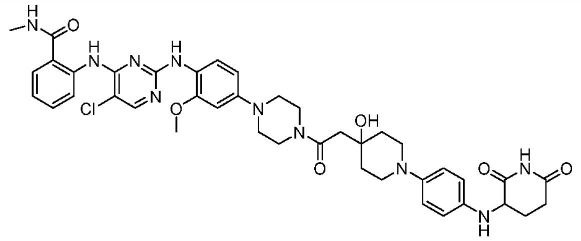 T-321 | 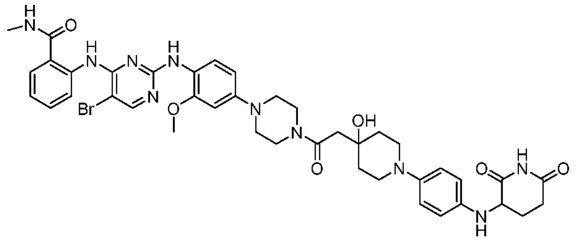 T-322 |
| 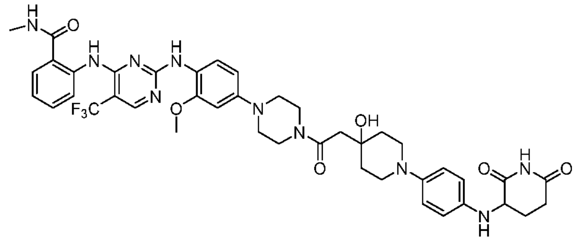 T-323 | 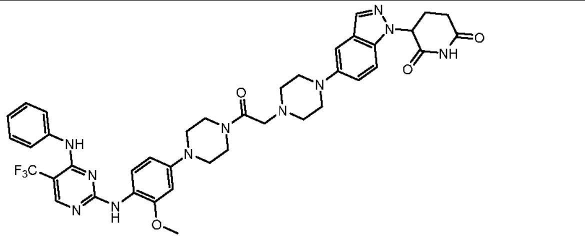 T-324 |
| 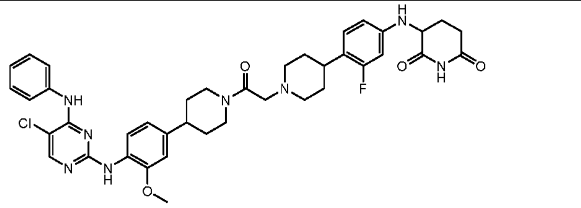 T-325 | 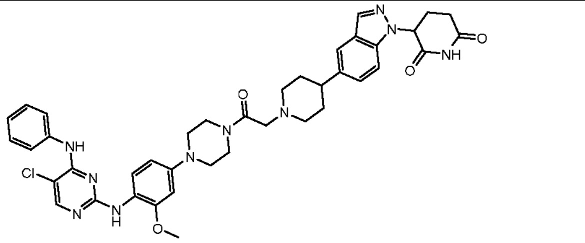 T-326 |
| 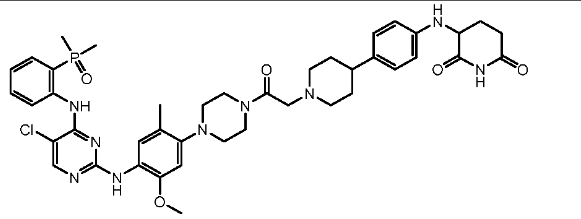 T-327 | 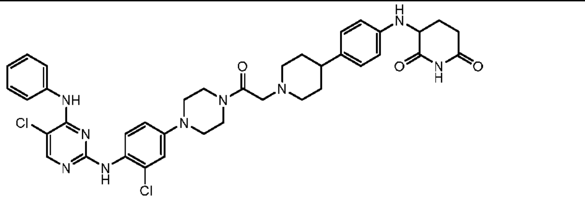 T-328 |
| 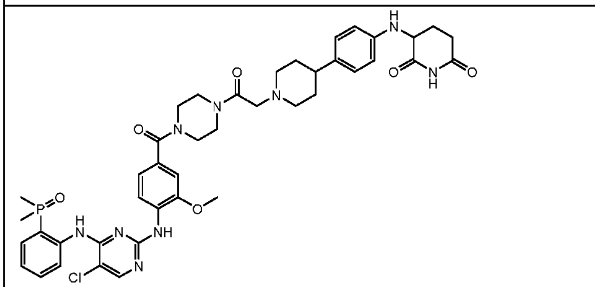 T-329 | 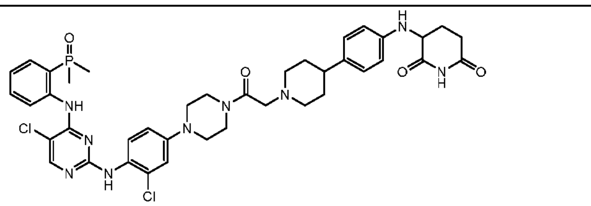 T-330 |
| 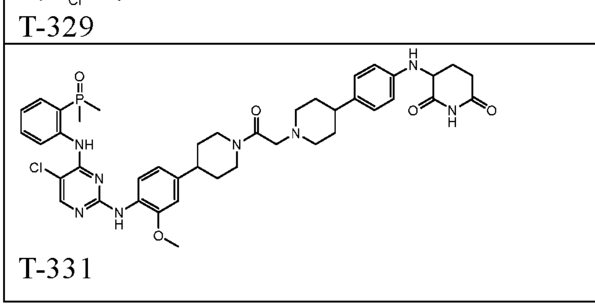 T-331 | 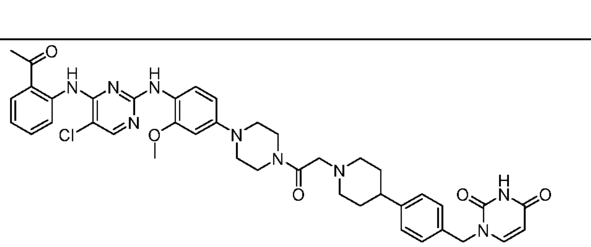 T-332 |

| | |
|---|---|
| 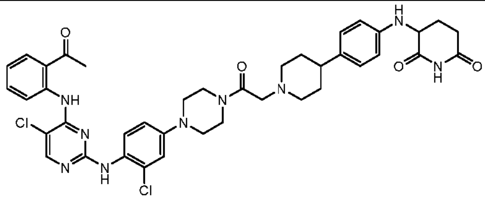 T-333 | 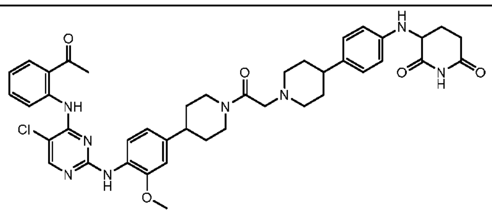 T-334 |
| 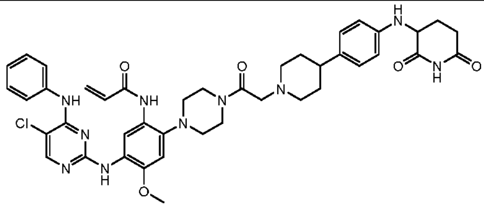 T-335 | 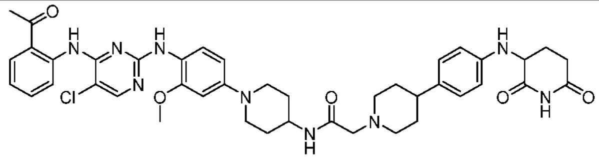 T-336 |
| 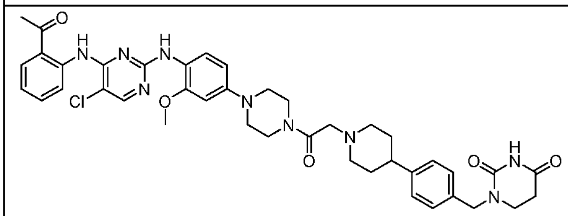 T-337 | 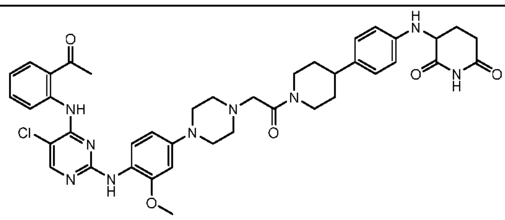 T-338 |
| 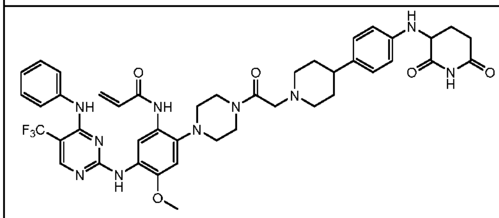 T-339 | 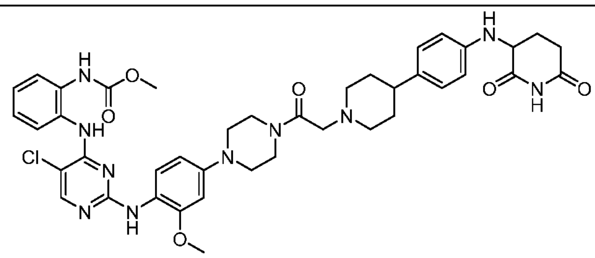 T-340 |
| 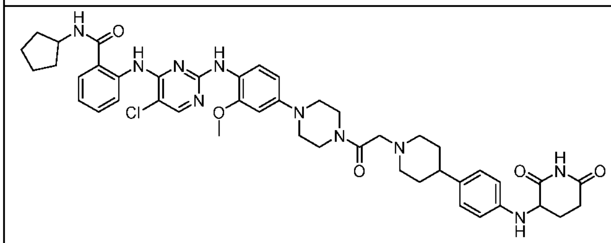 T-341 | 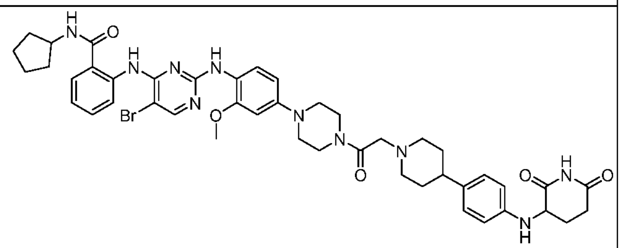 T-342 |
| 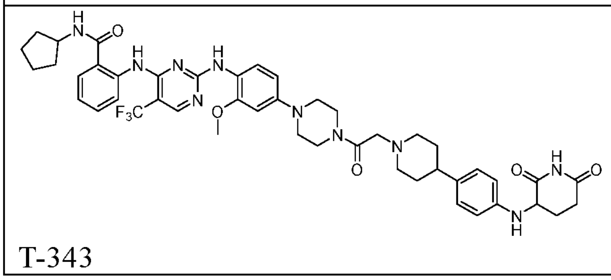 T-343 | 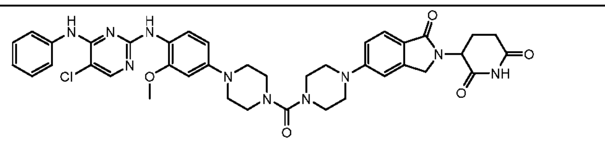 T-344 |

| | |
|---|---|
| 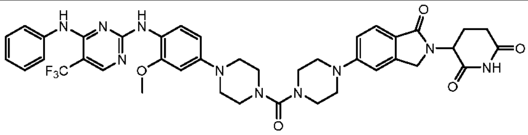 T-345 | 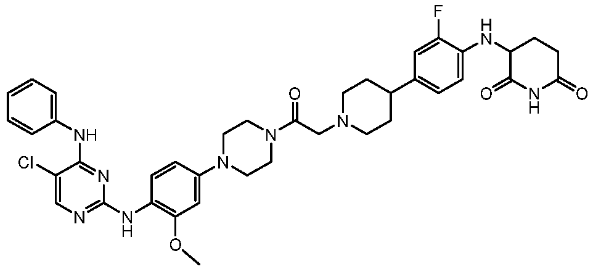 T-346 |
| 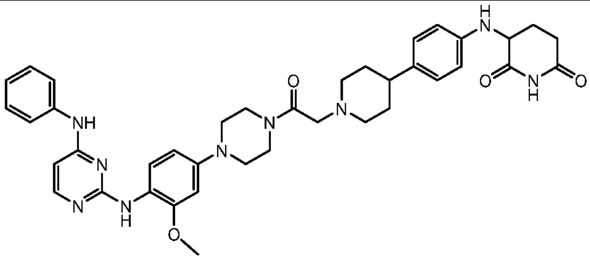 T-347 | 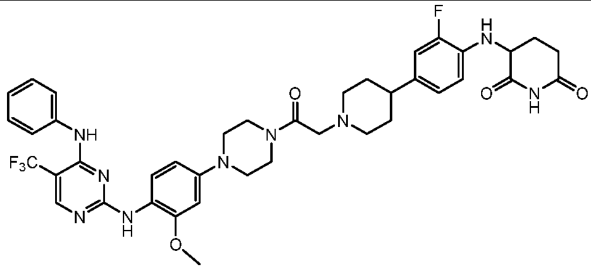 T-348 |
| 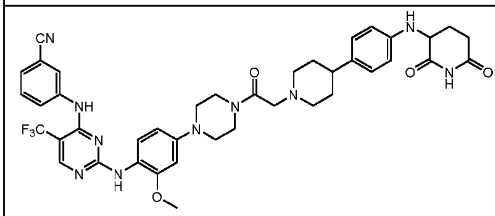 T-349 | 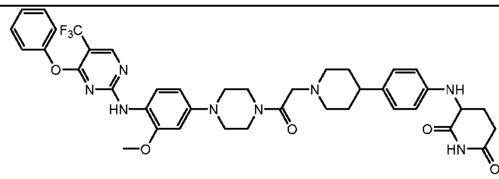 T-350 |
| 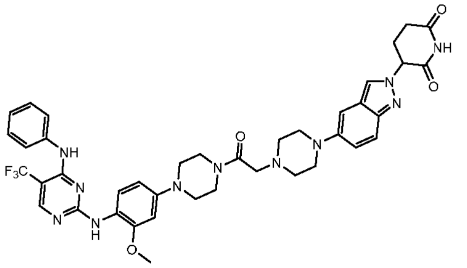 T-351 | 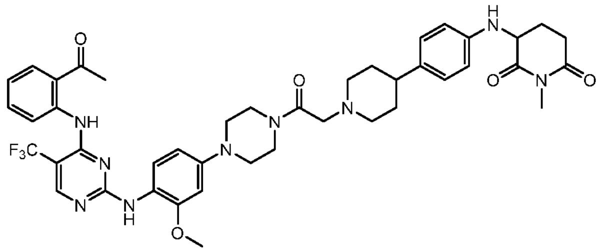 T-352 |
| 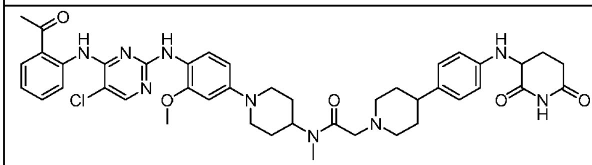 T-353 | 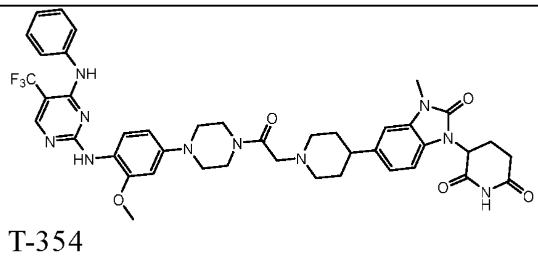 T-354 |
| 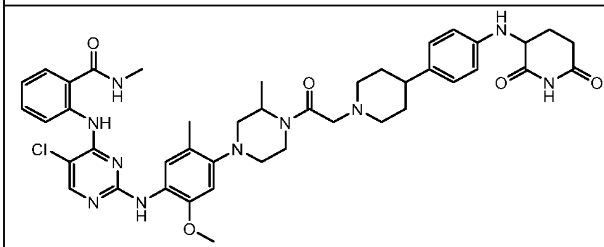 T-355 | 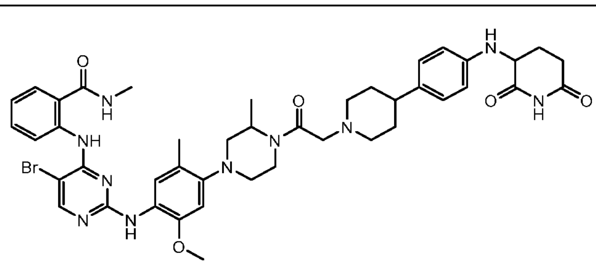 T-356 |

| | |
|---|---|
| 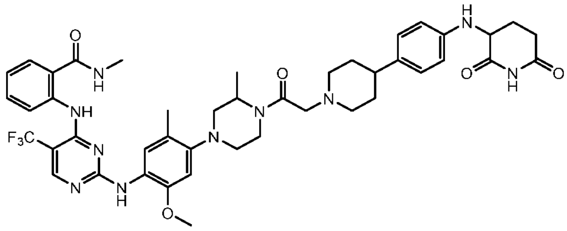 T-357 | 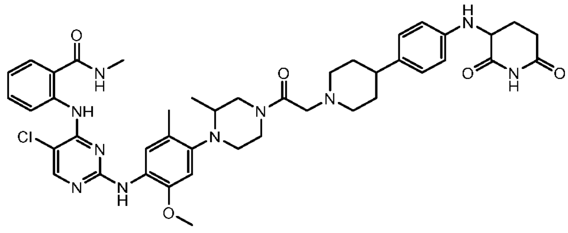 T-358 |
| 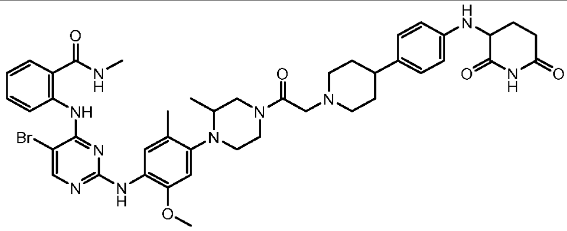 T-359 | 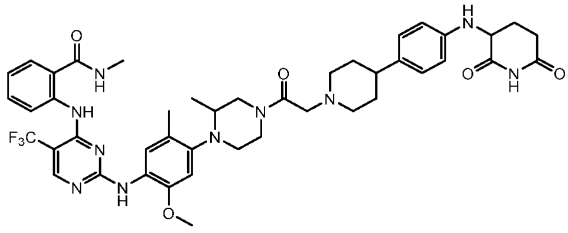 T-360 |
| 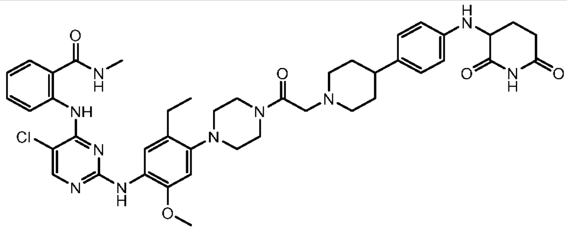 T-361 | 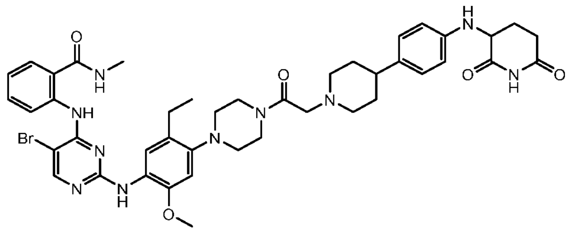 T-362 |
| 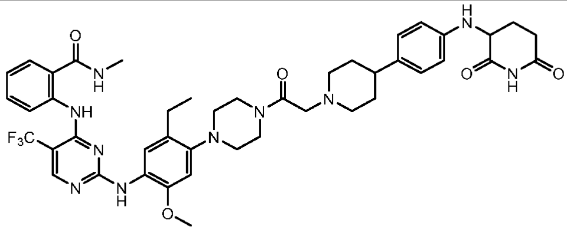 T-363 | 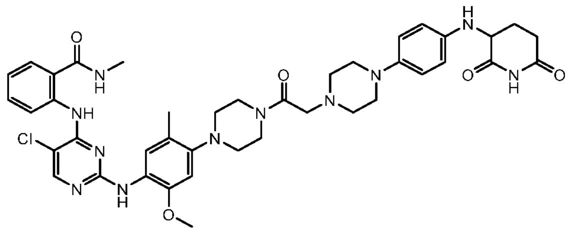 T-364 |
| 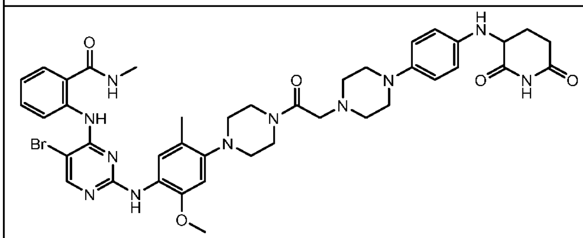 T-365 | 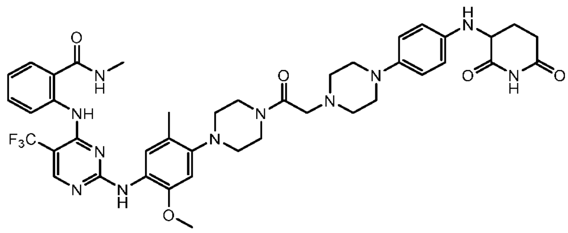 T-366 |
| 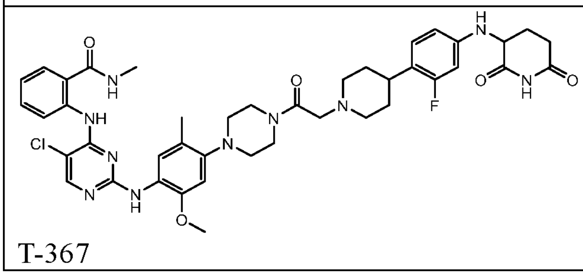 T-367 | 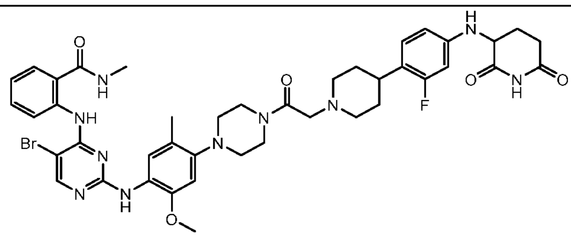 T-368 |

| | |
|---|---|
| 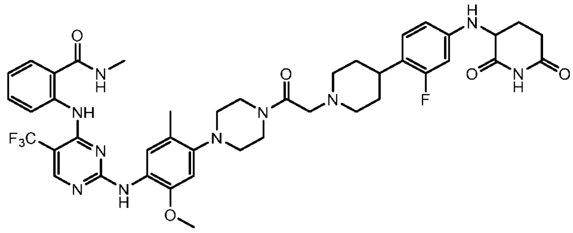 T-369 | 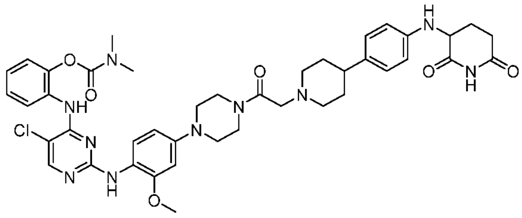 T-370 |
| 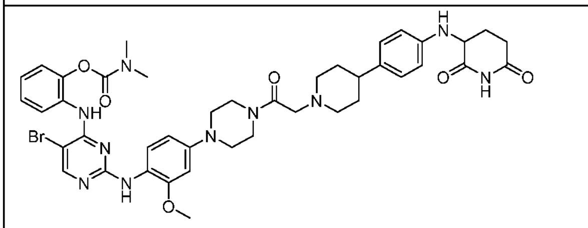 T-371 | 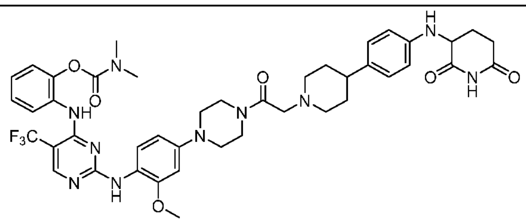 T-372 |
| 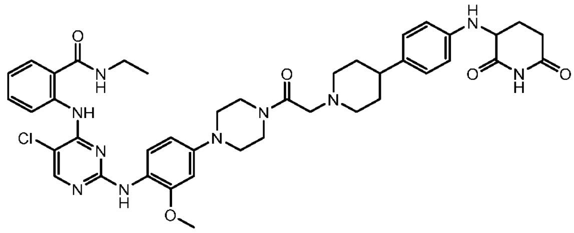 T-373 | 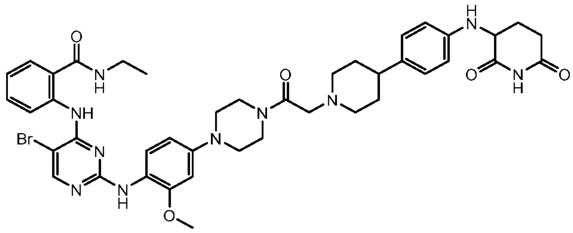 T-374 |
| 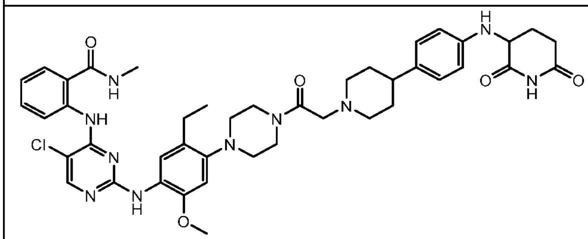 T-375 | 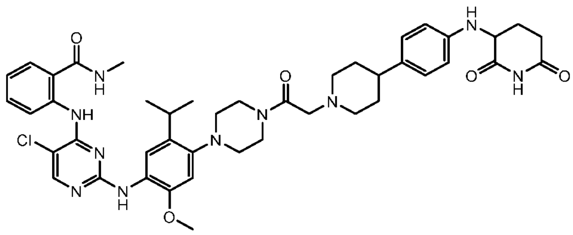 T-376 |
| 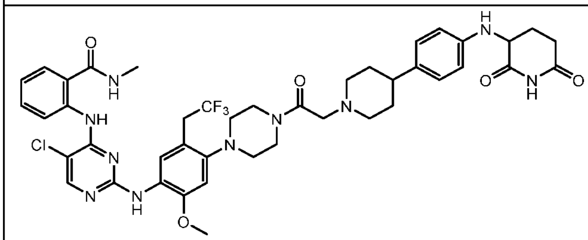 T-377 | 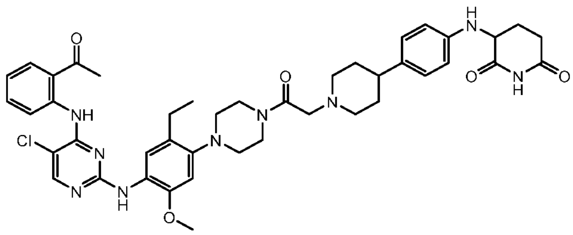 T-378 |
| 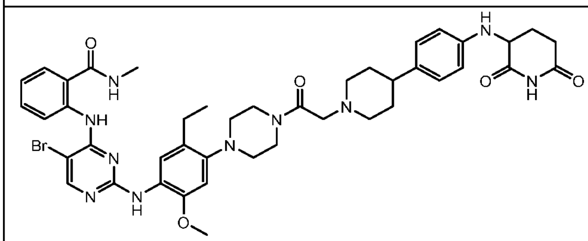 T-379 | 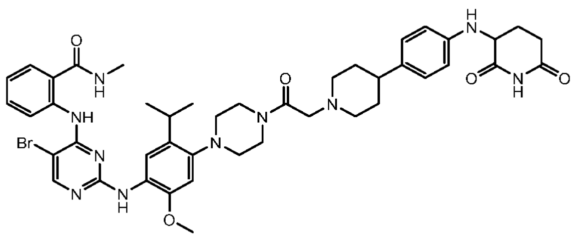 T-380 |

| | |
|---|---|
| 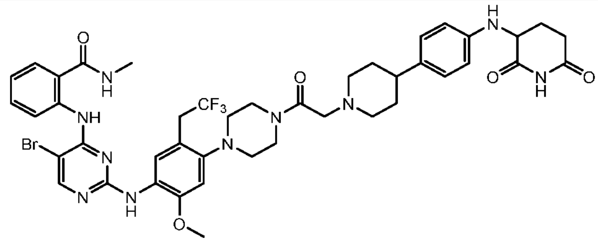 T-381 | 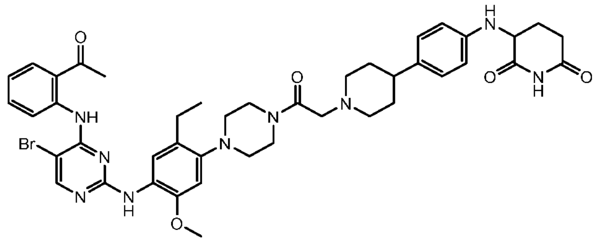 T-382 |

**7.** The compound according to claim 1, wherein the pharmaceutically acceptable salt is an inorganic acid salt or an organic acid salt;

wherein, the inorganic acid salt is selected from the group consisting of: hydrochloride, hydrobromide, hydriodate, sulfate, bisulfate, nitrate, phosphate, and acid phosphate;
the organic acid salt is selected from the group consisting of: formate, acetate, trifluoroacetate, propionate, pyruvate, glycolate, oxalate, malonate, fumarate, maleate, lactate, malate, citrate, tartrate, methanesulfonate, ethane sulfonate, benzenesulfonate, p-toluenesulfonate, salicylate, picrate, glutamate, ascorbate, camphorate, and camphorsulfonate.

**8.** A pharmaceutical composition, comprising the compound according to claim 1, and pharmaceutically acceptable carriers.

**9.** A use of the compound according to claim 1 or a composition comprising the same in the use selected from the group consisting of:

1) Preparation of drugs for modulating EGFR kinase activity or treating EGFR-related diseases;
2) Preparation of drugs for degrading EGFR proteins; and
3) Preparation of drugs for degrading an EGFR mutated protein selected from the group consisting of: DEL19, L858R, L858R/T790M, L858R/C797S, DEL19/T790M/C797S, L858R/T790M/C797S, exon20 insertion mutation, L861Q, DEL19-G724S and L858R-L792H.

**10.** The use according to claim 9, wherein the EGFR-related disease is selected from the group consisting of: inflammation, cancer, cardiovascular disease, infection, immune disease and metabolic disease.

**INTERNATIONAL SEARCH REPORT**

International application No.
**PCT/CN2022/132632**

**A. CLASSIFICATION OF SUBJECT MATTER**

C07D401/12(2006.01)i;C07D417/12(2006.01)i;C07D401/02(2006.01)i;C07D401/14(2006.01)i;A61K31/427(2006.01)i;A61K31/496(2006.01)i;A61K31/506(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNTXT, DWPI, ENTXT, VEN, WPABS, stn: 浙江同源康, protac, EGFR, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| A | CN 110204532 A (ZHEJIANG UNIVERSITY OF TECHNOLOGY) 06 September 2019 (2019-09-06)<br>entire document | 1-10 |
| A | CN 112321566 A (SHANGHAITECH UNIVERSITY) 05 February 2021 (2021-02-05)<br>entire document | 1-10 |
| A | CN 113735828 A (SOUTHERN MEDICAL UNIVERSITY) 03 December 2021 (2021-12-03)<br>entire document | 1-10 |
| PA | CN 115304606 A (TSINGHUA UNIVERSITY; ZEXINSHENG (BEIJING) PROTEIN DEGRADATION TECHNOLOGY CO., LTD.) 08 November 2022 (2022-11-08)<br>entire document | 1-10 |
| PX | KR 20220035014 A (J2H BIOTECH CO., LTD.) 21 March 2022 (2022-03-21)<br>embodiment compounds, and claims 9-13 | 1-10 |
| PA | TW 202216686 A (BEIGENE, LTD.) 01 May 2022 (2022-05-01)<br>entire document | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 February 2023** | **16 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

INTERNATIONAL SEARCH REPORT

International application No.
PCT/CN2022/132632

C. DOCUMENTS CONSIDERED TO BE RELEVANT

| Category* | Citation of document, with indication, where appropriate, of the relevant passages | Relevant to claim No. |
|---|---|---|
| A | WO 2020092662 A1 (THE WISTAR INSTITUTE OF ANATOMY AND BIOLOGY; THOMAS JEFFERSON UNIVERSITY) 07 May 2020 (2020-05-07) entire document | 1-10 |
| A | WO 2020172655 A1 (NEW YORK UNIVERSITY) 27 August 2020 (2020-08-27) entire document | 1-10 |
| X | WO 2021127561 A1 (C4 THERAPEUTICS INC.) 24 June 2021 (2021-06-24) embodiments, and claims 177-178 and 208-234 | 1-10 |
| PX | WO 2022012622 A1 (BEIGENE, LTD.; LEI BAILIN;) 20 January 2022 (2022-01-20) embodiments, and claims 53-54 | 1-10 |
| PX | WO 2022068849 A1 (BEIGENE, LTD.; LEI BAILIN;) 07 April 2022 (2022-04-07) entire document | 1-10 |
| PX | WO 2022171123 A1 (BEIGENE, LTD.; LEI BAILIN;) 18 August 2022 (2022-08-18) entire document | 1-10 |
| PX | WO 2022194269 A1 (SHANGHAI QILU PHARMACEUTICAL RESEARCH AND DEVELOPMENT CENTRE LTD.) 22 September 2022 (2022-09-22) entire document | 1-10 |
| PX | WO 2022228547 A1 (SICHUAN HAISCO PHARMACEUTICAL CO., LTD.) 03 November 2022 (2022-11-03) entire document | 1-10 |
| PX | WO 2022228556 A1 (BEIGENE, LTD.; LEI BAILIN;) 03 November 2022 (2022-11-03) entire document | 1-10 |

Form PCT/ISA/210 (second sheet) (July 2022)

**INTERNATIONAL SEARCH REPORT**
**Information on patent family members**

International application No.
**PCT/CN2022/132632**

| Patent document cited in search report | | | Publication date (day/month/year) | Patent family member(s) | | | Publication date (day/month/year) |
|---|---|---|---|---|---|---|---|
| CN | 110204532 | A | 06 September 2019 | None | | | |
| CN | 112321566 | A | 05 February 2021 | None | | | |
| CN | 113735828 | A | 03 December 2021 | None | | | |
| CN | 115304606 | A | 08 November 2022 | None | | | |
| KR | 20220035014 | A | 21 March 2022 | WO | 2022055181 | A1 | 17 March 2022 |
| TW | 202216686 | A | 01 May 2022 | WO | 2022012623 | A1 | 20 January 2022 |
| WO | 2020092662 | A1 | 07 May 2020 | EP | 3873906 | A1 | 08 September 2021 |
| | | | | EP | 3873906 | A4 | 27 July 2022 |
| | | | | US | 2022002291 | A1 | 06 January 2022 |
| WO | 2020172655 | A1 | 27 August 2020 | US | 2022143183 | A1 | 12 May 2022 |
| WO | 2021127561 | A1 | 24 June 2021 | KR | 20220119415 | A | 29 August 2022 |
| | | | | CA | 3154073 | A1 | 24 June 2021 |
| | | | | PE | 20221724 | A1 | 04 November 2022 |
| | | | | EP | 4076450 | A1 | 26 October 2022 |
| | | | | CO | 2022008604 | A2 | 08 July 2022 |
| | | | | IL | 293999 | A | 01 August 2022 |
| | | | | TW | 202136265 | A | 01 October 2021 |
| | | | | BR | 112022011827 | A2 | 30 August 2022 |
| | | | | AU | 2020405237 | A1 | 07 July 2022 |
| WO | 2022012622 | A1 | 20 January 2022 | None | | | |
| WO | 2022068849 | A1 | 07 April 2022 | TW | 202221000 | A | 01 June 2022 |
| WO | 2022171123 | A1 | 18 August 2022 | None | | | |
| WO | 2022194269 | A1 | 22 September 2022 | None | | | |
| WO | 2022228547 | A1 | 03 November 2022 | None | | | |
| WO | 2022228556 | A1 | 03 November 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019149922 A
- WO 2021127561 A
- WO 2021127561 A1
- WO 2022012622 A1